# EUROPEAN PATENT SPECIFICATION

(11) **EP 3 573 963 B1**
(45) Date of publication and mention of the grant of the patent: **01.09.2021**
(21) Application number: 18704135.5
(22) Date of filing: 26.01.2018
(51) Int. Cl.: C07D 311/74, C07D 319/16, A61K 31/352, A61K 31/47

(54) **NRF2 ACTIVATOR**
NRF2-AKTIVATOR
ACTIVATEUR DE NRF2

(30) Priority: 30.01.2017 US 201762452108 P
(43) Date of publication of application: 04.12.2019
(73) Proprietor: Biogen MA Inc., Cambridge, MA 02142 (US)
(72) Inventor: CAPACCI, Andrew, George, Cambridge, MA 02138 (US); LIN, Edward, Yin-Shiang, Ashland, MA 01721 (US); LUCAS, Brian, Stuart, Arlington, MA 02476 (US)
(74) Representative: HGF
(86) International application number: PCT/US2018/015465
(87) International publication number: WO 2018/140738

(56) References cited:
- WO-A1-2011/156889
- WO-A2-2009/146218
- Hannah Schuster ET AL: "Synthesis of the B-seco limonoid scaffoldw", Chem. Commun. Chem. Commun, 21 June 2011 (2011-06-21), pages 6545-6547, XP055463363, Retrieved from the Internet: URL:http://pubs.rsc.org/en/content/article pdf/2011/cc/c1cc11388g [retrieved on 2018-03-28]

## Description

Nuclear factor erythroid 2 (NF-E2)-related factor 2 (Nrf2) belongs to the Cap 'N' Collar (CNC) family of transcription factors and contains a conserved basic leucine zipper (bZIP) structure. The main function of Nrf2 is to activate the cellular antioxidant response by inducing the production of proteins that are able to combat the harmful effects of oxidative stress.

Activation of the Nrf2 pathway to treat diseases caused by oxidative stress, such as a neurodegenerative disease, inflammation and/or an inflammatory disease, an autoimmune disease, an ischemic fibrotic disease, a cancer, premature aging, a cardiovascular disease, a liver disease, a hemoglobinopathy and a metabolic disorder, is being studied.

Moreover, Nrf2 activation has been shown to upregulate fetal hemoglobin which can ameliorates symptoms of hemoglobinopathy such as sickle cell disease and thalassemia (e.g. beta-thalassemia).

WO2009/146218 A2 discloses particular compounds with antioxidant and antiinflammatory properties. WO2011/156889 A1 discloses particular modulators of Nrf2 and uses thereof. Schuster et al. (Synthesis of the B-seco limonoid scaffold", Chem. Communications, 1 Jan 2011, pages 6545-6547) discloses certain fused cyclohexanone compounds.

Therefore, a need exists for Nrf2 activators to treat these diseases.

### SUMMARY

Disclosed herein are potent activators of Nrf2 (see Example 20). These compounds can be used in the treatment of diseases treatable by activting Nrf2. A first embodiment of the invention is a compound of Formula I: 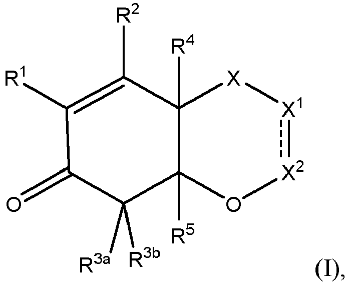 or a pharmaceutically acceptable salt thereof, wherein
R¹ is -CN, -C(O)R^{1a} or C₁₋₈alkyl substituted with one or more fluorine atoms;
R^{1a} is H, C₁₋₁₂alkyl, C₂₋₁₂alkenyl, C₂₋₁₂alkynyl, a 3 to 12-membered carbocyclyl, a 3 to 12-membered heterocyclyl, -OR^{10a}, -SR^{10a}, -N(R^{10a})₂, -NR^{10a}OR^{10a},-NR^{10a}S(O)₂R^{10a}, -NR^{10a}C(O)R^{10a}, -N(R^{10a})N(R^{10a})₂, -N(R^{10a})C(O)OR^{10a}, or-N(R^{10a})C(O)N(R^{10a})₂, wherein the C₁₋₁₂alkyl, C₂₋₁₂alkenyl, C₂₋₁₂alkynyl, 3 to 12-membered carbocyclyl, and 3 to 12-membered heterocyclyl are each optionally substituted with one or more R¹⁵;
R² is H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl, -C(O)R^{2a}, -C(S)R^{2a}, -C(O)OR^{2a}, -C(S)SR^{2a}, -C(O)SR^{2a}, -C(S)OR^{2a}, -SC(O)R^{2a}, -OC(S)R^{2a},-SC(S)R^{2a}, -C(O)N(R^{2a})₂, -OR^{2a}, -SR^{2a}, -N(R^{2a})₂, -N(R^{2a})OR^{2a},-N(R^{2a})S(O)₂R^{2a}, -N(R^{2a})C(O)R^{2a}, -N(R^{2a})N(R^{2a})₂, -N(R^{2a})C(O)OR^{2a},-N(R^{2a})C(O)N(R^{2a})₂, -S(O)₂R^{2a}, -S(O)R^{2a}, -S(O)N(R^{2a})₂, -S(O)₂N(R^{2a})₂,-N⁺(R^{2a})₃, -S⁺(R^{2a})₂ or -Si(R^{2a})₃, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R²⁵;
R^{3a} is H, C₁₋₁₂ alkyl, C₂₋₁₂ alkenyl, C₂₋₁₂ alkynyl, a 3 to 12-membered carbocyclyl, a 3 to 12-membered heterocyclyl, -C(O)R^{30a}, -C(O)OR^{30a}, -C(O)N(R^{30a})₂, -OR^{30a}, -N(R^{30a})₂, -N(R^{30a})OR^{30a}, -N(R^{30a})S(O)₂R^{30a}, -N(R^{30a})C(O)R^{30a},-N(R^{30a})N(R^{30a})₂, -N(R^{30a})C(O)OR^{30a}, -N(R^{30a})C(O)N(R^{30a})₂, -S(O)R^{30a},-S(O)N(R^{30a})₂, or -S(O)₂N(R^{30a})₂, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R³⁵; and
R^{3b} is C₁₋₁₂alkyl, C₂₋₁₂alkenyl, C₂₋₁₂alkynyl, a 3 to 12-membered carbocyclyl, a 3 to 12-membered heterocyclyl, -C(O)R^{30b}, -C(O)OR^{30b}, -C(O)N(R^{30b})₂, -OR^{30b},-N(R^{30b})₂, -N(R^{30b})OR^{30b}, -N(R^{30b})S(O)₂R^{30b}, -N(R^{30b})C(O)R^{30b},-N(R^{30b})N(R^{30b})₂, -N(R^{30b})C(O)OR^{30b}, -N(R^{30b})C(O)N(R^{30b})₂, -S(O)R^{30b},-S(O)N(R^{30b})₂ or -S(O)₂N(R^{30b})₂, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R³⁵; or
R^{3a} and R^{3b}, taken together, are C₂₋₁₂ alkylene, C₂₋₁₂ alkenylene or C₂₋₁₂ alkynylene, wherein the C₂₋₁₂ alkylene, C₂₋₁₂ alkenylene, and C₂₋₁₂ alkynylene are each optionally substituted with one or more R³⁵ ;
R⁴ is H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl, -C(O)R^{4a}, -C(S)R^{4a}, -C(O)OR^{4a}, -C(S)SR^{4a}, -C(O)SR^{4a}, -C(S)OR^{4a} -SC(O)R^{4a}, -OC(S)R^{4a},-SC(S)R^{4a}, -C(O)N(R^{4a})₂, -OR^{4a}, -SR^{4a}, -N(R^{4a})₂, -N(R^{4a})OR^{4a},-N(R^{4a})S(O)₂R^{4a}, -N(R^{4a})C(O)R^{4a}, -N(R^{4a})N(R^{4a})₂, -N(R^{4a})C(O)OR^{4a},-N(R^{4a})C(O)N(R^{4a})₂, -S(O)₂R^{4a}, -S(O)R^{4a}, -S(O)N(R^{4a})₂, -S(O)₂N(R^{4a})₂,-N⁺(R^{4a})₃, -S⁺(R^{4a})₂ or -Si(R^{4a})₃, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R⁴⁵;
R⁵ is H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl, -C(O)R^{5a}, -C(S)R^{5a}, -C(O)OR^{5a}, -C(S)SR^{5a}, -C(O)SR^{5a}, -C(S)OR^{5a}, -SC(O)R^{5a}, -OC(S)R^{5a},-SC(S)R^{5a}, -C(O)N(R^{5a})₂, -OR^{5a}, -SR^{5a}, -N(R^{5a})₂, -N(R^{5a})OR^{5a},-N(R^{5a})S(O)₂R^{5a}, -N(R^{5a})C(O)R^{5a}, -N(R^{5a})N(R^{5a})₂, -N(R^{5a})C(O)OR^{5a},-N(R^{5a})C(O)N(R^{5a})₂, -S(O)₂R^{5a}, -S(O)R^{5a}, -S(O)N(R^{5a})₂, -S(O)₂N(R^{5a})₂,-N⁺(R^{5a})₃, -S⁺(R^{5a})₂ or -Si(R^{5a})₃, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R⁵⁵;
" 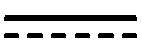 " is either a single bond or a double bond, wherein when " 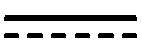 " is a double bond, then X¹ is CR⁶ and X² is CR⁷; and when " 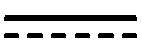 " is a single bond, then X¹ is C(R⁶)₂ and X² is C(R⁷)₂;
R⁶,
in each occurrence, is independently H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl, -C(O)R^{6a}, -C(S)R^{6a}, -C(O)OR^{6a}, -C(S)SR^{6a}, -C(O)SR^{6a},-C(S)OR^{6a}, -SC(O)R^{6a}, -OC(S)R^{6a}, -SC(S)R^{6a}, -C(O)N(R^{6a})₂, -OR^{6a}, -SR^{6a},-N(R^{6a})₂, -N(R^{6a})OR^{6a}, -N(R^{6a})S(O)₂R^{6a}, -N(R^{6a})C(O)R^{6a}, -N(R^{6a})N(R^{6a})₂,-N(R^{6a})C(O)OR^{6a}, -N(R^{6a})C(O)N(R^{6a})₂, -S(O)₂R^{6a}, -S(O)R^{6a}, -S(O)N(R^{6a})₂,-S(O)₂N(R^{6a})₂, -N⁺(R^{6a})₃, -S⁺(R^{6a})₂, or -Si(R^{6a})₃; or the two R⁶ groups, taken together, are oxo, C₁₋₁₂ alkylidene or =NR^{6a}, wherein the C₁₋₁₂alkyl, C₂₋₁₂alkenyl, C₂₋₁₂alkynyl, 3 to 12-membered carbocyclyl, 3 to 12-membered heterocyclyl, and C₁₋₁₂alkylidene are each optionally substituted with one or more R⁶⁵;
R⁷, in each occurrence, is independently H, halo, -NO₂, -CN, -N₃, C₁₋₁₂alkyl, C_{2- 12}alkenyl, C₂₋₁₂alkynyl, a 3 to 12-membered non-aromatic carbocyclyl, a 3 to 12-membered heterocyclyl, -C(O)R^{7a}, -C(S)R^{7a}, -C(O)OR^{7a}, -C(S)SR^{7a},-C(O)SR^{7a}, -C(S)OR^{7a}, -SC(O)R^{7a}, -OC(S)R^{7a}, -SC(S)R^{7a}, -C(O)N(R^{7a})₂, -OR^{7a}, -SR^{7a}, -N(R^{7a})₂, -N(R^{7a})OR^{7a}, -N(R^{7a})S(O)₂R^{7a}, -N(R^{7a})C(O)R^{7a},-N(R^{7a})N(R^{7a})₂, -N(R^{7a})C(O)OR^{7a}, -N(R^{7a})C(O)N(R^{7a})₂, -S(O)₂R^{7a}, -S(O)R^{7a},-S(O)N(R^{7a})₂, -S(O)₂N(R^{7a})₂, -N⁺(R^{7a})₃, -S⁺(R^{7a})₂, or -Si(R^{7a})₃; or the two R⁷ groups, taken together, are oxo, C₁₋₁₂ alkylidene, or =NR^{7a}, wherein the C₁₋₁₂alkyl, C₂₋₁₂alkenyl, C₂₋₁₂alkynyl, 3 to 12-membered non-aromatic carbocyclyl, 3 to 12-membered heterocyclyl, and C₁₋₁₂alkylidene are each optionally substituted with one or more R⁷⁵;
X is -C(R⁸)₂- or -O-;
R⁸, in each occurrence, is independently H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl, -C(O)R^{8a}, -C(S)R^{Sa}, -C(O)OR^{8a}, -C(S)SR^{8a}, -C(O)SR^{8a},-C(S)OR^{8a}, -SC(O)R^{8a}, -OC(S)R^{8a}, -SC(S)R^{8a}, -C(O)N(R^{8a})₂, -OR^{8a}, -SR^{8a},-N(R^{8a})₂, -N(R^{8a})OR^{8a}, -N(R^{8a})S(O)₂R^{8a}, -N(R^{8a})C(O)R^{8a}, -N(R^{8a})N(R^{8a})₂,-N(R^{8a})C(O)OR^{8a}, -N(R^{8a})C(O)N(R^{8a})₂, -S(O)₂R^{8a}, -S(O)R^{8a}, -S(O)N(R^{8a})₂,-S(O)₂N(R^{8a})₂, -N⁺(R^{8a})₃, -S⁺(R^{8a})₂, or -Si(R^{8a})₃; or the two R⁸ groups taken together are oxo, C₁₋₁₂ alkylidene, or =NR^{8a}, wherein the C₁₋₁₂alkyl, C₂₋₁₂alkenyl, C₂₋₁₂alkynyl, 3 to 12-membered carbocyclyl, 3 to 12-membered heterocyclyl, and C₁₋₁₂alkylidene are each optionally substituted with one or more R⁸⁵;
R^{10a}, R^{2a}, R^{30a}, R^{30b}, R^{4a}, R^{5a}, R^{6a}, R^{7a}, and R^{8a}, in each occurrence, are independently selected from H, C₁₋₁₂alkyl, C₂₋₁₂alkenyl, C₂₋₁₂alkynyl, C₁₋₁₂alkoxy, C₁₋₁₂acyl,-Si(R¹⁶)₃, a 3 to 12-membered carbocyclyl, and a 3 to 12-membered heterocyclyl, wherein the C₁₋₁₂alkyl, C₂₋₁₂alkenyl, C₂₋₁₂alkynyl, C₁₋₁₂alkoxy, C₁₋₁₂acyl, 3 to 12-membered carbocyclyl, and 3 to 12-membered heterocyclyl are each optionally substituted with one or more R¹⁷;
R¹⁶, in each occurrence, is independently selected from H, C₁₋₁₂alkyl, C₂₋₁₂alkenyl, C₂₋₁₂alkynyl, a 3 to 12-membered carbocyclyl, and a 3 to 12-membered heterocyclyl, wherein the C₁₋₁₂alkyl, C₂₋₁₂alkenyl, C₂₋₁₂alkynyl, 3 to 12-membered carbocyclyl, and 3 to 12-membered heterocyclyl are each optionally substituted with one or more R¹⁸;
R¹⁵, R²⁵, R³⁵, R⁴⁵, R⁵⁵, R⁶⁵, R⁷⁵, and R⁸⁵,
in each occurrence, are independently selected from halo, -OH, -CN, C₁₋₁₂alkyl, C₂₋₁₂alkenyl, C₂₋₁₂alkynyl, C₁₋₁₂alkoxy, a 3 to 12-membered carbocyclyl and a 3 to 12-membered heterocyclyl, wherein the C₁₋₁₂alkyl, C₂₋₁₂alkenyl, C₂₋₁₂alkynyl, C₁₋₁₂alkoxy, 3 to 12-membered carbocyclyl and 3 to 12-membered heterocyclyl are each optionally substituted with one or more R¹⁹; and
R¹⁷, R¹⁸, and R¹⁹, in each occurrence, are independently selected from halo, -OH, -CN, C₁₋₁₂alkyl, C₂₋₁₂alkenyl, C₂₋₁₂alkynyl, C₁₋₁₂alkoxy, a 3 to 12-membered carbocyclyl and a 3 to 12-membered heterocyclyl, wherein the C₁₋₁₂alkyl, C₂₋₁₂alkenyl, C₂₋₁₂alkynyl, C₁₋₁₂alkoxy, 3 to 12-membered carbocyclyl, and 3 to 12-membered heterocyclyl are each optionally substituted with one or more groups independently selected from halo, -OH, and C₁₋₄alkoxy.

Also provided is a pharmaceutical composition comprising at least one compound described herein, or a pharmaceutically acceptable salt thereof, and at least one pharmaceutically acceptable excipient.

Also provided is a method for activating Nrf2 in a subject in need thereof, the method comprising administering to the subject a therapeutically effective amount of a compound described herein, or a pharmaceutically acceptable salt thereof, thereby activating Nrf2 in the subject.

Also provided is a method for treating a disease caused by oxidative stress in a subject, the method comprising administering to the subject a therapeutically effective amount of a compound described herein, or a pharmaceutically acceptable salt thereof.

Also provided is a method for treating a disorder in a subject, wherein the disorder is selected from the group consisting of a neurodegenerative disease, inflammation/an inflammatory disease, an autoimmune disease, an ischemic fibrotic disease, a cancer, premature aging, a cardiovascular disease, a liver disease, a hemoglobinopathy, thalassemia (e.g., beta-thalassemia), and a metabolic disorder, the method comprising administering to the subject a therapeutically effective amount of a compound described herein, or a pharmaceutically acceptable salt thereof.

Other features or advantages will be apparent from the following detailed description of several embodiments, and also from the appended claims.

### BRIEF DESCRIPTION OF THE DRAWINGS

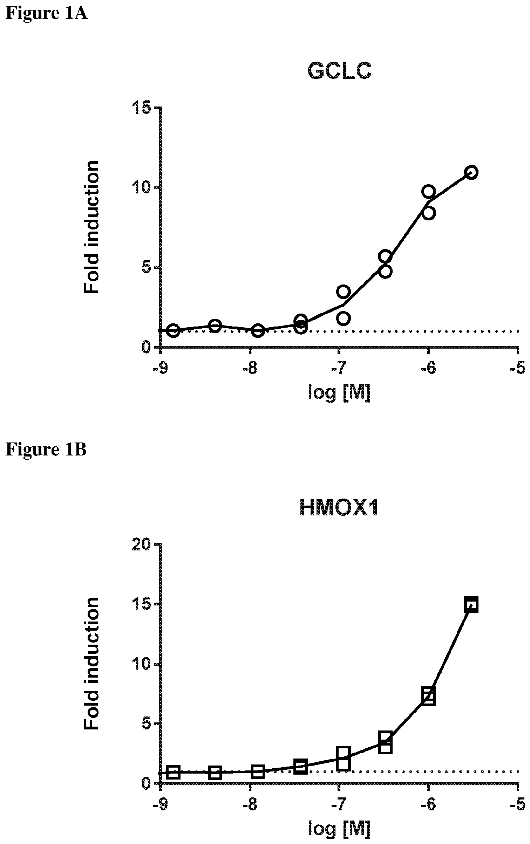

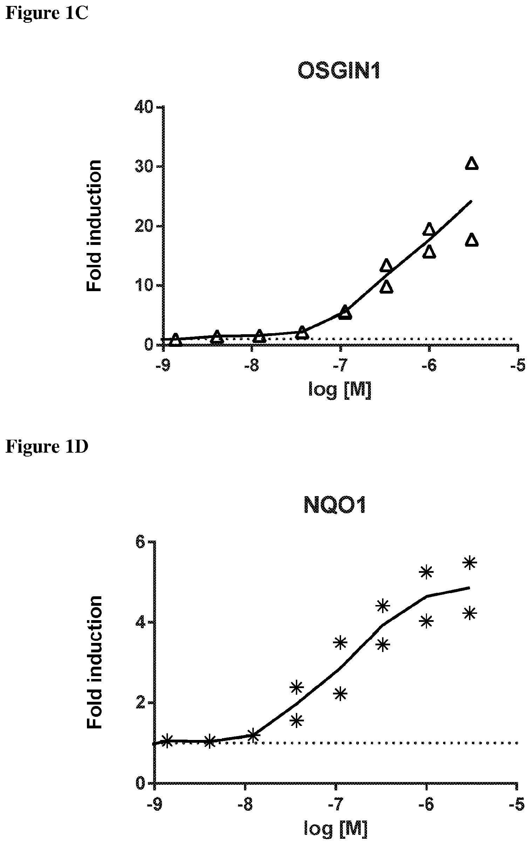

Figures 1A to 1D show transcription of GCLC (Figure 1A), HMOX1 (Figure 1B), OSGIN1 (Figure 1C) and NQ01 (Figure 1D) in human astrocytes treated with increasing concentrations of Compound 3-Ent1 for 20 hours. The x-axis represents log (molar concentrations of compound 3-Ent1). Values shown are mean ± S.E.M. n=2 experiments.

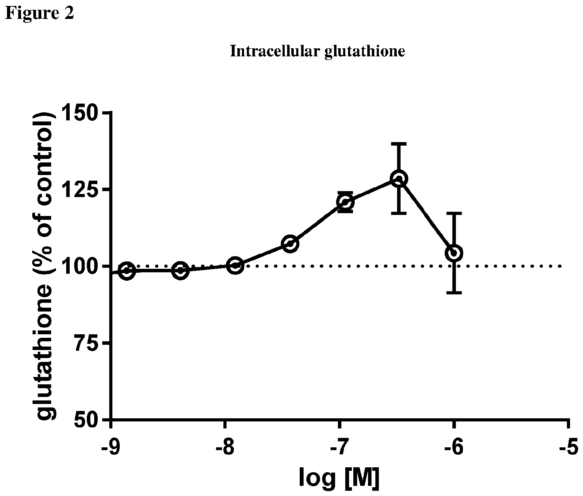

Figure 2 shows levels of intracellular glutathione Compound 3-Ent1 in human astrocytes treated with increasing concentrations of Compound 3-Entl for 20 hours. The x-axis represents log (molar concentrations of compound 3-Ent1). Values shown are mean ± S.E.M. n=3 experiments.

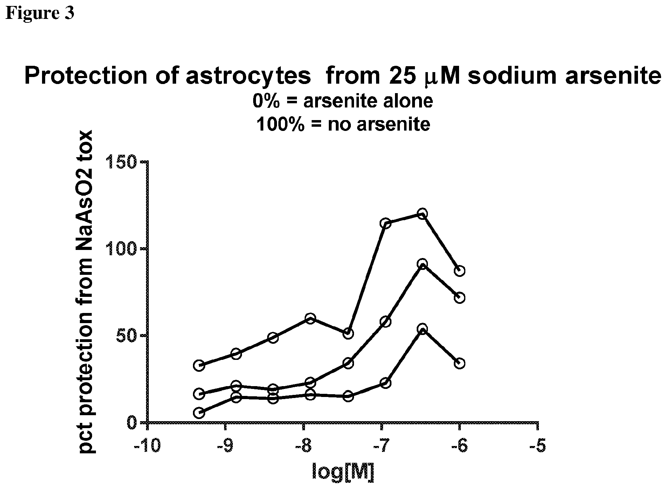

Figure 3 shows levels of protection of astrocytes by increasing concentrations of Compound 3-Ent1 from oxidative stress-induced cell death caused by 25 µM sodium arsenite. The compound was added to human astrocytes 20 hrs prior to addition of arsenite and the astrocytes were further incubated for 22 hours after addition of arsenite. The x-axis represents log (molar concentrations of compound 3-Ent1). This figure shows results from 3 separate experiments.

### DETAILED DESCRIPTION

The compounds or pharmaceutically acceptable salts thereof as described herein are Nrf2 activators.

In a second embodiment of the invention, the compound is represented by Formula II: 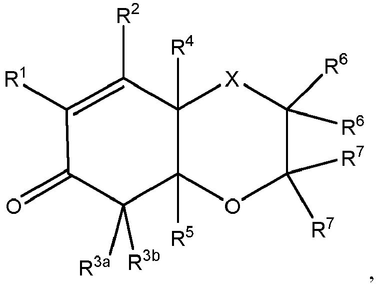 or a pharmaceutically acceptable salt thereof, wherein the values of the variables are as defined for the first embodiment.

In a third embodiment of the invention, the compound is represented by Formula IIA, IIB, IIC, or IID: 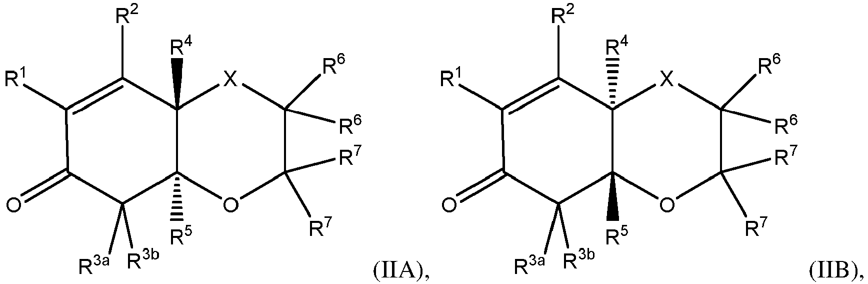 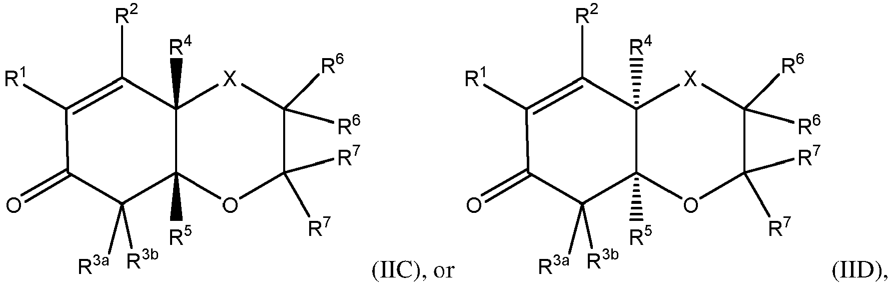 or a pharmaceutically acceptable salt thereof, wherein the values of the variables are as defined for the first embodiment.

In a fourth embodiment of the invention, the compound is represented by Formula III or IV: 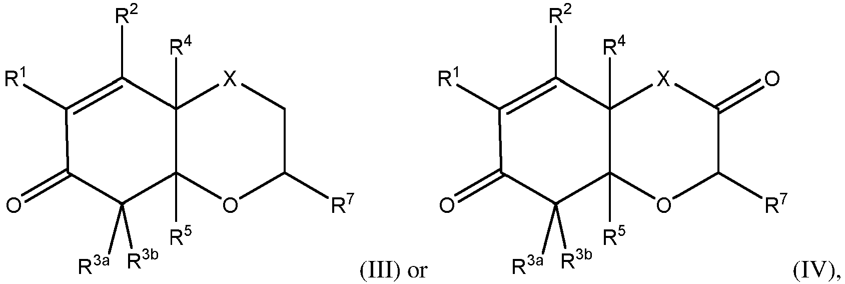 or a pharmaceutically acceptable salt thereof, wherein the values of the variables are as defined for the first embodiment.

In a fifth embodiment of the invention, the compound is represented by Formula IIIA, IIIB, IIIC, IIID, IVA, IVB, IVC, or IVD: 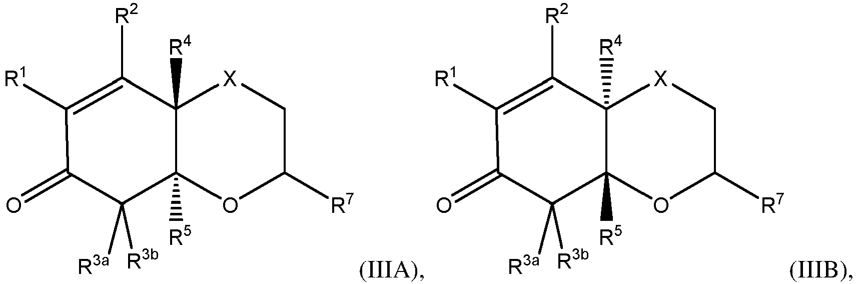 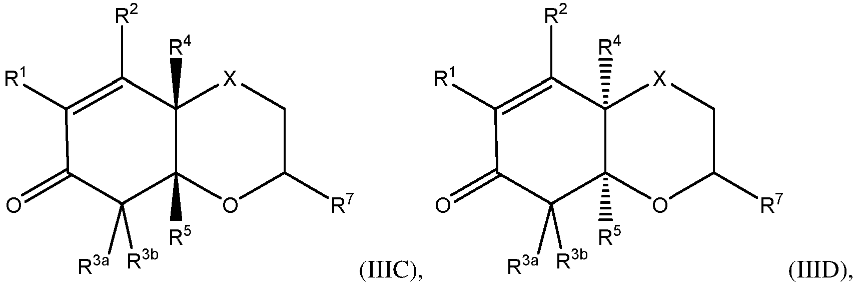 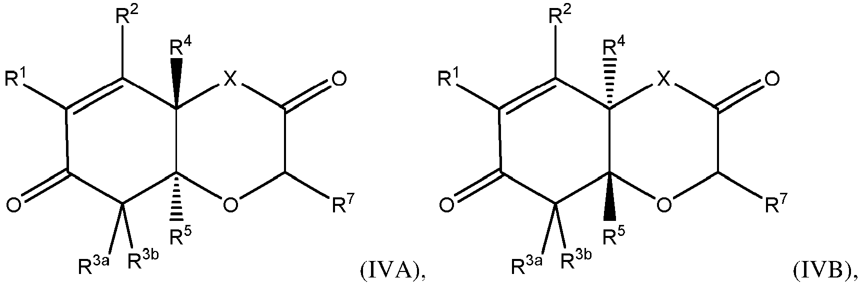 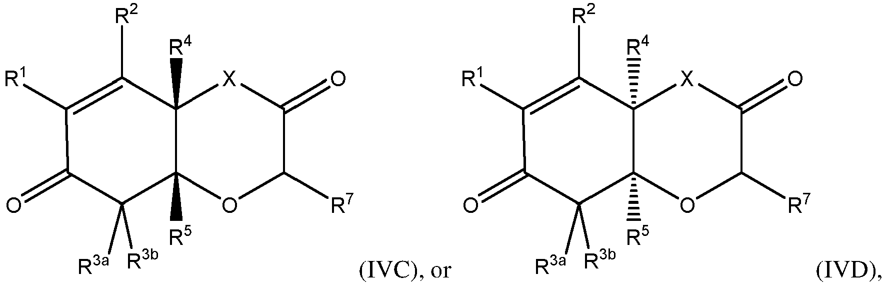 or a pharmaceutically acceptable salt thereof, wherein the values of the variables are as defined for the first embodiment.

In a sixth embodiment of the invention, the compound is represented by Formula III(A): 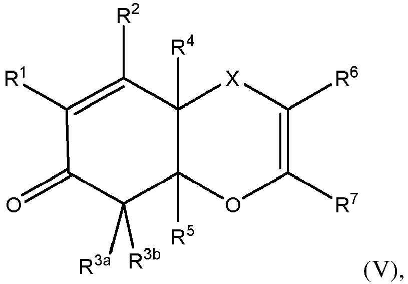 or a pharmaceutically acceptable salt thereof, wherein the values of the variables are as defined for the first embodiment.

In a seventh embodiment of the invention, the compound is represented by Formula VA, VB, VC, or VD: 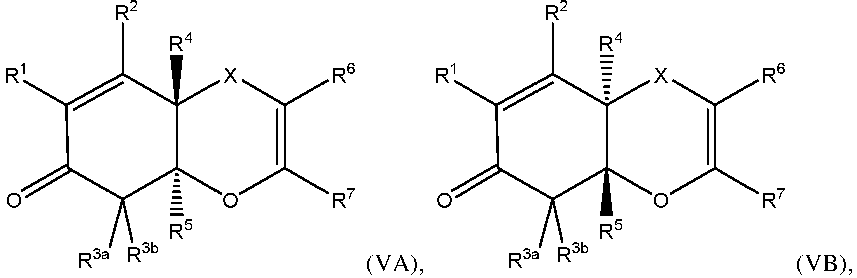 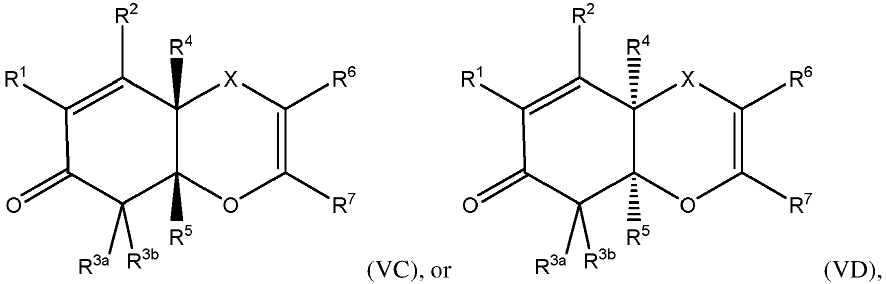 or a pharmaceutically acceptable salt thereof, wherein the values of the variables are as defined for the first embodiment.

In an eighth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R⁶, in each occurrence, is independently H, halo, -CN, -OR^{6a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R⁶⁵; or the two R⁶ groups, taken together, are oxo; R⁷, in each occurrence, is independently H, halo, -CN, -OR^{7a}, C₁₋₁₂alkyl, C₂₋₁₂alkenyl, C₂₋₁₂alkynyl, or a 3 to 8 membered non-aromatic carbocyclyl, wherein the C₁₋₁₂alkyl, C₂₋₁₂alkenyl, C₂₋₁₂alkynyl, and 3 to 8 membered carbocyclyl are each optionally substituted with one to eight R⁷⁵; R⁶⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, - OH and C₁₋₄alkoxy; R⁷⁵, in each occurrence, is independently selected from halo, -OH, -CN, C₁₋₁₂alkyl, C₂₋₁₂alkenyl, C₂₋₁₂alkynyl, a 3 to 8 membered carbocyclyl, and C₁₋₁₂alkoxy, wherein the C₁₋₁₂alkyl, C₂₋₁₂alkenyl, C₂₋₁₂alkynyl, 3 to 8 membered carbocyclyl, and C₁₋₁₂alkoxy are each optionally substituted with one to eight groups independently selected from halo, -OH and C₁₋₄alkoxy; R^{6a} and R^{7a}, in each occurrence, are independently selected from H, C₁₋₁₂alkyl, C₂₋₁₂alkenyl, and C₂₋₁₂alkynyl, wherein the C₁₋₁₂alkyl, C₂₋₁₂a|kenyl, and C₂₋₁₂alkynyl are each optionally substituted with one to six R¹⁷; and R¹⁷, in each occurrence, as an optional substituent of R^{6a} or R^{7a}, is independently selected from halo, -CN, -OH, C₁₋₆alkyl, and C₁-₆alkoxy, wherein the C₁₋₆alkyl and C₁-₆alkoxy are each optionally substituted with one to six halo; and wherein the values of the other variables are as defined for the first embodiment.

In a ninth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R⁶, in each occurrence, is independently H or C₁₋₆alkyl, or the two R⁶ groups, taken together, are oxo, wherein the C₁₋₆alkyl is optionally substituted with one to six groups independently selected from halo, -OH, C₁₋₄alkoxy, and -CN; and R⁷, in each occurrence, is independently H, -OH, halo, C₁₋₁₂alkyl, C₁₋₁₂alkoxy, or a 3 to 8 membered cycloalkyl, wherein the C₁₋₁₂alkyl, C₁₋₁₂alkoxy, and 3 to 8 membered cycloalkyl are each optionally substituted with one to six groups independently selected from phenyl, halo, -OH, C₁₋₄alkoxy, and -CN; and wherein the values of the other variables are as defined for the first embodiment.

In a tenth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R⁶, in each occurrence, is independently H or C₁₋₄alkyl; or the two R⁶ groups, taken together, are oxo; and R⁷, in each occurrence, is independently H, C₁₋₆alkyl, benzyl, cyclopropyl, cyclobutyl, cyclopentyl, or cyclohexyl; and wherein the values of the other variables are as defined for the first embodiment.

In an eleventh embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein X is -C(R⁸)₂-; R⁸, in each occurrence, is independently H, halo, -CN, -OR^{8a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R⁸⁵; R^{8a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷; R⁸⁵, in each occurrence, is independently selected from halo, - OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, -OH, and C₁₋₄alkoxy; and R¹⁷, in each occurrence, as an optional substituent of R^{8a}, is independently is selected from halo, -CN, -OH, C₁₋₆alkyl, and C₁-₆alkoxy, wherein the C₁₋₆alkyl and C₁-₆alkoxy are each optionally substituted with one to six halo; and wherein the values of the other variables are as defined for the first, eighth, ninth and/or tenth embodiments.

In a twelfth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein X is - C(R⁸)₂-; and R⁸, in each occurrence, is independently H or C₁₋₄alkyl; or preferably, R⁸, in each occurrence, is H; and wherein the values of the other variables are as defined for the first, eighth, ninth and/or tenth embodiments.

In a thirteenth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein X is -O-; and wherein the values of the other variables are as defined for the first, eighth, ninth and/or tenth embodiments.

In a fourteenth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R¹ is -CN; and wherein the values of the other variables are as defined for the first, eighth, ninth, tenth, eleventh, twelfth, thirteenth embodiments and/or the preferred embodiment thereof.

In a fifteenth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R¹ is - CF₃; and wherein the values of the other variables are as defined for the first, eighth, ninth, tenth, eleventh, twelfth, and/or thirteenth embodiments, and/or the preferred embodiment thereof.

In a sixteenth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R¹ is -C(O)R^{1a}; R^{1a} is H, C₁₋₁₂alkyl, C₂₋₁₂alkenyl, C₂₋₁₂alkynyl, a 3 to 12-membered carbocyclyl, or a 3 to 12-membered heterocyclyl, wherein the C₁₋₁₂alkyl, C₂₋₁₂alkenyl, C₂₋₁₂alkynyl, 3 to 12-membered carbocyclyl, and 3 to 12-membered heterocyclyl are each optionally substituted with one to six R¹⁵; and R¹⁵, in each occurrence, is independently selected from halo, -OH, C₁₋₁₂alkyl, and C₁₋₁₂alkoxy, wherein the C₁₋₁₂alkyl and C₁₋₁₂alkoxy are each optionally substituted with one to three groups independently selected from halo, - OH, and C₁₋₄alkoxy; and wherein the values of the other variables are as defined for the first, eighth, ninth, tenth, eleventh, twelfth, and/or thirteenth embodiments, and/or the preferred embodiment thereof. In a preferred embodiment, R^{a} is H or C₁₋₆alkyl (which is optionally substituted with one to four halo groups). In a more preferred embodiment, R^{a} is H or C₁₋₄alkyl. In the preferred or more preferred embodiments, the values of the other variables are as defined for the first, eighth, ninth, tenth, eleventh, twelfth, and/or thirteenth embodiments, and/or the preferred embodiment thereof.

In a seventeenth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R² is H, halo, -CN, -OR^{2a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R²⁵; R^{2a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷; R²⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, - OH, and C₁₋₄alkoxy; and R¹⁷, in each occurrence, as an optional substituent of R^{2a}, is independently selected from halo, -CN, -OH, C₁₋₆alkyl, and C₁-₆alkoxy, wherein the C₁₋₆alkyl and C₁₋₆alkoxy are each optionally substituted with one to six halo; and wherein the values of the other variables are as defined for the first, eighth, ninth, tenth, eleventh, twelfth, thirteenth, fourteenth, fifteenth, and/or sixteenth embodiments, and/or the preferred embodiments thereof.

In an eighteenth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R² is H, halo, -OH, -CN, C₁-₆alkyl, or C₁-₆alkoxy, wherein the C₁-₆alkyl and C₁-₆alkoxy are each optionally substituted with one to six groups independently selected from halo, -CN, -OH, C₁₋₄alkyl, and C₁-₄alkoxy; and wherein the values of the other variables are as defined for the first, eighth, ninth, tenth, eleventh, twelfth, thirteenth, fourteenth, fifteenth, and/or sixteenth embodiments, and/or the preferred embodiments thereof. In a preferred embodiment, R² is H or C₁₋₄alkyl. In a more preferred embodiment, R² is H. In the preferred or more preferred embodiment, the values of the other variables are as defined for the first, eighth, ninth, tenth, eleventh, twelfth, thirteenth, fourteenth, fifteenth, and/or sixteenth embodiments, and/or the preferred embodiments thereof.

In a nineteenth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R^{3a} is H, halo, -CN, -OR^{30a}, C₁₋₁₂ alkyl, C₂₋₁₂ alkenyl, or C₂₋₁₂ alkynyl, wherein the C₁₋₁₂alkyl, C₂₋₁₂alkenyl, and C₂₋₁₂alkynyl are each optionally substituted with one to eight R³⁵; R^{3b} is halo, -CN, -OR^{30b}, C₁₋₁₂ alkyl, C₂₋₁₂ alkenyl, or C₂₋₁₂ alkynyl, wherein the C₁₋₁₂alkyl, C₂₋₁₂alkenyl, and C₂₋₁₂alkynyl are each optionally substituted with one to eight R³⁵; R^{30a} and R^{30b} are each independently selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷; R³⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, -OH and C₁₋₄alkoxy; and R¹⁷, in each occurrence, as an optional substituent of R^{30a} or R^{30b}, is independently selected from halo, -CN, -OH, C₁₋₆alkyl, and C₁₋₆alkoxy, wherein the C₁₋₆alkyl and C₁-₆alkoxy are each optionally substituted with one to six halo; and wherein the values of the other variables are as defined for the first, eighth, ninth, tenth, eleventh, twelfth, thirteenth, fourteenth, fifteenth, sixteenth, seventeenth, and/or eighteenth embodiments, and/or the preferred embodiments thereof.

In a twentieth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R^{3a} and R^{3b} are each independently halo, -OH, -CN, C₁-₆alkyl, or C₁-₆alkoxy, wherein the C₁₋₆alkyl and C₁-₆alkoxy are each optionally substituted with one to six groups independently selected from halo, -CN, -OH, and C₁₋₄alkoxy; and wherein the values of the other variables are as defined for the first, eighth, ninth, tenth, eleventh, twelfth, thirteenth, fourteenth, fifteenth, sixteenth, seventeenth, and/or eighteenth embodiments, and/or the preferred embodiments thereof. In a preferred embodiment, R^{3a} and R^{3b} are each independently C₁₋₆alkyl. In a more preferred embodiment, R^{3a} and R^{3b} are each independently C₁₋₄alkyl. In an even more preferred embodiment, R^{3a} and R^{3b} are each methyl. In the preferred, more preferred, or even more preferred embodiment, the values of the other variables are as defined for the first, eighth, ninth, tenth, eleventh, twelfth, thirteenth, fourteenth, fifteenth, sixteenth, seventeenth, and/or eighteenth embodiments, and/or the preferred embodiments thereof.

In a twenty-first embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R⁴ is H, halo, -CN, -OR^{4a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R⁴⁵; R^{4a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷; R⁴⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, - OH and C₁₋₄alkoxy; and R¹⁷, in each occurrence, as an optional substituent of R^{4a}, is independently selected from halo, -CN, -OH, C₁₋₆alkyl, and C₁-₆alkoxy, wherein the C₁₋₆alkyl and C₁-₆alkoxy are each optionally substituted with one to six halo; and wherein the values of the other variables are as defined for the first, eighth, ninth, tenth, eleventh, twelfth, thirteenth, fourteenth, fifteenth, sixteenth, seventeenth, eighteenth, nineteenth and/or, twentieth embodiments, and/or the preferred embodiments thereof.

In a twenty-second embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R⁴ is H, halo, -OH, -CN, C₁-₆alkyl, or C₁-₆alkoxy, wherein the C₁-₆alkyl and C₁-₆alkoxy are each optionally substituted with one to six groups independently selected from halo, -CN, -OH, and C₁₋₄alkoxy; and wherein the values of the other variables are as defined for the first, eighth, ninth, tenth, eleventh, twelfth, thirteenth, fourteenth, fifteenth, sixteenth, seventeenth, eighteenth, nineteenth, and/or twentieth embodiments, and/or the preferred embodiments thereof. In a preferred embodiment, R⁴ is H or C₁₋₄alkyl. In a more preferred embodiment, R⁴ is C₁₋₄alkyl. In an even more preferred embodiment, R⁴ is methyl or ethyl. In the preferred, more preferred, or even more preferred embodiment, the values of the other variables are as defined for the first, eighth, ninth, tenth, eleventh, twelfth, thirteenth, fourteenth, fifteenth, sixteenth, seventeenth, eighteenth, nineteenth, and/or twentieth embodiments, and/or the preferred embodiments thereof.

In a twenty-third embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R⁵ is H, halo, -CN, -OR^{5a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R⁵⁵; R^{5a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷; R⁵⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, - OH, and C₁₋₄alkoxy; and R¹⁷, in each occurrence, as an optional substituent of R^{5a}, is independently selected from halo, -CN, -OH, C₁₋₆alkyl, and C₁-₆alkoxy, wherein the C₁₋₆alkyl and C₁-₆alkoxy are each optionally substituted with one to six halo; and wherein the values of the other variables are as defined for the first, eighth, ninth, tenth, eleventh, twelfth, thirteenth, fourteenth, fifteenth, sixteenth, seventeenth, eighteenth, nineteenth, twentieth, twenty-first, and/or twenty-second embodiments, and/or the preferred embodiments thereof.

In a twenty-fourth embodiment of the invention, the compound is represented by Formula I, II, II(A), II(B), II(C), II(D), III, III(A), III(B), III(C), III(D), IV, IV(A), IV(B), IV(C), IV(D), V, V(A), V(B), V(C), or V(D), or a pharmaceutically acceptable salt thereof, wherein R⁵ is H, halo, -OH, -CN, C₁-₆alkyl, or C₁-₆alkoxy, wherein the C₁-₆alkyl and C₁-₆alkoxy are each optionally substituted with one to six groups independently selected from halo, -CN, -OH, and C₁-₄alkoxy; and wherein the values of the other variables are as defined for the first, eighth, ninth, tenth, eleventh, twelfth, thirteenth, fourteenth, fifteenth, sixteenth, seventeenth, eighteenth, nineteenth, twentieth, twenty-first, and/or twenty-second embodiments, and/or the preferred embodiments thereof. In a preferred embodiment, R⁵ is H or C₁₋₄alkyl. In a more preferred embodiment, R⁵ is H. In the preferred or more preferred embodiment, the values of the other variables are as defined for the first, eighth, ninth, tenth, eleventh, twelfth, thirteenth, fourteenth, fifteenth, sixteenth, seventeenth, eighteenth nineteenth, twentieth, twenty-first, and/or twenty-second embodiments, and/or the preferred embodiments thereof.

In a twenty-fifth embodiment of the invention, the compound is represented by Formula I, II, II(A), II(B), II(C), II(D), III, III(A), III(B), III(C), III(D), IV, IV(A), IV(B), IV(C), or IV(D), or a pharmaceutically acceptable salt thereof, wherein R¹ is -CN or -CF₃; R² is H, halo, -OH, -CN, C₁-₆alkyl, or C₁-₆alkoxy, wherein the C₁-₆alkyl and C₁-₆alkoxy are each optionally substituted with one to six groups independently selected from halo, -CN, - OH, C₁₋₄alkyl, and C₁-₄alkoxy; R^{3a} and R^{3b} are each independently halo, -OH, -CN, C₁-₆alkyl, or C₁-₆alkoxy, wherein the C₁-₆alkyl and C₁-₆alkoxy are each optionally substituted with one to six groups independently selected from halo, -CN, -OH, and C₁-₄alkoxy; R⁴ is H, halo, - OH, -CN, C₁-₆alkyl, or C₁-₆alkoxy, wherein the C₁-₆alkyl and C₁-₆alkoxy are each optionally substituted with one to six groups independently selected from halo, -CN, -OH, and C₁-₄alkoxy; R⁵ is H, halo, -OH, -CN, C₁-₆alkyl, or C₁-₆alkoxy, wherein the C₁-₆alkyl and C₁-₆alkoxy are each optionally substituted by one to six groups independently selected from halo, -CN, -OH, and C₁-₄alkoxy; X is -C(R⁸)₂- or -O-, wherein R⁸, in each occurrence, is independently H, halo, -CN, -OH, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight groups independently selected from halo, -OH, -CN, and C₁₋₄alkoxy; " 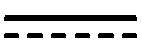 " is a single bond; X¹ is C(R⁶)₂, wherein R⁶, in each occurrence, is independently H or C₁₋₄alkyl, or the two R⁶ groups, taken together, are oxo, wherein the C₁₋₄alkyl is optionally substituted with one to six groups independently selected from halo, -OH, C₁₋₄alkoxy, and -CN; and X² is C(R⁷)₂, wherein R⁷, in each occurrence, is independently H, -OH, halo, C₁₋₁₂alkyl, C₁₋₁₂alkoxy, or a 3 to 8 membered cycloalkyl, wherein the C₁₋₁₂alkyl, C₁₋₁₂alkoxy, and 3 to 8 membered cycloalkyl are each optionally substituted with one to six groups independently selected from phenyl, halo, -OH, C₁₋₄alkoxy, and -CN.

In a twenty-sixth embodiment of the invention, the compound is represented by Formula I, II, II(A), II(B), II(C), II(D), III, III(A), III(B), III(C), III(D), IV, IV(A), IV(B), IV(C), or IV(D), or a pharmaceutically acceptable salt thereof, wherein R¹ is -CN; R ² is H or C₁₋₄alkyl; R^{3a} and R^{3b} are each independently C₁₋₆alkyl; R⁴ is H or C₁₋₄alkyl; R⁵ is H or C₁₋₄alkyl; X is -C(R⁸)₂- or -O-, wherein R⁸, in each occurrence, is independently H or C₁-₄alkyl; " 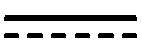 " is a single bond; X¹ is C(R⁶)₂ or -C(O)-, wherein R⁶, in each occurrence, is independently H or C₁₋₄alkyl; and X² is -CHR₇-, wherein R⁷ is H, C₁₋₆alkyl, benzyl, cyclopropyl, cyclobutyl, cyclopentyl, or cyclohexyl.

In a twenty-seventh embodiment of the invention, the compound is represented by Formula I, II, II(A), II(B), II(C), II(D), III, III(A), III(B), III(C), III(D), IV, IV(A), IV(B), IV(C), or IV(D), or a pharmaceutically acceptable salt thereof, wherein R¹ is -CN; R ² is H; R^{3a} and R^{3b} are each independently C₁₋₄alkyl; R⁴ is C₁₋₄alkyl; R⁵ is H; X is -CH₂- or -O-; " 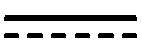 " is a single bond; X¹ is -CH₂- or -C(O)-; and X² is -CHR₇-, wherein R⁷ is H, C₁₋₄alkyl, benzyl or cyclopropyl.

In a twenty-eighth embodiment of the invention, the compound is selected from the group consisting of:
(4aS,8aR)-4a,8,8-trimethyl-7-oxo-3,4,4a,7,8,8a-hexahydro-2H-chromene-6-carbonitrile;
(4aR,8aS)-4a,8,8-trimethyl-7-oxo-3,4,4a,7,8,8a-hexahydro-2H-chromene-6-carbonitrile;
(2S,4aS,8aR)-2,4a,8,8-tetramethyl-7-oxo-2,3,4,8a-tetrahydrochromene-6-carbonitrile;
(2R,4aR,8aS)-2,4a,8,8-tetramethyl-7-oxo-2,3,4,8a-tetrahydrochromene-6-carbonitrile;
(2R,4aS,8aR)-2,4a,8,8-tetramethyl-7-oxo-2,3,4,8a-tetrahydrochromene-6-carbonitrile;
(2S,4aR,8aS)-2,4a,8,8-tetramethyl-7-oxo-2,3,4,8a-tetrahydrochromene-6-carbonitrile;
(4aS,8aR)-4a-ethyl-8,8-dimethyl-7-oxo-2,3,4,8a-tetrahydrochromene-6-carbonitrile;
(4aR,8aS)-4a-ethyl-8,8-dimethyl-7-oxo-2,3,4,8a-tetrahydrochromene-6-carbonitrile;
(2S,4aS,8aR)-4a-ethyl-2,8,8-trimethyl-7-oxo-2,3,4,8a-tetrahydrochromene-6-carbonitrile;
(2R,4aR,8aS)-4a-ethyl-2,8,8-trimethyl-7-oxo-2,3,4,8a-tetrahydrochromene-6-carbonitrile;
(2R,4aS,8aR)-4a-ethyl-2,8,8-trimethyl-7-oxo-2,3,4,8a-tetrahydrochromene-6-carbonitrile;
(2S,4aR,8aS)-4a-ethyl-2,8,8-trimethyl-7-oxo-2,3,4,8a-tetrahydrochromene-6-carbonitrile;
(2S,4aS,8aR)-2-ethyl-4a,8,8-trimethyl-7-oxo-2,3,4,8a-tetrahydrochromene-6-carbonitrile;
(2R,4aR,8aS)-2-ethyl-4a,8,8-trimethyl-7-oxo-2,3,4,8a-tetrahydrochromene-6-carbonitrile;
(2R,4aS,8aR)-2-ethyl-4a,8,8-trimethyl-7-oxo-2,3,4,8a-tetrahydrochromene-6-carbonitrile;
(2S,4aR,8aS)-2-ethyl-4a,8,8-trimethyl-7-oxo-2,3,4,8a-tetrahydrochromene-6-carbonitrile;
(2S,4aS,8aR)-2,4a-diethyl-8,8-dimethyl-7-oxo-2,3,4,8a-tetrahydrochromene-6-carbonitrile;
(2R,4aR,8aS)-2,4a-diethyl-8,8-dimethyl-7-oxo-2,3,4,8a-tetrahydrochromene-6-carbonitrile;
(2R,4aS,8aR)-2,4a-diethyl-8,8-dimethyl-7-oxo-2,3,4,8a-tetrahydrochromene-6-carbonitrile;
(2S,4aR,8aS)-2,4a-diethyl-8,8-dimethyl-7-oxo-2,3,4,8a-tetrahydrochromene-6-carbonitrile;
(2R,4aS,8aR)-2-cyclopropyl-4a,8,8-trimethyl-7-oxo-2,3,4,8a-tetrahydrochromene-6-carbonitrile;
(2S,4aR,8aS)-2-cyclopropyl-4a,8,8-trimethyl-7-oxo-2,3,4,8a-tetrahydrochromene-6-carbonitrile;
(2S,4aS,8aR)-2-isopropyl-4a,8,8-trimethyl-7-oxo-2,3,4,8a-tetrahydrochromene-6-carbonitrile;
(2R,4aR,8aS)-2-isopropyl-4a,8,8-trimethyl-7-oxo-2,3,4,8a-tetrahydrochromene-6-carbonitrile;
(2R,4aS,8aR)-2-isopropyl-4a,8,8-trimethyl-7-oxo-2,3,4,8a-tetrahydrochromene-6-carbonitrile;
(2S,4aR,8aS)-2-isopropyl-4a,8,8-trimethyl-7-oxo-2,3,4,8a-tetrahydrochromene-6-carbonitrile;
(2S,4aS,8aR)-2-benzyl-4a,8,8-trimethyl-7-oxo-2,3,4,8a-tetrahydrochromene-6-carbonitrile;
(2R,4aR,8aS)-2-benzyl-4a,8,8-trimethyl-7-oxo-2,3,4,8a-tetrahydrochromene-6-carbonitrile;
(2R,4aS,8aR)-2-benzyl-4a,8,8-trimethyl-7-oxo-2,3,4,8a-tetrahydrochromene-6-carbonitrile;
(2S,4aR,8aS)-2-benzyl-4a,8,8-trimethyl-7-oxo-2,3,4,8a-tetrahydrochromene-6-carbonitrile;
(2R,4aS,8aR)-2,4a,8,8-tetramethyl-3,7-dioxo-3,4,4a,7,8,8a-hexahydro-2H-chromene-6-carbonitrile;
(2S,4aR,8aS)-2,4a,8,8-tetramethyl-3,7-dioxo-3,4,4a,7,8,8a-hexahydro-2H-chromene-6-carbonitrile;
(4aS,8aS)-4a,8,8-trimethyl-7-oxo-3,8a-dihydro-2H-1,4-benzodioxine-6-carbonitrile;
(4aR,8aR)-4a,8,8-trimethyl-7-oxo-3,8a-dihydro-2H-1,4-benzodioxine-6-carbonitrile;
4a,8,8,8a-tetramethyl-7-oxo-3,4,4a,7,8,8a-hexahydro-2H-chromene-6-carbonitrile (cis fused, racemic); and
4a,8,8-trimethyl-7-oxo-4a,7,8,8a-tetrahydro-4H-chromene-6-carbonitrile (cis-fused, racemic),
and a pharmaceutically acceptable salt thereof.

As used herein, the term "alkyl" refers to a fully saturated branched or unbranched hydrocarbon moiety. Unless otherwise specified, the alkyl comprises 1 to 12 carbon atoms, preferably 1 to 8 carbon atoms, more preferably 1 to 6 carbon atoms or most preferably 1 to 4 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl and n-hexyl.

As used herein, the term "alkylene" refers to a non-aromatic divalent group, wherein the alkylene group is attached with two σ-bonds, with two saturated carbon atoms as the points of attachment. An alkylene is a linear or branched acyclic structure with no carbon-carbon double or triple bonds, and no atoms other than carbon and hydrogen. The groups, - CH₂- (methylene), -CH₂CH₂-, -CH₂C(CH₃)₂CH₂- and -CH₂CH₂CH₂-, are non-limiting examples of alkylene groups.

As used herein, the term "alkenyl" refers to an unsaturated hydrocarbon group which may be linear or branched and has at least one carbon-carbon double bond. Alkenyl groups with 2-12 carbon atoms or 2-6 carbon atoms are preferred. The alkenyl group may contain 1, 2 or 3 carbon-carbon double bonds, or more. Preferably, alkenyl groups contain one or two double bonds, most preferably one double bond. Examples of alkenyl groups include ethenyl, n-propenyl, isopropenyl, n-but-2-enyl, n-hex-3-enyl and the like.

As used herein, the term "alkenylene" refers to a non-aromatic divalent group, wherein the alkenylene group is attached with two σ-bonds, with two carbon atoms as points of attachment. An alkenylene is linear or branched acyclic structure havingat least one nonaromatic carbon-carbon double bond with no carbon-carbon triple bonds and no atoms other than carbon and hydrogen. The groups, -CH=CH-, -CH=C(CH₃)CH₂- and - CH=CHCH₂-, are non-limiting examples of alkenylene groups.

As used herein, the term "alkynyl" refers to an unsaturated hydrocarbon group which is linear or branched and has at least one carbon-carbon triple bond. Alkynyl groups with 2-12 carbon atoms or 2-6 carbon atoms can be preferred. The alkynyl group may contain 1, 2 or 3 carbon-carbon triple bonds, or more. Preferably, alkynyl groups contain one or two triple bonds, most preferably one triple bond. Examples of alkynyl groups include ethynyl, n-propynyl, n-but-2-ynyl, n-hex-3-ynyl and the like.

As used herein, the term "alkynylene" refers to a non-aromatic divalent group, wherein the alkynylene group is attached with two σ-bonds, with two carbon atoms as points of attachment. An alkynylene is a linear or branched acyclic structure having at least one carbon-carbon triple bond withno atoms other than carbon and hydrogen. The groups, -C=C-, -C≡CCH₂- and -C≡CCH(CH₃)-, are non-limiting examples of alkynylene groups.

As used herein, the term "alkylidene" refers to the divalent group =CRR', wherein the alkylidene group is attached with one σ-bond and one π-bond, in which R and R' are independently hydrogen, alkyl, or R and R' are taken together to represent alkylene. Non-limiting examples of alkylidene groups include: =CH₂, =CH(CH₂CH₃) and =C(CH₃)₂.

As used herein, the term "acyl" refers to a monovalent group with a carbon atom of a carbonyl group as the point of attachment, further having a linear or branched, cyclo, cyclic or acyclic structure, further having no additional atoms that are not carbon or hydrogen, beyond the oxygen atom of the carbonyl group. The groups, -CHO, -C(O)CH₃ (acetyl, Ac), - C(O)CH₂CH₃, -C(O)CH₂CH₂CH₃, -C(O)CH(CH₃)₂, -C(O)CH(CH₂)₂, -C(O)C₆H₅, - C(O)C₆H₄CH₃, -C(O)C₆H₄CH₂CH₃, -COC₆H₃(CH₃)₂ and -C(O)CH₂C₆H₅, are non-limiting examples of acyl groups. The term "acyl" therefore encompasses, but is not limited to, groups sometimes referred to as "alkyl carbonyl" and "aryl carbonyl" groups.

As used herein, the term "alkoxy" refers to the group -OR, in which R is a C₁₋₁₂alkyl, as that term is defined above. Non-limiting examples of alkoxy groups include: -OCH₃, - OCH₂CH₃, -OCH₂CH₂CH₃, -OCH(CH₃)₂, -OCH(CH₂)₂, -O-cyclopropyl, -O-cyclobutyl, -O-cyclopentyl and -O-cyclohexyl.

The number of carbon atoms in a group is specified herein by the prefix "Cₓ₋ₓₓ", wherein x and xx are integers. For example, "C₁₋₄alkyl" is an alkyl group which has from 1 to 4 carbon atoms.

As used herein, the term "halogen" or "halo" may be fluoro, chloro, bromo or iodo.

As used herein, the term "heterocyclyl" refers to a saturated or unsaturated, monocyclic or bicyclic (e.g., bridged, fused or spiro ring systems) ring system which has from 3- to 12-ring members, or in particular 3- to 6- ring members or 5- to 7- ring members, at least one of which is a heteroatom, and up to 4 (*e.g*., 1, 2, 3 or 4) of which may be heteroatoms, wherein the heteroatoms are independently selected from O, S and N, and wherein C can be oxidized (e.g., C(O)), N can be oxidized (e.g., N(O)) or quaternized, and S can be optionally oxidized to sulfoxide and sulfone. Unsaturated heterocyclic rings include heteroaryl rings and heterocyclic rings that is not aromatic (i.e., "non-aromatic heterocycles"). As used herein, the term "heteroaryl" refers to an aromatic 5 to 12 membered monocyclic or bicyclic ring system, having 1 to 4 heteroatoms independently selected from O, S and N, and wherein N can be oxidized (e.g., N(O)) or quaternized, and S can be optionally oxidized to sulfoxide and sulfone. A non-aromatic heterocyclyl is a 3- to 7-membered saturated monocyclic or a 3- to 6-membered saturated monocyclic or a 5- to 7-membered saturated monocyclic ring. A non-aromatic heterocyclyl is a 3- to 7-membered unsaturated monocyclic or a 3- to 6-membered unsaturated monocyclic or a 5- to 7-membered unsaturated monocyclic ring. In another embodiment, a heterocyclyl is a 6 or-7-membered bicyclic ring. The heterocyclyl group can be attached at a heteroatom or a carbon atom. Examples of non-aromatic heterocyclyls include aziridinyl, azetidinyl, oxetanyl, thietanyl, pyrrolidinyl, tetrahydrofuranyl, thiolanyl, imidazolidinyl, pyrazolidinyl, isoxazolidinyl, isothiazolidinyl, piperidinyl, tetrahydropyranyl, thianyl, piperazinyl, morpholinyl, thiomorpholinyl, dioxanyl, dithianyl, azepanyl, oxepanyl, thiepanyl, dihydrofuranyl, imidazolinyl, dihydropyranyl, hydantoinyl, pyrrolidinonyl, tetrahydrothiopyranyl, tetrahydropyridinyl, and thiopyranyl, and examples of heteroaryls including pyrrolyl, furanyl, imidazolyl, pyrazolyl, oxazolyl, isoxazolyl, thiazolyl, isothiazolyl, oxadiazolyl, thiadiazolyl, triazolyl, tetrazolyl, pyridyl, pyranyl, pyrazinyl, pyrimidyl, pyridazinyl, oxazinyl, thiazinyl, dioxinyl, triazinyl, tetrazinyl, azepinyl, oxepinyl, thiepinyl, thiazepinyl, 1-oxo-pyridyl, thienyl, valerolactamyl, azaindolyl, benzimidazolyl, benzo[1,4]dioxinyl, benzofuryl, benzoisoxazolyl, benzoisothiazolyl, benzothiadiazolyl, benzothiazolyl, benzothienyl, benzotriazolyl, benzoxadiazolyl, benzoxazolyl, cyclopentaimidazolyl, cyclopentatriazolyl, imidazo[1,2-a]pyridyl, indazolyl, indolizinyl, indolyl, isoquinolinyl, oxazolopyridinyl, purinyl, pyrazolo[3,4]pyrimidinyl, pyridopyazinyl, pyridopyrimidinyl, pyrrolo[2,3]pyrimidinyl, pyrrolopyrazolyl, pyrroloimidazolyl, pyrrolotriazolyl, quinazolinyl, quinolinyl, thiazolopyridinyl, and the like. Examples of bicyclic nonaromatic heterocyclic ring systems include benzo[1,3]dioxolyl, tetrahydroindolyl, and 2-azaspiro[3.3]heptanyl.

As used herein, the term "carbocyclyl" refers to saturated, partially unsaturated, or aromatic monocyclic or bicyclic hydrocarbon groups of 3-12 carbon atoms, 3-6 carbon atoms or 5-7 carbon atoms. The term "carbocyclyl" encompasses cycloalkyl groups and aromatic groups. The term "cycloalkyl" refers to completely saturated monocyclic or bicyclic (bridged, fused or spiro) hydrocarbon groups of 3-12 carbon atoms, 3-6 carbon atoms or 5-7 carbon atoms. "Aromatic group or "aryl" refers to an aromatic 6-12 membered monocyclic or bicyclic ring system. Exemplary monocyclic carbocyclyl groups include, but are not limited to, cyclopropyl, cyclobutyl, cyclopentyl, cyclohexyl, cycloheptyl, cyclopropenyl, cyclobutenyl, cyclopenentyl, cyclohexenyl, cycloheptenyl, cyclobutadienyl, cyclopentadienyl, cyclohexadienyl, cycloheptadienyl, phenyl and cycloheptatrienyl.

The term "bridged ring system," as used herein, is a ring system that has a carbocyclyl or heterocyclyl ring wherein two non-adjacent atoms of the ring are connected (bridged) by one or more (preferably from one to three) atoms selected from C, N, O or S. A bridged ring system may have 6-12 ring members. Exemplary bridged carbocyclyl groups include decahydro-2,7-methanonaphthyl, bicyclo[2.2.1]heptyl, bicyclo[2.1.1]hexyl, bicyclo[2.2.1]heptenyl, tricyclo[2.2.1.0^{2,6}]heptanyl, 6,6-dimethylbicyclo[3.1.1]heptyl, and 2,6,6-trimethylbicyclo[3.1.1]heptyl.

The term "fused ring system," as used herein, is a ring system that has a carbocyclyl or heterocyclyl ring wherein two adjacent atoms of the ring are connected (bridged) by one or more (preferably from one to three) atoms selected from C, N, O or S. A fused ring system may have from 4-10 ring members.

The term "spiro ring system," as used herein, is a ring system that has two rings each of which are independently selected from a carbocyclyl or a heterocyclyl, wherein the two ring structures having one ring atom in common. Spiro ring systems have from 5 to 7 ring members. Exemplary sprio ring carbocyclyl groups include spiro[2.2]pentanyl and spiro[3.3]heptanyl.

Pharmaceutical acceptable salts of the compounds disclosed herein are also included in the invention. In cases where a compound provided herein is sufficiently basic or acidic to form stable nontoxic acid or base salts, preparation and administration of the compounds as pharmaceutically acceptable salts may be appropriate. Examples of pharmaceutically acceptable salts are organic acid addition salts formed with acids which form a physiological acceptable anion, for example, tosylate, methanesulfonate, acetate, citrate, malonate, tartarate, succinate, benzoate, ascorbate, α-ketoglutarate or α-glycerophosphate. Inorganic salts may also be formed, including hydrochloride, sulfate, nitrate, bicarbonate and carbonate salts.

Pharmaceutically acceptable salts may be obtained using standard procedures well known in the art, for example by reacting a sufficiently basic compound such as an amine with a suitable acid affording a physiologically acceptable anion. Alkali metal (for example, sodium, potassium or lithium) or alkaline earth metal (for example calcium) salts of carboxylic acids can also be made.

Pharmaceutically-acceptable base addition salts can be prepared from inorganic and organic bases. Salts from inorganic bases can include, but are not limited to, sodium, potassium, lithium, ammonium, calcium and magnesium salts. Salts derived from organic bases can include, but are not limited to, salts of primary, secondary or tertiary amines, such as alkyl amines, dialkyl amines, trialkyl amines, substituted alkyl amines, di(substituted alkyl) amines, tri(substituted alkyl) amines, alkenyl amines, dialkenyl amines, trialkenyl amines, substituted alkenyl amines, di(substituted alkenyl) amines, tri(substituted alkenyl) amines, cycloalkyl amines, di(cycloalkyl) amines, tri(cycloalkyl) amines, substituted cycloalkyl amines, disubstituted cycloalkyl amine, trisubstituted cycloalkyl amines, cycloalkenyl amines, di(cycloalkenyl) amines, tri(cycloalkenyl) amines, substituted cycloalkenyl amines, disubstituted cycloalkenyl amine, trisubstituted cycloalkenyl amines, aryl amines, diaryl amines, triaryl amines, heteroaryl amines, diheteroaryl amines, triheteroaryl amines, heterocycloalkyl amines, diheterocycloalkyl amines, triheterocycloalkyl amines or mixed di- and tri-amines where at least two of the substituents on the amine can be different and can be alkyl, substituted alkyl, alkenyl, substituted alkenyl, cycloalkyl, substituted cycloalkyl, cycloalkenyl, substituted cycloalkenyl, aryl, heteroaryl, heterocycloalkyl and the like. Also included are amines where the two or three substituents, together with the amino nitrogen, form a heterocycloalkyl and heteroaryl group. Non-limiting examples of amines can include, isopropylamine, trimethyl amine, diethyl amine, tri(iso-propyl) amine, tri(n-propyl) amine, ethanolamine, 2-dimethylaminoethanol, tromethamine, lysine, arginine, histidine, caffeine, procaine, hydrabamine, choline, betaine, ethylenediamine, glucosamine, N-alkylglucamines, theobromine, purines, piperazine, piperidine, morpholine, N-ethylpiperidine and the like. Other carboxylic acid derivatives can be useful, for example, carboxylic acid amides, including carboxamides, lower alkyl carboxamides or dialkyl carboxamides and the like.

The disclosed compounds, or pharmaceutically acceptable salts thereof, can contain one or more asymmetric centers in the molecule. In accordance with the present disclosure any structure that does not designate the stereochemistry is to be understood as embracing all the various stereoisomers (*e.g*., diastereomers and enantiomers) in pure or substantially pure form, as well as mixtures thereof (such as a racemic mixture, or an enantiomerically enriched mixture). It is well known in the art how to prepare such optically active forms (for example, resolution of the racemic form by recrystallization techniques, synthesis from optically-active starting materials, by chiral synthesis or chromatographic separation using a chiral stationary phase). The disclosed compounds may exist in tautomeric forms and mixtures and separate individual tautomers are contemplated. In addition, some compounds may exhibit polymorphism.

When a particular steroisomer (e.g., enantiomer, diasteromer, etc.) of a compound used in the disclosed methods is depicted by name or structure, the stereochemical purity of the compounds is at least 60%, 70%, 80%, 90%, 95%, 97%, 99%, 99.5% or 99.9%. "Stererochemical purity" means the weight percent of the desired stereoisomer relative to the combined weight of all stereoisomers.

When the stereochemistry of a disclosed compound is named or depicted by structure, and the named or depicted structure encompasses more than one stereoisomer (e.g., as in a diastereomeric pair), it is to be understood that one of the encompassed stereoisomers or any mixture of the encompassed stereoisomers are included. It is to be further understood that the stereoisomeric purity of the named or depicted stereoisomers at least 60%, 70%, 80%, 90%, 99% or 99.9% by weight. The stereoisomeric purity in this case is determined by dividing the total weight in the mixture of the stereoisomers encompassed by the name or structure by the total weight in the mixture of all of the stereoisomers.

In one embodiment, any position occupied by hydrogen is meant to include enrichment by deuterium above the natural abundance of deuterium as well. For example, one or more hydrogen atoms are replaced with deuterium at an abundance that is at least 3340 times greater than the natural abundance of deuterium, which is 0.015% (i.e., at least 50.1% incorporation of deuterium), at least 3500 (52.5% deuterium incorporation at each designated deuterium atom), at least 4000 (60% deuterium incorporation), at least 4500 (67.5% deuterium incorporation), at least 5000 (75% deuterium), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation). In one embodiment, hydrogen is present at all positions at its natural abundance. The compounds or pharmaceutically acceptable salts thereof as described herein, may exist in tautomeric forms and mixtures and separate individual tautomers are contemplated.

Another embodiment is a pharmaceutical composition comprising at least one compound described herein, or a pharmaceutically acceptable salt thereof, and at least one pharmaceutically acceptable carrier.

The compounds provided herein can be useful to activate the NRF2 pathway in a cell. In one embodiment, the method comprises contacting a cell with an effective amount of a compound provided herein, or a pharmaceutically acceptable salt thereof. In one embodiment, the cell is contacted in vitro or in vivo. In one embodiment, contacting the cell includes administering the compound to a subject.

One embodiment of the invention includes a method for activating Nrf2 in a subject in need thereof, the method comprising administering to the subject a therapeutically effective amount of a compound described herein, or a pharmaceutically acceptable salt thereof, thereby activating Nrf2 in the subject.

One embodiment of the invention includes a method for inhibiting a KEAP1 protein in a cell, the method comprising contacting a cell with an effective amount of a compound provided herein, or a pharmaceutically acceptable salt thereof, thereby inhibiting a KEAP1 protein in the cell.

One embodiment of the invention includes a method for increasing a cell's ability to resist a stress, the method comprising administering to the subject a therapeutically effective amount of a compound described herein, or a pharmaceutically acceptable salt thereof, thereby increasing the cell's ability to resist the stress. The stress is selected from the group consisting of heat shock, oxidative stress, osmotic stress, DNA damage, inadequate salt level, inadequate nitrogen level and inadequate nutrient level.

One embodiment of the invention includes a method for mimiking the effect of nutrient restriction on the cell, the method comprising administering to the subject a therapeutically effective amount of a compound described herein, or a pharmaceutically acceptable salt thereof, thereby mimiking the effect of the nutrient restriction on the cell.

One embodiment of the invention includes a method for promoting survival of a eukaryotic cell (e.g., a mammalian cell) or increasing the lifespan of the cell, the method comprising administering to the subject a therapeutically effective amount of a compound provided herein, or a pharmaceutically acceptable salt thereof, thereby promoting survival of the eukaryotic cell or increasing the lifespan of the cell.

One embodiment of the invention includes a method for treating a disease associated with cell death in a subject, the method comprising administering to the subject a therapeutically effective amount of a compound described herein, or a pharmaceutically acceptable salt thereof.

One embodiment of the invention includes a method for treating a disease caused by oxidative stress in a subject, the method comprising administering to the subject a therapeutically effective amount of a compound provided herein, or a pharmaceutically acceptable salt thereof.

One embodiment of the invention includes a method for treating a disorder in a subject, wherein the disorder is selelcted from the group consisting of a neurodegenerative disease, inflammation/an inflammatory disease, an autoimmune disease, an ischemic fibrotic disease, a cancer, premature aging, a cardiovascular disease, a liver disease, a hemoglobinopathy, thalassemia (e.g. beta-thalassemia) and a metabolic disorder, the method comprising administering to the subject a therapeutically effective amount of a compound provided herein, or a pharmaceutically acceptable salt thereof. Hemoglobinopathy includes sickle cell disease (SCD). In one embodiment, the disorder is sickle cell disease or thalassemia (e.g. beta-thalassemia). More specifically, the disorder is sickle cell disease.

The neurodegenerative disease can be selected from the group consisting of Alzheimer's disease (AD), Parkinson's disease (PD), Huntington disease (HD) and other CAG-triplet repeat (or polyglutamine) diseases, amyotrophic lateral sclerosis (ALS; Lou Gehrig's disease), diffuse Lewy body disease, chorea-acanthocytosis, primary lateral sclerosis, multiple sclerosis (MS), frontotemporal dementia, Friedreich's ataxia and epilepsy (repression of microglia activation). More preferably, the neurodegenerative disease is Parkinson's disease or amyotrophic lateral sclerosis.

The inflammatory disease can be selected from the group consisting of chronic cholecystitis, aortic valve stenosis, restenosis, a skin disease, a pulmonary diseases and a disease of the airway, inflammatory uveitis, atherosclerosis, arthritis, conjunctivitis, pancreatitis, a chronic kidney disease (CDK), an inflammatory condition associated with diabetes,an ischemia, a transplant rejection, a CD14 mediated sepsis, a non-CD 14 mediated sepsis, Behcet's syndrome, ankylosing spondylitis, sarcoidosis and gout. In some embodiments, the skin disease is selected from the group consisting of rash, contact dermatitis and atopic dermatitis. In one embodiment, the pulmonary disease and disease of the airway is selected from the group consisting of Adult Respiratory Disease Syndrome (ARDS), Chronic Obstructive Pulmonary Disease (COPD), pulmonary fibrosis, an interstitial lung disease, asthma, chronic cough, allergic rhinitis, bronchiectasis and bronchitis. In one embodiment, the inflammatory condition associated with diabetes is selected from a diabetic retinopathy, a diabetic cardiomyopathy and a diabetes-induced aortic damage.

The autoimmune disease is selected from the group consisting of psoriasis, inflammatory bowel disease, rheumatoid arthritis, systemic lupus erythematosus, multiple sclerosis, type 1 diabetes, systemic sclerosis and Sjogren's syndrome. In one embodiment, the inflammatory bowel disease is Crohn's disease or ulcerative colitis. In one embodiment, the autoimmune disease is type 1 diabetes. Alternatively, the autoimmune disease is multiple sclerosis.

The ischemic fibrotic disease is selected from the group consisting of stroke, acute lung injury, acute kidney injury, ischemic cardiac injury, acute liver injury and ischemic skeletal muscle injury.

The cancer is selected from the group consisting of prostate cancer, bladder cancer, ovarian cancer, breast cancer (e.g., breast cancer with mutated BRCA1), head and neck cancer, chronic lymphocytic leukemia, thymus cancer, hepatocellular carcinoma, colorectal cancer, colon cancer, skin cancer, pancreatic cancer, leukemia, lung cancer, glioblastoma, cervical cancer, lymphoma, Waldenström's macroglobulinemia and multiple myeloma.

The cardiovascular disease can be selected from the group consisting of pulmonary arterial hypertension, systemic hypertension, coronary artery disease, peripheral artery disease and atherosclerosis.

The liver disease can be selected from the group consisting of non-alcoholic steatohepititis (NASH), alcoholic liver disease, primary biliary cirrhosis and primary sclerosing cholangitis.

The hemoglobinopathy is a condition that involves a mutation in human beta-globin or an expression control sequence thereof, such as sickle cell disease (SCD) or beta-thalassemia. SCD typically arises from a mutation substituting thymine for adenine in the sixth codon of the beta-chain gene of hemoglobin (i.e., GAG to GTG of the HBB gene). This mutation causes glutamate to valine substitution in position 6 of the Hb beta chain. The resulting Hb, referred to as HbS, has the physical properties of forming polymers under conditions of low oxygen tension. SCD is typically an autosomal recessive disorder. Beta-Thalassemias are a group of inherited blood disorders caused by a variety of mutational mechanisms that result in a reduction or absence of synthesis of β-globin and leading to accumulation of aggregates of unpaired, insoluble α-chains that cause ineffective erythropoiesis, accelerated red cell destruction, and severe anemia. Subjects with beta-thalassemia exhibit variable phenotypes ranging from severe anemia to clinically asymptomatic individuals. The genetic mutations present in β thalassemias are diverse, and can be caused by a number of different mutations. The mutations can involve a single base substitution or deletions or inserts within, near or upstream of the β globin gene. For example, mutations occur in the promoter regions preceding the beta-globin genes or cause production of abnormal splice variants. β⁰ is used to indicate a mutation or deletion which results in no functional β globin being produced. β⁺ is used to indicate a mutation in which the quantity or β globin is reduced or in which the β globin produced has a reduced functionality.

Examples of thalassemias include thalassemia minor, thalassemia intermedia, and thalassemia major.

Thalassemia minor refers to thalassemia where only one of beta-globin alleles bears a mutation. Individuals typically suffer from microcytic anemia. Detection usually involves lower than normal MCV value (<80 fL) plus an increase in fraction of Hemoglobin A2 (>3.5%) and a decrease in fraction of Hemoglobin A (<97.5%). Genotypes can be β⁺/β or β⁰/β.

Thalassemia intermedia refers to a thalassemia intermediate between the major and minor forms. Affected individuals can often manage a normal life but may need occasional transfusions, e.g., at times of illness or pregnancy, depending on the severity of their anemia. Genotypes can be β⁺/β⁺ or β⁰/β.

Thalassemia major refers to a thalassemia where both beta-globin alleles have thalassemia mutations. This is a severe microcytic, hypochromic anemia. If left untreated, it causes anemia, splenomegaly, and severe bone deformities and typically leads to death before age 20. Treatment consists of periodic blood transfusion; splenectomy if splenomegaly is present, and treatment of transfusion-caused iron overload. Cure is possible by bone marrow transplantation. Genotypes include β⁺/β⁰ or β⁰/β⁰ or β⁺/β⁺. Mediteranean anemia or Cooley's anemia has a genotype of β⁰/β⁰ so that no hemoglobin A is produced. It is the most severe form of P-thalasemia.

Although carriers of sickle cell trait do not suffer from SCD, individuals with one copy of HbS and one copy of a gene that codes for another abnormal variant of hemoglobin, such as HbC or Hb beta-thalassemia, typically will have a less severe form of sickle cell disease. For example, another specific defect in beta-globin causes another structural variant, hemoglobin C (HbC). Hemoglobin C (abbreviated as Hb C or HbC) is an abnormal hemoglobin in which substitution of a glutamic acid residue with a lysine residue at the 6th position of the β-globin chain has occurred. A subject that is a double heterozygote for HbS and HbC (HbSC disease) is typically characterized by symptoms of moderate clinical severity.

Another common structural variant of beta-globin is hemoglobin E (HbE). HbE is an abnormal hemoglobin in which substitution of a glutamic acid residue with a lysine residue at the 26th position of the β-globin chain has occurred. A subject that is a double heterozygote for HbS and HbE has HbS/HbE syndrome, which usually causes a phenotype similar to HbS/b+ thalassemia, discussed below.

A subject that is a double heterozygote for HbS and β⁰ thalassemia (i.e., HbS/β⁰ thalassemia) can suffer symptoms clinically indistinguishable from sickle cell anemia.

A subject that is a double heterozygote for HbS and β⁺ thalassemia (i.e., HbS/β⁺ thalassemia) can have mild-to-moderate severity of clinical symptoms with variability among different ethnicities.

Rare combinations of HbS with other abnormal hemoglobins include HbD Los Angeles, G-Philadelphia, HbO Arab, and others.

Nrf2 upregulates fetal hemoglobin which alleviates some of the symptoms of these disorders. Therefore, in some embodiments, the disclosed compositions are used to treated SCD or thalassemia (e.g. beta-thalassemia), including those that involve a mutation in human beta-globin or an expression control sequence thereof, as described above.

In some embodiments, the disclosed compositions and methods are used to treat a subject with an HbS/β⁰ genotype, an HbS/β⁺ genotype, an HBSC genotype, an HbS/HbE genotype, an HbD Los Angeles genotype, a G-Philadelphia genotype, or an abHbO Arab genotype.

In some embodiments, the compositions disclosed herein are administered to a subject in an effective amount to treat one or more symptoms of sickle cell disease, a thalassemia (e.g. beta-thalassemia), or a related disorder. In subjects with sickle cell disease, or a related disorder, physiological changes in RBCs can result in a disease with the following signs: (1) hemolytic anemia; (2) vaso-occlusive crisis; and (3) multiple organ damage from microinfarcts, including heart, skeleton, spleen, and central nervous system. Thalassemia can include symptoms such as anemia, fatigue and weakness, pale skin or jaundice (yellowing of the skin), protruding abdomen with enlarged spleen and liver, dark urine, abnormal facial bones and poor growth, and poor appetite.

Retinopathy due to SCD can also be treated by administering an effective amount of a compound according to any one of described herein. Sickle retinopathy occurs when the retinal blood vessels get occluded by sickle red blood cells and the retina becomes ischemic, angiogenic factors are made in retina. In sickle cell disease, this occurs mostly in the peripheral retina, which does not obscure vision at first. Eventually, the entire peripheral retina of the sickle cell patient becomes occluded and many neovascular formations occur. Administration of a compound according to any one of described herein can reduce or inhibit the formation of occlusions in the peripheral retina of a sickle cell patient.

As used herein, the term "subject" and "patient" may be used interchangeably, and means a mammal in need of treatment, *e.g*., companion animals (*e.g*., dogs, cats and the like), farm animals (*e.g*., cows, pigs, horses, sheep, goats and the like) and laboratory animals (*e.g*., rats, mice, guinea pigs and the like). Typically, the subject is a human in need of treatment.

As used herein, the term "treating" or 'treatment" refers to obtaining desired pharmacological and/or physiological effect. The effect can be therapeutic, which includes achieving, partially or substantially, one or more of the following results: partially or totally reducing the extent of the disease, disorder or syndrome; ameliorating or improving a clinical symptom or indicator associated with the disorder; and delaying, inhibiting or decreasing the likelihood of the progression of the disease, disorder or syndrome.

Administering a compound described herein, or a pharmaceutically acceptable salt thereof, to a mammal comprises any suitable delivery method. Administering a compound described herein, or a pharmaceutically acceptable salt thereof, to a mammal includes administering a compound described herein, or a pharmaceutically acceptable salt thereof, orally, topically, enterally (e.g. orally), parenterally, transdermally, transmucosally, via inhalation, intracisternally, epidurally, intravaginally, intravenously, intramuscularly, subcutaneously, intradermally and intravitreally to the mammal. Administering a compound described herein, or a pharmaceutically acceptable salt thereof, to a mammal also includes administering topically, enterally (e.g. orally), parenterally, transdermally, transmucosally, via inhalation, intracisternally, epidurally, intravaginally, intravenously, intramuscularly, subcutaneously, intradermally and intravitreally to a mammal a compound that metabolizes within or on a surface of the body of the mammal to a compound described herein, or a pharmaceutically acceptable salt thereof.

Thus, a compound or pharmaceutically acceptable salt thereof as described herein, may be systemically administered, e.g., orally, in combination with a pharmaceutically acceptable vehicle such as an inert diluent or an assimilable edible carrier. They may be enclosed in hard or soft shell gelatin capsules, may be compressed into tablets, or may be incorporated directly with the food of the patient's diet. For oral therapeutic administration, the compound or pharmaceutically acceptable salt thereof as described herein may be combined with one or more excipients and used in the form of ingestible tablets, buccal tablets, troches, capsules, elixirs, suspensions, syrups or wafers and the like. Such compositions and preparations should contain at least about 0.1% of active compound. The percentage of the compositions and preparations may, of course, be varied and may conveniently be between about 2 to about 60% of the weight of a given unit dosage form. The amount of active compound in such therapeutically useful compositions can be such that an effective dosage level will be obtained.

The tablets, troches, pills, capsules and the like can include the following: binders such as gum tragacanth, acacia, corn starch and gelatin; excipients such as dicalcium phosphate; a disintegrating agent such as corn starch, potato starch, alginic acid and the like; a lubricant such as magnesium stearate; a sweetening agent such as sucrose, fructose, lactose and aspartame; and a flavoring agent.

The active compound may also be administered intravenously or intraperitoneally by infusion or injection. Solutions of the active compound or its salts can be prepared in water, optionally mixed with a nontoxic surfactant.

Exemplary pharmaceutical dosage forms for injection or infusion can include sterile aqueous solutions or dispersions and sterile powders comprising the active ingredient which are adapted for the extemporaneous preparation of sterile injectable or infusible solutions or dispersions. In all cases, the ultimate dosage form should be sterile, fluid and stable under the conditions of manufacture and storage.

Sterile injectable solutions can be prepared by incorporating the active compound in the required amount in the appropriate solvent with various of the other ingredients enumerated above, as required, followed by filter sterilization. In the case of sterile powders for the preparation of sterile injectable solutions, the preferred methods of preparation can be vacuum drying and the freeze drying techniques, which can yield a powder of the active ingredient plus any additional desired ingredient present in the previously sterile-filtered solutions.

Exemplary solid carriers can include finely divided solids such as talc, clay, microcrystalline cellulose, silica, alumina and the like. Useful liquid carriers include water, alcohols, glycols and water-alcohol/glycol blends, in which the compounds or pharmaceutically acceptable salts thereof as described herein can be dissolved or dispersed at effective levels, optionally with the aid of non-toxic surfactants.

Useful dosages of a compound or pharmaceutically acceptable salt thereof as described herein can be determined by comparing their in vitro activity and in vivo activity in animal models. Methods for the extrapolation of effective dosages in mice and other animals, to humans are known to the art; for example, see U.S. Pat. No. 4,938,949, which is incorporated by reference in its entirety.

"A therapeutically effective amount" and "an effective amount" are interchangeable and refer to an amount that, when administered to a subject, achieves a desired effect for treating a disease treatable with a compound or pharmaceutically acceptable salt thereof as described herein. The therapeutically effective amount of a compound or pharmaceutically acceptable salt thereof as described herein, required for use in treatment can vary not only with the particular salt selected but also with the route of administration, the nature of the condition being treated and the age and condition of the patient and can be ultimately at the discretion of the attendant physician or clinician. In general, however, a dose can be in the range of from about 0.1 µg to about 100 mg/kg of body weight per day.

The desired dose may conveniently be presented in a single dose or as divided doses administered at appropriate intervals.

The disclosed method can include a kit comprising a compound or pharmaceutically acceptable salt thereof as described herein and instructional material which can describe administering a compound or pharmaceutically acceptable salt thereof as described herein or a composition comprising a compound or pharmaceutically acceptable salt thereof as described herein to a cell or a subject. This should be construed to include other embodiments of kits that are known to those skilled in the art, such as a kit comprising a (such as sterile) solvent for dissolving or suspending a compound or pharmaceutically acceptable salt thereof as described herein or composition prior to administering a compound or pharmaceutically acceptable salt thereof as described herein or composition to a cell or a subject. In some embodiments, the subject can be a human.

### EXAMPLE

The terms "Ent1" and Ent2" do not infer structural assignment as to one enantiomer or the other. The absolute configuration of final compounds was only determined in certain instances as described below:
Key:
- A:: Absolute configuration determined by x-ray crystallography and/or circular dichroism
- B:: Absolute configuration assigned by comparison to a class A compound or derived from a common intermediate in the synthesis of a class A compound
- C:: Absolute configuration assigned based on literature precedent
- D:: Absolute configuration unknown

| Compound | Stereochemical Determination |
|---|---|
| 1-Ent1 | D |
| 1-Ent2 | D |
| 2-Ent1 | D |
| 2-Ent2 | D |
| 3-Ent1 | D |
| 3-Ent2 | D |
| 4-Ent1 | D |
| 4-Ent2 | D |
| 5-Ent1 | D |
| 5-Ent2 | D |
| 6-Ent1 | D |
| 6-Ent2 | D |
| 7-Ent1 | D |
| 7-Ent2 | D |
| 8-Ent1 | D |
| 8-Ent2 | D |
| 9-Ent1 | D |
| 9-Ent2 | D |
| 10-Ent1 | D |
| 10-Ent2 | D |
| 11-Ent1 | D |
| 11-Ent2 | D |
| 40-Ent1 | D |
| 40-Ent2 | D |
| 41-Ent1 | D |
| 41-Ent2 | D |
| 42-Ent1 | D |
| 42-Ent2 | D |
| 43-Ent1 | D |
| 43-Ent2 | D |
| 44-Ent1 | D |
| 44-Ent2 | D |
| 45-Ent1 | D |
| 45-Ent2 | D |

### Example 1. Synthesis of Compounds 1-Ent1 and 1-Ent2

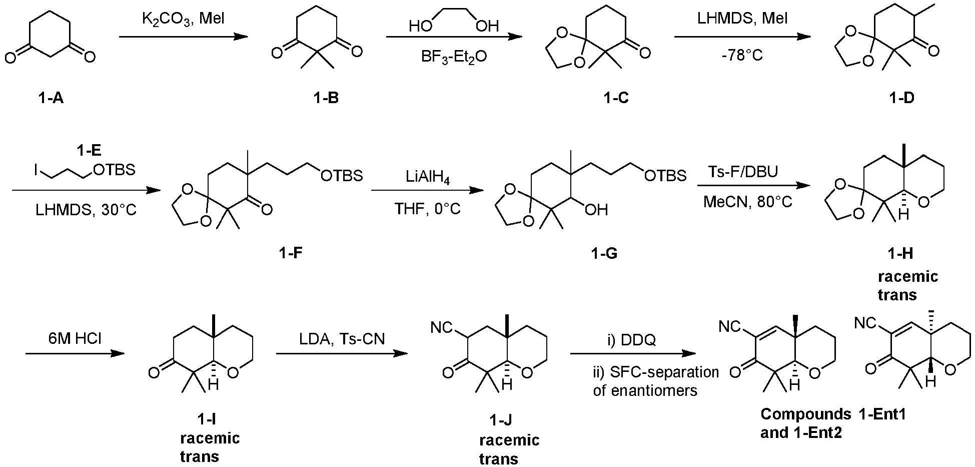

### Preparation of Compound 1-B

To a solution of compound **1-A** (50 g, 445.92 mmol, 1.0 eq.) in acetone (900 mL) was added K₂CO₃ (123.26 g, 891.85 mmol, 2.0 eq.). After stirring for 10 min, CH₃I (162.12 g, 1.14 mol, 2.5 eq.) was added drop-wise. The reaction mixture was stirred at 60°C for 13 hours. TLC (PE/EA = 3:1) showed the starting material was consumed completely. The reaction was cooled to rt. The reaction mixture was filtered to remove most of the solid and the filtrate was concentrated to give a yellow oil. A 300 mL portion of CH₃Cl was added and a precipitate appeared. The mixture was stirred at 15°C for 5 hours, then filtered. The filtrate was concentrated and the residue was purified by silica gel chromatography eluted with petroleum ether/EtOAc = 3:1 to give compound **1-B** (28 g, 44.8% Yield) as pale-yellow oil. **¹H NMR: (400MHz, CDCl₃)** δ: 2.61 (t, *J* = 6.8 Hz, 4H), 1.87 (quin, *J* = 6.8 Hz, 2H), 1.21 (s, 6H).

### Preparation of Compound 1-C

BF₃-Et₂O (2.83 g, 19.97 mmol, 0.1 eq.) was added by syringe to a stirred solution of compound **1-B** (28.0 g, 199.74 mmol, 1.0 eq.) and ethylene glycol (27.27 g, 439.44 mmol, 2.2 eq.) in THF (200 mL) at 15°C under N₂ atmosphere. After stirring at 15°C for 26 hours, the reaction mixture was diluted with THF (100 mL) and washed with brine (150 mL). The organic phase was diluted with petroleum ether until cloudy and dried over Na₂SO₄. The mixture was filtered and concentrated to give a yellow oil. The residue was purified by silica gel chromatography eluted with Petroleum ether/EtOAc = 10:1 ∼ 5:1 to give compound **1-C** (30.0 g, 81.5% Yield) as a colorless oil.
**¹H NMR (400MHz, CDCl₃)** δ: 3.88 (s, 4H), 2.35 (t, *J* = 6.8 Hz, 2H), 1.88 - 1.80 (m, 2H), 1.76 - 1.66 (m, 2H), 1.07 (s, 6H).

### Preparation of Compound 1-D

To a solution of LiHMDS (81.42 mL, 81.42 mmol, 1.0 M in THF, 1.0 eq.) was added compound **1-C** (15.0 g, 81.42 mmol, 1.0 eq.) in THF (60 mL) drop-wise at -78°C under N₂ atmosphere. After stirring for 1.5 hours, the mixture was treated with iodomethane (16.0 g, 112.74 mmol, 1.4 eq.) in THF (40 mL) drop-wise over 20 min. The mixture was allowed to warm to 10°C over 3 hours. TLC (PE/EA = 10:1) showed the reaction was complete. The reaction was quenched with 30% of NH₄Cl (100 mL) and extracted with EtOAc (2 x 100 mL). The combined organic layers were washed with brine (200 mL), dried over Na₂SO₄, filtered and concentrated. The residue was purified by silica gel chromatography eluted with PE/EtOAc = 20:1 to give compound **1-D** (14.3 g, 88.6% Yield) as pale-yellow oil.
**¹HNMR: (400 MHz, CDCl₃**) δ: 4.00 - 3.89 (m, 4H), 2.65 (t, *J* = 6.6 Hz, 1H), 2.13 - 2.08 (m, 1H), 1.90 -1.80 (m, 1H), 1.78 - 1.71 (m, 1H), 1.38 (dd, *J* = 4.2 Hz, 1H), 1.26 (s, 3H), 1.05 - 0.98 (m, 6H).
**LCMS: (M+H:** 199.0)

### Preparation of Compound 1-E

To a solution of (3-bromopropoxy)(tert-butyl)dimethylsilane (10 g, 39.49 mmol, 1.0 eq) in acetone (30 mL, dried over MgSO₄) was added NaI (14.80 g, 98.72 mmol, 2.5 eq.). The reaction was heated at 60°C for 30 minutes. The solution was then diluted with petroleum ether (200 mL) and the solid was removed by filtration. The filtrate was concentrated to give the desired product **1-E** as yellow oil, which was used for the next step directly without further purification (12.0 g, crude).
**¹HNMR: (400 MHz, CDCl₃)** δ: 3.70 - 3.64 (m, 2 H), 3.29 (t, *J* = 6.6 Hz, 2 H), 2.00 (quin, *J* = 6.0 Hz, 2 H), 0.90 (s, 9 H), 0.08 (s, 6 H).

### Preparation of Compound 1-F

To a solution of compound **1-D** (2.5 g, 12.61 mmol, 1.0 eq.) in THF (60 mL) at 30°C was added LiHMDS (16.39 mL, 16.39 mmol, 1.0 M in THF, 1.3 eq.). After stirring for 1.5 hours, the mixture was treated with compound **1-E** (5.68 g, 18.91 mmol, 1.5 eq.) in THF (10 mL) drop-wise. The mixture was stirred at 30°C for 10 hours. TLC (PE/EA = 5:1) showed the reaction was complete. The reaction was quenched with 50% of NH₄Cl (100 mL) and extracted with EtOAc (2 x 100 mL). The combined organic layers were washed with brine (100 mL), dried over Na₂SO₄, filtered and concentrated. The residue was purified by silica gel chromatography eluted with PE/EtOAc = 10:1 to give compound **1-F** (4.3 g, 92.1% Yield) as colorless oil.
**¹HNMR: (400 MHz, CDCl₃)** δ: 3.95 - 3.89 (m, 4H), 3.59 - 3.47 (m, 2H), 2.01 - 1.92 (m, 1H), 1.90 - 1.82 (m, 1H), 1.81 - 1.73 (m, 1H), 1.66 - 1.55 (m, 2H), 1.50 - 1.35 (m, 2H), 1.34 - 1.26 (m, 1H), 1.11 (s, 3H), 1.09 (s, 3H), 1.06 (s, 3H), 0.87 (s, 9H), 0.00 (s, 6H).

### Preparation of Compound 1-G

To a solution of compound **1-F** (4.3 g, 11.60 mmol, 1.0 eq.) in THF (50 mL) was added LiAlH₄ (660.57 mg, 17.40 mmol, 1.5 eq.) in portions at 0°C. The resulting slurry was then stirred at 15°C for 4 hours. TLC (PE/EA = 5:1) showed the most of the starting material was consumed. 4.3 g of Na₂SO₄.10H₂O was added at 0 ∼ 5°C in portions until gas evolution ceased. The slurry was stirred at 10°C for another 2 hours, and then filtered. The filtrate was concentrated to give the crude product. The residue was purified by silica gel chromatography eluted with PE/EtOAc = 20:1 ∼ 10:1 to give compound **1-G** (1.2 g)
**¹HNMR: (400 MHz, CDCl₃)** δ: 3.94 (d, *J* = 4.0 Hz, 4H), 3.58 (t, *J* = 6.4 Hz, 2H), 3.32 (d, *J* = 4.4 Hz, 1H), 1.82 - 1.73 (m, 1H), 1.62 (br. s., 1H), 1.55 - 1.40 (m, 5H), 1.38 - 1.30 (m, 1H), 1.04 (s, 3H), 0.98 (s, 3H), 0.97 - 0.92 (m, 3H), 0.90 (s, 9H), 0.06 (s, 6H).

### Preparation of Compound 1-H

To a solution of compound **1-G** (1.2 g, 3.22 mmol, 1.0 eq.) in MeCN (30 mL) were added TsF (673.18 mg, 3.86 mmol, 1.2 eq.) and DBU (1.47 g, 9.66 mmol, 3.0 eq.) successively. The mixture turned yellow and then was heated to 83 °C for 2 hours. TLC (PE/EA = 5:1) showed the reaction was complete. The solvent was removed under vacuum. The residue was treated with water (50 mL) and extracted with EtOAc (2 x 50 mL). The combined organic layers were washed with brine (100 mL), dried over Na₂SO₄, filtered and concentrated. The residue was purified by silica gel chromatography eluted with PE/EtOAc = 20:1 ∼ 10:1 to give racemic, trans compound **1-H** (670 mg) as colorless oil which was solidified on standing.
**¹HNMR: (400 MHz, CDCl₃)** δ: 4.07 (m, 1H), 4.00 - 3.90 (m, 4H), 3.49 - 3.40 (m, 1H), 2.92 (s, 1H), 1.94 - 1.81 (m, 2H), 1.45 (dd, *J* = *3.2,* 13.6 Hz, 2H), 1.33 (d, *J* = 9.2 Hz, 4H), 1.05 (s, 3H), 0.98 (s, 3H), 0.93 (s, 3H).

### Preparation of Compound 1-I

Racemic, trans compound **1-H** (670 mg, 2.79 mmol, 1.0 eq.) was added to a co-solvent of THF/H₂O (20 mL, v/v = 1:1), followed by addition of 6M HCl (20 mL) at 0 ∼ 5°C. After addition, the reaction mixture was heated to 30 °C for 1.5 hrs. TLC (PE: EA = 5:1) showed the reaction was complete. The reaction mixture was diluted with water (10 mL) and EtOAc (2 x 10 mL). The combined organic layers were washed with saturated NaHCO₃ (100 mL, aq.), brine (20 mL), dried over Na₂SO₄, filtered and concentrated. The residue was used for the next step directly without further purification (560 mg, crude).
**¹HNMR: (400 MHz, CDCl₃)** δ: 4.13 (dd, *J* = 5.0, 11.0 Hz, 1H), 3.49 - 3.38 (m, 1H), 2.85 (s, 1H), 2.77 - 2.67 (m, 1H), 2.30 - 2.23 (m, 1H), 2.05 - 1.85 (m, 1H), 1.69 - 1.59 (m, 2H), 1.44 - 1.39 (m, 1H), 1.37 - 1.33 (m, 1H), 1.31 - 1.25 (m, 1H), 1.21 (s, 3H), 1.14 (s, 3H), 1.08 (s, 3H).

### Preparation of Compound 1-J

To a solution of compound **1-I** (600 mg, 3.06 mmol, 1.0 eq.) in anhydrous THF (30 mL) was added LDA (2 M in THF/heptane, 1.7 mL, 3.4 mmol, 1.1 eq.) dropwise via syringe at -70 °C. The mixture was stirred at -70 °C for 1 h before TsCN (616 mg, 3.4 mmol, 1.1 eq.) in anhydrous THF (5 mL) was added. The yellow solution was stirred at -70 °C for another 1 h. TLC (PE: EA = 4:1) indicated completion. Quenched with Sat. NH₄Cl (5 mL), diluted with EA (30 mL), H₂O (30 mL). After separation, the water layer was extracted with EA (20 mL x 4). The combined organic layers were dried over anhydrous sodium sulfate. The mixture was filtered and concentrated, and the residue was purified by column chromatography (PE: EA = 5:1∼2:1) to give compound **1-J** (555 mg, 81.0%) as white solid. **¹HNMR: (400 MHz, CDCl₃)** δ: 4.19 - 4.07 (m, 2H), 3.49 - 3.39 (m, 2H), 2.97 (s, 1H), 2.14 - 1.81 (m, 6H), 1.69 - 1.60 (m, 2H), 1.24 (s, 3H), 1.14 (s, 3H), 1.08 - 1.04 (m, 3H).

### Preparation of Compounds 1 Ent1/Ent2

To a suspension of compound **1-J** (555.0 mg, 2.51 mmol, 1.0 eq.) in toluene (20 mL) was added DDQ (853.97 mg, 3.76 mmol, 1.5 eq.). The resulting red mixture was heated to 120°C for 2 hours. LCMS showed the desired MS was detected and the starting material was consumed completely. The solvent was evaporated under vacuum. The residue was treated with DCM (20 mL) and stirred for 10 min, then filtered to remove the solid. The filtrate was concentrated. The residue was purified by column chromatography (PE: EA = 20:1 ∼ 10:1) to give 0.4 g of desired product as colorless oil which was solidified on standing, then separated by SFC (column: AY 250 mm x 30 mm x 10 µm, condition: Base-EtOH) to give Compound **1-Ent1** (Rt = 3.01 min, 143.7 mg) as the first eluting enantiomer and Compound **1-Ent2** (Rt = 3.94 min, 155.8 mg) as the second eluting enantiomer, both as white solid. The absolute configuration was not determined.

### Spectra for 1-Ent2

**¹HNMR: (400 MHz, CDCl₃)** δ: 7.41 (s, 1H), 4.15 (dd, *J* = 5.2, 11.2 Hz, 1H), 3.55 - 3.46 (m, 1H), 3.25 (s, 1H), 2.12 - 1.99 (m, 1H), 1.78 - 1.71 (m, 1H), 1.68 - 1.54 (m., 2H), 1.37 (s, 3H), 1.27 (s, 3H), 1.12 (s, 3H).
**SFC:** (ee: 100%)
**HPLC:** (Purity: 99.73%)
**LCMS:** (M+H: 219.9)

### Spectra for 1-Ent2

**¹HNMR: (400 MHz, CDCl₃)** δ: 7.41 (s, 1H), 4.16 (dd, *J* = 5.6, 11.2 Hz, 1H), 3.55 - 3.46 (m, 1H), 3.25 (s, 1H), 2.12 - 1.99 (m, 1H), 1.78 - 1.71 (m, 1H), 1.68 - 1.54 (m., 2H), 1.37 (s, 3H), 1.27 (s, 3H), 1.12 (s, 3H).
**SFC:** (ee: 100%)
**HPLC:** (Purity: 100.00%)
**LCMS:** (M+H: 220.0)

### Example 2. Synthesis of Compounds 2-Ent1 and 2-Ent2

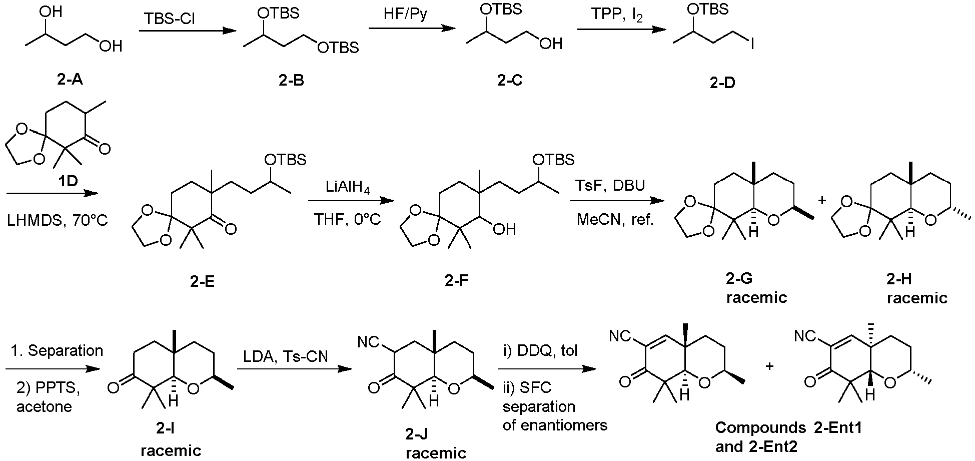

### Preparation of Compound 2-B

To a solution of butane-1,3-diol (**2-A,** 50 g, 0.55 mol, 1.0 eq) in DCM (1 L) was added imidazole (94 g, 1.38 mol, 2.5 eq.) and TBS-Cl (184 g, 1.22 mol, 2.2 eq) in portions. The white suspension was stirred at 25°C for 16 h. TLC (PE: EA = 30:1) indicated reaction completion. The reaction was quenched with sat. NaHCO₃ (250 mL) carefully and slowly under ice-water bath cooling. After separation, the organic layer was washed with brine (100 mL x 3), dried over Na₂SO₄. The mixture was filtered and concentrated and the residue was purified by column chromatography (PE: EA=50:1∼15:1) to give compound **2-B** (181 g, >100%) as colorless oil.
**¹HNMR: (400 MHz, CDCl₃)** δ: 3.96 (m, 1H), 3.74 - 3.61 (m, 2H), 1.71 - 1.60 (m, 2H), 1.14 (d, *J* = 5.9 Hz, 3H), 0.95 - 0.84 (m, 18H), 0.11 - 0.01 (m, 12H).

### Preparation of Compound 2-C

To a solution of compound **2-B** (55 g, 172.6 mmol, 1.0 eq) in HF/Py./THF solution (1120 mL, prepared by addition of 1 mL HF/Py. in 4 mL pyridine, then diluted with 10 mL THF). The reaction mixture was stirred at 25°C for 2 h. TLC (PE) indicated completion. The reaction mixture was poured into sat. NaHCO₃ (700 mL), cooled with ice-water bath carefully and slowly. After separation, the water layer was extracted with EA (400 mL). The combined organic layers of two batches were washed with brine (500 mL x 2), dried over Na₂SO₄. Filtered and concentrated without heating, the residue was re-dissolved with EA (500 mL), washed with 1 N HCl (500 mL x 3), brine (500 mL x 2), dried over Na₂SO₄. Filtered and concentrated to give a crude product as yellow oil, which was purified by column chromatography (PE: EA = 15:1∼12:1) to give compound **2-C** (33 g, 46.9%) as colorless oil.
**¹HNMR: (400 MHz, CDCl₃)** δ: 4.14 - 4.12 (m, 2H), 3.85 - 3.83(m, 1H), 3.75- 3.72m, 1H), 2.58 (t, *J* = 5.2 Hz, 1H), 1.80 - 1.77 (m, 1H), 1.65 - 1.61 (m, 1H), 1.21 (d, *J* = 6.2 Hz, 3H), 0.93 - 0.87 (m, 9H), 0.10 (d, *J* = 3.1 Hz, 6H).

### Preparation of Compound 2-D

To a solution of triphenylphosphine (39 g, 148.5 mmol, 1.1 eq) in DCM (680 mL) was added I₂ (37.7 g, 148.5 mmol, 1.1 eq.). The dark-red solution was stirred at 22°C for 30 min then imidazole (9.2 g, 135 mmol, 1.0 eq) was added in one portion. The yellow suspension was stirred for 5 min before a solution of compound **2-C** (27.6 g, 135 mmol, 1.0 eq) in DCM (50 mL) was added. After addition, the yellow suspension was stirred at 22°C for 1.5 h. TLC (PE: EA = 6:1) indicated completion. The reaction mixture was quenched with Sat. NaHCO₃ (500 mL). After separation, the organic layer was washed with brine (300 mL x 3), dried over Na₂SO₄. Filtered and concentrated, the residue was purified by column chromatography (PE: EA =80:1∼50:1) to give compound **2-D** (39.4 g, 92.9%) as colorless oil. **¹HNMR: (400 MHz, CDCl₃)** δ: 3.89 (qd, J = 6.0, 11.7 Hz, 1H), 3.28 - 3.18 (m, 2H), 2.00 - 1.87 (m, 2H), 1.16 (d, *J* = 6.3 Hz, 3H), 0.95 - 0.85 (m, 9H), 0.10 (d, *J* = 6.3 Hz, 6H).

### Preparation of Compound 2-E

To a solution of compound **1-D** (21.5 g, 108.2 mmol, 1.0 eq.) in THF (220 mL) at 22°C was added LiHMDS (141 mL, 141 mmol, 1.0 M in THF, 1.3 eq.). After stirring for 1 h, the mixture was treated with compound **2-D** (51 g, 162.3 mmol, 1.5 eq.) in THF (30 mL) drop-wise. The reaction mixture was heated to reflux for 8 hours. TLC (PE/EA = 10:1) showed the reaction was complete. The reaction was quenched with Sat. NH₄Cl (200 mL). Combined the crude product on page 82 and separated, the solvent (THF) was removed under reduced pressure and the water layer was extracted with EtOAc (200 mL). The combined organic layers were washed with brine (200 mL x 2), dried over Na₂SO₄, filtered and concentrated. The residue was purified by silica gel chromatography eluted with PE/EtOAc = 30:1∼20:1 to give compound **2-E** (36.5 g, 71.2% yield) as a light yellow oil.
**¹HNMR: (400 MHz, CDCl₃)** δ: 3.95 (m, 4H), 3.79 - 3.65 (m, 1H), 2.07 - 1.68 (m, 5H), 1.58 - 1.22 (m, 3H), 1.17 - 1.05 (m, 12H), 0.92 - 0.86 (m, 9H), 0.06 - 0.01 (s, 6H).

### Preparation of Compound 2-F

To a suspension of LiA1H₄ (3.9 g, 101.4 mmol, 1.5 eq.) in THF (600 mL) was added a solution of compound 2-E (26 g, 67.6 mmol, 1.0 eq.) in THF (80 mL) in portions at 0°C. The white suspension was then stirred at 25°C for 1 h. TLC (PE/EA = 6:1) indicated completion. Quenched with Na₂SO₄.10H₂O at 0 ∼ 5 °C in portions until there was no gas released. Filtered and concentrated, the residue was purified by silica gel chromatography eluted with PE/EtOAc = 30:1 ∼ 20:1 to give compound **2-F** (19 g, 72.8%) as colorless oil.
**¹HNMR: (400 MHz, CDCl₃)** δ: 4.05 - 3.87 (m, 4H), 3.81 - 3.66 (m, 1H), 3.33 - 3.17 (m, 1H), 2.04 (m, 1H), 1.91 - 1.67 (m, 2H), 1.62 - 1.56 (m, 3H), 1.53 - 1.23 (m, 5H), 1.16 - 1.10 (m, 3H), 1.07 - 1.02 (m, 3H), 1.01 - 0.96 (s, 3H), 0.91 - 0.85 (s, 9H), 0.05 (s, 6H).

### Preparation of Compounds 2-G and 2-H

To a solution of compound **2-F** (11.27 g, 29.1 mmol, 1.0 eq.) in MeCN (290 mL) were added TsF (6.1 g, 34.9 mmol, 1.2 eq.) and DBU (8.7 mL, 58.2 mmol, 2.0 eq.) at 25°C. The resulting clean solution was heated to reflux for 20 hours. TLC (PE/EA = 10:1) showed the reaction was complete. The solvent was removed under vacuum. The residue was purified by silica gel chromatography eluted with PE/EtOAc = 50:1 ∼ 30:1 to give crude compound **2-G** and pure compound **2-H,** both racemic.

### Spectra for 2-H:

**¹HNMR: (400 MHz, CDCl₃)** δ: 4.25 (m, 1H), 4.02 - 3.88 (m, 4H), 3.25 (s, 1H), 2.14 - 1.99 (m, 1H), 1.91 - 1.74 (m, 1H), 1.56 - 1.41 (m, 2H), 1.36 - 1.29 (m, 2H), 1.27 - 1.17 (m, 5H), 1.03 (s, 3H), 0.96 (s, 3H), 0.90 - 0.85 (m, 3H).

### Preparation of Compound 2-I

To a solution of crude, racemic compound **2-G** (2 g, 7.87 mmol, 1.0 eq.) in Acetone/H₂O (78 mL, v/v = 9:1) was added PPTS (0.8 g, 3.15 mmol, 0.4 eq.). The resulting yellow solution was heated to reflux for 15 hrs. TLC (PE: EA = 10:1) showed trace **2-G** still remained. The solvent acetone was removed under reduced pressure. The residue was diluted with water (40 mL) and extracted with EtOAc (2 x 40 mL). The combined organic layers were dried over Na₂SO₄, filtered and concentrated. The residue was purified by a flash column (PE: EA = 12:1) to give impure compound **2-I** (1.7 g, >100%)
**¹HNMR: (400 MHz, CDCl₃)** δ: 3.54 - 3.39 (m, 1H), 2.88 (s, 1H), 2.77 - 2.64 (m, 1H), 2.30 - 2.20 (m, 1H), 1.66 (dt, *J* = 2.0, 6.8 Hz, 1H), 1.57 - 1.44 (m, 3H), 1.38 - 1.29 (m, 2H), 1.22 (d, *J* = 6.2 Hz, 3H), 1.17 (s, 3H), 1.13 (s, 3H), 1.08 (s, 3H).

### Preparation of Compound 2-J

To a solution of compound **2-I** (1.7 g, 8.1 mmol, 1.0 eq.) in anhydrous THF (20 mL) was added LDA (2 M in THF/heptane, 5.3 mL, 10.5 mmol, 1.3 eq.) dropwise via syringe at - 78 °C. The resulting dark solution was stirred at -78 °C for 1 h before TsCN (1.6 g, 8.9 mmol, 1.1 eq.) in anhydrous THF (10 mL) was added. The resulting brown suspension was stirred at -70 °C for another 1 h. TLC (PE: EA = 10:1) indicated the starting material was remained. Quenched with Sat. NH₄Cl (20 mL). The solvent (THF) was removed under reduced pressure, and the water layer was extracted with EA (20 mL). The combined organic layer was dried over anhydrous sodium sulfate. Filtered and concentrated, the residue was purified by column chromatography (PE: EA = 15:1∼10:1) to give compound **2-J** (600 mg, 31.6%) as white solid.

### Preparation of Compounds 2-Ent1 and 2-Ent2

To a suspension of racemic compound **2-J** (0.6 g, 2.55 mmol, 1.0 eq.) in toluene (12 mL) was added DDQ (870 mg, 3.83 mmol, 1.5 eq.) in one portion. The resulting brown mixture was heated to reflux for 2 hours. The solvent was evaporated under vacuum. The residue was treated with DCM (20 mL) then filtered to remove the solid. The filtrate was concentrated. The residue was purified by column chromatography (PE: EA = 15:1) then p-TLC (PE: EA= 4:1) to give the racemic product (0.4 g, 67.3%) as brown oil. Separation of the enantiomers was achieved by SFC (column: AY 250 mm x 30 mm x 5 um, condition: Base-EtOH) to give compound **2-Entl** as the first eluting enantiomer (Rt = 1.194 min, 94.8 mg) and compound **2-Ent2** as the second eluting enantiomer (Rt = 1.648 min, 110.4 mg), both as white solid. The absolute configuration was not determined.

### Spectra for 2-Entl

**¹HNMR: (400 MHz, CDCl₃)** δ: 7.43 (s, 1H), 3.58 - 3.55 (m, 1H), 3.29 (s, 1H), 1.73 - 1.62 (m, 4H), 1.33 (s, 3H), 1.29 - 1.24 (m, 6H), 1.12 (s, 3H).
**SFC:** (ee: 99.8%)
**HPLC:** (Purity: 97.69%)
**LCMS:** (M+H: 234.1)

### Spectra for 2-Ent2

**¹HNMR: (400 MHz, CDCl₃)** δ: 7.43 (s, 1H), 3.57 (m, 1H), 3.29 (s, 1H), 1.71 - 1.61 (m, 4H), 1.33 (s, 3H), 1.29 - 1.25 (m, 6H), 1.12 (s, 3H).
**SFC:** (ee: 94.6%)
**HPLC:** (Purity: 97.73%)
**LCMS:** (M+H: 234.1)

### Example 3. Synthesis of Compounds 3-Ent1 and 3-Ent2

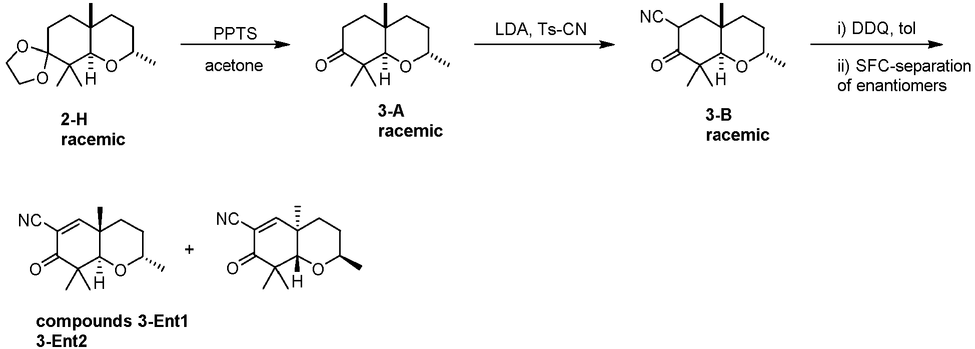

### Preparation of Compound 3-A

To a solution of racemic compound **2-H** (1.1 g, 4.33 mmol, 1.0 eq.) in acetone/H₂O (43 mL, v/v = 9:1), was added PPTS (0.43 g, 1.73 mmol, 0.4 eq.). The resulting clean solution was heated to reflux for 15 hrs. TLC (PE: EA = 10:1) indicated the completion. The solvent acetone was removed under reduce pressure then the residue was diluted with water (30 mL) and extracted with EtOAc (2 x 30 mL). The combined organic layers were dried over Na₂SO₄, filtered and concentrated. The residue was purified by a short column (PE: EA = 15:1) to give compound **3-A** (0.8 g, 88%) as colorless oil.
**¹HNMR: (400 MHz, CDCl₃)** δ: 4.31 (m, 1H), 3.17 (s, 1H), 2.72 (dt, *J* = 6.5, 15.0 Hz, 1H), 2.30 - 2.20 (m, 1H), 2.18 - 2.05 (m, 1H), 1.73 - 1.58 (m, 2H), 1.55 - 1.44 (m, 1H), 1.42 - 1.35 (m, 2H), 1.23 - 1.18 (m, 6H), 1.09 (s, 3H), 1.06 (s, 3H).

### Preparation of Compound 3-B

To a solution of compound **3-A** (0.8 g, 3.8 mmol, 1.0 eq.) in anhydrous THF (10 mL) was added LDA (2 M in THF/heptane, 2.5 mL, 4.9 mmol, 1.3 eq.) dropwise via syringe at - 78 °C. The clean solution was stirred at -78 °C for 1 h before TsCN (0.83 g, 4.6 mmol, 1.2 eq.) in anhydrous THF (5 mL) was added. The resulting colorless mixture was stirred at -70 °C for another 1 h. TLC (PE: EA = 10:1) indicated trace starting material remained. Quenched with Sat. NH₄Cl (10 mL). Diluted with H₂O (20 mL), EA (20 mL). After separation, the solvent was removed under reduce pressure then water layer was extracted with EA (30 mL). The combined organic layers were dried over anhydrous sodium sulfate. Filtered and concentrated, the residue was purified by column chromatography (PE: EA = 15:1∼10:1) to give compound **3-B** (0.5 g, 56%) as yellow solid.

**¹HNMR: (400 MHz, CDCl₃)** δ: 4.37 - 4.26 (m, 1H), 3.93 (dd, *J* = 5.3, 13.9 Hz, 1H), 3.16 (s, 1H), 2.17 - 2.03 (m, 2H), 1.71 (t, *J* = 13.7 Hz, 1H), 1.58 - 1.40 (m, 2H), 1.27 - 1.20 (m, 6H), 1.16 (s, 3H), 1.09 (s, 3H).

### Preparation of Compounds 3-Ent1 and 3-Ent2

To a suspension of racemic compound **3-B** (500 mg, 2.13 mmol, 1.0 eq.) in toluene (10 mL) was added DDQ (730 mg, 3.19 mmol, 1.5 eq.). The resulting dark-red mixture was heated to reflux for 2 hours. The reaction mixture was turned to a brown suspension. On cooling, the suspension was filtered and concentrated, the residue was purified by column chromatography (PE: EA = 12:1) to give the racemic product (0.32 g, 64.5%) as pink solid. Separation of enantiomers by SFC (column: AY 250 mm x 30 mm x10 um, condition: Neu-EtOH) gave **3-Entl** (Rt = 2.291 min, 126.7 mg) as the first eluting enantiomer and **3-Ent2** (Rt = 2.633 min, 104.0 mg) as the second eluting enantiomer, both as white solid. The absolute configuration was not determined.

### Spectra for 3-Entl

**¹HNMR: (400 MHz, CDCl₃)** δ: 7.41 (s, 1H), 4.35 (m, 1H), 3.59 (s, 1H), 2.32 - 2.15 (m, 1H), 1.79 (m, 1H), 1.58 - 1.51 (m, 1H), 1.41 (dd, *J* = 3.7, 13.9 Hz, 1H), 1.35 (s, 3H), 1.26 (d, *J* = 7.0 Hz, 3H), 1.23 (s, 3H), 1.10 (s, 3H). **SFC:** (ee: 100%)
**HPLC:** (Purity: 98.47%)
**LCMS:** (M+H: 234.1)

### Spectra for 3-Ent2

**¹HNMR: (400 MHz, CDCl₃)** δ: 7.41 (s, 1H), 4.34 (m, 1H), 3.59 (s, 1H), 2.33 - 2.16 (m, 1H), 1.79 (m, 1H), 1.57 - 1.51 (m, 1H), 1.41 (d, *J* = 14.5 Hz, 1H), 1.35 (s, 3H), 1.26 (d, *J* = 7.0 Hz, 3H), 1.23 (s, 3H), 1.10 (s, 3H).
**SFC:** (ee: 100%)
**HPLC:** (Purity: 98.36%)
**LCMS:** (M+H: 234.1)

### Example 4. Synthesis of Compounds 4-Ent1 and 4-Ent2

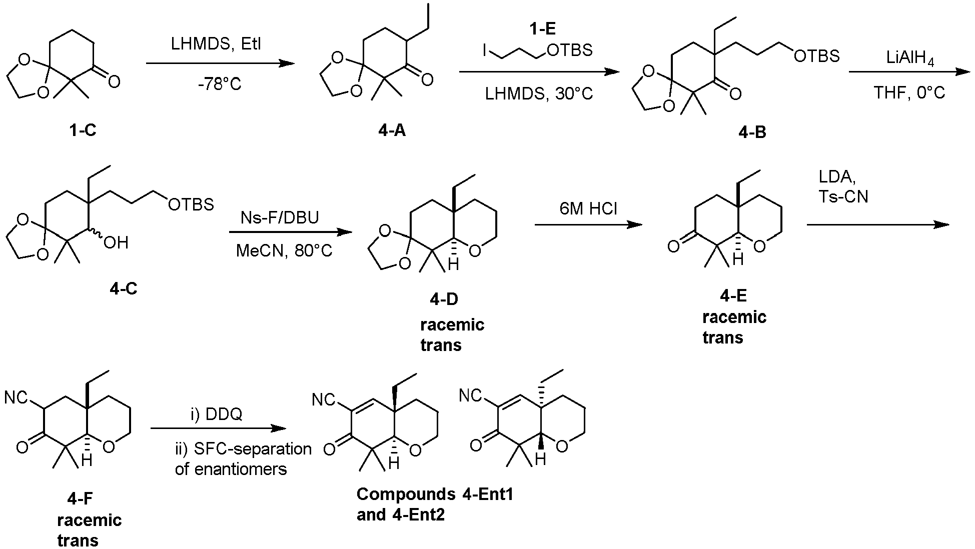

### Preparation of Compound 4-A

To a solution of 1 M of Lithium hexamethyldisilazide in Tetrahydrofuran (10.8 mL, 10.8 mmol) at -78°C was added [A] 6,6-dimethyl-1,4-dioxaspiro[4.5]decan-7-one **1-C** (2.0 g, 11 mmol) in 5 mL THF dropwise. The reaction was allowed to stir for 1h. The reaction was then treated with Iodoethane (2.20 g, 14.1 mmol) dropwise over 10 min and allowed to warm to RT overnight. The reaction was quenched with 50% saturated NH₄Cl solution and extracted with ethyl ether. The organic phases were combined and washed with brine, dried over MgSO₄, filtered, and concentrated. Purification by column chromatography using an 120g silica gel column eluted with 5-10% ethyl acetate in heptanes afforded compound **4-A** (1.3g, 56% yield).

### Spectra for 4-A:

**¹H NMR (400 MHz,** CH**LOROFORM-*d***) δ ppm 3.87 - 4.07 (m, 4 H) 2.46 (dq, *J*=12.6, 6.3 Hz, 1 H) 2.13 (td, *J*=13.6, 4.6 Hz, 1 H) 1.94 (dddd, *J*=13.2, 6.2, 4.6, 2.8 Hz, 1 H) 1.74 - 1.89 (m, 2 H) 1.36 (qd, *J*=13.3, 4.3 Hz, 1 H) 1.19 - 1.30 (m, 4 H) 1.05 (s, 3 H) 0.92 (t, *J*=7.5 Hz, 3 H).

### Preparation of Compound 4-B

To a solution of 8-ethyl-6,6-dimethyl-1,4-dioxaspiro[4.5]decan-7-one **4-A** (1.3 g, 6.0 mmol) in 30 mL THF at rt was added 1 M of Lithium hexamethyldisilazide in Tetrahydrofuran (7.9 mL, 7.9 mmol). After 1h, tert-butyl(3-iodopropoxy)dimethylsilane (**1-E,** 3.6 g, 12 mmol) was added. After 6h, the reaction was quenched with NH₄Cl solution and diluted with ether. The phases were separated and the organic phase was washed with brine, dried over MgSO₄, filtered, and concentrated. Purification by column chromatography (heptane/ethyl acetate) afforded 8-(3-((tert-butyldimethylsilyl)oxy)propyl)-8-ethyl-6,6-dimethyl-1,4-dioxaspiro[4.5]decan-7-one (**4-B,** 1.37 g; Yield = 59%).

### Spectra for 4-B:

**¹H NMR (400 MHz, CHLOROFORM-*d***) δ ppm 3.85 - 3.99 (m, 4 H) 3.52 (qt, *J*=9.9, 6.3 Hz, 2 H) 1.83 - 1.97 (m, 2 H) 1.64 - 1.72 (m, 2 H) 1.17-1.61 (m, 9 H) 1.09 (d, *J*=2.8 Hz, 5 H) 0.82 - 0.91 (m, 10 H) 0.71 (t, *J*=7.4 Hz, 3 H) 0.00 (d, *J*=1.8 Hz, 5 H).

### Preparation of Compound 4-C

To a solution of 8-(3-((tert-butyldimethylsilyl)oxy)propyl)-8-ethyl-6,6-dimethyl-1,4-dioxaspiro[4.5]decan-7-one (**4-B,** 1.35 g, 3.51 mmol) in 15 mL THF at 0°C was added 1 M of Lithium aluminum hydride in tetrahydrofuran(3.5 mL, 3.5 mmol) dropwise. The reaction was stirred at 0°C for 1.5h and then quenched by addition of saturated Rochelle's salt. The mixture was stirred for 2h and diluted with ethyl acetate. The organic phase was separated and washed with brine, dried over MgSO₄, filtered, and concentrated. Purification by column chromatography afforded 8-(3-((tert-butyldimethylsilyl)oxy)propyl)-8-ethyl-6,6-dimethyl-1,4-dioxaspiro[4.5]decan-7-ol (**4-C,** 800 mg; Yield = 60%) as a crude mixture of isomers.

### Preparation of Compound 4-D

To a reaction vessel containing 8-(3-((tert-butyldimethylsilyl)oxy)propyl)-8-ethyl-6,6-dimethyl-1,4-dioxaspiro[4.5]decan-7-ol (**4-C,** 800.0 mg, 2.069 mmol) in acetonitrile (10 mL) was added 4-nitrophenylsulfonyl fluoride (0.4670 g, 2.276 mmol) followed by 1,8-Diazabicyclo[5.4.0]undec-7-ene (0.3780 g, 2.483 mmol). The reaction was heated to reflux. After 6h, the solvent was removed under vacuum and the residue was partioned between ethyl acetate and ∼ 0.1N HCL. The layers were separated layers and the organic phase was washed with brine, dried over MgSo4, filtered, and concentrated. The reaction mixture was deposited on silica gel and purified by column chromatography eluted with 0-15% ethyl acetate in heptane to afford two major products. Two-dimensional ¹H-NMR nOe experiments identified the first eluting isomer as the trans-product **4-D.**

### Spectra for 4-D:

**¹H NMR (400 MHz, CHLOROFORM-*d***) δ ppm 4.03 - 4.12 (m, 1 H) 3.87 - 4.01 (m, 4 H) 3.41 - 3.53 (m, 1 H) 1.65 - 1.88 (m, 4 H) 1.56 - 1.61 (m, 1 H) 1.49 - 1.54 (m, 1 H) 1.40 (dt, *J*=13.8, 3.4 Hz, 1 H) 1.24 - 1.34 (m, 1 H) 1.09 (tdd, *J*=14.1, 14.1, 3.8, 1.8 Hz, 1 H) 0.95 - 1.04 (m, 1H), 0.99 (s, 3H), 0.93 (s, 3 H) 0.76 (t, *J*=7.5 Hz, 3 H).

### Preparation of Compound 4-E

Compound **4-D** (140 mg, 0.55 mmol) was dissolved in acetone (10 mL) and treated with p-toluenesulfonic acid monohydrate (0.010 g, 0.055 mmol). The reaction was heated to reflux for 8h then allowed to stir at RT overnight. The solvent was removed in vacuo and the reaction was diluted with 30 mL of ethyl acetate and 5 mL of sat sodium bicarbonate. The organic phase was separated and washed with brine. The organic phase was dried over MgSO₄, filtered, and concentrated to afford **4-E,** of approximately 93% purity by ¹HNMR. The compound was taken directly into the next step without further purification.

### Preparation of Compound 4-F

A solution of freshly prepared lithium diisopropylamide (LDA) was made as follows: 0.514 mL of N,N-diisopropylamine (0.370 g, 3.66 mmol) was taken up in 1.5 mL of THF and cooled to -78C. The solution was treated with 2.5 M of n-Butyllithium in hexanes(1.33 mL, 3.33 mmol) and warmed to 0°C for 30 minutes.

A solution of compound **4-E** in 2 mL of THF at -78°C was treated with 0.963 mL of the LDA solution prepared above. After 30 minutes, p-toluenesulfonyl cyanide (0.181 g, 0.998 mmol) in 0.5 mL THF was added. The reaction was allowed to stir for 1h then quenched by addition of 1.0 mL of 1N HCL and warmed to 0°C. The mixture was diluted with ethyl acetate and the layers were separated. The organic phase was washed with brine, dried over MgSO₄, filtered, and concentrated. Purification using 5-15% ethyl acetate in heptane afforded **4-F** (140 mg; Yield = 89%) as a white solid.

### Spectra for 4-F:

**¹H NMR (400 MHz, DMSO-d*₆*)** δ ppm 9.91 (s, 1 H) 3.97 - 4.07 (m, 1 H) 3.35 - 3.41 (m, 1 H) 3.33 - 3.33 (m, 1 H) 3.02 (s, 1 H) 1.95 - 2.06 (m, 1 H) 1.80 (m, 2 H) 1.48 - 1.65 (m, 2 H) 1.25 - 1.37 (m, 2 H) 1.14 (s, 3 H) 1.04 (s, 3 H) 0.99 (d, *J*=3.8 Hz, 1 H) 0.97 - 1.06 (m, 1 H) 0.97 (m, 1 H) 0.74 (t, *J*=7.5 Hz, 3 H).

### Preparation of Compounds 4-Ent1 and 4-Ent2

Compound **4-F** (120 mg, 0.51 mmol) was dissolved in 5 mL of benzene and treated with Dichlorodicyanoquinone (DDQ, 0.14 g, 0.61 mmol) at reflux for 2h. The reaction was cooled to RT and filtered and rinsed with benzene. Purification by column chromatography using 12g 30 µM silica gel cartridge afforded racemic compound 4-E. (54 mg; Yield = 44%; Purity = 97%; Supplier = BIOGEN). The racemic compound was purified by chiral SFC using a CHIRALPAK AD-H 30x250mm, 5um column eluted with 10% Methanol (w/o any modifier) in CO2 (flow rate: 100mL/min). The first eluting enantiomer was called compound **4-Entl** and the second eluting isomer was called compound **4-Ent2.** The absolute configuration was not determined.

### Spectra for 4-Entl

**¹HNMR: (400 MHz, DMSO-d*₆*)** δ ppm 8.07 (s, 1 H) 4.04 (dd, *J*=11.0, 5.5 Hz, 1 H) 3.44 (ddd, *J*=12.7, 11.2, 3.1 Hz, 1 H) 3.40 (s, 1 H) 2.17 (dq, *J*=14.1, 7.8 Hz, 1 H) 2.04 (br d, *J*=13.1 Hz, 1 H) 1.71 - 1.85 (m, 1 H) 1.33 - 1.56 (m, 3 H) 1.16 (s, 3 H) 1.05 (s, 3 H) 0.88 (t, *J*=7.5 Hz, 3 H)
**SFC:** (ee:100%)
**LCMS:** (M+H: 234)

### Spectra for 4-Ent2

**¹HNMR: (400 MHz, DMSO-d*₆*)** δ ppm 8.07 (s, 1 H) 4.04 (dd, *J*=11.0, 5.5 Hz, 1 H) 3.44 (ddd, *J*=12.7, 11.2, 3.1 Hz, 1 H) 3.40 (s, 1 H) 2.17 (dq, *J*=14.1, 7.8 Hz, 1 H) 2.04 (br d, *J*=13.1 Hz, 1 H) 1.71 - 1.85 (m, 1 H) 1.33 - 1.56 (m, 3 H) 1.16 (s, 3 H) 1.05 (s, 3 H) 0.88 (t, *J*=7.5 Hz, 3 H)
**SFC:** (ee: 98%)
**LCMS:** (M+H:234)

### Example 5. Synthesis of Compounds 5-Ent1 and 5-Ent2

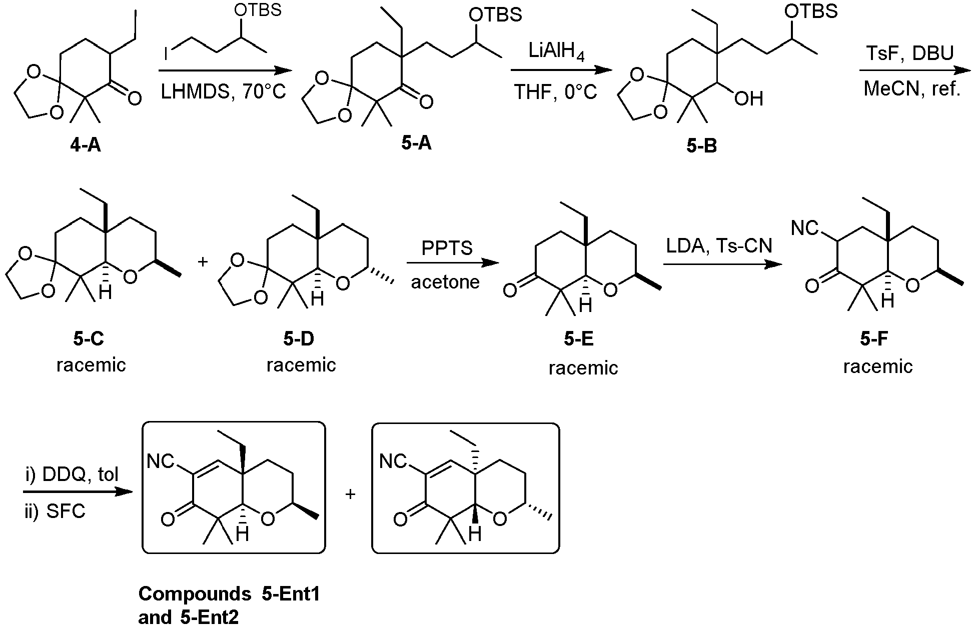

### Preparation of Compound 5-A

To a solution of compound 4-A (21.2 g, 100 mmol, 1.0 eq.) in THF (400 mL) was added LiHMDS (150 mL, 150 mmol, 1.0 M in THF, 1.5 eq.) at 18°C. After stirring for 1 h, the mixture was treated with tert-butyl((4-iodobutan-2-yl)oxy)dimethylsilane (Nagasawa, K.; Georgieva, A.; Takahashi, H.; Nakata, T. Tetrahedron, 2001, 57:8959) (180 mmol, 1.8 eq.) in THF (100 mL) dropwise. The resulting brown solution was heated to reflux for 8 hours. TLC (PE/EtOAc = 20:1) showed the reaction was complete. The reaction was quenched with Sat. NH₄Cl (200 mL). The solvent (THF) was removed under reduced pressure and the water layer was extracted with EtOAc (100 mL x 2). The combined organic layers were washed with water (200 mL x 3) and brine (200 mL x 3), dried over Na₂SO₄, filtered and concentrated. The residue was purified by silica gel chromatography eluted with PE/EtOAc = 90:1∼30:1 to give compound **5-A** as a light yellow oil.
**¹HNMR: (400 MHz, CDCl₃)** δ: 3.99 - 3.92 (m, 4H), 3.78 - 3.65 (m, 1H), 2.14 - 1.90 (m, 4H), 1.85 - 1.78 (m, 2H), 1.74 - 1.67 (m, 3H), 1.58 - 1.54 (m, 1H), 1.14 - 1.09 (m, 9H), 0.88 (s, 9H), 0.75 (m, 3H), 0.10 - -0.01 (m, 6H).

### Preparation of Compound 5-B

To a solution of compound **5-A** (24.8 g, 62.2 mmol, 1.0 eq.) in THF (620 mL) was added LiA1H₄ (4.7 g, 124.4 mmol, 2.0 eq.) in portions at 0°C under N₂ atmosphere. The gray suspension was then stirred at 0-5°C for 2 h. TLC (PE/ EtOAc = 10:1) indicated completion. The reaction was quenched with H₂O (4.7 mL) carefully and slowly, then treated with 15% NaOH aq. (4.7 mL), H₂O (15 mL). The white suspension was filtered and concentrated, the residue was purified by silica gel chromatography eluted with PE/EtOAc = 20:1 ∼ 10:1 to give compound **5-B**(20 g) as a yellow gum.
**¹HNMR: (400 MHz, CDCl₃)** δ: 3.95 (m., 4H), 3.69 - 3.62 (m, 1H), 3.32 (m, 1H), 1.79 - 1.67 (m, 2H), 1.50 - 1.40 (m, 5H), 1.18 - 1.11 (m, 3H), 1.09 - 1.03 (m, 9H), 0.91 - 0.84 (m, 12H), 0.05 (s, 6H).

### Preparation of Compounds 5-C and 5-D

To a solution of compound **5-B** (21.7 g, 54.2 mmol, 1.0 eq.) in MeCN (540 mL) were added TsF (11.3g, 65 mmol, 1.2 eq.) and DBU (16.5 g, 108.4 mmol, 2.0 eq.) at 25 °C. The resulting solution was heated to reflux for 20 hours. TLC (PE/EtOAc = 20:1) showed the reaction worked and five spots were observed. The solvent was removed under vacuum. The residue was purified by silica gel chromatography eluted with PE/EtOAc = 90:1 ∼ 30:1 to give compound **5-C** (2.87 g) and compound **5-D** (1.0 g, 6.9% yield) as yellow oil.

### Spectra for 5-C:

**¹H-NMR: (400 MHz, CDCl₃)** δ: 4.04 - 3.86 (m, 4H), 3.54 - 3.42 (m, 1H), 3.00 (s, 1H), 1.87 - 1.69 (m, 3H), 1.57 - 1.47 (m, 2H), 1.44 - 1.24 (m, 4H), 1.18 (d, *J* = 5.9 Hz, 3H), 1.11 - 1.04 (m, 1H), 1.00 - 0.96 (m, 3H), 0.93 (s, 3H), 0.74 (t, *J* = 7.4 Hz, 3H).

### Spectra for 5-D:

**¹H-NMR: (400 MHz, CDCl₃)** δ: 4.25 (m, 1H), 4.03 - 3.85 (m, 4H), 3.31 (s, 1H), 1.96 - 1.71 (m, 3H), 1.61 - 1.48 (m, 3H), 1.40 (m, 1H), 1.25 (d, *J* = 6.7 Hz, 3H), 1.19 - 1.04 (m, 3H), 0.97 (s, 3H), 0.88 (s, 3H), 0.75 (t, *J* = 7.4 Hz, 3H).

### Preparation of Compound 5-E

To a solution of compound **5-C** (2.87 g, 10.7 mmol, 1.0 eq.) in acetone/H₂O (54 mL, v/v = 9:1), was added PPTS (2.15 g, 8.56 mmol, 0.8 eq.). The resulting yellow solution was heated to reflux for 16 hrs. TLC (PE/ EtOAc = 10:1) indicated completion. The solvent acetone was removed under reduce pressure. The residue was diluted with water (50 mL) and extracted with EtOAc (3 x 50 mL). The combined organic layers were dried over Na₂SO₄, filtered and concentrated. The residue was purified by a short column (PE/ EtOAc = 50:1) to give compound **5-E** (2.2 g, 91.7%) as yellow oil.
**¹H-NMR: (400 MHz, CDCl₃)** δ: 3.51 - 3.44 (m, 1H), 2.98 (s, 1H), 2.60 (m, 1H), 2.29 - 2.19 (m, 1H), 1.90 - 1.78 (m, 3H), 1.71 - 1.60 (m, 1H), 1.46 - 1.34 (m, 2H), 1.22 (d, *J* = 6.3 Hz, 3H), 1.14 (s, 3H), 1.12 - 1.06 (m, 4H), 1.00 (m, 1H), 0.82 (t, *J* = 7.4 Hz, 3H).

### Preparation of Compound 5-F

To a yellow solution of compound **5-E** (1.53 g, 7.13 mmol, 1.0 eq.) in anhydrous THF (18 mL) was added LDA (2 M in THF/heptane, 4.6 mL, 9.27 mmol, 1.3 eq.) dropwise via syringe at -78 °C. The resulting dark solution was stirred at -78 °C for 1 h before TsCN (1.42 g, 7.84 mmol, 1.1 eq.) in anhydrous THF (20 mL) was added. The resulting brown suspension was stirred at -70 °C for another 1 h. TLC (PE/ EtOAc = 20:1) indicated completion. The reaction was quenched with Sat. NH₄Cl (50 mL). The solvent (THF) was removed under reduce pressure, and water layer was extracted with EtOAc (50 mL x 2). The combined organic layers were dried over anhydrous sodium sulfate. Filtered and concentrated, the residue was purified by column chromatography (PE/EtOAc = 20:1 ∼ 10:1) to give compound **5-F** (330 mg, 19.4%) as white solid.
**¹HNMR: (400 MHz, CDCl₃)** δ: 3.85 (dd, *J* = 5.3, 13.9 Hz, 1H), 3.58 - 3.40 (m, 1H), 2.96 (s, 1H), 2.28 (dd, *J* = 5.3, 13.9 Hz, 1H), 2.03 - 1.88 (m, 2H), 1.53 - 1.36 (m, 3H), 1.24 - 1.20 (m, 6H), 1.15 - 1.12 (m, 3H), 0.88 (t, *J* = 7.4 Hz, 3H).

### Preparation of Compounds 5-Ent1 and 5-Ent2

To a solution of compound **5-F** (330 mg, 1.32 mmol, 1.0 eq.) in toluene (13 mL) was added DDQ (449 mg, 1.98 mmol, 1.5 eq.) in one portion. The resulting red suspension was heated to reflux for 2 hours. TLC (PE/ EtOAc = 10:1) indicated completion. The solvent was evaporated under vacuum. The residue was purified by column chromatography (PE/EtOAc = 12:1∼10:1) then p-TLC (PE/ EtOAc = 6:1) to give the final product (0.19 g, 59.3%) as yellow oil. The racemic compound was separated by SFC (Mobile phase: Supercritical CO₂/EtOH; Column: Chiralpak AY 250*30mm*10 um; Detection wavelength: 220 nm) to give Compound **5-Entl** (Rt = 1.1873 min) and Compound **5-Ent2** (Rt = 2.127 min) both as yellow solids. Compound **5-Ent2** was further purified by Preparatory TLC (PE/ EtOAc = 6:1). The absolute configuration was not determined.

### Spectra for 5-Entl

**¹HNMR: (400 MHz, CDCl₃)** δ: 7.52 (s, 1H), 3.66 - 3.49 (m, 1H), 3.33 (s, 1H), 2.36 - 2.21 (m, 1H), 2.09 (d, *J* = 12.9 Hz, 1H), 1.60 - 1.47 (m, 3H), 1.45 - 1.34 (m, 1H), 1.31 - 1.22 (m, 6H), 1.17 (s, 3H), 0.93 (t, *J* = 7.6 Hz, 3H).
**SFC:** (ee: 99.94%)
**HPLC:** (Purity: 96.82%)
**LCMS:** (M+H: 248.1)

### Spectra for 5-Ent2

**¹HNMR: (400 MHz, CDCl₃)** δ: 7.51 (s, 1H), 3.65 - 3.48 (m, 1H), 3.34 (s, 1H), 2.35 - 2.20 (m, 1H), 2.07 (d, *J* = 12.9 Hz, 1H), 1.65 - 1.47 (m, 3H), 1.45 - 1.35 (m, 1H), 1.31 - 1.23 (m, 6H), 1.17 (s, 3H), 0.93 (t, *J* = 7.6 Hz, 3H).
**SFC:** (ee: 100%)
**HPLC:** (Purity: 95.79%)
**MS:** (M+H: 248.1)

### Example 6. Synthesis of Compounds 6-Ent1 and 6-Ent2

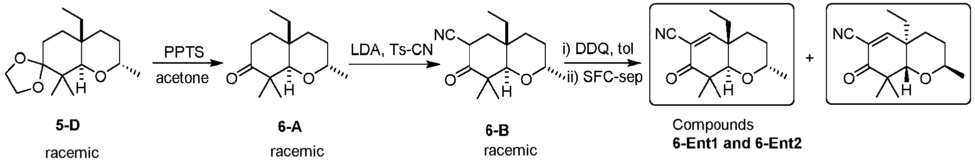

### Preparation of Compound 6-A

To a solution of compound **5-D** (1 g, 3.73 mmol, 1.0 eq.) in Acetone/H₂O (19 mL, v/v = 9:1), was added PPTS (0.75 g, 1.73 mmol, 0.8 eq.). The resulting clear solution was heated to reflux for 16 hrs. TLC (PE/EtOAc = 20:1) indicated completion. Water (30 mL) was added and the solvent acetone was removed under reduced pressure. The residue was extracted with EtOAc (20 mL x 3). The combined organic layers were dried over Na₂SO₄, filtered and concentrated. The residue was purified by a short column (PE/EA = 50:1) to give compound **6-A** (0.79 g, 94.6%) as yellow oil.
**¹HNMR: (400 MHz, CDCl₃)** δ: 4.31 (m, 1H), 3.28 (s, 1H), 2.69 - 2.54 (m, 1H), 2.28 - 2.19 (m, 1H), 2.03 - 1.92 (m, 2H), 1.83 (m, 1H), 1.76 - 1.67 (m, 1H), 1.66 - 1.61 (m, 1H), 1.28 - 1.21 (m, 5H), 1.19 - 1.14 (m, 1H), 1.09 (d, *J* = 12.1 Hz, 6H), 0.84 (t, *J* = 7.4 Hz, 3H).

### Preparation of Compound 6-B

To a solution of compound **6-A** (0.56 g, 2.5 mmol, 1.0 eq.) in anhydrous THF (6.3 mL) was added LDA (2 M in THF/heptane, 2.5 mL, 5 mmol, 2.0 eq.) dropwise via syringe at -78 °C. The yellow solution was stirred at -78 °C for 1 h before TsCN (0.91 g, 5 mmol, 2.0 eq.) in anhydrous THF (10 mL) was added via syringe. The resulting brown mixture was stirred at -78 °C for another 1 h. TLC (PE/EtOAc = 20:1) indicated trace starting material remaining. The reaction mixture was quenched with Sat. NH₄Cl (50 mL). The solvent was removed under reduce pressure then the water layer was extracted with EA (30 mL x 2).

The combined organic layers were dried over anhydrous sodium sulfate. Filtered and concentrated, the residue was purified by column chromatography (PE/EtOAc = 20:1∼10:1) to give compound **6-B** (0.37 g, 59.4%) as yellow solid.

### Preparation of Compounds 6-Ent1 and 6-Ent2

To a suspension of compound 6-A (370 mg, 1.48 mmol, 1.0 eq.) in toluene (15 mL) was added DDQ (504 mg, 2.22 mmol, 1.5 eq.). The resulting red mixture was heated to reflux for 2 hours. TLC (PE/EtOAc = 6:1) indicated completion. On cooling, the solvent was removed under reduce pressure and the residue was purified by column chromatography (PE/EtOAc = 12:1) to give a yellow solid (246 mg, 67.4%). Chiral separation by SFC (column: AY 250 mm*30 mm*10 um, condition: Neu-EtOH) afforded **6-Entl** (Rt = 2.023 min, 107.4 mg) and **6-Ent2** (Rt = 2.521 min, 88.3 mg) both as yellow solid.

### Spectra for 6-Entl

**¹HNMR: (400 MHz, CDCl₃)** δ: 7.51 (s, 1H), 4.42 - 4.26 (m, 1H), 3.63 (s, 1H), 2.45 - 2.29 (m, 1H), 2.19 - 2.05 (m, 1H), 1.99 - 1.89 (m, 1H), 1.61 - 1.48 (m, 2H), 1.38 (d, *J* = 13.7 Hz, 1H), 1.27 (d, *J* = 6.8 Hz, 3H), 1.23 (s, 3H), 1.15 (s, 3H), 0.94 (t, *J* = 7.6 Hz, 3H).
**SFC:** (ee: 100%)
**HPLC:** (Purity: 97.43%)
**MS:** (M+H: 248.1)

### Spectra for 6-Ent2

**¹HNMR: (400 MHz, CDCl₃)** δ: 7.51 (s, 1H), 4.33 (m, 1H), 3.63 (m, 1H), 2.44 - 2.30 (m, 1H), 2.19 - 2.03 (m, 1H), 1.97 - 1.89 (m, 1H), 1.63 - 1.46 (m, 2H), 1.42 - 1.34 (m, 1H), 1.27 (d, *J* = 6.8 Hz, 3H), 1.23 (s, 3H), 1.15 (s, 3H), 0.94 (t, *J* = 7.6 Hz, 3H).
**SFC:** (ee: 99.64%)
**HPLC:** (Purity: 98.09%)
**MS:** (M+H: 248.1)

### Example 7. Synthesis of Compounds 7-Ent1 and 7-Ent2

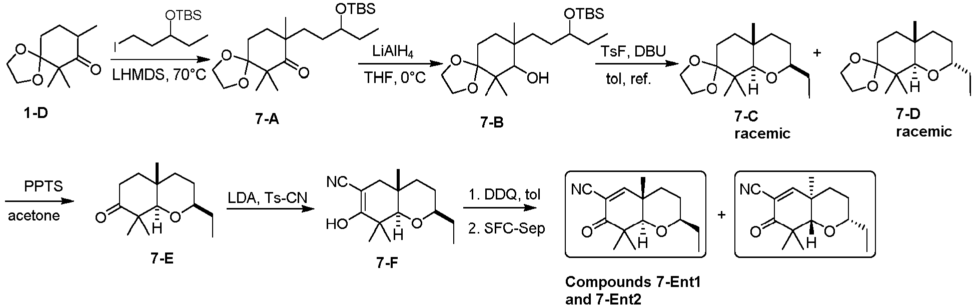

### Preparation of Compound 7-A

To a solution of compound **1-D** (19.8 g, 0.1 mol, 1.0 eq.) in THF (200 mL) was added LiHMDS (150 mL, 150 mmol, 1.0 M in THF, 1.5 eq.) at 24 °C. After stirring for 1 h, the mixture was treated with tert-butyl((1-iodopentan-3-yl)oxy)dimethylsilane (Bollbuck, B.; Tochtermann, W. Tetrahedron 1999, 55, 7209-7220) (42 g, 0.13 mol, 1.3 eq.) in THF (50 mL) drop-wise. The mixture was heated to reflux for 16 hours. The mixture turned to clear. TLC (PE/EA = 10:1) showed the reaction was complete. Most of the starting material was consumed (R_{f} = 0.5) and a new main spot was detected (R_{f} = 0.6). The reaction mixture was quenched with water (100 mL) and extracted with EA (100 mL x 3). The combined organic phase was dried over Na₂SO₄, and filtered. The filtrate was concentrated *in vacuo* to give the residue, which was purified by silica gel chromatography (eluting with PE/EtOAc = 50:1) to give compound **7-A** (32 g, 80.4% Yield) as a yellow oil.
**¹H NMR (400MHz, CDCl₃)** δ: 3.92 (m, 4H), 3.50 - 3.48 (m, 1H), 2.06 - 1.58 (m, 5H), 1.50 - 1.19 (m, 5H), 1.14 - 0.98 (m, 9H), 0.85 (m, 12H), 0.00 (s, 6H).

### Preparation of Compound 7-B

To a suspension of LiAlH₄ (4.8 g, 0.13 mol, 1.8 eq.) in THF (500 mL) was added a solution of compound **7-A** (28 g, 0.07 mol, 1.0 eq.) in THF (100 mL) in portions at 0°C. The white suspension was then stirred at 24°C for 1 h. TLC (PE/EA = 5:1) indicated the completion. Most of the starting material was almost consumed (R_{f} = 0.7) and a new spot was detected (R_{f} = 0.6). The mixture was quenched with water (4.8 mL), aq.NaOH (15%, 4.8 mL) and water (14.4 mL). The mixture was filtered and the filtrate was concentrated *in vacuo,* the residue was purified by silica gel chromatography (eluting with PE/EtOAc = 5:1) to give compound **7-B** (27 g, 84%) as a colorless oil.
**¹H NMR (400MHz,CDCl₃)** δ: 3.91 (m, 4H), 3.56 - 3.43 (m, 1H), 3.28 - 3.16 (m, 1H), 1.65 - 1.50 (m, 3H), 1.49 - 1.15 (m, 7H), 1.10 - 0.92 (m, 9H), 0.91 - 0.75 (m, 12H), 0.00 (s, 6H).

### Preparation of Compounds 7-C and 7-D

To a solution of compound **7-B** (15 g, 0.037 mol, 1.0 eq.) and DBU (22.5 g, 0.148 mol, 4.0 eq.) in toluene (150 mL) was added TsF (13.05 g, 0.075 mol, 2.0 eq.). The mixture was stirred at 130 °C for 16 hours. TLC (PE/EA = 20:1) showed the starting material still remained and several spots were detected. To the mixture was added TsF (13.05 g, 0.075 mol, 2.0 eq.) again, and stirred at 130 °C for another 16 hours. TLC (PE/EA = 20:1) showed the reaction was complete. The solvent was removed under vacuum. The residue was purified by silica gel chromatography eluted with PE/EtOAc = 100/1 to give compound **7-C** (2.78 g, impure) and compound **7-D** (0.8 g, impure) as colorless oils.

### Spectra for Compound 7-C

**¹HNMR: (400 MHz, CDCl₃)** δ: 4.08 - 3.88 (m, 4H), 3.23 - 3.12 (m, 1H), 2.88 (s, 1H), 1.89 - 1.77 (m, 1H), 1.55 - 1.34 (m, 6H), 1.33 - 1.20 (m, 3H), 0.96 - 0.89 (m, 12H).

### Spectra for Compound 7-D

**¹HNMR: (400 MHz, CDCl₃)** δ: 3.95 - 3.91 (m, 4H), 3.88 - 3.82 (m, 1H), 3.16 (s, 1H), 2.07 - 1.98 (m, 1H), 1.93 - 1.79 (m, 2H), 1.47 - 1.16 (m, 7H), 1.09 - 0.84 (m, 12H).

### Preparation of Compound 7-E

To a solution of compound **7-C** (3.78 g, 14 mmol, 1.0 eq.) in Acetone/H₂O (60 mL, v/v = 9:1), was added PPTS (1.7 g, 7 mmol, 0.5 eq.). The resulting yellow solution was heated to 65°C and stirred for 15 hrs. TLC (PE: EA = 20:1) showed the reaction was completed. Most of the starting material was almost consumed (Rf = 0.5) and a new spot was detected (Rf = 0.4). The organic solvent was removed off under reduced pressure. The aqueous was diluted with water (10 mL) and extracted with EtOAc (25 mL x 3). The combined organic layers were dried over Na₂SO₄, filtered and concentrated. The residue was purified by column chromatography on silica gel (PE: EA = 100:1) to give compound **7-E** (1.88 g, 60%) as colorless oil.
**¹HNMR: (400 MHz, CDCl₃)** δ: 3.17 - 3.03 (m, 1H), 2.78 (s, 1H), 2.70 - 2.58 (m, 1H), 2.21 - 2.13 (m, 1H), 1.59 (m, 1H), 1.53 - 1.33 (m, 5H), 1.31 - 1.19 (m, 2H), 1.17 - 0.99 (m, 9H), 0.89 (t, *J* = 7.6 Hz, 3H).

### Preparation of Compound 7-F

To a solution of compound **7-E**(1.78 g, 7.9 mmol, 1.0 eq.) in anhydrous THF (20 mL) was added LDA (2 M in THF/heptane, 7.9 mL, 15.8 mmol, 2.0 eq.) dropwise via syringe at - 78 °C. The resulting dark solution was stirred at -78 °C for 1 h before TsCN (2.88 g, 15.8 mmol, 2.0 eq.) in anhydrous THF (5 mL) was added. The resulting brown suspension was stirred at -78 °C for another 1 h. TLC (PE: EA = 10:1) indicated the reaction was completed. Most of the starting material was almost consumed (R_{f} = 0.6) and a new spot was detected (R_{f} = 0.2). The mixture was quenched with Sat. NH₄Cl (10 mL) and extracted with EA (20 mL x 3). The combined organic layers were dried over anhydrous sodium sulfate. Filtered and concentrated, the residue was purified by column chromatography (eluted with PE: EA = 15:1∼10:1) to give compound **7-F** (940 mg, 47.3%) as colorless oil.
**LCMS:** (M+H: 250.0).

### Preparation of Compounds 7-Ent1 and 7-Ent2

To a suspension of compound **7-F** (940 mg, 3.78 mmol, 1.0 eq.) in toluene (10 mL) was added DDQ (1.28 g, 5.67 mmol, 1.5 eq.) in one portion. The resulting brown mixture was heated to reflux for 2 hours. TLC showed the starting material was almost consumed (Rf = 0.5) and a new spot was detected (R_{f} = 0.55). The mixture was concentrated *in vacuo* to give the residue, which was purified by column chromatography (PE: EA = 30:1 to 15:1) to give racemic product (495 mg, 53%). The racemic mixture was further separated by SFC (column: C2 250mm*30mm, 10 um, condition: Base-IPA) to give **7-Entl** (19242-114-1, Rt = 3.227 min, 173.6 mg) and **7-Ent2** (19242-114-2, Rt = 3.486 min, 162.6 mg) both as pale yellow solid.

### Spectra for 7-Entl

**¹HNMR: (400 MHz, CDCl₃)** δ: 7.40 (s, 1H), 3.31 - 3.29 (m, 1H), 3.25 (s, 1H), 1.72 - 1.55 (m, 5H), 1.52 - 1.43 (m, 1H), 1.29 (s, 3H), 1.24 (s, 3H), 1.10 (s, 3H), 0.95 (t, *J* = 7.6 Hz, 3H).
**SFC:** (ee: 99.6%)
**HPLC:** (Purity: 100%)
**LCMS:** (M+H: 248.2)

### Preparation of Compound 8-B

To a solution of compound **8-A** (850 mg, 3.8 mmol, 1.0 eq.) in anhydrous THF (10 mL) was added LDA (2 M in THF, 3.8 mL, 7.6 mmol, 2.0 eq.) dropwise via syringe at -78 °C. The resulting dark solution was stirred at -78 °C for 1 h before TsCN (1.37 g, 7.6 mmol, 2.0 eq.) in anhydrous THF (5 mL) was added. The resulting brown suspension was stirred at -78 °C for another 1 h. TLC (PE: EA = 15:1) indicated the reaction was complete. Most of the starting material was almost consumed (R_{f} = 0.6) and a new spot was detected (R_{f} = 0.2). The mixture was quenched with Sat. NH₄Cl (20 mL) and extracted with Ethyl Acetate (20 mL x 3). The combined organic layers were dried over anhydrous sodium sulfate. Filtered and concentrated, the residue was purified by column chromatography (PE: EA = 5:1) to give compound **8-B** (610 mg, 64.5%) as white solid.
**LCMS:** (M+H: 250.0)

### Preparation of Compounds 8-Ent1 and 8-Ent2

To a suspension of compound **8-B** (610 mg, 2.4 mmol, 1.0 eq.) in toluene (10 mL) was added DDQ (834 mg, 3.7 mmol, 1.5 eq.) in one portion. The mixture was stirred at 130 °C for 2 hours. TLC (PE: EA = 5:1) showed the starting material was almost consumed (R_{f} = 0.5) and a new spot was detected (R_{f} = 0.55). The mixture was concentrated *in vacuo* to give the residue, which was purified by column chromatography (eluting with PE: EA = 5:1) to give a racemic mixture of **8-Entl and 8-Ent2** (310 mg, 52.3%). The racemic mixture was further separated by SFC (AY 250 mm x 30 mm, 10 um, condition: Base-IPA) to give **8-Entl** (Rt = 2.543 min, 116.6 mg) and **8-Ent2** (Rt = 2.725 min, 117.7 mg) both as pale yellow solid.

### Spectra for 8-Entl

**¹HNMR: (400 MHz, CDCl₃)** δ: 7.40 (s, 1H), 3.97 - 3.92 (m, 1H), 3.53 (s, 1H), 2.25 - 2.14 (m, 1H), 1.90 - 1.80 (m, 1H), 1.77 - 1.68 (m, 1H), 1.54 - 1.39 (m, 3H), 1.36 (s, 3H), 1.26 (s, 3H), 1.11 (s, 3H), 0.93 (t, *J* = 7.4 Hz, 3H).
**SFC:** (ee: 99.9%)
**HPLC:** (Purity: 98.7%)
**LCMS:** (M+H: 248.1)

### Spectra for 8-Ent2

**¹HNMR: (400 MHz, CDCl₃)** δ: 7.38 (s, 1H), 3.96 - 3.89 (m, 1H), 3.51 (s, 1H), 2.23 - 2.13 (m, 1H), 1.88 - 1.78 (m, 1H), 1.76 - 1.67 (m, 1H), 1.52 - 1.36 (m, 3H), 1.34 (s, 3H), 1.24 (s, 3H), 1.09 (s, 3H), 0.91 (t, *J* = 7.2 Hz, 3H).
**SFC:** (ee: 99.3%)
**HPLC:** (Purity: 100%)
**LCMS:** (M+H: 248.1)

### Example 9. Synthesis of Compounds 9-Ent1 and 9-Ent2

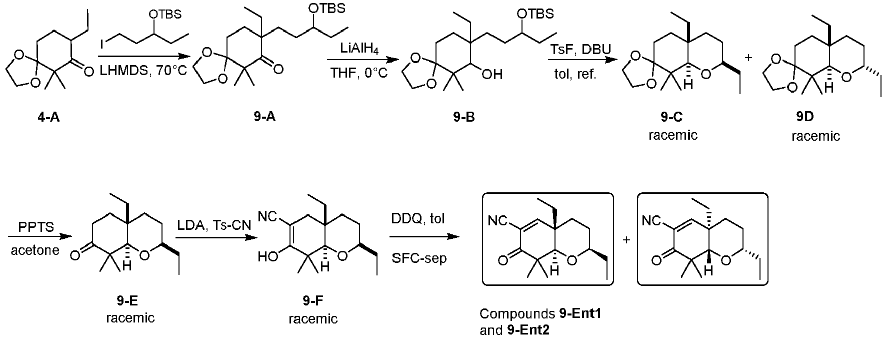

### Preparation of Compound 9-A

To a solution of compound **4-A** in THF (400 mL) was added LiHMDS (150 mL, 150 mmol, 1.0 M in THF, 1.5 eq.) at 18°C. After stirring for 1 h, the mixture was treated with tert-butyl((1-iodopentan-3-yl)oxy)dimethylsilane (Bollbuck, B.; Tochtermann, W. Tetrahedron 1999, 55, 7209-7220) (48.6 g, 150 mmol, 1.5 eq.) in THF (50 mL) dropwise. The resulting brown solution was heated to reflux for 16 hours. TLC (PE/EtOAc = 20:1) showed the reaction was complete. The reaction was quenched with Sat. NH₄Cl (200 mL). The solvent (THF) was removed under reduce pressure and the water layer was extracted with EtOAc (100 mL x 2). The combined organic layers were washed with water (200 mL x 2), brine (200 mL x 3), dried over Na₂SO₄, filtered and concentrated. The residue was purified by silica gel chromatography eluted with PE/EtOAc = 100:1∼30:1 to give compound **9-A** (34 g, 82.5%) as a yellow oil.
**¹HNMR: (400 MHz, CDCl₃)** δ: 3.99- 3.91 (m, 4H), 3.53 - 3.50 (m, 1H), 1.92 - 1.90 (m, 2H), 1.76 - 1.68 (m, 2H), 1.63 - 1.50 (m, 3H), 1.48 - 1.36 (m, 3H), 1.18 - 1.08 (m, 6H), 0.92 - 0.81 (m, 14H), 0.75 (t, *J* = 7.4 Hz, 3H), 0.11 - 0.03 (m, 6H).

### Preparation of Compound 9-B

To a solution of compound **9-A** (34 g, 82.4 mmol, 1.0 eq.) in THF (500 mL) was added LiAlH₄ (6.3 g, 164.8 mmol, 2.0 eq.) in portions at 0∼10 °C under N₂ atmosphere. The white suspension was then stirred at 10°C for 2 h. TLC (PE/ EtOAc = 20:1) indicated completion. The reaction was quenched with H₂O (6.3 mL) carefully and slowly, then treated with 15% NaOH aq. (6.3 mL), H₂O (12.4 mL). The white suspension was filtered and concentrated, the residue was purified by silica gel chromatography eluted with PE/EtOAc = 30:1 ∼ 10:1 to give compound **9-B** (29 g, 84.8) as colorless oil.
**¹HNMR: (400 MHz, CDCl₃)** δ: 3.94- 3.90 (m, 4H), 3.55 - 3.52 (m, 1H), 3.36 -3.30 (m, 1H), 1.67 - 1.56 (m, 3H), 1.45 - 1.38 (m, 9H), 1.07 - 1.04 (m, 6H), 0.89 - 0.85 (m, 16H), 0.04 (s, 6H).

### Preparation of Compounds 9-C and 9-D

To a solution of compound **9-B** (29 g, 69.9 mmol, 1.0 eq.) in toluene (175 mL) were added TsF (24.3 g, 139.8 mmol, 2 eq.) and DBU (21.2 g, 139.8 mmol, 2.0 eq.) at 25 °C. The resulting clean solution was heated to reflux for 16 hours. TLC (PE/EtOAc = 20:1) showed the reaction worked and five spots were observed. The solvent was removed under vacuum. The residue was purified by silica gel chromatography eluted with PE/EtOAc = 100:1 to give compound **9-C** (8.6 g, not pure, mixed with PPTS or its derivation) and compound **9-D** (1.6 g, 8.1% yield) as colorless oil.

### Spectra for 9-C:

**¹HNMR: (400 MHz, CDCl₃)** δ: 4.09 - 3.81 (m, 4H), 3.31 - 3.16 (m, 1H), 2.98 (s, 1H), 1.89 - 1.67 (m, 3H), 1.57 - 1.28 (m, 7H), 1.11 - 0.91 (m, 11H), 0.74 (t, *J* = 7.6 Hz, 3H).

### Spectra for 9-D:

**¹HNMR: (400 MHz, CDCl₃)** δ: 3.98 - 3.90 (m, 4H), 3.89 - 3.82 (m, 1H), 3.26 (s, 1H), 1.88 - 1.78 (m, 4H), 1.58 - 1.54 (m, 3H), 1.43 - 1.34 (m, 2H), 1.28 - 1.19 (m, 1H), 1.17 - 1.04 (m, 2H), 1.00 (s, 3H), 0.95 - 0.89 (m, 6H), 0.76 (t, *J* = 7.5 Hz, 3H).

### Preparation of Compound 9-E

To a solution of compound **9-C** (8.6 g, 30.4 mmol, 1.0 eq.) in Acetone/H₂O (80 mL, v/v = 9:1), was added PPTS (2 g, 8 mmol, 0.26 eq.). The resulting yellow solution was heated to reflux for 16 hrs. TLC (PE/ EtOAc = 20:1) indicated the completion. The solvent acetone was removed under reduce pressure. The residue was diluted with water (50 mL) and extracted with EtOAc (2 x 50 mL). The combined organic layers were dried over Na₂SO₄, filtered and concentrated to give a mixture (7.2 g). PE (20 mL) was added then stirred at -40°C vigorously. The suspension was filtered, the filtrate was concentrated to give a crude product of compound **9-E** (3 g) as a yellow oil.
**¹HNMR: (400 MHz, CDCl₃)** δ: 3.26 - 3.24 (m, 1H), 2.96 (s, 1H), 2.60 (m, 1H), 2.25 - 2.24 (m, 1H), 1.88 - 1.85 (m, 3H), 1.60 - 1.47 (m, 3H), 1.40 - 1.39 (m, 2H), 1.18 - 1.07 (m, 7H), 1.05 - 0.92 (m, 4H), 0.82 (t, *J* = 7.5 Hz, 3H).

### Preparation of Compound 9-F

To a yellow solution of compound **9-E** (3 g, 12.6 mmol, 1.0 eq.) in anhydrous THF (63 mL) was added LDA (2 M in THF/heptane, 9.5 mL, 18.9 mmol, 1.5 eq.) dropwise via syringe at -78 °C. The resulting dark solution was stirred at -78 °C for 1 h before TsCN (3.4 g, 18.9 mmol, 1.5 eq.) in anhydrous THF (20 mL) was added. The resulting brown suspension was stirred at -70 °C for another 1 h. TLC (PE/ EtOAc = 20:1) indicated completion. The reaction was quenched with Sat. NH₄Cl (100 mL). The solvent (THF) was removed under reduce pressure, and water layer was extracted with EtOAc (100 mL x 2). The combined organic layers were washed with water (100 mL x 2), brine (80 mL x 2), dried over anhydrous sodium sulfate. Filtered and concentrated, the residue was purified by column chromatography (PE/EtOAc = 15:1 ∼ 6:1) to give compound **9-F** (2 g, 60%) as yellow gum.
**LCMS:** (M+H: 263.9)

### Preparation of Compounds 9-Ent1 and 9-Ent2

A mixture of compound **9-F** (2 g, 7.6 mmol, 1.0 eq.) and DDQ (3.5 g, 15.2 mmol, 2.0 eq.) in toluene (40 mL) was heated to 50°C for 2 hours. TLC (PE/ EtOAc = 6:1) indicated completion. The solvent was evaporated under vacuum. The residue was purified by column chromatography (PE/EtOAc = 20:1) twice to give the final product (0.8 g, 40.2%) as yellow solid. Further separated by SFC (Mobile phase: Supercritical CO₂/MeOH; Column: Chiralpak AD 250 x 30 mm x 5 um; Detection wavelength: 220 nm) to give Compound **9-Ent1** (Rt = 1.337 min, 313.9 mg, 39.2%) and Compound **9-Ent2** (Rt = 1.555 min, 350 mg) both as yellow solid. The compound of **9-Ent2** was further purified by SFC (Mobile phase: Supercritical CO₂/MeOH; Column: Chiralpak AD 250 x 30 mm x 5 um; Detection wavelength: 220 nm) to give **9-Ent2** (Rt = 1.555 min, 211.3 mg, 10.6%) as yellow solid.

### Spectra for 9-Entl

1HNMR: (400 MHz, CDCl₃) δ: 7.52 (s, 1H), 3.35 - 3.29 (m, 2H), 2.26 - 2.24 (m, 1H), 2.11 - 2.08 (m, 1H), 1.60 - 1.53 (m, 5H), 1.45 - 1.33 (m, 1H), 1.27 (s, 3H), 1.18 (s, 3H), 0.95 (m, 6H).
SFC: (ee: 99.24%)
HPLC: (Purity: 100%)
LCMS: (M+H: 262.1)

### Spectra for 9-Ent2

**¹HNMR: (400 MHz, CDCl₃)** δ: 7.52 (s, 1H), 3.35 - 3.29 (m, 2H), 2.26 - 2.24 (m, 1H), 2.11 - 2.08 (m, 1H), 1.60 - 1.48 (m, 5H), 1.44 - 1.33 (m, 1H), 1.27 (s, 3H), 1.17 (s, 3H), 0.95 (m, 6H).
**SFC:** (ee: 99.48%)
**HPLC:** (Purity: 100%)
**MS:** (M+H: 262.1)

### Example 10. Synthesis of Compounds 10-Ent1 and 10-Ent2

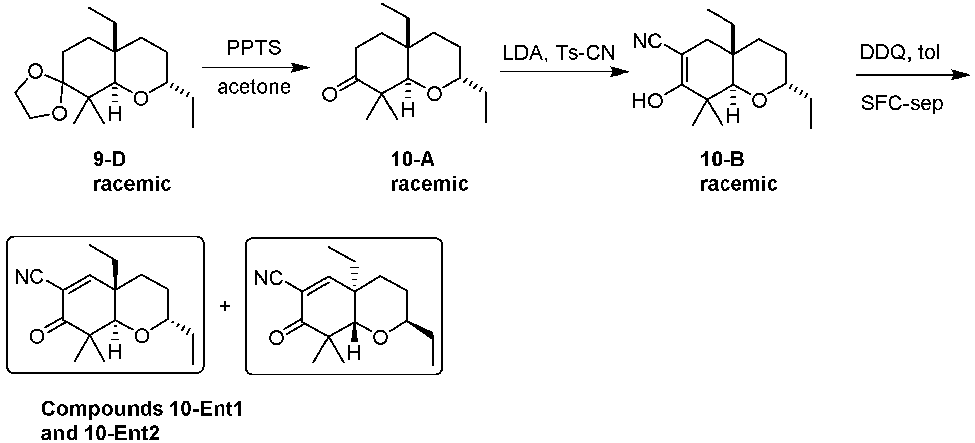

### Preparation of Compound 10-A

To a solution of compound **9-D** (1.6 g, 5.67 mmol, 1.0 eq.) in Acetone/H₂O (28 mL, v/v = 9:1), was added PPTS (1.14 g, 4.54 mmol, 0.8 eq.). The resulting clear solution was heated to reflux for 16 hrs. TLC (PE/EtOAc = 20:1) indicated completion. Water (40 mL) was added and the solvent acetone was removed under reduced pressure. The residue was extracted with EtOAc (40 mL x 2). The combined organic layers were dried over Na₂SO₄, filtered and concentrated to give compound **10-A** (1.1 g, 81%) as colorless oil.
**¹HNMR: (400 MHz, CDCl₃)** δ: 3.98 - 3.84 (m, 1H), 3.22 (s, 1H), 2.60 (m, 1H), 2.32 - 2.20 (m, 1H), 2.03 - 1.77 (m, 4H), 1.74 - 1.57 (m, 2H), 1.48 - 1.36 (m, 1H), 1.35 - 1.22 (m, 1H), 1.18 - 1.07 (m, 8H), 0.97 - 0.88 (m, 3H), 0.83 (t, *J* = 7.4 Hz, 3H).

### Preparation of Compound 10-B

To a solution of compound **10-A** (1.1 g, 4.6 mmol, 1.0 eq.) in anhydrous THF (23 mL) was added LDA (2 M in THF/heptane, 4.6 mL, 9.2 mmol, 2.0 eq.) dropwise via syringe at - 78 °C. The yellow solution was stirred at -78 °C for 1 h before TsCN (1.7 g, 9.2 mmol, 2.0 eq.) was added in portions. The resulting yellow solution was stirred at -78 °C for another 1 h. TLC (PE/EtOAc = 20:1) indicated completion. The reaction mixture was quenched with Sat. NH₄Cl (50 mL). The solvent was removed under reduced pressure then the water layer was extracted with EA (50 mL x 2). The combined organic layers were dried over anhydrous sodium sulfate. Filtered and concentrated to give compound **10-B** (1.6 g, crude) as yellow gum. The crude product was used in next step directly without further purification.

### Preparation of Compounds 10-Ent1 and 10-Ent2

To a suspension of compound **10-B** (1.6 g, 6 mmol, 1.0 eq.) in toluene (30 mL) was added DDQ (2 g, 9 mmol, 1.5 eq.). The resulting red mixture was heated to 50°C for 2 hours. TLC (PE/EtOAc = 6:1) indicated completion. On cooling, the solvent was removed under reduced pressure and the residue was dissolved in EA (100 mL), washed with Sat NaHCO₃ (50 mL x 3), brine (50 mL x 2). The organic layer was dried over Na₂SO₄, filtered and concentrated, the residue was purified by column chromatography (PE/EtOAc = 15:1) to give a racemic mixture of **10-Entl** and **10-Ent2** (550 mg, purity 70%). The crude product was recrystallized with PE: EA = 10:1 (10 mL) to give 0.42g of a white solid. The enantiomers were separated by SFC (column: IC 250 mm*30 mm*10 um, condition: Neu-EtOH) to give **10-Ent1** (Rt = 1.713 min, 161.0 mg) and **10-Ent2** (Rt = 1.836 min, 147.0 mg) both as white solid.

### Spectra for 10-Ent1

**¹HNMR: (400 MHz, CDCl₃)** δ: 7.50 (s, 1H), 3.97 - 3.92 (m, 1H), 3.58 (s, 1H), 2.41 - 2.39 (m, 1H), 2.37 - 2.01 (m, 1H), 1.97 - 1.80 (m, 2H), 1.53 - 1.44 (m, 4H), 1.27 (s, 3H), 1.17 (s, 3H), 0.94 (t, *J* = 7.4 Hz, 6H).
**SFC:** (ee: 99.4%)
**HPLC:** (Purity: 99.06%)
**MS:** (M+H: 262.1)

### Spectra for 10-Ent2

**¹HNMR: (400 MHz, CDCl₃)** δ: 7.50 (s, 1H), 3.97 - 3.91 (m, 1H), 3.58 (s, 1H), 2.41 - 2.39 (m, 1H), 2.37 - 2.01 (m, 1H), 1.97 - 1.80 (m, 2H), 1.53 - 1.43 (m, 4H), 1.27 (s, 3H), 1.17 (s, 3H), 0.94 (t, *J* = 7.2 Hz, 6H).
**SFC:** (ee: 96.42%)
**HPLC:** (Purity: 100%)
**MS:** (M+H: 262.1)

### Example 11. Synthesis of Compounds 11-Ent1 and 11-Ent2

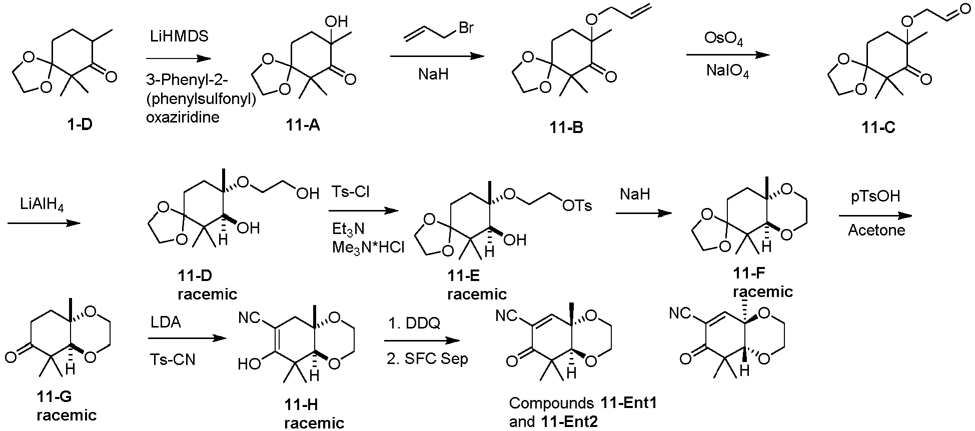

### Preparation of Compound 11-A

To a round-bottomed flask containing racemic compound **1-D** (2760 mg, 13.9 mmol) in 50 mL THF at -78°C was added Potassium bis(trimethylsilyl)amide (3.0 g, 15 mmol) (1M in THF, 15.0 ml). After 30 min, 3-Phenyl-2-(phenylsulfonyl)oxaziridine (4.0 g, 15 mmol) in 50 mL THF was added and the reaction was allowed to warm to RT slowly. The reaction was quenched by addition of Sat. NH₄Cl solution. Ethyl ether was added and the organic phase was separated and washed with brine, dried over MgSO₄, filtered, and concentrated. Purification by column chromatography using 10-30% ethyl acetate in heptane afforded racemic compound 11-A.
**¹H NMR (400 MHz, DMSO-d6)** δ ppm 5.29 (d, J=1.0 Hz, 1 H) 3.68 - 4.05 (m, 4 H) 2.05 - 2.21 (m, 1 H) 1.63 - 1.83 (m, 2 H) 1.52 - 1.63 (m, 1 H) 1.25 (s, 3 H) 1.14 (s, 3 H) 0.95 (s, 3 H).

### Preparation of Compound 11-B

Compound **11-A** (410 mg, 1.9 mmol) in 12 mL of anhydrous THF was treated with sodium hydride (0.084 g, 2.1 mmol) and heated to 60 °C for 15 min, then treated with allyl bromide (0.35 g, 2.9 mmol) and heated to reflux. The reaction was monitored for disappearance of starting material and then cooled to RT and stirred overnight. The reaction was quenched by addition of 50% saturated NH₄Cl solution and diluted with ether. The layers were separated and the organic phase was washed with brine, dried over MgSO₄, filtered, and concentrated. Purification using 10-30% ethyl acetate in heptane on 25g silica gel column afforded compound **11-B.**
**¹H NMR (400 MHz, CHLOROFORM-d)** δ ppm 5.86 (ddt, J=17.1, 10.5, 5.3, 5.3 Hz, 1 H) 5.25 (dq, J=17.3, 1.7 Hz, 1 H) 5.12 (dq, J=10.4, 1.5 Hz, 1H) 3.86 - 4.04 (m, 5 H) 3.57 (ddt, J=12.5, 5.2, 1.6, 1.6 Hz, 1 H) 2.40 (td, J=13.5, 4.1 Hz, 1 H) 2.04 (dt, J=14.6, 3.8 Hz, 1 H) 1.71 - 1.83 (m, 1 H)1.63 (dt, J=13.6, 3.8 Hz, 1 H) 1.36 (s, 3 H) 1.25 (s, 3 H) 1.05 (s, 3 H).

### Preparation of Compound 11-C

To a solution of compound **11-B** (207 mg, 0.814 mmol) in 6 mL dioxane and 2 mL water was added 2,6-Lutidine (0.700 g, 6.53 mmol) and Sodium periodate (0.696 g, 3.26 mmol). The reaction was treated with Osmium tetroxide, 2.5 wt. % solution in 2-methyl-2-propanol (0.214 g, 0.0163 mmol) and allowed to stir at room temperature. A voluminous white precipitate formed almost immediately. The reaction was allowed to stir for 2h, then it was diluted with 10 mL water and extracted with 3 x 10 mL dichloromethane. The combined organic phases were washed with brine, dried over Sodium Sulfate, filtered and concentrated. Purification using 10-50% ethyl acetate in heptane on a 12g silica gel column compound 11-C (0.12 g; Yield = 58%).
**¹H NMR (400 MHz, CHLOROFORM-d)** δ ppm 9.68 (t, J=0.9 Hz, 1 H) 3.88 - 4.06 (m, 6 H) 3.77 (dd, J=17.8, 0.8 Hz, 1 H) 2.36 (ddd, J=13.7, 12.4, 4.3 Hz, 1 H) 2.11 (dt, J=14.4, 4.4 Hz, 1 H) 1.78 - 1.91 (m, 1 H) 1.72 (dt, J=13.7, 4.5 Hz, 1 H) 1.30 (s, 3 H) 1.26 (s, 3 H) 1.08 (s, 3 H).

### Preparation of Compound 11-D

To a reaction vessel containing **11-C** (0.12 g, 0.47 mmol) in 2 mL THF at 0°C was added lithium aluminum hydride (0.036 g, 0.94 mmol) (940 uL of a 1M Solution in THF). After 2h at 0°C, sat. Rochelle's salt was added and the reaction was allowed to warm to room temperature and stir for 2h. The aqueous phase was extracted with diethyl ether, washed with brine, and dried over MgSO₄, filtered, and concentrated. Purification by column chromatography: 15-30% acetone in heptane on 12g column afforded two separated diastereomers, a minor diastereomer (39 mg; Yield = 32%) and a major diastereomer (57 mg; Yield = 47%). The major diastereomer was confirmed to be structure **11-D** after subsequent derivatization to **11-F** and 2-Dimensional nOe NMR analysis.
**¹H NMR (400 MHz, CHLOROFORM-d)** δ ppm 3.84 - 4.02 (m, 4 H) 3.72 (br s, 2 H) 3.39 - 3.49 (m, 2 H) 3.27 (d, J=10.3 Hz, 1 H) 2.38 (br d, J=10.5 Hz, 1 H) 1.98 - 2.09 (m, 1 H) 1.87 - 1.96 (m, 1 H) 1.77 - 1.87 (m, 1 H) 1.44 - 1.55 (m, 1 H) 1.34 - 1.42 (m, 1 H) 1.27 (s, 6 H) 1.09 (s, 3 H) 0.97 (s, 3 H).

### Preparation of Compound 11-E

To a solution of **11-D** in 10 mL of anhydrous dichloromethane was added triethylamine (0.350 g, 3.46 mmol), trimethylamine hydrochloride (0.0110 g, 0.115 mmol), and p-toluenesulfonyl Chloride (0.283 g, 1.21 mmol). The reaction was allowed to stir at room temperature for 4h, then deposited on 2g silica gel and purified using 20% acetone in heptane on a 12g silica gel column to afford compound **11-E** (450 mg, 94%).
**¹H NMR (400 MHz, CHLOROFORM-d)** δ ppm 7.67 - 7.87 (m, 2 H) 7.29 - 7.40 (m, 2 H) 4.04 - 4.19 (m, 2 H) 3.85 - 4.01 (m, 4 H) 3.49 - 3.64 (m, 2H) 3.16 (d, J=8.5 Hz, 1 H) 2.87 (br s, 1 H), 2.38 (s, 3H), 1.60 - 1.85 (m, 3 H) 1.40 (dt, J=13.1, 4.2 Hz, 1 H) 1.18 (s, 3 H) 1.08 (s, 3 H) 0.97 (s, 3 H).

### Preparation of Compound 11-F

Compound **11-E** (450 mg, 1.1 mmol) in 0.5 mL THF was treated with Sodium hydride (0.087 g, 2.2 mmol). After 10 min, the reaction was heated at 65 °C for 1h hour. The reaction was deemed complete by LC/MS and TLC. The reaction was cooled to RT and quenched with sat NH₄Cl solution and diluted with ethyl acetate. The layers were separated and the ethyl acetate layer was washed twice with sat NaHCO₃ solution and then brine, dried over MgSO₄, filtered, and concentrated to afford crude **11-F.** The crude material was purified on a 12g silica column using 0-10% acetone in heptane to afford 198 mg (75%) of **11-F** as a white solid.
**¹H NMR (300 MHz, CHLOROFORM-d)** δ ppm 3.65 - 4.14 (m, 7 H) 3.48 (dd, J=11.9, 3.2 Hz, 1 H) 3.37 (s, 1 H) 1.46 - 1.95 (m, 6 H) 1.41 (s, 3 H) 0.95 (s, 3H), 0.94 (s, 3H).

### Preparation of Compound 11-G

Compound **11-F** (210 mg, 0.87 mmol) was dissolved in acetone (10 mL) and treated with p-toluenesulfonic acid monohydrate (0.0082 g, 0.043 mmol). The reaction was heated to 45°C overnight, then heated to reflux for 8h. The reaction was heated at 45°C overnight again to afford -95% conversion by NMR analysis. The reaction was worked up as follows: the solvent was removed *in vacuo* and the reaction was diluted with 30 mL of ethyl acetate and 5 mL of sat sodium bicarbonate. The organic phase was separated and washed with brine. The organic phase was dried over MgSO₄, filtered, and concentrated to afford **11-G** Yield = 91%. **¹H NMR (400 MHz, CHLOROFORM-d)** δ ppm 4.07 (td, J=12.2, 3.3 Hz, 1 H) 3.94 (dd, J=11.5, 3.3 Hz, 1 H) 3.71 - 3.83 (m, 1 H) 3.55 (dd, J=12.2, 3.4 Hz, 1 H) 3.49 (dd, J=12.0, 3.3 Hz, 1 H) 3.30 (s, 1 H) 2.55 - 2.67 (m, 1 H) 2.41 - 2.51 (m, 1 H) 1.88 (ddd, J=13.2, 6.9, 2.5 Hz, 1 H) 1.71 (td, J=13.3, 6.3 Hz, 1 H) 1.54 (s, 3 H) 1.19 (s, 3 H) 1.06 (s, 3 H).

### Preparation of Compound 11-H

Freshly prepared lithium diisoproylamide was prepared as follows: : 640 uL of N,N-Diisopropylamine (0.463 g, 4.58 mmol) was taken up in 2.7 mL of THF and cooled to -78°C. The solution was treated with 2.5 M of n-Butyllithium in hexanes (1.66 mL, 4.16 mmol) and warmed to 0°C for 30 minutes. Compound **11-G** (165 mg, 0.832 mmol) in 3 mL of THF at - 78°C was treated with 1.20 mL of Lithium Diisopropylamide solution prepared above. After 30 minutes, p-toluenesulfonyl cyanide (0.226 g, 1.25 mmol) in 0.5 mL THF was added. The reaction was allowed to stir for 1h then quenched by addition of 1.25 mL of 1N HCL and warmed to RT. The mixture was diluted with ethyl acetate and the layers were separated. The organic phase was washed with brine, dried over MgSO₄, filtered, and concentrated. Purification using 5-20% acetone in heptane afforded **11-H** (65 mg; Yield = 35%) as a white solid. LCMS (M+H) = 224.1.

### Preparation of Compounds 11-Ent1 and 11-Ent2

Compound **11-H** (65 mg, 0.29 mmol) in 5 mL of benzene was treated with Dichlorodicyanoquinone (DDQ, 0.079 g, 0.35 mmol) and heated to reflux for 4 hours. The reaction was cooled to room temperature and filtered through Celite™. The crude material was deposited on 500 mg of silica gel and purified twice using 5-15% gradient of acetone in heptane on a 4g silica gel cartridge to afford a white solid (52 mg; Yield = 77%; Purity = 95%). The racemic product was separated into the individual enantiomers by chiral SFC chromatography (CHIRALPAK AD-H 30x250mm, 5um Co-solvent: 5% Isopropanol (w/o any modifier) in CO2 (flow rate: 100mL/min)) to afford two peaks. The first eluting peak was called 11-Entl and the second eluting peak was called 11-Ent2. The absolute configuration was not determined.

### Data for 11-Ent1:

**¹H NMR (400 MHz, DMSO-d6)** δ ppm 8.01 (s, 1 H) 3.87 - 4.07 (m, 2 H) 3.65 - 3.79 (m, 2 H) 3.60 (dd, J=12.0, 3.3 Hz, 1 H) 1.53 (s, 3 H) 1.20 (s, 3H) 1.01 (s, 3 H).
**LC/MS (M+H)** = 222.1
**Chiral HPLC** = 100% ee

### Data for 11-Ent2:

**¹H NMR (400 MHz, DMSO-d6)** δ ppm 8.01 (s, 1 H) 3.87 - 4.07 (m, 2 H) 3.65 - 3.79 (m, 2 H) 3.60 (dd, J=12.0, 3.3 Hz, 1 H) 1.53 (s, 3 H) 1.20 (s, 3H) 1.01 (s, 3 H)
**LC/MS (M+H)** = 222.1
**Chiral HPLC** = 96% ee

### Example 12. Synthesis of Compounds 40-Ent1 and 40-Ent2

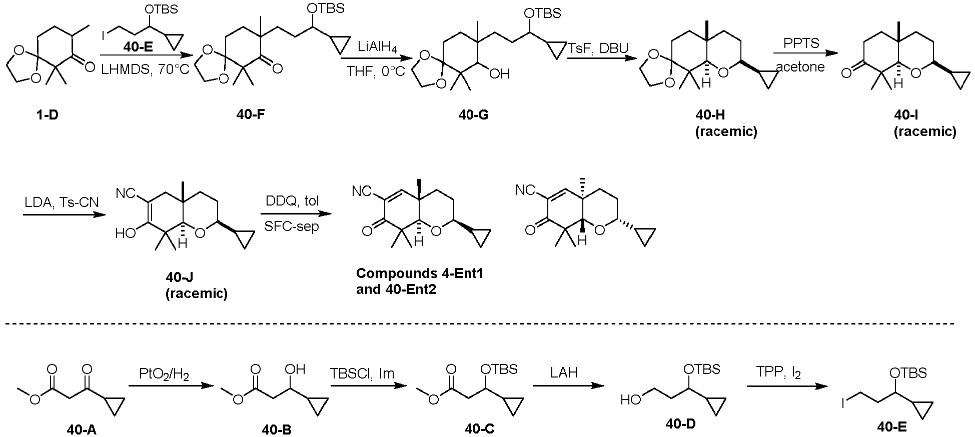

### Preparation of Compound 40-B

A mixture of compound **40-A** (Durham, T. B.; Miller, M. J. Journal of Organic Chemistry, 2003, 68, 27. ; 200 g, 1.28 mol, 1.0 eq.), PtO₂ (6.8 g) and TEA (4 mL) in MeOH (1000 mL) was stirred at 50°C for 48 hours under 50 psi H₂ atmosphere. TLC (EA) showed the reaction was completed. The mixture was filtered and concentrated *in vacuo* to give compound **40-B** (200 g, crude) as a yellow solid, which was used directly in the next step without purification.

### Preparation of Compound 40-C

A mixture of compound **40-B** (150 g, 0.63 mol, 1.0 eq), TBSCl (150 g, 0.63 mol, 1.0 eq), and imidazole (109 g, 1.6 mol, 2.5 eq) in DCM (500 mL) was stirred at 40°C for 12 hours. TLC (PE/EA = 5/1) showed the reaction completed. The mixture was diluted with water (500 mL), and extracted with DCM (500 mL x 3). The combined organic phase was dried over Na₂SO₄, and filtered. The filtrate was concentrated *in vacuo* to give the residue, which was purified by column chromatography (PE/EA = 1/0 ∼ 20/1) to give compound **40-C** (190 g, yield: 90%) as yellow oil.

### Preparation of Compound 40-D

To a solution of compound **40-C** (50 g, 184 mmol, 1.0 eq.) in THF (500 mL) was added LAH (4 g, 110.4 mmol, 0.6 eq) in portions at -10°C. The mixture was stirred at -10°C for 30 min. TLC (PE/EA = 5/1) showed the reaction completed. The mixture was quenched with water (50 mL), 15% NaOH (50 mL), and followed by addition of water (150 mL). The mixture was extracted with EtOAc (500 mL x 2). The combined organic phase was dried over Na₂SO₄, and filtered. The filtrate was concentrated *in vacuo* to give compound **40-D** (24 g, crude) as a yellow solid.

### Preparation of Compound 40-E

A mixture of PPh₃ (45 g, 0.17 mol, 1.1 eq.), and I₂ (43 g, 0.17 mol, 1.1 eq) in DCM (400 mL) was stirred at 30°C for 1 h. Imidazole (20 g, 0.3 mol, 2 eq) was added to adjust pH to 6-7. Then compound **40-D** (35 g, 0.15 mol, 1.0 eq.) was added. The mixture was stirred 30°C for 1h. TLC (PE) showed the reaction was completed. The mixture was diluted with water (400 mL), extracted with DCM (400 mL x 2). The combined organic phase was dried over Na₂SO₄, filtered and concentrated in vacuum. The residue was purified by column chromatography (PE/EA = 10/1) to give compound **40-E**(35 g, 69 % yield) as a yellow oil. **¹H NMR: (400MHz, CDCl₃)** δ: 3.37 - 3.23 (m, 2H), 3.13 - 3.03 (m, 1H), 2.63 - 2.55 (m, 1H), 2.15 - 2.06 (m, 2H), 0.92 - 0.90 (m, 9H), 0.56 - 0.43 (m, 2H), 0.37 - 0.27 (m, 1H), 0.23 - 0.16 (m, 1H), 0.10 (d, *J* = 7.2 Hz, 6H). The spectra was from the pilot run.

### Preparation of Compound 40-F:

To a solution of compound **1-D** (21.8 g, 110.3 mmol, 1.0 eq.) in THF (220 mL) was added LiHMDS (220 mL, 220 mmol, 1.0 M in THF, 2 eq.) at 18°C. After stirring for 1 h, the mixture was added compound **40-E** (45 g, 132.3 mmol, 1.2 eq.) in THF (100 mL) dropwise. The resulting brown solution was heated to reflux for 16 hours. TLC (PE/EA = 10:1) showed the reaction completed. The reaction was quenched with Sat. NH₄Cl (600 mL). The organic solvent was removed off under reduced pressure and the water layer was extracted with EA (250 mL x 2). The combined organic layers were washed with water (250 mL x 2), brine (250 mL x 3), dried over Na₂SO₄, filtered and concentrated. The residue was purified by silica gel chromatography (eluted with PE/EtOAc = 80:1) to give compound **40-F** (21 g, 46.5%) as yellow gum.
**¹HNMR: (400 MHz, CDCl₃)** δ: 4.02 - 3.89 (m, 4H), 3.01 (br d, *J* = 5.3 Hz, 1H), 2.04 - 1.93 (m, 1H), 1.92 - 1.73 (m, 3H), 1.70 - 1.60 (m, 2H), 1.54 - 1.44 (m, 1H), 1.33 - 1.21 (m, 1H), 1.18 - 1.07 (m, 9H), 0.88 (m, 10H), 0.49 - 0.36 (m, 2H), 0.29 - 0.21 (m, 1H), 0.17 - 0.09 (m, 1H), 0.06 - 0.02 (m, 6H).

### Preparation of Compound 40-G

To a solution of compound **40-F** (21 g, 51.2 mmol, 1.0 eq.) in THF (512 mL) was added LiAlH₄ (3.9 g, 102.4 mmol, 2.0 eq.) in portions at 0∼10°C under N₂ atmosphere. The white suspension was then stirred at 10°C for 2 h. TLC (PE/EA = 10:1) indicated completion. Quenched with H₂O (3.9 mL) carefully and slowly, then treated with 15% NaOH aq. (3.9 mL), H₂O (12 mL). The white suspension was filtered and concentrated, the residue was purified by silica gel chromatography (eluting with PE/EtOAc = 100:1 ∼ 30:1) to give compound **40-G** (19.4 g, 94%) as colorless gum.
**¹HNMR: (400 MHz, CDCl₃)** δ: 3.95 (br s, 4H), 3.36 - 3.17 (m, 1H), 3.10 - 2.95 (m, 1H), 1.66 - 1.54 (m, 5H), 1.27 (br s, 4H), 1.08 - 1.03 (m, 6H), 1.00 (br d, *J* = 4.1 Hz, 3H), 0.89 (s, 10H), 0.47 - 0.38 (m, 2H), 0.31 - 0.22 (m, 1H), 0.17 (br d, *J* = 4.9 Hz, 1H), 0.08 - 0.00 (m, 6H).

### Preparation of Compound 40-H

To a solution of compound **40-G** (13.7 g, 33.2 mmol, 1.0 eq.) in toluene (83 mL) was added TsF (11.6 g, 66.4 mmol, 2 eq.) and DBU (10 g, 66.4 mmol, 2.0 eq.) at 25 °C. The resulting solution was heated to reflux for 16 hours. TLC (PE/EtOAc = 10:1) showed five spots. The solvent was removed under vacuum. The residue was purified by silica gel chromatography eluted with PE/EtOAc = 200:1 to give compound **40-H** (0.6 g, 6.5% yield) as colorless gum.
**¹HNMR: (400 MHz, CDCl₃)** δ:3.96 - 3.79 (m, 4H), 2.78 (s, 1H), 2.71 (m, 1H), 1.78 (m, 1H), 1.62 - 1.53 (m, 1H), 1.46 - 1.32 (m, 3H), 1.28 - 1.16 (m, 3H), 0.92 (d*, J* = 12.9 Hz, 6H), 0.87 - 0.77 (m, 4H), 0.43 - 0.26 (m, 3H), 0.20 - 0.13 (m, 1H).

### Preparation of Compound 40-I

To a solution of compound **40-H** (0.6 g, 2.14 mmol, 1.0 eq.) in Acetone/H₂O (20 mL, v/v = 9:1), was added PPTS (0.43 g, 1.71 mmol, 0.8 eq.). The resulting clean solution was heated to reflux for 16 hrs. TLC (PE/ EtOAc = 10:1) indicated completion. The solvent acetone was removed under reduced pressure. The residue was diluted with water (50 mL) and extracted with EtOAc (3 x 30 mL). The combined organic layers were dried over Na₂SO₄, filtered and concentrated to give compound **40-I** (0.47 g, 93% yield) as colorless gum.
**¹HNMR: (400 MHz, CDCl₃)** δ: 2.85 - 2.67 (m, 3H), 2.30 - 2.20 (m, 1H), 1.76 - 1.63 (m, 2H), 1.58 - 1.53 (m, 2H), 1.40 - 1.23 (m, 2H), 1.19 (s, 3H), 1.11 (d*, J* = 11.3 Hz, 6H), 0.98 - 0.88 (m, 1H), 0.55 - 0.43 (m, 2H), 0.42 - 0.34 (m, 1H), 0.30 - 0.22 (m, 1H)

### Preparation of Compound 40-J

To a yellow solution of compound **40-I** (0.42 g, 1.79 mmol, 1.0 eq.) in anhydrous THF (9 mL) was added LDA (2 M in THF/heptane, 1.8 mL, 3.6 mmol, 2 eq.) dropwise via syringe at -78 °C. The resulting dark solution was stirred at -78 °C for 1 h before TsCN (0.48 g, 2.7 mmol, 1.5 eq.) in anhydrous THF (5 mL) was added. The resulting brown suspension was stirred at -78 °C for another 1 h. TLC (PE/ EtOAc = 10:1) indicated completion. The reaction was quenched with Sat. NH₄Cl (30 mL) then extracted with EtOAc (30 mL x 3). The combined organic layers were dried over anhydrous sodium sulfate. Filtered and concentrated, the residue was used in next step without purification.

### Preparation of Compounds 40 Ent1 and 40 Ent2

A mixture of compound **40-J** (0.46 g, 1.79 mmol, 1.0 eq.) and DDQ (0.41 g, 1.79 mmol, 1.0 eq.) in toluene (18 mL) was heated to 50 °C for 2 hours. LCMS indicated completion. The solvent was evaporated under vacuum. The residue was purified by Prep-TLC (PE: EA = 4:1) to give the final product (0.3 g, 64.5%) as yellow gum. Further separated by SFC (Mobile phase: Superaritical CO₂/MeOH; Column: Chiralpak AD 250*30mm*5 um; Detection wavelength: 220 nm) to give **40-Ent1** (Rt = 1.694 min, 83.6 mg, 39.2%) and **40-Ent2** (19112-150-2, Rt = 1.981 min, 86.2 mg, 37.3%) both as white solid. The absolute stereochemistry was not determined.

### Spectra for 40-Ent1

**¹HNMR: (400 MHz, CDCl₃)** δ: 7.43 (s, 1H), 3.20 (s, 1H), 2.89 - 2.82 (m, 1H), 1.90 - 1.79 (m, 1H), 1.79 - 1.69 (m, 2H), 1.66 - 1.50 (m, 1H), 1.34 (s, 3H), 1.26 (s, 3H), 1.13 (s, 3H), 0.95 (m, 1H), 0.57 - 0.47 (m, 2H), 0.44 - 0.34 (m, 1H), 0.31 - 0.23 (m, 1H).
**SFC:** (ee: 99.72%)
HPLC: (Purity: 99.7%)
MS: (M+H: 260.1)

### Spectra for 40-Ent2

**¹HNMR: (400 MHz, CDCl₃)** δ: 7.45 (s, 1H), 3.23 (s, 1H), 2.91 - 2.84 (m, 1H), 1.91 (s, 1H), 1.79 - 1.71 (m, 2H), 1.67 - 1.61 (m, 1H), 1.37 (s, 3H), 1.28 (s, 3H), 1.15 (s, 3H), 1.02 - 0.91 (m, 1H), 0.59 - 0.49 (m, 2H), 0.47 - 0.36 (m, 1H), 0.33 - 0.25 (m, 1H).
**SFC:** (ee: 97.02%).
**HPLC:** (Purity: 98.95%).
**MS:** (M+H: 260.1).

### Example 13. Synthesis of Compounds 41-Ent1 and 41-Ent2

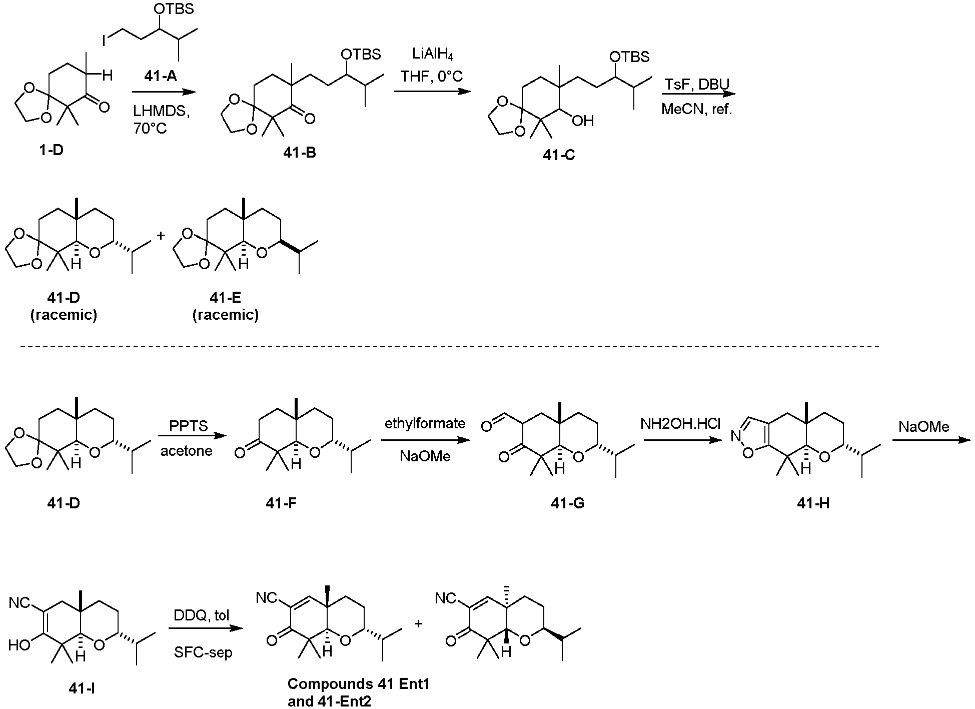

### Preparation of Compound 41-B

To a solution of compound **41-A** (Vettel, S.; Vaupel, A.; Knochel, P. Journal of Organic Chemistry, 1996, vol. 61, 7473.; 30 g, 151 mmol, 1.0 eq) in THF (300 mL) was added LiHMDS (1.0 M in THF, 303 mL, 303 mmol, 1.3 eq) at 26 °C by dropwise. After addition the mixture was stirred for 1 h. Compound **5** (67 g, 0.196 mmol, 1.3 eq) in THF (50 mL) was added and the reaction was stirred at reflux for 19 hours. TLC (PE: EA = 10:1) showed the starting material remained little. The mixture was quenched by saturated aqueous NH₄Cl (600 mL) and extracted with EA (300 mL x 2). The combined organic layers was concentrated and purified by column chromatography on silica gel (glutted with PE to PE: EtOAc =300:1-150:1-100:1) to supply compound **41-B** (27g, 27% yield) as pale-yellow gum. **¹H NMR (400MHz, CDCl₃)** δ: 3.98 - 3.93 (m, 4H), 3.39 - 3.33 (m, 1H), 1.98 - 1.59 (m, 6H), 1.49 - 1.23 (m, 3H), 1.13 (m, 6H), 1.08 (d, *J*=1.8 Hz, 3H), 0.89 (s, 9H), 0.87 - 0.80 (m, 6H), 0.06 - 0.00 (m, 6H)

### Preparation of Compound 41-C

To a solution of compound **41-B** (25 g, 60.68 mmol, 1.0 eq.) in THF (300 mL) was added LiAlH₄ (5.76 g, 151.7 mmol, 2.5 eq.) at 0°C by dropwise. After addition the mixture was allowed to warm to room temperature and stirred for 18h. TLC (PE: EA = 10:1) showed compound **9** remained little. The reaction mixture was quenched by adding water (5.8 mL) slowly under ice-bath, then 15% NaOH aq (5.8 mL) and water (17.4 mL). The mixture was diluted with THF (500 mL), then stirred with Na₂SO₄ (30 g). The suspension was filtered through a pad of celite, the cake was washed with EtOAc/EtOH (500 mL / 100 mL) and filtered again. The combined filtrate was concentrated and purified by column chromatography on silica gel (PE: EA = 150:1-100:1-80:1) to supply compound **41-C** (18 g, 72% yield) as pale-yellow gum.
**¹H NMR (400MHz,CDCl₃)** δ: 3.94-3.93 (m, 4H), 3.38 - 3.37 (m, 1H), 3.29 - 3.19 (m, 1H), 1.69 - 1.26 (m, 10H), 1.05 - 0.99 (m, 8H), 0.89 - 0.83 (m, 16H), 0.04 - 0.02 (m, 6H).

### Preparation of Compounds 41-D and 41-E

To a solution of compound **41-C** (118 g, 43.48 mmol, 1.0 eq) in toluene (100 mL) was added TsF (22.7 g, 130.43 mmol, 3.0 eq) and DBU (19.8 g, 130.43 mmol, 3.0 eq). The mixture was stirred at 120°C for 18 hours. TLC (PE: EA = 10:1) showed little compound **41-C** remained and several spots were observed. The mixture was concentrated and purified by column chromatography on silica gel (silica: 300-400. elute:PE: EA = 150:1-100:1-60:1) to supply compound crude **41-D** (racemic, 480 mg) as pale-yellow gum and compound **41-E** (racemic, 1.27g, contaminated with TsF) as pale-yellow gum.
**¹HNMR: (400 MHz, CDCl₃) (41-E, impure)** δ: 3.98-3.91 (m, 4H), 3.03 - 3.01 (m, 1H), 2.89 (s, 1H), 1.46-1.43 (m, 2H),1.32 - 1.26 (m, 7H), 0.97 - 0.89 (m, 15H).
**¹HNMR: (400 MHz, CDCl₃) (41-D, impure)** δ: 4.00-3.96 (m, 4H), 2.81 (s, 1H), 2.67 (m,1H),1.65-1.59 (m, 2H),1.41 - 1.38 (m, 7H), 0.97 - 0.94 (m, 15H).

### Preparation of Compound 41-F

To a solution of compound **41-D** (480 mg, 1.7 mmol, 1.0 eq) in acetone/H₂O (10 mL, v : v = 9 : 1) was added PPTS (259 mg, 1.02 mmol, 0.6 eq). The resulting mixture was stirred at 85°C for 18 hrs. TLC (PE: EtOAc = 10:1) indicated the completion. The mixture was concentrated to remove acetone and diluted with water (15 mL) and extracted with EtOAc (20 mL x 2). The combined organic layers were dried over Na₂SO₄, filtered and concentrated. The residue was purified by column chromatography on silica gel (PE: EA =100:1-80:1-60:1) to supply compound **41-F** (330 mg, 81%) as pale-yellow oil.

### Preparation of Compound 41-G

To a solution of compound **41-F** (330 mg, 1.39 mmol, 1.0 eq) in 2.2 mL of ethyl formate (1.05 g, 27.73 mmol, 20.0 eq) was added NaOMe (0.9 mL, 4.87 mmol, 3.5 eq, 5.4 M in MeOH) under ice-bath. After addition the mixture was stirred at 20-26 °C under N₂ atmosphere for 4h. It turned into white suspension. TLC (PE: EA = 10:1) indicated the completion. The mixture was added into 20 mL of ice-water, extracted with EtOAc (15 mL×3). The combined organic layer was dried over anhydrous Na₂SO₄, filtered, concentrated under reduced pressure to supply crude compound **41-G** (330 mg) as pale-yellow gum.

### Preparation of Compound 41-H

Compound **41-G** (330 mg, 1.24 mmol, 1.0 eq) and hydrochloride hydroxylamine (104 mg, 1.49 mmol, 1.2 eq) were added into 11 mL of EtOH/H₂O (V: V = 10:1). The mixture was stirred at 50 °C for 18h. TLC (PE: EA = 10:1) indicated the completion. The reaction mixture was diluted with water (30 mL) and extracted with EtOAc (15 mL × 3). The combined organic layer was concentrated and purified by column chromatography on silica gel (PE: EA =200:1-100:1) to supply impure compound **41-H** (270 mg crude, 82.6%) as pale-yellow gum.

### Preparation of Compound 41-1

To a solution of compound **41-H** (270 mg, 1.03 mmol, 1.0 eq) in 2 mL of anhydrous methanol was added NaOMe (2.05 mL, 4.11 mmol, 4.0 eq, 2 M in methanol). The mixture was stirred at 16 -22 °C for 18 h. TLC (PE/EA = 10:1) showed compound **41-H** was consumed completely. The mixture was concentrated and the residue was diluted with water (40 mL), extracted with EtOAc (30 mL × 3). The combined organic layer was dried over anhydrous Na₂SO₄, filtered, concentrated under reduced pressure to supply crude compound **41-1** (250 mg) as pale-yellow gum.

### Preparation of Compounds 41 Ent1 and 41-Ent2

Compound **41-I** (250 mg, 0.95 mmol, 1.0 eq) and DDQ (259 mg, 1.14 mmol, 1.2 eq) were added into 5 mL of anhydrous toluene. The mixture was stirred at 80 °C for 2 h. TLC (PE: EA = 8:1) showed two spot were observed. The mixture was concentrated under reduced pressure and purified by prep-TLC (PE: EA = 6:1) for twice to and then prep-HPLC (condition: water (10mM NH₄HCO₃)-ACN, column: C18, 150*25mm*5um) to supply the racemic product (25 mg, 10% yield) as off-white solid.
**¹HNMR: (400 MHz, MeOD)** δ: 7.68 (s, 1H), 3.68 (s, 1H),3.50 - 3.456 (m, 1H), 2.29 - 2.26 (m, 1H), 2.24 - 2.09 (m, 1H),1.81 - 1.76 (m, 2H), 1.58 - 1.55 (m, 1H), 1.37 (s, 3H), 1.27 (s, 3H), 1.10 (s, 3H), 0.98 (d, *J* = 6.4 Hz, 3H), 0.87 (d, *J* = 6.8 Hz, 3H).

The racemic product was further separated by SFC (column: AY 250mm*30mm, 5 um, condition: Neu-EtOH) to give **41-Ent1** (19202-75-1, Rt = 2.270 min, 7.0 mg, 28% yield) as off-white solid and **41-Ent2** (19202-75-2, Rt = 2.722 min, 5.5 mg, 22% yield) as off-white solid.

### Spectra for 41-Ent1

**¹HNMR: (400 MHz, CDCl₃**) δ: 7.42 (s, 1H), 3.58 - 3.46 (m, 2H), 2.19 - 2.04 (m, 2H), 1.78 - 1.65 (m, 2H), 1.56 - 1.49 (m, 1H), 1.38 (s, 3H), 1.31 (s, 3H), 1.13 (s, 3H), 0.98 (d, *J* = 6.5 Hz, 3H), 0.87 (d, *J* = 6.7 Hz, 3H).
**SFC:** (ee: 99.54%)
**HPLC:** (Purity: 93.37%)
**LCMS:** (M+H:262.1)

### Spectra for 41-Ent2

**¹HNMR: (400 MHz, CDCl₃**) δ: 7.42 (s, 1H), 3.57 - 3.46 (m, 2H), 2.22 - 2.02 (m, 2H), 1.78 - 1.66 (m, 2H), 1.56 - 1.50 (m, 1H), 1.38 (s, 3H), 1.31 (s, 3H), 1.13 (s, 3H), 0.99 (d, *J* = 6.5 Hz, 3H), 0.87 (d, *J* = 6.5 Hz, 3H).
**SFC:** (ee: 99.09: %)
**HPLC:** (Purity: 96.11%)
**LCMS:** (M+H:262.1)

### Example 14. Synthesis of Compounds 42-Ent1 and 42-Ent2

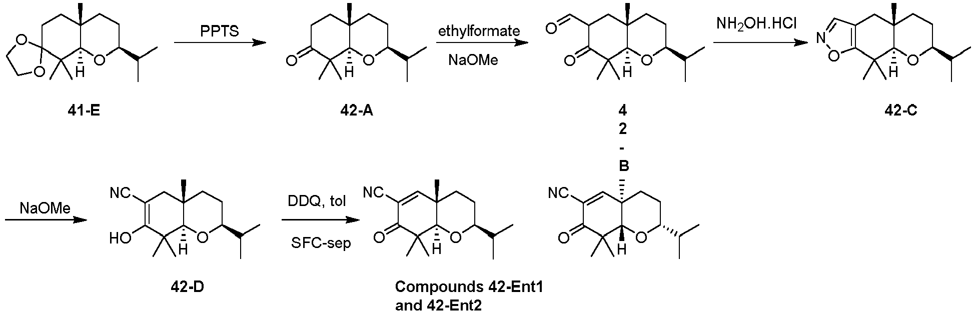

### Preparation of Compound 42-A

To a solution of compound **41-E** (1.27 g, 4.5 mmol, 1.0 eq,) in acetone/H₂O (10 mL, v : v = 9: 1) was added PPTS (686 mg, 2.7 mmol, 0.6 eq.). The resulting mixture was stirred at 85°C for 18 hrs. TLC (PE: EA = 10:1) indicated the completion. The reaction mixture was concentrated to remove acetone, diluted with water (15 mL) and extracted with EtOAc (10 mL x 2). The combined organic layers were dried over Na₂SO₄, filtered and concentrated. The residue was purified by column chromatography on silica gel (PE: EA =100:1-80:1-60:1) to supply compound **42-A** (900 mg, 84%) as pale-yellow oil.

### Preparation of Compound 42-B

To a solution of compound **42-A** (900 mg, 3.78 mmol, 1.0 eq) in 6.0 mL of ethyl formate (5.59 g, 75.6 mmol, 20.0 eq) was added NaOMe (2.45 mL, 13.23 mmol, 3.5 eq, 5.4 M in MeOH) by dropwise under ice-bath. After addition the mixture was allowed to room temperature and stirred under N₂ atmosphere for 4h. It turned into white suspension. TLC (PE/EA = 10:1) indicated the completion. The mixture was added into 25 mL of ice-water, extracted with EtOAc (20 mL × 3). The combined organic layer was dried over anhydrous Na₂SO₄, filtered, concentrated under reduced pressure to supply crude compound **42-B** (301 mg) as pale-yellow gum.

### Preparation of Compound 42-C

To a solution of compound **42-B** (840 mg, 3.15 mmol, 1.0 eq) in 22 mL of EtOH/H₂O (V: V = 10:1) was added hydrochloride hydroxylamine (265 mg, 3.79 mmol, 1.2 eq). After addition the mixture was stirred at 50 °C for 18h. TLC (PE: EA = 10:1) indicated the completion. The mixture was concentrated, the residue was diluted with water (20 mL), extracted with EtOAc (15 mL × 3). The combined organic layer was concentrated and purified by column chromatography on silica gel (PE: EA =200:1-100:1) to supply compound **42-C** (600 mg, 72.5%) as pale-yellow gum.
**LCMS:** (M+H:264.4)
**¹HNMR: (400 MHz, CDCl₃)** δ: 8.00 (s, 1H), 3.18 (s, 1H),3.09 - 3.05 (m, 1H), 2.27 (d, *J* = 15.2, 1H), 2.16 (d, ***J*** = 15.2, 1H),1.74 - 1.50 (m, 2H), 1.49 - 1.46 (m, 3H), 1.37 (s, 3H), 1.26 (s, 3H), 1.00 - 094 (m, 9H).

### Preparation of Compound 42-D

To a solution of compound **42-C** (600 mg, 2.28 mmol, 1.0 eq) in 6 mL of anhydrous methanol was added NaOMe (4.0 mL, 7.98 mmol, 3.5 eq, 2 M in methanol). The mixture was stirred at 18 -24 °C for 18 h. TLC (PE/EA = 10:1) showed compound **42-C** was consumed completely. The mixture was concentrated, diluted with water (40 mL), adjusted pH to 7 by adding 1N HCl aq. and extracted with EtOAc (30 mL × 3). The combined organic layer was dried over anhydrous Na₂SO₄, filtered, concentrated under reduced pressure to supply crude compound **42-D** (610 mg) as pale-yellow solid.

### Preparation of Compounds 42-Entl and 42-Ent2

To a solution of compound **42-D**(600 mg, 2.28 mmol, 1.0 eq) in 10 mL of anhydrous was added DDQ (621 mg, 2.73 mmol, 1.2 eq). The mixture was stirred at 70 °C for 1.5 h. TLC (PE: EA = 9:1) showed only one spot was observed. The mixture was concentrated under reduced pressure and purified by column chromatography on silica gel (PE: EA =300:1-150:1-80:1) to supply racemic product (330 mg, 55.5% yield) as off-white solid. **¹HNMR: (400 MHz, CDCl₃)** δ: 7.44 (s, 1H), 3.26 (s, 1H), 3.16 - 3.09 (m, 1H), 1.80 - 1.60 (m, 5H), 1.31 (s, 3H), 1.27 (s, 3H), 1.13 (s, 3H), 1.00 (d, *J* = 6.7 Hz, 3H), 0.94 (d, *J* = 6.8Hz, 3H).

The racemic product was further separated by SFC (column: AD 250mm*30mm, 5 um, condition: Neu-MeOH) two times to give **42-Ent2** (Rt = 2.271 min, 90.2 mg, 27.3% yield) as white solid and **42-Ent1** (Rt = 1.639 min, 55 mg) as white solid which was purified for the third time by SFC (column: AS 250mm*30mm, 5 um, condition: Neu-MeOH) to give **42-Ent1** (Rt = 2.206 min, 30.1 mg) as white solid.

### Spectra for 42-Ent1

**¹HNMR: (400 MHz, CDCl₃)** δ: 7.44 (s, 1H), 3.26 (s, 1H), 3.16 - 3.09 (m, 1H), 1.80 - 1.60 (m, 5H), 1.31 (s, 3H), 1.27 (s, 3H), 1.13 (s, 3H), 1.00 (d, *J* = 6.7 Hz, 3H), 0.94 (d, *J* = 6.8Hz, 3H).
**SFC:** (ee: 100%)
**HPLC:** (Purity: 100%)
**LCMS:** (M+H:262.1)

### Spectra for 42-Ent2

**¹HNMR: (400 MHz, CDCl₃)** δ: 7.44 (s, 1H), 3.26 (s, 1H), 3.16 - 3.09 (m, 1H), 1.80 - 1.61 (m, 5H), 1.31 (s, 3H), 1.27 (s, 3H), 1.12 (s, 3H), 0.99 (d, *J* = 6.8 Hz, 3H), 0.94 (d, *J* = 6.8Hz, 3H).
**SFC:** (ee: 98.28%)
**HPLC:** (Purity: 99.43%)
**LCMS:** (M+H:262.1)

### Example 15. Synthesis of Compounds 43-Ent1 and 43-Ent2

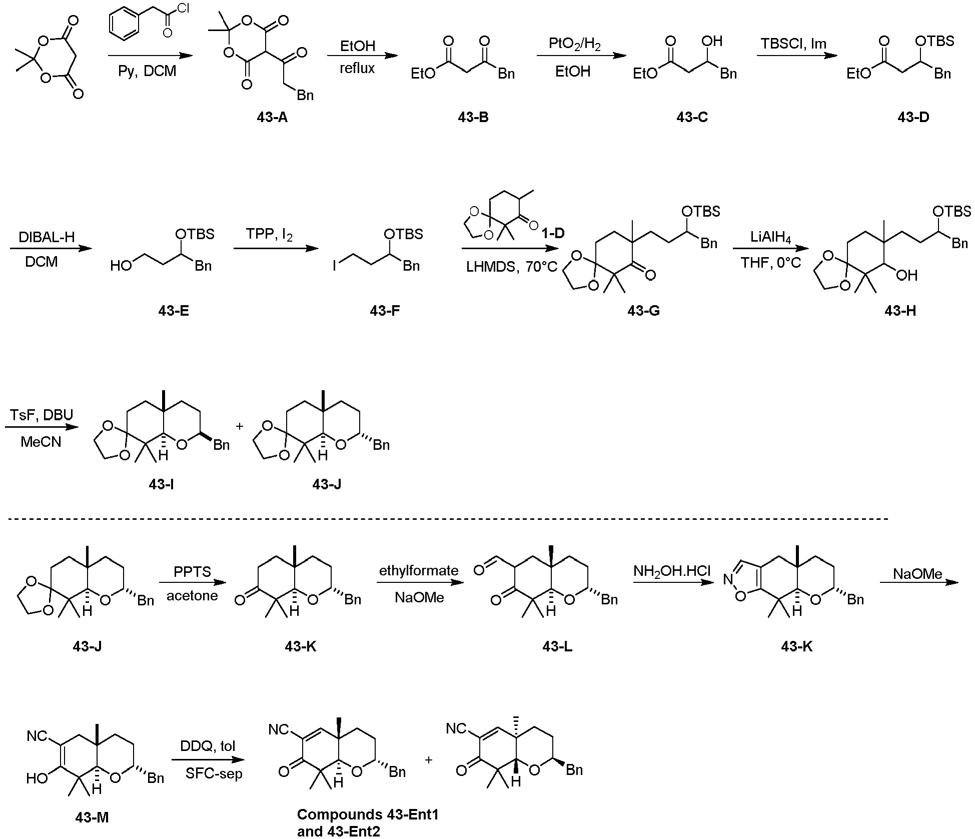

### Preparation of Compound 43-A

The reaction was set into two batches and the work-up was combined together.

The solution of Meldrum's Acid (150 g, 1.04 mol, 1.0 eq) and DIEA (268.7 g, 2.08 mol, 2.0 eq) in 1.5 L of DCM was added compound 2-phenyl acetyl chloride (168 g, 1.09 mol, 1.05 eq) in 300 mL of DCM at 0-5 °C by dropwise. After addition the mixture was stirred at 3-13 °C for 18 h. The mixture was washed with 10% aqueous HCl (500 mL × 2), water (500 mL × 2). The aqueous phase was back extracted with DCM (500 mL × 2). The combined organic layer was dried over anhydrous Na₂SO₄, filtered, concentrated under reduced pressure to provide crude compound **43-A** (272 g+270 g) as pale-yellow solid used for next step directly.

### Preparation of Compound 43-B

Compound **43-A** (272 g, 1.04 mol, 1.0 eq) was added into 2.0 L of anhydrous EtOH. The mixture was heated to reflux for 12 h. TLC (PE: EA = 6:1) showed one main new spot was observed. The solution was concentrated and purified by column chromatography on silica gel (PE: EA = 100:1-80:1-60:1-30:1) to supply compound **43-B** (165 g, 77% yield) as yellow liquid.
**¹HNMR: (400 MHz, CDCl₃)** δ: 7.44 - 7.20 (m, 5H), 4.22 (q, *J* = 7.0 Hz, 2H), 3.88 (s, 2H), 3.50 (s, 2H), 1.33 - 1.29 (m, 3H).

### Preparation of Compound 43-C

The reaction was set into two batches and the work-up was combined together.

To the solution of compound **43-B** (50 g, 0.243 mol, 1.0 eq) and TEA (0.2 mL) in 400 mL of EtOH was added PtO₂ (636 mg, 2.43 mmol, 0.01 eq) under Ar atmosphere. The reaction mixture was degassed with Ar for 3 times and H₂ for 3 times. Then the mixture was stirred at 50°C under H₂ (50 psi). TLC (PE: EA = 5:1) showed compound **43-B** remained about 30%. The mixture was filtered through a pad of celite, the pad was washed with EtOAc (300mL × 2). The combined filtrate was concentrated and purified by column chromatography on silica gel (PE: EA = 100:1-80:1-50:1) to supply compound **43-C** (60 g, 59% yield) as yellow liquid.
**¹HNMR: (400 MHz, CDCl₃)** δ: 7.36 - 7.15 (m, 1H), 4.31 - 4.22 (m, 1H), 4.15 (q, *J* = 7.1 Hz, 2H), 2.99 (d, *J* = 3.3 Hz, 1H), 2.92 - 2.83 (m, 1H), 2.80 - 2.73 (m, 1H), 2.56 - 2.39(m, 2H), 1.26 (t, *J* = 7.2 Hz, 3H).

### Preparation of Compound 43-D

To the solution of compound **43-C** (60 g, 0.288 mol, 1.0 eq) in DMF (600 mL) was added imidazole (39.2 g, 0.576 mol, 2.0 eq.) and TBSCl (64.9 g, 0.433 mol, 1.5 eq) under ice-bath. After addition the reaction mixture was stirred at 10-18 °C for 18 h. TLC (PE: EA = 5:1) showed the starting material was consumed. The mixture was poured into water (1.0 L), extracted with MTBE (500 mL x 2). The combined organic layer was washed with brine (500 mL) and water (300 mL), dried with anhydrous Na₂SO₄, filtered and concentrated. The residue was purified by column chromatography on silica gel (PE: EA = 200:1∼100:1∼80:1) to supply compound **43-D** (89 g, 95.8% yield) as yellow liquid.
**¹HNMR: (400 MHz, CDCl₃)** δ: 7.42 - 7.30 (m, 5H), 4.45 (t, *J* = 6.3 Hz, 1H), 4.26 - 4.22 (m, 2H), 2.94 - 2.92 (m, 2H), 2.54 - 2.53 (m, 2H), 1.38 (t, *J* = 7.0 Hz, 3H), 0.97 (s, 9H), 0.11 (s, 3H), 0.00 (s, 3H).

### Preparation of Compound 43-E

The reaction was set into two batches and the work-up was combined together.

To the solution of compound **43-D** (44 g, 0.137 mol, 1.0 eq) in DCM (700 mL) was added DIBAL-H (409 mL, 409 mmol, 3.0 eq, 1M in toluene) at -70°C by dropwise (about 2.5 h). After addition the mixture was allowed to warm to 0°C and stirred for 1h. TLC (PE: EA = 5:1) indicated completion. The mixture was quenched by adding water (16.36 mL) slowly under ice-bath, then 15% NaOH aq (36 mL) and water (41 mL). The suspension was diluted with DCM (1.5 L) and stirred with Na₂SO₄ (200 g) for 3h, filtered, concentrated and purified by column chromatography on silica gel ((PE: EA = 80:1∼60:1∼30:1)) to supply compound **43-E** (50 g, 65.2% yield) as yellow liquid.
**¹HNMR: (400 MHz, CDCl₃)** δ: 7.41 - 7.34 (m, 2H), 7.33 - 7.22 (m, 3H), 4.25 - 4.17 (m, 1H), 3.98-3.97 (m ,1H), 3.83-3.81 (m, 1H), 3.02 - 2.97 (m, 1H), 2.89- 2.84 (m, 1H), 1.95 - 1.84 (m, 1H), 1.76 - 1.66 (m, 1H), 0.99 (s, 9H), 0.16 (s, 3H), 0.01 (s, 3H).

### Preparation of Compound 43-F

To a solution of triphenylphosphine (TPP) (41.17 g, 0.157 mol, 1.1 eq) in DCM (800 mL) was added I₂ (39.85 g, 0.157 mol, 1.1 eq.). The dark-red solution was stirred at 16°C for 30 min, and then imidazole (29.17 g, 0.429 mol, 3.0 eq) was added by portion-wise. The yellow solution was stirred at 16°C for 10 min. Then a solution of compound **43-E** (40 g, 0.143 mol, 1.0 eq) in DCM (100 mL) was added by dropewise. After addition the mixture was stirred at 16°C for 2.5 h. TLC (PE: EtOAc = 6:1) showed the starting material was consumed. The reaction was diluted with DCM (500 mL), washed with Sat. NaHCO₃ (500 mL) and water (500 mL), dried over anhydrous Na₂SO₄, filtered and concentrated. The residue was purified by column chromatography on silica gel with PE to give product **43-F** (53 g, 95% yield) as colorless liquid.
**¹HNMR: (400 MHz, CDCl₃)** δ: 7.41 - 7.24 (m, 5H), 4.07 - 4.01 (m, 1H), 3.36 - 3.25 (m, 2H), 2.93 - 2.78 (m, 2H), 2.09 - 1.96 (m, 2H), 0.98 (s, 9H), 0.16 (s, 3H), 0.02 (s, 3H).

### Preparation of Compound 43-G

To a solution of compound **1-D** (10 g, 50.5 mmol, 1.0 eq.) in THF (100 mL) was added LHMDS (101 mL, 101 mmol, 2.0 eq. 1.0 M in THF) by dropwise at 18°C. After stirring for 1 h, the mixture was treated with compound **43-F** (23.63 g, 60.6 mmol, 1.2 eq.) in THF (20 mL) by drop-wise. The mixture was stirred at 70°C for 18 hours. TLC (PE: EA = 10:1) showed compound **1-D** was consumed completely. The reaction mixture was poured into 300 mL of Sat. NH₄Cl (500 mL), extracted with EtOAc (100 mL x 2). The combined organic phase was washed with brine (100 mL), water (100 mL), dried over Na₂SO₄, filtered and concentrated. The residue was purified by column chromatography on silica gel ((PE: EA = 300:1∼150:1∼90:1)) to supply compound **43-G** (20 g, 86.1% yield) as yellow liquid.
**LCMS:** (M+H:461.3)
**¹H NMR (400MHz, CDCl₃)** δ:7.50 - 7.38 (m, 5H), 4.23 - 4.13 (m, 4H), 4.07 - 3.98 (m, 1H), 3.00 - 2.86 (m, 2H), 2.25 - 1.61 (m, 8H), 1.38 - 1.27 (m, 9H), 1.13 (m, 9H), 0.26 - 0.11 (m, 3H), 0.06 - 0.05 (m, 3H).

### Preparation of Compound 43-H

To a solution of compound **43-G** (18 g, 39.13 mmol, 1.0 eq.) in THF (200 mL) was added LiAlH₄ (3.47 g, 89.4 mmol, 2.3 eq.) at 0°C by dropwise. After addition the mixture was allowed to warm to room temperature and stirred for 5h. TLC (PE: EA = 10:1) showed little compound **43-G** remaining. The reaction mixture was diluted with THF (600 mL), quenched by adding water (3.47 mL) slowly under ice-bath, then 15% NaOH aq (3.47 mL) and water (10.4 mL), then stirred with Na₂SO₄ (35 g) overnight. The suspension was filtered through a pad of celite, the cake was washed with EtOAc (500 mL) for twice and filtered. The combined filtrate was concentrated and purified by column chromatography on silica gel ((PE: EA = 150:1-100:1-80:1-40:1) to supply compound **43-H** (10 g, 55.6% yield) as colorless gum.
**LCMS:** (M+H:463.4)
**¹H NMR (400MHz,CDCl₃)** δ: 7.56 - 7.49 (m, 2H), 7.45 - 7.43 (m, 3H), 4.21 - 4.20 (m, 4H), 4.09 (m, 1H), 3.55 - 3.45 (m, 1H), 3.01 - 2.94 (m, 2H), 1.85 - 1.31 (m, 8H), 1.24 - 1.11 (m, 9H), 1.10 (s, 9H), 0.21 - 0.20 (m, 3H), 0.04 -0.00 (m, 3H).

### Preparation of Compounds 43-I and 43-J

To a solution of compound **43-H** (9.0 g, 19.48 mmol, 1.0 eq.) in toluene (60 mL) was added TsF (6.78 g, 38.96 mmol, 2.0 eq.) and DBU (5.92 g, 38.96 mmol, 2.0 eq.). The mixture was stirred at 120°C for 18 hours. TLC (PE: EA = 10:1) showed compound **43-H** was largely consumed and several spots were detected. The mixture was concentrated and purified by column chromatography on silica gel (silica: 300-400. elute: PE: EA = 100:1-80:1-60:1) to supply compound **43-1** (racemic, 400 mg) as colorless gum and compound **43-J** (racemic, 1.5g, impure) as pale-yellow gum.
**¹HNMR: (400 MHz, CDCl₃) (43-1)** δ: 7.54 - 7.37 (m, 5H), 4.46 - 4.16 (m, 1H), 4.15-4.09(m,4H),3.57 (s, 1H), 3.23-3.22 (m, 1H), 3.19 - 3.13 (m, 1H), 2.05 - 1.17 (m, 2H), 1.54 - 1.43 (m, 8),1.23 (s, 3H), 1.14(s, 3H), 1.01(s, 31H).
**¹HNMR: (400 MHz, CDCl₃) (43-J)** δ:7.26 - 7.12 (m, 5H), 3.98 - 3.85 (m, 4H), 3.56 - 3.54 (m, 1H), 2.91 (s, 1H), 2.89 - 2.87 (m, 1H), 2.75-2.70 (m, 1H), 1.88-1.83 (m, 1H), 1.54 - 1.29 (m, 7H), 0.99 (s, 6H), 0.83 (s, 3H).

### Preparation of Compound 43-K

To a solution of compound **43-J** (1.5 g, 1.14 mmol, 1.0 eq, 25%) in acetone/H₂O (10 mL, v/v = 9:1), was added PPTS (232 mg, 0.91 mmol, 0.8 eq.). The resulting mixture was stirred at 85°C for 18 hrs. LCMS showed the reaction was completed. The mixture was concentrated to remove acetone; the aqueous layer was diluted with water (10 mL) and extracted with EtOAc (8 mL x 2). The combined organic layers were dried over Na₂SO₄, filtered and concentrated. The residue was purified by prep-TLC gel (PE: EA =12:1) for twice to give compound **43-K** (260 mg, 79.8%) as pale-yellow gum.
**LCMS:** (M+H:287.2)

### Preparation of Compound 43-L

To a solution of compound **43-K** (260 mg, 0.91 mmol, 1.0 eq) in 1.44 mL of ethyl formate (1.35 g, 18.2 mmol, 20.0 eq) was added NaOMe (0.95 mL, 3.18 mmol, 3.5 eq, 5.4 M in MeOH) under ice-bath. After addition the mixture was stirred at 25 °C under N₂ atmosphere for 18h. TLC (PE/EA = 10:1) indicated the completion. The mixture was added into 15 mL of ice-water, extracted with EtOAc (10 mL×3). The combined organic layer was dried over anhydrous Na₂SO₄, filtered, concentrated under reduced pressure to supply crude compound **43-L** (301 mg) as pale-yellow gum.

### Preparation of Compound 43-M

To a solution of compound **43-L** (301 mg, 0.95 mmol, 1.0 eq) in 11 mL of EtOH/H₂O (V: V = 10:1) was added hydrochloride hydroxylamine (81 mg, 1.15 mmol, 1.2 eq). After addition the mixture was stirred at 50 °C for 18h. TLC (PE: EA = 10:1) indicated the completion. The mixture was concentrated, the residue was diluted with water (20 mL), extracted with EtOAc (15 mL×3). The combined organic layer was concentrated and purified by prep-TLC (PE: EA = 10:1) for twice to supply compound **43-M** (200 mg. 67.8% yield) as pale-yellow gum.
**LCMS:** (M+H:312.8)
**¹HNMR: (400 MHz, CDCl₃)** δ: 7.95 (s, 1H), 7.27 - 7.18 (m, 5H), 3.53-3.51 (m, 1H), 3.12 (s, 1H), 2.88 - 2.86 (m, 1H), 2.76 - 2.75 (m, 1H), 2.13 (d*, J* = 16 Hz, 1H), 2.09 (d, *J* = 15.6 Hz, 1H), 1.62-1.44 (m, 5H), 1.23 (*J* = 7.2 Hz, 6H), 0.92 (s, 3H).

### Preparation of Compound 43-N

To a solution of compound **43-M** (200 mg, 0.64 mmol, 1.0 eq) in 2 mL of anhydrous methanol was added NaOMe (1.29 mL, 2.57 mmol, 4.0 eq, 2 M in methanol). The mixture was stirred at 16 °C ∼22 °C for 18 h. TLC (PE/EA = 10:1) showed compound **43-M** was consumed completely. The mixture was concentrated and the residue was portioned into 15 mL of EtOAc and 20 mL of water, the organic layer was separated and the aqueous layer was extracted with EtOAc (15 mL × 2). The combined organic layer was dried over anhydrous Na₂SO₄, filtered, concentrated under reduced pressure to supply crude compound **43-N** (190 mg) as off-white solid.

### Preparation of Compounds 43-Entl and 43-Ent2

Racemic Compound **43-N** (190 mg, 0.61 mmol, 1.0 eq) and DDQ (166 mg, 0.73 mmol, 1.2 eq) were added into 6 mL of anhydrous toluene. The mixture was stirred at 60 °C for 1 h. TLC (PE/ EtOAc = 8:1) showed only one spot was observed. The mixture was concentrated under reduced pressure and purified by prep-TLC (PE: EtOAc = 8:1) for twice to supply compound racemic product (140 mg, 74.2% yield) as white solid.
**¹HNMR: (400 MHz, CDCl₃)** δ: 7.41 (s, 1H), 7.33 - 7.28 (m, 2H), 7.26 - 7.21 (m, 3H), 3.68 - 3.59 (m, 1H), 3.26 (s, 1H), 2.98 - 2.90 (m, 1H), 2.80 (dd, *J* = 5.0, 13.8 Hz, 1H), 1.75 - 1.59 (m, 4H), 1.30 (s, 3H), 1.17 (s, 3H), 1.14 (s, 3H).

The racemic mixture was further separated by SFC (column: AD 250 mm x 30 mm, 5 um, condition: Neu-EtOH), (purified twice) to give **43-Ent1** (Rt = 2.436 min, 24.0 mg, 17.1% yield) as white solid and **43-Ent2** (Rt = 2.620 min, 34 mg, 24.3% yield) as white solid.

### Spectra for 43-Ent1

**¹HNMR: (400 MHz, CDCl₃)** δ: 7.41 (s, 1H), 7.33 - 7.28 (m, 2H), 7.26 - 7.21 (m, 3H), 3.68 - 3.59 (m, 1H), 3.26 (s, 1H), 2.98 - 2.90 (m, 1H), 2.80 (dd, *J* = 5.0, 13.8 Hz, 1H), 1.75 - 1.59 (m, 4H), 1.30 (s, 3H), 1.17 (s, 3H), 1.14 (s, 3H).
**SFC:** (ee: 98.03%)
**HPLC:** (Purity: 96.71%)
**MS:** (M+H: 310.1)

### Spectra for 43-Ent2

**¹HNMR: (400 MHz, CDCl₃)** δ: 7.41 (s, 1H), 7.32 - 7.28 (m, 2H), 7.26 - 7.22 (m, 3H), 3.69 - 3.59 (m, 1H), 3.26 (s, 1H), 2.98 - 2.90 (m, 1H), 2.80 (dd, *J* = 5.0, 13.8 Hz, 1H), 1.76 - 1.59 (m, 4H), 1.30 (s, 3H), 1.17 (s, 3H), 1.14 (s, 3H).
**SFC:** (ee: 97.908%)
**HPLC:** (Purity: 97.43%)
**MS:** (M+H: 310.1)

### Example 16. Synthesis of Compounds 44-Entl and 44-Ent2

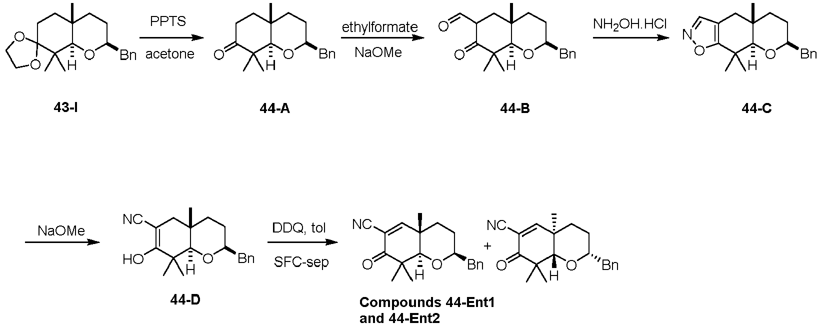

### Preparation of Compound 44-A

To a solution of compound **43-1** (400 mg, 1.21 mmol, 1.0 eq) in acetone/H₂O (10 mL, v/v = 9:1) was added PPTS (184 mg, 0.73 mmol, 0.6 eq.). The resulting mixture was stirred at 85°C for 18 hrs. TLC (PE: EA = 9:1) indicated the completion. The mixture was concentrated to remove acetone. The aqueous layer was diluted with water (10 mL) and extracted with EtOAc (8 mL x 2). The combined organic layers were dried over Na₂SO₄, filtered and concentrated. The residue was purified by prep-TLC gel (PE: EA =8:1) to give compound **44-A** (200 mg, 57.8%) as pale-yellow gum.
**LCMS:** (M+H:287.1)

### Preparation of Compound 44-B

To a solution of compound **44-A** (200 mg, 0.7 mmol, 1.0 eq) in 1.1 mL of ethyl formate (1.03 g, 14 mmol, 20.0 eq) was added NaOMe (0.45 mL, 2.45 mmol, 3.5 eq, 5.4 M in MeOH) under ice-bath by dropwise. After addition the mixture was stirred at 20-26 °C under N₂ atmosphere for 18h. TLC (PE/EA = 10:1) indicated the completion. The mixture was added into 15 mL of ice-water, extracted with EtOAc (10 mL×3). The combined organic layer was dried over anhydrous Na₂SO₄, filtered, concentrated under reduced pressure to supply crude compound **44-B** (200 mg) as pale-yellow gum.

### Preparation of Compound 44-C

To a solution of compound **44-B** (200 mg, 0.64 mmol, 1.0 eq) in 11 mL of EtOH/H₂O (V: V = 10:1) was added hydrochloride hydroxylamine (54 mg, 0.76 mmol, 1.2 eq). After addition the mixture was stirred at 50 °C for 18h. TLC (PE: EA = 10:1) indicated the completion. The mixture was concentrated, the residue was diluted with water (15 mL), extracted with EtOAc (10 mL x 3). The combined organic layer was concentrated and purified by prep-TLC (PE: EA = 10:1) for twice to supply compound **44-C** (150 mg. 75.4% yield) as white solid.
**LCMS:** (M+H:312.8)
**¹HNMR: (400 MHz, CDCl₃)** δ: 7.97 (s, 1H), 7.27 - 7.17 (m, 5H), 4.38 - 3.32 (m, 1H), 3.52 (s, 1H), 3.00 - 2.90 (m, 2H), 2.29 - 2.24 (m, 2H), 2.01 - 1.96 (m, 1H), 1.74 - 1.73 (m, 1H), 1.53 - 1.52 (m, 1H), 1.51 - 1.41 (m, 1H), 1.17 (s, 6H), 0.98 (s, 3H).

### Preparation of Compound 44-D

To a solution of compound **44-C** (150 mg, 0.48 mmol, 1.0 eq) in 2 mL of anhydrous methanol was added NaOMe (0.96 mL, 1.93 mmol, 4.0 eq, 2 M in methanol). The mixture was stirred at 16 ∼ 22°C for 18 h. TLC (PE/EA = 10:1) showed compound **44-C** was consumed completely. The mixture was concentrated and the residue was diluted with water (30 mL), extracted with EtOAc (25 mL × 2). The combined organic layer was dried over anhydrous Na₂SO₄, filtered, concentrated under reduced pressure to supply crude compound **44-D** (150 mg) as white solid.

### Preparation of Compounds 44-Entl and 44-Ent2

Compound **44-D** (150 mg, 0.48 mmol, 1.0 eq) and DDQ (131 mg, 0.52 mmol, 1.2 eq) were added into 6 mL of anhydrous toluene. The mixture was stirred at 60 °C for 1.5 h. TLC (PE: EA = 8:1) showed only one spot was observed. The mixture was concentrated under reduced pressure and purified by prep-TLC (PE: EA = 8:1) for twice to supply racemic product (100 mg, 67.6% yield) as white solid.
**¹HNMR: (400 MHz, CDCl₃)** δ: 7.45 (s, 1H), 7.34 - 7.28 (m, 2H), 7.26 - 7.17 (m, 3H), 4.45 - 4.37 (m, 1H), 3.67(s, 1H), 3.04 - 2.91 (m, 2H), 2.13 - 2.11 (m, 1H), 1.89 - 1.87 (m, 1H), 1.63-1.58 (m, 1H), 1.56 - 1.50 (m, 1H), 1.37 (s, 3H), 1.09 (d, *J* = 2.8 Hz, 6H).

The racemic product was further separated by SFC (column: OJ 250mm * 30mm, 5um, condition: 0.1% NH₃.H₂O - EtOH) for twice to give **44-Ent1** (Rt = 3.697 min, 39.0 mg, 39% yield) as white solid and **44-Ent2** (Rt = 4.520 min, 31 mg, 31% yield) as white solid.

### Spectra for 44-Ent1

**¹HNMR: (400 MHz, CDCl₃)** δ: 7.45 (s, 1H), 7.34 - 7.28 (m, 2H), 7.26 - 7.17 (m, 3H), 4.45 - 4.37 (m, 1H), 3.67(s, 1H), 3.04 - 2.91 (m, 2H), 2.13 - 2.11 (m, 1H), 1.89 - 1.87 (m, 1H), 1.63-1.58 (m, 1H), 1.56 - 1.50 (m, 1H), 1.37 (s, 3H), 1.09 (d, *J* = 2.8 Hz, 6H).
**SFC:** (ee: 100%)
**HPLC:** (Purity: 100%)
**MS:** (M+H: 310.1)

### Spectra for 44-Ent2

**¹HNMR: (400 MHz, CDCl₃)** δ: 7.45 (s, 1H), 7.34 - 7.28 (m, 2H), 7.26 - 7.17 (m, 3H), 4.45 - 4.37 (m, 1H), 3.67(s, 1H), 3.04 - 2.91 (m, 2H), 2.13 - 2.11 (m, 1H), 1.89 - 1.87 (m, 1H), 1.63-1.58 (m, 1H), 1.56 - 1.50 (m, 1H), 1.37 (s, 3H), 1.09 (d, *J* = 2.8 Hz, 6H).
**SFC:** (ee: 100%)
**HPLC:** (Purity: 100%)
**MS:** (M+H: 310.1)

### Example 17. Synthesis of Compounds 45-Entl and 45-Ent2

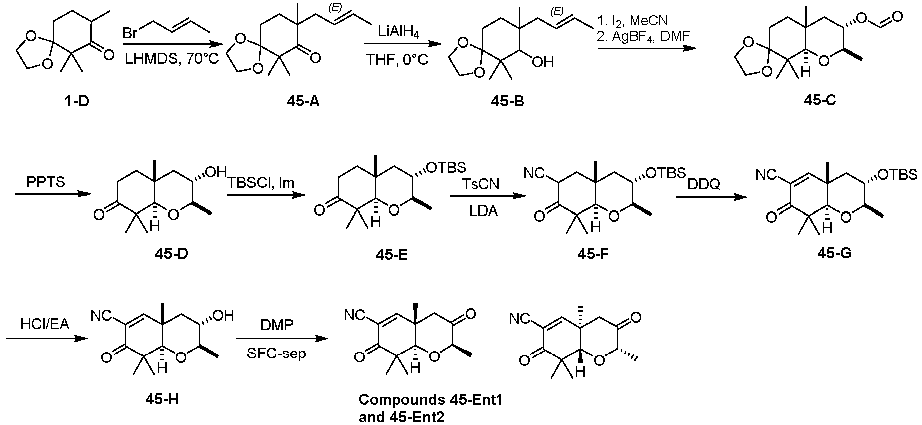

### Preparation of Compound 45-A

To a solution of compound 1-D (25 g, 0.13 mol, 1eq) in THF (25 mL) was added LiHMDS (260 mL, 0.26 mol, 2eq, 1M). The mixture was stirred at 20°C for 1 hour. Then a solution of (*E*)-1-bromobut-2-ene (21g, 0.15 mol, 1.2 eq) in THF (10 mL) was added. The mixture was stirred at 70°C for 16 hours. TLC (PE : EA = 10:1) showed a little starting material still remained and a new main spot was detected (R_{f} = 0.6). The mixture was quenched with aq. NH₄Cl (500 mL) and extracted with EA (100 mL x 3). The combined organic phase was dried over Na₂SO₄, and filtered. The filtrate was concentrated *in vacuo* to give the residue, which was purified by column chromatography on silica gel (PE: EA = 30:1) to give compound **45-A** (25 g, 78%) as yellow oil.
**¹H NMR** (400MHz, CDCl₃) δ 5.48 - 5.37 (m, 1H), 5.32 - 5.21 (m, 1H), 3.93 (m, 4H), 2.28 - 2.15 (m, 2H), 1.94 - 1.86 (m, 2H), 1.82 - 1.72 (m, 1H), 1.67 - 1.60 (m, 3H), 1.58 - 1.51 (m, 1H), 1.17 - 1.01 (m, 9H).

### Preparation of Compound 45-B

To a solution of compound **45-A** (25 g, 0.11 mol, 1eq) in THF (270 mL) was added LiAlH₄ (5.6 g, 0.15 mol, 1.3eq) portion wise at 0°C. The mixture was stirred at 15°C for 2 hours. The mixture turned to a suspension. TLC (PE: EA = 10:1) showed the reaction was completed. Most of the starting material was almost consumed (R_{f} = 0.5) and two new spots were detected (R_{f1} = 0.3, R_{f2} = 0.4). The reaction mixture was quenched with water (5.6 mL), aq.NaOH (15%, 5.6 mL) followed by water (5.6 mL) and stirred for further 30min. Then the mixture was filtered and the filtrate was concentrated *in vacuo* to give the residue, which was purified by column chromatography on silica gel (PE: EA = 50:1) to give compound **45-B** (14 g, 51%) as yellow oil.
**¹H NMR** (400MHz,CDCl₃) δ 5.62 - 5.41 (m, 2H), 3.99 - 3.84 (m, 4H), 3.35 - 3.13 (m, 1H), 2.36 - 1.85 (m, 3H), 1.82 - 1.71 (m, 1H), 1.65 - 1.50 (m, 3H), 1.48 - 1.37 (m, 1H), 1.34 - 1.20 (m, 1H), 1.09 - 0.88 (m, 9H).

### Preparation of Compound 45-C

To a solution of compound **45-B** (2.4 g, 10 mmol, 1eq) in MeCN (50 mL) was added I₂ (6.3 g, 25 mmol, 2.5eq) at 0°C. The reaction was stirred in the dark at 0°C for 5 hours. The mixture turned to a black-brown suspension. TLC (PE: EA = 10:1) showed the reaction was completed. Most of the starting material was almost consumed (R_{f} = 0.5) and several spots were detected (R_{f} = 0.6-0.9). The mixture was diluted with EA (50 mL) and washed with Sat. Na₂SO₃ (100 mL), then dried over Na₂SO₄, and filtered, concentrated *in vacuo* to give the residue. The residue was dissolved in DMF (40 mL) and AgBF₄ (2.9 g, 15 mmol, 1.5 eq) was added. The mixture was stirred at 70°C for 16 hours. TLC (PE: EA = 5:1) showed several spots were detected (R_{f} = 0.2-0.6). The mixture was poured into water (10 ml) and extracted with EA (10 mL x 3). The combined organic phase was dried over Na₂SO₄, and filtered. The filtrate was concentrated *in vacuo* to give the residue, which was purified by column chromatography on silica gel (PE: EA = 10:1) to give compound **45-C** (380 mg, 13%) as colorless oil.
**¹H NMR** (400MHz, CDCl₃) δ 8.02 (s, 1H), 4.82 - 4.71 (m, 1H), 3.98 - 3.86 (m, 4H), 3.44 - 3.32 (m, 1H), 3.01 (s, 1H), 1.46 - 1.31 (m, 2H), 1.19 (d, *J* = 6.0 Hz, 3H), 1.08 (s, 3H), 0.94 - 0.92 (m, 6H).

### Preparation of Compound 45-D

To a solution of compound **45-C** (380 mg, 1.27 mmol, 1eq) in acetone (9 mL) and water (1 mL) was added PPTS (128 mg, 0.51 mmol, 0.4 eq). The mixture was stirred at 75°C for 16 hours. TLC (PE: EA = 5:1) showed the reaction was completed. Most of the starting material was almost consumed and a new spot was detected. The mixture was diluted with water (10 mL) and extracted with EA (25 mL x 3). The combined organic phase was dried over Na₂SO₄, filtered and concentrated under reduced pressure. The residue was purified by column chromatography on silica gel (PE: EA = 5:1) to give compound **45-D** (220 mg, 76.7%) as a white solid.
**¹H NMR** (400MHz, CDCl₃) δ 3.52 - 3.46 (m, 1H), 3.18 - 3.10 (m, 1H), 2.91 (s, 1H), 2.71 - 2.62 (m, 1H), 2.26 - 2.21 (m, 1H), 1.85 - 1.84 (m, 1H), 1.68 - 1.63 (m, 1H), 1.43 - 1.31 (m, 1H), 1.31 (d, *J* = 6.0 Hz, 1H), 1.177 (m, 4H), 1.125 (s, 3H), 1.047 (s, 3H).

### Preparation of Compound 45-E

To a solution of compound **45-D** (100 mg, 0.44 mmol, 1eq) and imidazole (60 mg, 0.88 mmol, 2eq) in DMF (5mL) was added TBSCl (100 mg, 0.66 mmol, 1.5eq). The mixture was stirred at 80°C for 16 hours. TLC (PE: EA = 3:1) showed the starting material was almost consumed and a new spot was detected (R_{f} = 0.8). The mixture was quenched with aq.NaHCO₃ until pH = 8. The mixture was extracted with EA (10 mL x3). The combined organic phase was dried over Na₂SO₄, and filtered. The filtrate was concentrated *in vacuo* to give the residue, which was purified by column chromatography on silica gel to give compound **45-E** (170 mg, crude) as colorless oil.
**¹H NMR** (400MHz, CDCl₃) δ 3.50 - 3.47 (m, 1H), 3.20 - 3.11 (m, 1H), 2.92 (s, 1H), 2.67 - 2.62 (m, 1H), 2.28 - 2.19 (m, 1H), 1.75 - 1.71 (m, 1H), 1.65- 1.60 (m, 1H), 1.42 - 1.34 (m, 1H), 1.24 (d, *J*=6.1 Hz, 3H), 1.19 - 1.16 (s, 3H), 1.12 (s, 3H), 1.05 (s, 3H), 0.90 - 0.83 (m, 9H), 0.07 - 0.02 (m, 6H).

### Preparation of Compound 45-F

To a solution of compound **45-E** (170 mg, 0.5 mmol, 1eq) in THF (5 mL) was added LDA (0.3 mL, 0.6 mmol, 1.2 eq) at -78°C for 1 hour, then a solution of TsCN (108 mg, 0.6 mmol,1.2 eq) in THF (5 mL) was added. The mixture was stirred at -78°C for 3 hours. TLC (PE: EA = 3:1) showed the starting material still remained and a new spot was detected (R_{f} = 0.2). The mixture was quenched with aq.NH₄Cl (10 mL) and extracted with EA (10 mL x 3). The combined organic phase was dried over Na₂SO₄ and filtered. The filtrate was concentrated *in vacuo* to give the crude compound **45-F** (180 mg, crude), which was used directly in the next step.

### Preparation of Compound 45-G

To a solution of compound **45-F** (180 mg, 0.5 mmol, 1eq) in toluene (5 mL) was added DDQ (113 mg, 0.5 mmol, 1eq). The mixture was stirred at 50°C for 1 hour. The mixture turned to a suspension. TLC showed the reaction was completed. The mixture was concentrated *in vacuo* to give the residue, which was purified by column chromatography on silica gel to give compound **45-G** (50 mg, 28%).
**¹H NMR** (400MHz, CDCl₃) δ 7.35 (s, 1H), 3.52 - 3.46 (m, 1H), 3.25 (s, 1H), 3.23 - 3.16 (m, 1H), 1.83 (dd, *J* = 4.9, 12.1 Hz, 1H), 1.45 - 1.41 (m, 1H), 1.33 - 1.12 (m, 9H), 1.03 (s, 3H), 0.84 - 0.78 (m, 9H), 0.06 - 0.04 (m, 6H).

### Preparation of Compound 45-H

To a solution of compound **45-G** (40 mg, 0.11 mmol, 1eq) in EA (1 mL) was added HCl/EA (1 mL, 4M). The mixture was stirred at 18°C for 3 hours. The mixture turned to clear. TLC (PE: EA = 5:1) showed the reaction was completed. Most of the starting material was almost consumed and a new spot was detected. The mixture was concentrated *in vacuo* to give the crude compound **45-H** (35 mg, crude), which was used directly in the next step without purification.

### Preparation of Compounds 45-Entl and 45-Ent2

To a solution of compound **45-H** (35 mg, 0.14 mmol, 1eq) in DCM (5mL) was added Dess-martin reagent (119 mg, 0.28mmol, 2eq). The mixture was stirred at 25°C for 16 hours. The mixture turned to a white suspension. TLC (PE: EA = 3:1) showed the reaction was completed. Most of the starting material was almost consumed (R_{f}= 0.3) and a new main spot was detected (R_{f} = 0.5). The mixture was diluted with DCM (10 mL) and neutralized with aq.NaHCO₃ until pH = 7. The aqueous layer was extracted with DCM (10 mL x 3). The combined organic phase was dried over Na₂SO₄, and filtered. The filtrate was concentrated *in vacuo* to give the residue, which was purified by pre-TLC (PE: EA = 3:1), then separated by SFC (AD 250 mm x 30 mm, 5um, Neu-MeOH) and re-purified by pre-TLC (PE:EA = 3:1) to give **45-Ent1** (4.0 mg, Rt=1.912 min) and **45-Ent2** (3.1 mg, Rt =2.285 min), both as oil.

### Spectra for 45-Ent1

**¹H NMR** (400MHz, CDCl₃) δ 7.29 (s, 1H), 3.97 (q, *J* = 6.8 Hz, 1H), 3.73 (s, 1H), 2.47 (s, 2H), 1.34 (d, *J* = 6.8 Hz, 3H), 1.31 (s, 3H), 1.26 (s, 3H), 1.11 (s, 3H).
**SFC:** (ee: 95.7%)
**HPLC:** (Purity: 95.26%)
**MS:** (M+H 248.1)

### Spectra for 45-Ent2

**¹H NMR** (400MHz, CDCl₃) δ 7.29 (s, 1H), 3.97 (q, *J* = 6.8 Hz, 1H), 3.73 (s, 1H), 2.47 (s, 2H), 1.34 (d, *J* = 6.8 Hz, 3H), 1.31 (s, 3H), 1.26 (s, 3H), 1.11 (s, 3H).
**SFC:** (ee: 97.5%)
**HPLC:** (Purity: 100%)
**MS:** (M+H 248.1)

### Example 18. Synthesis Compound 54

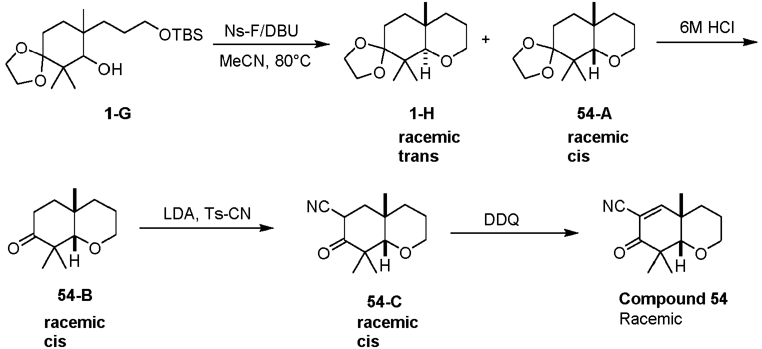

### Preparation of Compound 54-A

To a reaction vessel containing [**1-G**] 8-(3-((tert-butyldimethylsilyl)oxy)propyl)-6,6,8-trimethyl-1,4-dioxaspiro[4.5]decan-7-ol (725 mg, 1.94 mmol) in Acetonitrile (10 mL) was added Nosyl fluoride (0.44 g, 2.1 mmol) followed by 1,8-Diazabicyclo[5.4.0]undec-7-ene (0.36 g, 2.3 mmol). The reaction was heated to 70°C for 5h, then allowed to stir at RT overnight. The solvent was removed in vacuo and the residue was partioned between ethyl acetate and ∼ 0.1N HCL. The layers were separated layers and the organic phase was with brine, dried over MgSO4, filtered, and concentrated. The residue was deposited on 600 mg silica gel and purified by column chromatography using a 4g Redisep Gold ™ cartridge eluted with 5% ethyl acetate in heptane.. Isolated both cis (**54-A, 65 mg**) and trans isomers (1-H) of product.
**LCMS** for 54-A (M+H) = 241.1
**¹H NMR** (400 MHz, CHLOROFORM-*d*) δ ppm 4.10 - 4.19 (m, 1 H) 3.98 - 4.10 (m, 2 H) 3.78 - 3.93 (m, 2 H) 3.14 (ddd, *J*=12.4, 11.2, 2.3 Hz, 1 H) 2.86 (d, *J*=1.8 Hz, 1 H) 2.24 (td, *J*=13.6, 3.9 Hz, 1 H) 1.75 - 1.96 (m, 2 H) 1.57 - 1.64 (m, 1 H) 1.37 - 1.52 (m, 2 H) 1.26 - 1.35 (m, 1 H) 1.16 (s, 3 H) 1.05 (s, 3 H) 0.98 (s, 3 H) 0.93 - 0.98 (m, 1 H)

### Preparation of Compound 54-B

p-Toluenesulfonic acid monohydrate (5 mg, 0.03 mmol) was added to a solution of **54-A** (65 mg, 0.27 mmol) in 2 mL acetone. The reaction was allowed to stir at RT for 6h, then quenched with sodium bicarbonate solution and extracted with ethyl acetate, dried over MgSO₄, filtered, and concentrated. Purification using 2-5% ethyl acetate in heptanes afforded **54-B.** (39 mg; Yield = 73%)
**¹H NMR** (400 MHz, CHLOROFORM-*d*) δ ppm 4.01 (dd, *J*=11.3, 5.5 Hz, 1 H) 3.16 - 3.32 (m, 1 H) 3.07 (d, *J*=1.8 Hz, 1 H) 2.64 - 2.79 (m, 1 H) 2.47 (td, *J*=13.2, 5.3 Hz, 1 H) 2.31 (ddd, *J*=15.2, 5.3, 2.4 Hz, 1 H) 1.72 - 1.94 (m, 1 H) 1.43 - 1.57 (m, 2 H) 1.30 - 1.40 (m, 2 H) 1.28 (s, 3 H) 1.21 (s, 3 H) 1.11 (s, 3 H)

### Preparation of Compound 54-C

A freshly prepared 1 M solution of Lithium Diisopropylamide in Tetrahydrofuran/hexanes (0.367 mL, 0.367 mmol) was cooled to -78C and treated with **54-B** (48 mg, 0.24 mmol) in 1 mL of THF. AfFter 30 min, p-toluenesulfonyl cyanide (0.0665 g, 0.367 mmol) in 1 mL of THF was added. The reaction was stirred for 30 min and then quenched by addition of 0.5 mL 1N HCL. The reaction was diluted with ethyl acetate and brine. The organic phase was separated and washed with brine, dried over MgSO₄, filtered and concentrated. Purification by silica gel chromatography using 10% ethyl acetate in heptanes afforded a white solid upon standing, **54-C**(40 mg; Yield = 70%) mLCMS (M+H) = 222.

### Preparation of Compound 54

To a solution of **54-C** (40 mg, 0.2 mmol) in 5 mL benzene was added Dichlorodicyanoquinone (0.049 g, 0.22 mmol). The reaction was heated to reflux for 30 min. The reaction was cooled to rt and filtered through Celite ™ and rinsed with 10 mL benzene. The filtrate was concentrated and deposited on 300 mg silica gel. Purification on a 4g silica gel cartridge eluted using 15% ethyl acetate in heptane followed by a second chromatographic purification using 10% acetone in heptane to afford 19 mg of a white solid, compound 54.

### Data for 54:

**¹H NMR** (400 MHz, CHLOROFORM-*d*) δ ppm 1.26 (s, 12 H) 1.29 (s, 13 H) 1.35 (s, 12 H) 1.48 - 1.63 (m, 18 H) 1.65 - 1.78 (m, 4 H) 1.83 - 1.98 (m, 4 H) 3.32 (d, *J*=2.76 Hz, 7 H) 3.88 - 4.03 (m, 4 H) 7.19 (d, *J*=2.76 Hz, 4 H)
**LC/MS:** (M+H 220)
**HPLC:** (Purity: 99.6%)

Compound 54 can be further separated by chiral chromatography to produce the following two enantiomers: (4aS,8aS)-4a,8,8,8a-tetramethyl-7-oxo-3,4,4a,7,8,8a-hexahydro-2H-chromene-6-carbonitrile and (4aR,8aR)-4a,8,8,8a-tetramethyl-7-oxo-3,4,4a,7,8,8a-hexahydro-2H-chromene-6-carbonitrile.

### Example 19. Synthesis Compound 55

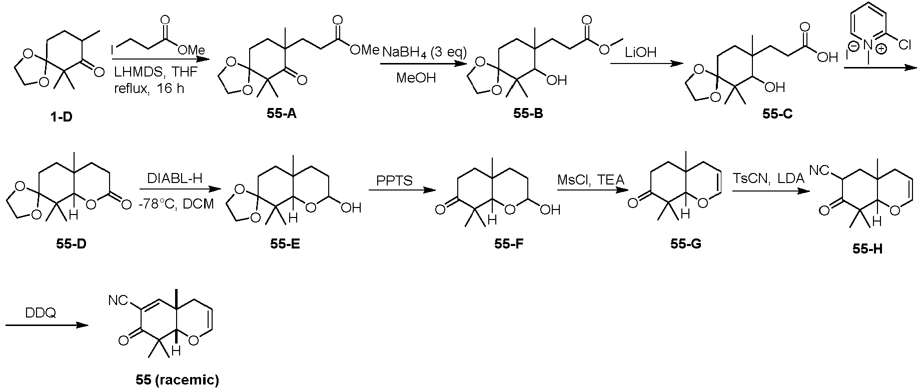

### Preparation of Compound 55-A

To a solution of compound **1-D** (40.2 g, 202.7 mmol, 1.0 eq.) in THF (450 mL) was added LiHMDS (304 mL, 304 mmol, 1.0 M in THF, 1.5 eq.) at 20°C drop-wise through addition funnel. The resulting brown solution was stirred at 20°C for 1 h, the mixture was treated with compound **methyl 3-ioodopropanoate** (65 g, 304 mmol, 1.5 eq.) in THF (50 mL) drop-wise. The resulting brown solution was heated to reflux for 16 hours. TLC (PE/EtOAc = 6:1) showed trace starting material remained. The reaction was quenched with Sat. NH₄Cl (500 mL). After separation, the solvent (THF) was removed under reduce pressure and the water layer was extracted with EtOAc (200 mL x 2). The combined organic layers were washed with water (200 mL x 3), brine (200 mL x 3), dried over Na₂SO₄, filtered and concentrated. The residue was purified by silica gel chromatography eluted with PE/EtOAc = 40:1-20:1 to give compound **55-A** (15 g, 26%) as yellow gum.
**¹HNMR: (400 MHz, CDCl₃)** δ: 3.95 (s, 4H), 3.72 - 3.61 (s, 3H), 2.33 - 2.10 (m, 2H), 2.01 - 1.76 (m, 5H), 1.66 - 1.53 (m, 1H), 1.17 - 1.10 (m, 9H).

### Preparation of Compound 55-B

The reaction was performed in two batches in parallel with the same scale, we will take one for example:
To a solution of compound **55-A** (7.5 g, 26.4 mmol, 1.0 eq.) in MeOH (132 mL) was added NaBH₄ (3 g, 79.2 mmol, 3 eq.) in portions. The clear solution was stirred at 18°C for 5 h. TLC (PE/ EtOAc = 4:1) indicated compound **55-A** still existed. The reaction was acidified by HOAc to pH 5-6. The solvent (MeOH) was removed under reduced pressure then dissolved in EA (150 mL). The solution was washed with Sat. NaHCO₃ (50 mL), dried over sodium sulfate. Filtered and concentrated, the two batches was combined then purified by silica gel chromatography eluted with PE/EtOAc = 20:1 ∼ 1:1 to give compound **55-B** (2.9 g, 19.2%) as yellow oil.
**¹HNMR: (400 MHz, CDCl₃)** δ: 4.01 - 3.89 (m, 4H), 3.66 (s, 3H), 3.16 (br. s., 1H), 2.50 - 2.35 (m, 2H), 2.34 - 2.22 (m, 1H), 2.03 - 1.93 (m, 1H), 1.72 - 1.48 (m, 3H), 1.37 - 1.29 (m, 1H), 1.09 - 0.99 (m, 9H).

### Preparation of Compound 55-C

To a solution of compound **55-B** (2.76 g, 9.6 mmol, 1.0 eq.) in THF (24 mL) were added H₂O (24 mL) and LiOH (0.6 g, 14.4 mmol, 1.5 eq.) in portions. The resulting clean solution was stirred at 18°C for 2.5 hours. TLC (PE/EtOAc = 4:1) showed trace compound **55-B** remained. The solvent (THF) was removed under vacuum. The residue was extracted with EA (20 mL). The water layer was acidified by 2N HCl to pH = 2-3, then extracted with EA (30 mL x 3). The combined organic layers were washed with brine (20 mL x 3), dried over Na₂SO₄, filtered and concentrated to give compound **55-C** (2.4 g, 92%) as white solid. **¹HNMR: (400 MHz, CDCl₃)** δ: 4.02 - 3.88 (m, 4H), 3.19 (s, 1H), 2.52 - 2.41 (m, 1H), 2.32 (m, 1H), 2.06 - 1.95 (m, 1H), 1.75 - 1.46 (m, 4H), 1.37 - 1.23 (m, 1H), 1.11 - 0.99 (m, 9H).

### Preparation of Compound 55-D

To a solution of compound **55-C** (2.4 g, 8.8 mmol, 1.0 eq.) in DCM (45 mL) was added TEA (3.7 mL, 26.4 mmol, 3 eq.), CMPI (3.4 g, 13.4 mmol, 1.5 eq.) in portions, The resulting yellow solution was stirred at 20°C for 16 hrs. TLC (EtOAc/MeOH = 9:1) indicated completion. The reaction mixture was washed with H₂O (20 mL x 3), dried over Na₂SO₄, filtered and concentrated. The residue was purified by a short column (PE/ EtOAc = 60:1-2:1) to give compound **55-D** (1.87 g, 83.5%) as yellow gum.
**¹HNMR: (400 MHz, CDCl₃)** δ: 4.10 (s, 1H), 4.08 - 3.92 (m, 4H), 2.67 - 2.57 (m, 2H), 2.27 (td, *J* = 9.4, 13.6 Hz, 1H), 1.90 - 1.78 (m, 1H), 1.68 (dt, *J* = 4.1, 13.8 Hz, 1H), 1.55 - 1.43 (m, 2H), 1.35 - 1.28 (m, 1H), 1.14 (s, 3H), 1.05 (d, *J* = 2.7 Hz, 6H).

### Preparation of Compound 55-E

To a solution of compound **55-D** (254 mg, 1 mmol, 1.0 eq.) in anhydrous DCM (10 mL) was added DIBAL-H (1 M in toluene, 1.4 mL, 1.4 mmol, 1.4 eq.) dropwise via syringe at -78°C. The colorless solution was stirred at -78°C for 1 h till TLC (PE/ EtOAc = 4:1) indicated the completion. The reaction mixture was quenched with Sat. NaHCO₃ (10 mL) and stirred for 30 min. After filtration and separation, the organic layer was washed with Sat. NH₄Cl (10 mL x 2). The combined organic layers were dried over anhydrous sodium sulfate. Filtered and concentrated to give compound **55-E** (200 mg, 78%) as white solid. The crude product was used in next step without further purification.

### Preparation of Compound 55-F

To a solution of compound **55-E** (200 mg, 0.78 mmol, 1.0 eq.) in ACT/H₂O (4 mL, v/v = 9:1), was added PPTS (78 mg, 0.31 mmol, 0.4 eq.) in one portion. The resulting mixture was heated to reflux for 1 h. TLC (PE/ EtOAc = 1:3) indicated the completion. The solvent acetone was removed under reduced pressure. The residue was diluted with EA (50 mL) and washed with H₂O (20 mL x 3). The combined organic layers were dried over Na₂SO₄, filtered and concentrated to give compound **55-F** (100 mg, 60.6%) as colorless gum.

### Preparation of Compound 55-G

To a yellow solution of compound **55-F** (110 mg, 0.52 mmol, 1.0 eq.) in anhydrous DCM (2 mL) was added TEA (100 mg, 1.0 mmol, 2 eq.), MsCl (100 mg, 0.88 mmol, 1.7 eq.) dropwise via syringe at 15°C. The resulting dark solution was stirred at 15°C for 4 h till TLC (PE/ EtOAc = 1:3) indicated completion. The reaction was diluted with DCM (20 mL) then washed with H₂O (10 mL x 2). The combined organic layers were dried over anhydrous sodium sulfate. Filtered and concentrated, the residue was purified by Prep-TLC (PE/ EtOAc = 10:1) to give compound **55-G** (50 mg, 50%) as colorless oil.
**¹HNMR: (400 MHz, CDCl₃)** δ: 6.41 - 6.35 (m, 1H), 4.68 - 4.65 (m, 1H), 3.50 (s, 1H), 2.59 (m, 1H), 2.47 - 2.36 (m, 1H), 2.11 (d*, J* = 17.6 Hz, 1H), 1.98 (m, 1H), 1.84 - 1.75 (m, 1H), 1.57 - 1.45 (m, 1H), 1.23 (s, 3H), 1.20 (s, 3H), 1.17 (s, 3H).

### Preparation of Compound 55

To a yellow solution of compound **55-G** (37 mg, 0.19 mmol, 1.0 eq.) in anhydrous THF (2 mL) was added LDA (2 M in THF/heptane, 0.15 mL, 0.29 mmol, 1.5 eq.) dropwise via syringe at -78°C. The resulting yellow solution was stirred at -78°C for 1 h before TsCN (52 mg, 0.29 mmol, 1.5 eq.) in anhydrous THF (1 mL) was added. The resulting mixture was stirred at -70 °C for another 1 h. TLC (PE/ EtOAc = 10:1) indicated completion. The reaction mixture was quenched with Sat. NH₄Cl (10 mL) then extracted with EtOAc (10 mL x 3). The organic layer was dried over anhydrous sodium sulfate. Filtered and concentrated to give compound **55-H** (30 mg, 73.1%) as yellow oil. The crude product was used in next step directly without purification.

To a solution of compound **55-H** (30 mg, 0.14 mmol, 1.0 eq.) in MeCN (1.5 mL) was added DDQ (32 mg, 0.14 mmol, 1.0 eq.) in one portion. The resulting dark suspension was heated to 50°C for 1.5 hours. TLC (PE/ EtOAc = 6:1) indicated completion. The residue was purified by Prep-TLC (PE/EtOAc = 6:1) to give **55** (9.5 mg, 31.7%) as white solid. Please note it was racemic product.
**¹HNMR: (400 MHz, CDCl₃)** δ: 7.52 (s, 1H), 6.44 (d, *J* = 6 Hz, 1H), 4.77 - 4.73 (m, 1H), 3.83 (s, 1H), 2.12 - 2.02 (m, 2H), 1.38 (s, 3H), 1.34 (s, 3H), 1.22 (s, 3H).
**HPLC:** (Purity: 95.80%)
**LCMS:** (M+H: 218.1)

Compound 55 can be further separated by chiral chromatography to produce the following two enantiomers: (4aS,8aS)-4a,8,8-trimethyl-7-oxo-4a,7,8,8a-tetrahydro-4H-chromene-6-carbonitrile and (4aR,8aR)-4a,8,8-trimethyl-7-oxo-4a,7,8,8a-tetrahydro-4H-chromene-6-carbonitrile.

### Example 20. Cellular Assay

The assay was performed by DiscoverX Corporation, 42501 Albrae Street, Suite 100, Fremont, CA 94538. The PathHunter® Nuclear Translocation assay detects translocation of a target protein to, or from, the nucleus. In this system, ProLinkTM (PK), a small enzyme fragment, is fused to the protein of interest and EA is localized in the nucleus. Activation of the signaling pathway induces the target protein to either transit into the nucleus, thus forcing complementation of the PK and EA fragments, or out of the nucleus, hindering complementation of the fragments.

EC₅₀ determinations were performed in duplicate at 10 concentrations with 3-fold serial dilutions at a 30 µM top concentration or an otherwise specified top concentration.

*Cell handling-.* PathHunter Pathway cell lines were expanded from freezer stocks according to standard procedures. 5000 cells were seeded in Cell Plating Reagent 0 (containing 1% FBS) to a total volume of 20 uL into white walled, 384-well microplates and incubated for the overnight prior to testing.

*Agonist format:* For Agonist determination, cells were incubated with sample to induce response. Sample stocks were serially diluted in DMSO to generate 100X sample. Intermediate dilution of sample stocks was performed to generate 5X sample in assay buffer (Cell Plating Reagent 0 containing 1% FBS). 5 µL of 5x sample was added to cells and incubated at room temperature for 6 hours. Vehicle concentration was 1%.

*Signal* detection: Assay signal was generated through a single addition of 25 µL (100% v/v) of PathHunter Flash Detection reagent, followed by a one hour incubation at room temperature. Microplates were read following signal generation with a PerkinElmer Envision™ instrument for chemiluminescent signal detection.

*Data analysis:* Compound activity was analyzed using CBIS data analysis suite (Chemlnnovation, CA). For agonist mode assays, percentage activity was calculated using the following formula: % Activity = 100% x (mean RLU of test sample - mean RLU of vehicle control) / (mean MAX RLU control ligand - mean RLU of vehicle control). For EC50 determination, data was normalized to the maximal and minimal response observed in the presence of the control ligand and vehicle respectively. CDDO methyl ester was used as a control compound.

The compounds described herein were tested for in the above nuclear translocation assay. The results are provided below, wherein the compound number corresponds to the numbers set forth in the examples above, a "+" represents an EC₅₀ of greater than 10 µM, a "++" represents an EC₅₀ of less than or equal to 10 µM, a "+++" represents an EC₅₀ of less than or equal to 1 µM and a "++++" represents an EC₅₀ of less than or equal to 0.1 µM.

| COMPOUNDS | EC₅₀ (NRF2 TRANSLOCATION) |
|---|---|
| 6-Ent1, 8-Ent1, 10-Ent1 and 41-Ent1 | xxxx (<0.1 µM) |
| 1-Ent1, 2-Ent1, 2-Ent2, 3-Ent1, 3-Ent2, 4-Ent1, 4-Ent2, 5-Ent1, 7-Ent1, 7-Ent2, 8-Ent2, 9-Ent1, 10-Ent2, 11-Ent1, 40-Ent1, 40-Ent2, 42-Ent2, 43-Ent1, 43-Ent2, 44-Ent1 and 45-Ent1 | xxx (<1 µM) |
| 1-Ent2, 6-Ent2, 9-Ent2, 11-Ent2, 41-Ent2, 42-Ent1, 44-Ent2, 45-Ent2 and 55 | xx (<10 µM) |
| 5-Ent2 and 54 | x (>10 µM) |

### Example 21. Testing Nrf2 activator compounds in cultured human astrocytes.

### Cells

Human astrocytes from ScienCell (cat # 1820) were grown in astrocyte medium per supplier's instructions. Cells cultured for no more than two passages were plated in 96-well plates at 40,000 cells per well for gene transcription experiments and 20,000 cell per well for glutathione and cytoprotection assays.

### Gene expression

Primary cultures of human spinal cord astrocytes were treated with compound 3-Ent1 for 20 hours. The cells were then rinsed in PBS, lysed, and processed for RNA using Ambion Taqman™ Cells-to-CT kit. The resulting cDNA was stored at -20°C until analysis by real-time polymerase chain reaction (RT-PCR). The cDNA mixture from Cells-to-CT was diluted 5x before loading into PCR. This yields results similar to using 6 ng of purified cDNA. RT-PCR was performed on Life Technologies QuantStudio platform using OpenArray technique according to manufacturer's protocol using the following Taqman primers:

| **Target** | **Taqman assay** | |
|---|---|---|
| GCLC | Hs00155249_m1 | glutamate-cysteine ligase, catalytic subunit |
| GCLM | Hs00157694_m1 | glutamate-cysteine ligase, modifier subunit |
| OSGIN1 | Hs00203539_m1 | oxidative stress induced growth inhibitor 1 |
| TBP | Hs00427620_m1 | TATA box binding protein [Homo sapiens (human)] peroxiredoxin |
| PRDX1 | Hs00602020_mH | 6 |
| SRXN1 | Hs00607800_m1 | sulfiredoxin 1 |
| TXNRD1 | Hs00917067_m1 | thioredoxin reductase 1 |
| ACTB | Hs01060665_g1 | actin, beta [Homo sapiens(human)] |
| HMOX1 | Hs01110250_m1 | heme oxygenase 1 [Homo sapiens(human)] ubiquitin C [Homo |
| UBC | Hs01871556_s1 | sapiens (human)] NAD(P)H dehydrogenase, quinone |
| NQO1 | Hs02512143_s1 | 1 |
| GAPDH | Hs02758991_g1 | glyceraldehyde-3-phosphate dehydrogenase [Homo sapiens (human)] |

The comparative CT method was used to calculate fold changes using ThermoFisher Cloud software for PCR analysis. Samples were compared to vehicle control.

As shown in Figures 1A to 1D, Compound 3-Ent1 induces transcription of Nrf2 target genes, including GCLC, HMOX1, OSGIN1 and NQO1.

### Glutathione assay

Intracellular glutathione was measured after a 20-hr exposure to test compound 3-Ent1 by a two-step process. First, cells were lysed and luciferin quantitatively generated from substrate, catalyzed by glutathione-S-transferase in the presence of analyte glutathione. Then luciferin was assayed using stabilized luciferase to produce a luminescent signal proportional to the concentration of glutathione (Promega GSH-Glo, cat #V6912).

As shown in Figure 2, Compound 3-Ent1 increases intracellular glutathione.

### Cytoprotection

Astrocytes were treated for 20 hrs as above, then the medium was removed and replaced with serum- and supplement-free growth medium with and without 25 µM sodium arsenite. After 22 hrs., cells were washed with PBS, fixed with 4% paraformaldehyde/4% sucrose in PBS, stained with 4',6-Diamidino-2-phenylindole dihydrochloride (DAPI) and counted by quantitative fluorescence microscopy.

As shown in Figure 3, Compound 3-Ent1 protects cells from oxidative stress-induced cell death caused by 25 µM sodium arsenite.

Other embodiments are within the scope of the following claims.

## Claims

1. A compound represented by Formula I: 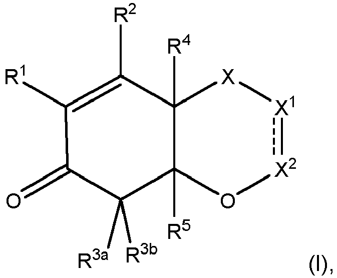 or a pharmaceutically acceptable salt thereof, wherein
R¹ is -CN, -C(O)R^{1a} or C₁-₈alkyl substituted with one or more fluorine atoms;
R^{1a} is H, C₁₋₁₂alkyl, C₂₋₁₂alkenyl, C₂₋₁₂alkynyl, a 3 to 12-membered carbocyclyl, a 3 to 12-membered heterocyclyl, -OR^{10a}, -SR^{10a}, -N(R^{10a})₂, -NR^{10a}OR^{10a}, -NR^{10a}S(O)₂R^{10a}, -NR^{10a}C(O)R¹⁰a, -N(R^{10a})N(R^{10a})₂, -N(R^{10a})C(O)OR^{10a}, or -N(R^{10a})C(O)N(R^{10a})_{2,} wherein the C₁₋₁₂alkyl, C₂₋₁₂alkenyl, C₂₋₁₂alkynyl, 3 to 12-membered carbocyclyl, and 3 to 12-membered heterocyclyl are each optionally substituted with one or more R¹⁵;
R² is H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl, -C(O)R^{2a}, -C(S)R^{2a}, -C(O)OR^{2a},-C(S)SR^{2a}, -C(O)SR^{2a}, -C(S)OR^{2a}, -SC(O)R^{2a}, -OC(S)R^{2a},-SC(S)R^{2a}, -C(O)N(R^{2a})_{2,} -OR^{2a}, -SR^{2a}, -N(R^{2a})₂, -N(R^{2a})OR^{2a}, -N(R^{2a})S(O)₂R^{2a},-N(R^{2a})C(O)R^{2a}, -N(R^{2a})N(R^{2a})₂, -N(R^{2a})C(O)OR^{2a}, -N(R^{2a})C(O)N(R^{2a} )₂, -S(O)₂R^{2a}, -S(O)R^{2a}, -S(O)N(R^{2a})_{2,} -S(O)₂N(R^{2a})₂, -N⁺(R^{2a})₃, -S⁺(R^{2a})₂ or -Si(R^{2a})₃, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R²⁵;
R^{3a} is H, C₁₋₁₂ alkyl, C₂₋₁₂ alkenyl, C₂₋₁₂ alkynyl, a 3 to 12-membered carbocyclyl, a 3 to 12-membered heterocyclyl, -C(O)R^{30a}, -C(O)OR^{30a}, -C(O)N(R^{30a})₂, -OR^{30a},-N(R^{30a})₂, -N(R^{30a})OR^{30a}, -N(R^{30a})S(O)₂R^{30a}, -N(R^{30a}₎C(O)R^{30a}, -N(R^{30a})N(R^{30a})₂,-N(R^{30a})C(O)OR^{30a}, -N(R^{30a})C(O)N(R^{30a})₂, -S(O)R^{30a}, -S(O)N(R^{30a})₂, or-S(O)₂N(R^{30a})₂, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R³⁵; and
R^{3b} is C₁₋₁₂alkyl, C₂₋₁₂alkenyl, C₂₋₁₂alkynyl, a 3 to 12-membered carbocyclyl, a 3 to 12-membered heterocyclyl, -C(O)R^{30b}, -C(O)OR^{30b}, -C(O)N(R^{30b})₂, -OR^{30b}, -N(R^{30b})₂, -N(R^{30b})OR^{30b}, -N(R^{30b})S(O)₂R^{30b}, -N(R^{30b})C(O)R^{30b}, -N(R^{30b})N(R^{30b})₂,-N(R^{30b})C(O)OR^{30b}, -N(R^{30b})C(O)N(R^{30b})₂, -S(O)R^{30b}, -S(O)N(R^{30b})₂ or-S(O)₂N(R^{30b})₂, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R³⁵; or
R^{3a} and R^{3b}, taken together, are C₂₋₁₂ alkylene, C₂₋₁₂ alkenylene or C₂₋₁₂ alkynylene, wherein the C₂₋₁₂ alkylene, C₂₋₁₂ alkenylene, and C₂₋₁₂ alkynylene are each optionally substituted with one or more R³⁵;
R⁴ is H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl, -C(O)R^{4a}, -C(S)R^{4a}, -C(O)OR^{4a},-C(S)SR^{4a}, -C(O)SR^{4a}, -C(S)OR^{4a}, -SC(O)R^{4a}, -OC(S)R^{4a}, -SC(S)R^{4a},-C(O)N(R^{4a})_{2,} -OR^{4a}, -SR^{4a}, -N(R^{4a})₂, -N(R^{4a})OR^{4a}, -N(R^{4a})S(O)₂R^{4a},-N(R^{4a})C(O)R^{4a}, -N(R^{4a})N(R^{4a})₂, -N(R^{4a})C(O)OR^{4a}, -N(R^{4a})C(O)N(R^{4a})₂, -S(O)₂R^{4a}, -S(O)R^{4a}, -S(O)N(R^{4a})₂, -S(O)₂N(R^{4a})₂, -N⁺(R^{4a})₃, -S⁺(R^{4a})₂ or -Si(R^{4a})₃, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R⁴⁵;
R⁵ is H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl, -C(O)R^{5a}, -C(S)R^{5a}, -C(O)OR^{5a},-C(S)SR^{5a}, -C(O)SR^{5a} , -C(S)OR^{5a}, -SC(O)R^{5a}, -OC(S)R^{5a}, -SC(S)R^{5a},-C(O)N(R^{5a})_{2,} -OR^{5a}, -SR^{5a}, -N(R^{5a})₂, -N(R^{5a})OR^{5a}, -N(R^{5a})S(O)₂R^{5a},-N(R^{5a})C(O)R^{5a}, -N(R^{5a})N(R^{5a})₂, -N(R^{5a})C(O)OR^{5a}, -N(R^{5a})C(O)N(R^{5a})₂, -S(O)₂R^{5a}, -S(O)R^{5a}, -S(O)N(R^{5a})₂, -S(O)₂N(R^{5a})₂, -N⁺(R^{5a})₃, -S⁺(R^{5a})₂ or -Si(R^{5a})₃, wherein the C₁₋₁₂alkyl, C₂₋₁₂alkenyl, C₂₋₁₂alkynyl, 3 to 12-membered carbocyclyl and 3 to 12-membered heterocyclyl are each optionally substituted with one or more R⁵⁵; " 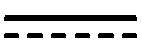 " is either a single bond or a double bond, wherein when " 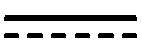 " is a double bond, then X¹ is CR⁶ and X² is CR⁷; and when " 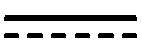 " is a single bond, then X¹ is C(R⁶)₂ and X² is C(R⁷)₂;
R⁶, in each occurrence, is independently H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl,-C(O)R^{6a}, -C(S)R^{6a}, -C(O)OR^{6a}, -C(S)SR^{6a}, -C(O)SR^{6a}, -C(S)OR^{6a}, -SC(O)R^{6a},-OC(S)R^{6a}, -SC(S)R^{6a}, -C(O)N(R^{6a})_{2,} -OR^{6a}, -SR^{6a}, -N(R^{6a})₂, -N(R^{6a})OR^{6a},-N(R^{6a})S(O)₂R^{6a}, -N(R^{6a})C(O)R^{6a}, -N(R^{6a})N(R^{6a})₂, -N(R^{6a}) C(O)OR^{6a},-N(R^{6a})C(O)N(R^{6a})₂, -S(O)₂R^{6a}, -S(O)R^{6a}, -S(O)N(R^{6a})₂, -S(O)₂N(R^{6a})₂, -N⁺(R^{6a})₃,-S⁺(R^{6a})₂, or -Si(R^{6a})₃; or the two R⁶ groups, taken together, are oxo, C₁₋₁₂ alkylidene or =NR^{6a}, wherein the C₁₋₁₂alkyl, C₂₋₁₂alkenyl, C₂₋₁₂alkynyl, 3 to 12-membered carbocyclyl, 3 to 12-membered heterocyclyl, and C₁₋₁₂alkylidene are each optionally substituted with one or more R⁶⁵;
R⁷, in each occurrence, is independently H, halo, -NO₂, -CN, -N₃, C₁₋₁₂alkyl, C₂₋₁₂alkenyl, C₂₋₁₂alkynyl, a 3 to 12-membered non-aromatic carbocyclyl, a 3 to 12-membered heterocyclyl, -C(O)R^{7a}, -C(S)R^{7a}, -C(O)OR^{7a}, -C(S)SR^{7a}, -C(O)SR^{7a}, -C(S)OR^{7a},-SC(O)R^{7a}, -OC(S)R^{7a}, -SC(S)R^{7a}, -C(O)N(R^{7a})₂, -OR^{7a}, -SR^{7a}, -N(R^{7a})₂,-N(R^{7a})OR^{7a}, -N(R^{7a})S(O)₂R^{7a}, -N(R^{7a})C(O)R^{7a}, -N(R^{7a})N(R^{7a})₂, -N(R^{7a})C(O)OR^{7a},-N(R^{7a})C(O)N(R^{7a})₂, -S(O)₂R^{7a}, -S(O)R^{7a}, -S(O)N(R^{7a})₂, -S(O)₂N(R^{7a})₂, -N⁺(R^{7a})₃,-S⁺(R^{7a})₂, or -Si(R^{7a})₃; or the two R⁷ groups, taken together, are oxo, C₁₋₁₂ alkylidene, or =NR^{7a}, wherein the C₁₋₁₂alkyl, C₂₋₁₂alkenyl, C₂₋₁₂alkynyl, 3 to 12-membered non-aromatic carbocyclyl, 3 to 12-membered heterocyclyl, and C₁₋₁₂alkylidene are each optionally substituted with one or more R⁷⁵;
X is -C(R⁸)₂- or -O-;
R⁸, in each occurrence, is independently H, halo, -NO₂, -CN, -N₃, C₁₋₁₂alkyl, C₂₋₁₂alkenyl, C₂₋₁₂alkynyl, a 3 to 12-membered carbocyclyl, a 3 to 12-membered heterocyclyl,-C(O)R^{8a}, -C(S)R^{8a}, -C(O)OR^{8a} , -C(S)SR^{8a}, -C(O)SR^{8a}, -C(S)OR^{8a}, -SC(O)R^{8a},-OC(S)R^{8a}, -SC(S)R^{8a}, -C(O)N(R^{8a})₂, -OR^{8a}, -SR^{8a}, -N(R^{8a})₂, -N(R^{8a})OR^{8a},-N(R^{8a})S(O)₂R^{8a}, -N(R^{8a})C(O)R^{8a}, -N(R^{8a})N(R^{8a})₂, -N(R^{8a})C(O)OR^{8a},-N(R^{8a})C(O)N(R^{8a})₂, -S(O)₂R^{8a}, -S(O)R^{8a}, -S(O)N(R^{8a})₂, -S(O)₂N(R^{8a})₂, -N⁺(R^{8a})₃,-S⁺(R^{8a})₂, or -Si(R^{8a})₃; or the two R⁸ groups taken together are oxo, C₁₋₁₂ alkylidene, or =NR^{8a}, wherein the C₁₋₁₂alkyl, C₂₋₁₂alkenyl, C₂₋₁₂alkynyl, 3 to 12-membered carbocyclyl, 3 to 12-membered heterocyclyl, and C₁₋₁₂alkylidene are each optionally substituted with one or more R⁸⁵;
R^{10a}, R^{2a}, R^{30a} , R^{30b}, R^{4a}, R^{5a}, R^{6a}, R^{7a}, and R^{8a}, in each occurrence, are independently selected from H, C₁₋₁₂alkyl, C₂₋₁₂alkenyl, C₂₋₁₂alkynyl, C₁₋₁₂alkoxy, C₁₋₁₂acyl,-Si(R¹⁶)₃, a 3 to 12-membered carbocyclyl, and a 3 to 12-membered heterocyclyl, wherein the C₁₋₁₂alkyl, C₂₋₁₂alkenyl, C₂₋₁₂alkynyl, C₁₋₁₂alkoxy, C₁₋₁₂acyl, 3 to 12-membered carbocyclyl, and 3 to 12-membered heterocyclyl are each optionally substituted with one or more R¹⁷;
R¹⁶, in each occurrence, is independently selected from H, C₁₋₁₂alkyl, C₂₋₁₂alkenyl, C₂₋₁₂alkynyl, a 3 to 12-membered carbocyclyl, and a 3 to 12-membered heterocyclyl, wherein the C₁₋₁₂alkyl, C₂₋₁₂alkenyl, C₂₋₁₂alkynyl, 3 to 12-membered carbocyclyl, and 3 to 12-membered heterocyclyl are each optionally substituted with one or more R¹⁸;
R¹⁵, R²⁵, R³⁵, R⁴⁵, R⁵⁵, R⁶⁵, R⁷⁵, and R⁸⁵, in each occurrence, are independently selected from halo, -OH, -CN, C₁₋₁₂alkyl, C₂₋₁₂alkenyl, C₂₋₁₂alkynyl, C₁₋₁₂alkoxy, a 3 to 12-membered carbocyclyl and a 3 to 12-membered heterocyclyl, wherein the C₁₋₁₂alkyl, C₂₋₁₂alkenyl, C₂₋₁₂alkynyl, C₁₋₁₂alkoxy, 3 to 12-membered carbocyclyl and 3 to 12-membered heterocyclyl are each optionally substituted with one or more R¹⁹; and
R¹⁷, R¹⁸, and R¹⁹, in each occurrence, are independently selected from halo, -OH, -CN, C₁₋₁₂alkyl, C₂₋₁₂alkenyl, C₂₋₁₂alkynyl, C₁₋₁₂alkoxy, a 3 to 12-membered carbocyclyl and a 3 to 12-membered heterocyclyl, wherein the C₁₋₁₂alkyl, C₂₋₁₂alkenyl, C₂₋₁₂alkynyl, C₁₋₁₂alkoxy, 3 to 12-membered carbocyclyl, and 3 to 12-membered heterocyclyl are each optionally substituted with one or more groups independently selected from halo, -OH, and C₁₋₄alkoxy.

2. The compound of claim 1, wherein the compound is represented by Formula II, III, IV, or V: 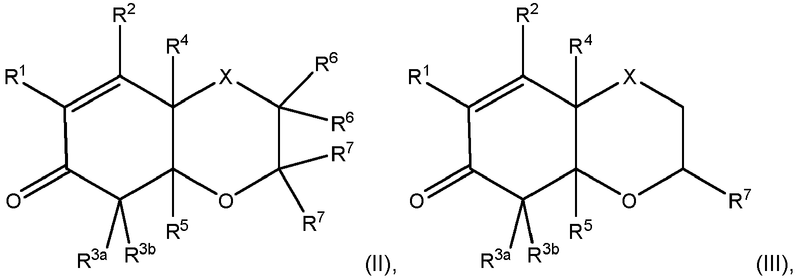 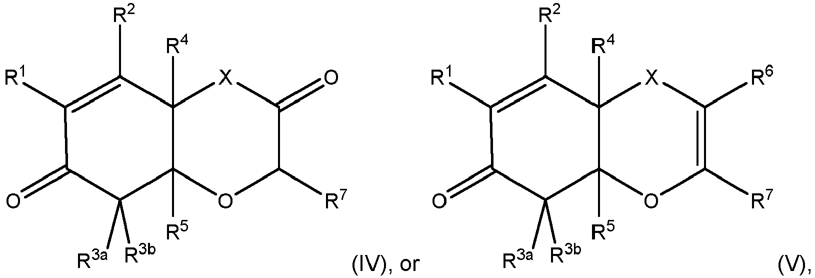 or a pharmaceutically acceptable salt thereof.

3. The compound of claim 1 or 2, wherein the compound is represented by Formula IIA, IIB, IIC, IID, IIIA, IIIB, IIIC, IIID, IVA, IVB, IVC, IVD, VA, VB, VC, or VD: 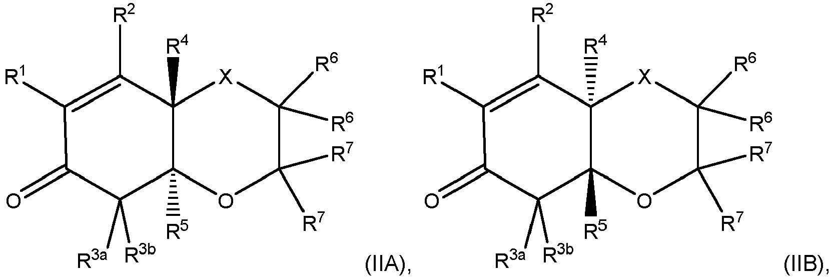 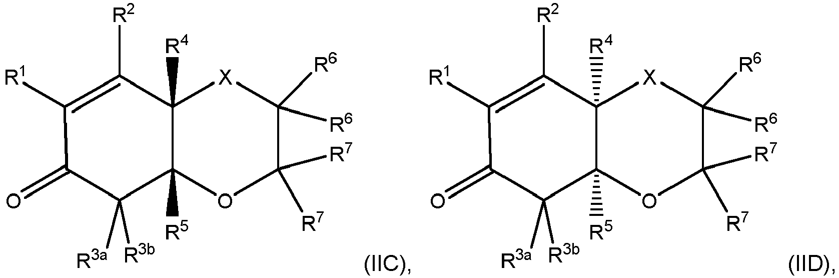 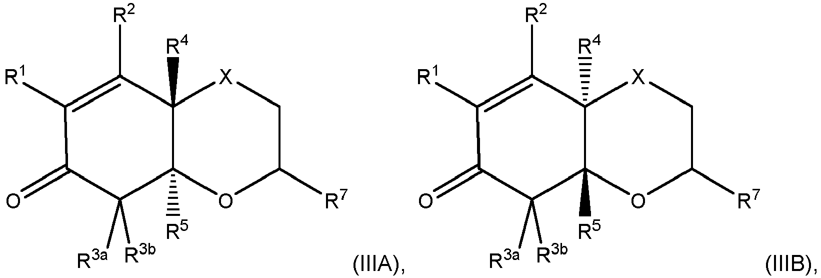 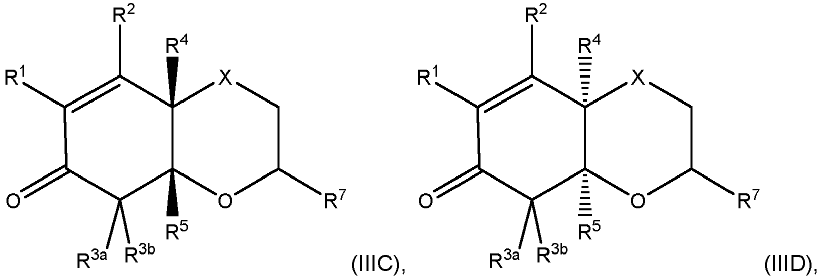 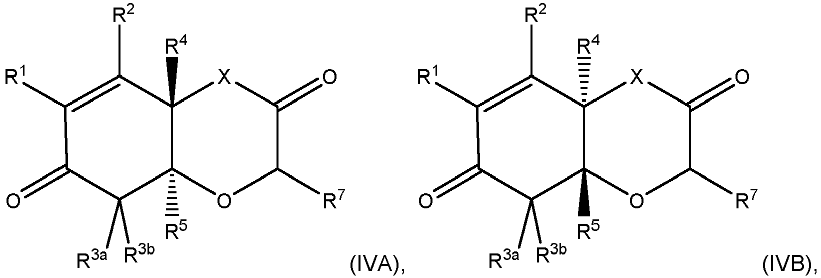 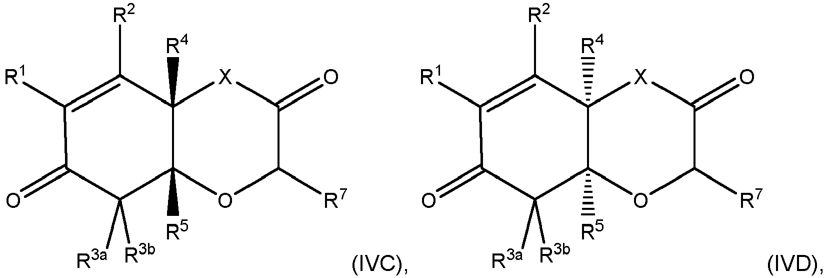 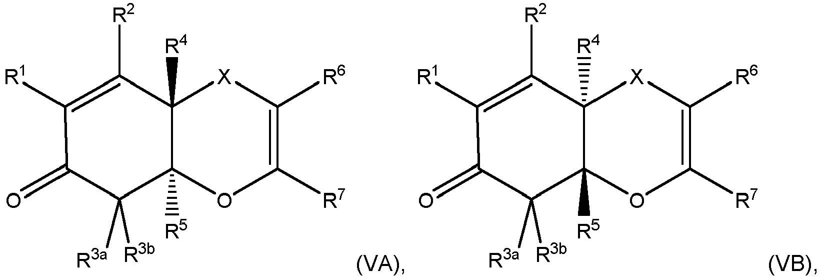 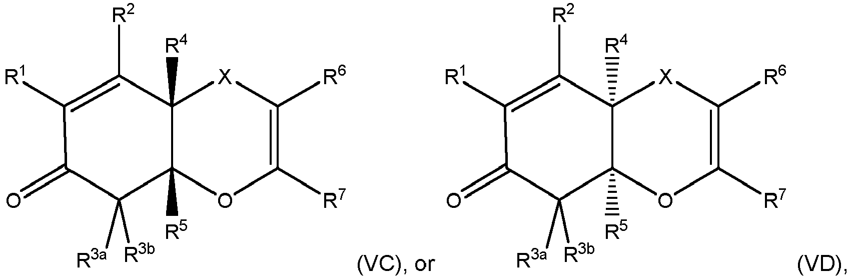 or a pharmaceutically acceptable salt thereof.

4. The compound of any one of claims 1-3, or a pharmaceutically acceptable salt thereof, wherein
R⁶, in each occurrence, is independently H, halo, -CN, -OR^{6a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R⁶⁵; or the two R⁶ groups, taken together, are oxo;
R^{6a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷;
R⁷, in each occurrence, is independently H, halo, -CN, -OR^{7a}, C₁₋₁₂alkyl, C₂₋₁₂alkenyl, C₂₋₁₂alkynyl, or a 3 to 8 membered non-aromatic carbocyclyl, wherein the C₁₋₁₂alkyl, C₂₋₁₂alkenyl, C₂₋₁₂alkynyl, and 3 to 8 membered carbocyclyl are each optionally substituted with one to eight R⁷⁵;
R^{7a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷;
R⁶⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, -OH and C₁₋₄alkoxy;
R⁷⁵, in each occurrence, is independently selected from halo, -OH, -CN, C₁₋₁₂alkyl, C₂₋₁₂alkenyl, C₂₋₁₂alkynyl, a 3 to 8 membered carbocyclyl, and C₁₋₁₂alkoxy, wherein the C₁₋₁₂alkyl, C₂₋₁₂alkenyl, C₂₋₁₂alkynyl, 3 to 8 membered carbocyclyl, and C₁₋₁₂alkoxy are each optionally substituted with one to eight groups independently selected from halo, -OH and C₁₋₄alkoxy; and
R¹⁷, in each occurrence, as an optional substituent of R^{6a} or R^{7a}, is independently selected from halo, -CN, -OH, C₁₋₆alkyl, and C₁₋₆alkoxy, wherein the C₁₋₆alkyl and C₁₋₆alkoxy are each optionally substituted with one to six halo.

5. The compound of any one of claims 1-4, or a pharmaceutically acceptable salt thereof, wherein
X is -C(R⁸)₂-;
R⁸, in each occurrence, is independently H, halo, -CN, -OR^{8a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R⁸⁵;
R^{8a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷;
R⁸⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, -OH, and C₁₋₄alkoxy;
and
R¹⁷, in each occurrence, as an optional substituent of R^{8a}, is independently is selected from halo, -CN, -OH, C₁₋₆alkyl, and C₁₋₆alkoxy, wherein the C₁₋₆alkyl and C₁-₆alkoxy are each optionally substituted with one to six halo.

6. The compound of any one of claims 1-4, or a pharmaceutically acceptable salt thereof, wherein X is -O-.

7. The compound of any one of claims 1-6, or a pharmaceutically acceptable salt thereof, wherein:
1)
R¹ is -CN;
2)
R¹ is -CF₃; or
3)
R¹ is -C(O)R^{1a};
R^{1a} is H, C₁₋₁₂alkyl, C₂₋₁₂alkenyl, C₂₋₁₂alkynyl, a 3 to 12-membered carbocyclyl, or a 3 to 12-membered heterocyclyl, wherein the C₁₋₁₂alkyl, C₂₋₁₂alkenyl, C₂₋₁₂alkynyl, 3 to 12-membered carbocyclyl, and 3 to 12-membered heterocyclyl are each optionally substituted with one to six R¹⁵; and
R¹⁵, in each occurrence, is independently selected from halo, -OH, C₁₋₁₂alkyl, and C_{1- 12}alkoxy, wherein the C₁₋₁₂alkyl and C₁₋₁₂alkoxy are each optionally substituted with one to three groups independently selected from halo, -OH, and C₁₋₄alkoxy.

8. The compound of any one of claims 1-7, or a pharmaceutically acceptable salt thereof, wherein
R² is H, halo, -CN, -OR^{2a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R²⁵;
R^{2a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷;
R²⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, -OH, and C₁₋₄alkoxy;
R^{3a} is H, halo, -CN, -OR^{30a}, C₁₋₁₂ alkyl, C₂₋₁₂ alkenyl, or C₂₋₁₂ alkynyl, wherein the C₁₋₁₂alkyl, C₂₋₁₂alkenyl, and C₂₋₁₂alkynyl are each optionally substituted with one to eight R³⁵;
R^{3b} is halo, -CN, -OR^{30b}, C₁₋₁₂ alkyl, C₂₋₁₂ alkenyl, or C₂₋₁₂ alkynyl, wherein the C₁₋₁₂alkyl, C₂₋₁₂alkenyl, and C₂₋₁₂alkynyl are each optionally substituted with one to eight R³⁵;
R^{30a} and R^{30b} are each independently selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷;
R³⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, -OH and C₁₋₄alkoxy;
R⁴ is H, halo, -CN, -OR^{4a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R⁴⁵;
R^{4a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷;
R⁴⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, -OH and C₁₋₄alkoxy;
R⁵ is H, halo, -CN, -OR^{5a}, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight R⁵⁵;
R^{5a} is selected from H, C₁₋₁₂alkyl, C₂₋₁₂alkenyl, and C₂₋₁₂alkynyl, wherein the C₁₋₁₂alkyl, C₂₋₁₂alkenyl, and C₂₋₁₂alkynyl are each optionally substituted with one to six R¹⁷;
R⁵⁵, in each occurrence, is independently selected from halo, -OH, -CN, C₁₋₁₂alkyl, C₂₋₁₂alkenyl, C₂₋₁₂alkynyl, and C₁₋₁₂alkoxy, wherein the C₁₋₁₂alkyl, C₂₋₁₂alkenyl, C₂₋₁₂alkynyl, and C₁₋₁₂alkoxy are each optionally substituted with one to eight groups independently selected from halo, -OH, and C₁₋₄alkoxy; and
R¹⁷, in each occurrence, is independently selected from halo, -CN, -OH, C₁₋₆alkyl, and C₁₋₆alkoxy, wherein the C₁₋₆alkyl and C₁₋₆alkoxy are each optionally substituted with one to six halo.

9. The compound of any one of claims 1-3, or a pharmaceutically acceptable salt thereof, wherein
R¹ is -CN or -CF₃;
R² is H, halo, -OH, -CN, C₁₋₆alkyl, or C₁₋₆alkoxy, wherein the C₁₋₆alkyl and C₁-₆alkoxy are each optionally substituted with one to six groups independently selected from halo, -CN, -OH, C₁₋₄alkyl, and C₁₋₄alkoxy;
R^{3a} and R^{3b} are each independently halo, -OH, -CN, C₁₋₆alkyl, or C₁₋₆alkoxy, wherein the C₁₋₆alkyl and C₁₋₆alkoxy are each optionally substituted with one to six groups independently selected from halo, -CN, -OH, and C₁-₄alkoxy;
R⁴ is H, halo, -OH, -CN, C₁₋₆alkyl, or C₁₋₆alkoxy, wherein the C₁₋₆alkyl and C₁-₆alkoxy are each optionally substituted with one to six groups independently selected from halo, -CN, -OH, and C₁₋₄alkoxy;
R⁵ is H, halo, -OH, -CN, C₁₋₆alkyl, or C₁₋₆alkoxy, wherein the C₁₋₆alkyl and C₁-₆alkoxy are each optionally substituted by one to six groups independently selected from halo, -CN, -OH, and C₁₋₄alkoxy;
X is -C(R⁸)₂- or-O-, wherein R⁸, in each occurrence, is independently H, halo,-CN, -OH, C₁₋₁₂alkyl, C₂₋₁₂alkenyl, or C₂₋₁₂alkynyl, wherein the C₁₋₁₂alkyl, C₂₋₁₂alkenyl, and C₂₋₁₂alkynyl are each optionally substituted with one to eight groups independently selected from halo, -OH, -CN, and C₁₋₄alkoxy;
" 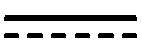 " is a single bond;
X¹ is C(R⁶)₂, wherein R⁶, in each occurrence, is independently H or C₁₋₄alkyl, or the two R⁶ groups, taken together, are oxo, wherein the C₁₋₄alkyl is optionally substituted with one to six groups independently selected from halo, -OH, C₁₋₄alkoxy, and -CN; and
X² is C(R⁷)₂, wherein R⁷, in each occurrence, is independently H, -OH, halo, C₁₋₁₂alkyl, C₁₋₁₂alkoxy, or a 3 to 8 membered cycloalkyl, wherein the C₁₋₁₂alkyl, C₁₋₁₂alkoxy, and 3 to 8 membered cycloalkyl are each optionally substituted with one to six groups independently selected from phenyl, halo, -OH, C₁₋₄alkoxy, and -CN.

10. The compound of claim 9, or a pharmaceutically acceptable salt thereof, wherein
R¹ is -CN;
R² is H or C₁₋₄alkyl;
R^{3a} and R^{3b} are each independently C₁₋₆alkyl;
R⁴ is H or C₁₋₄alkyl;
R⁵ is H or C₁₋₄alkyl;
X is -C(R⁸)₂- or-O-, wherein R⁸, in each occurrence, is independently H or C₁-₄alkyl;
" 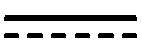 " is a single bond;
X¹ is C(R⁶)₂ or-C(O)-, wherein R⁶, in each occurrence, is independently H or C₁₋₄alkyl; and
X² is -CHR₇-, wherein R⁷ is H, C₁₋₆alkyl, benzyl, cyclopropyl, cyclobutyl, cyclopentyl, or cyclohexyl.

11. The compound of claim 10, or a pharmaceutically acceptable salt thereof, wherein
R¹ is -CN;
R² is H;
R^{3a} and R^{3b} are each independently C₁₋₄alkyl;
R⁴ is C₁₋₄alkyl;
R⁵ is H;
X is -CH₂- or -O-;
" 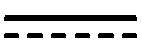 " is a single bond;
X¹ is -CH₂- or-C(O)-; and
X² is -CHR₇-, wherein R⁷ is H, C₁₋₄alkyl, benzyl or cyclopropyl.

12. The compound of claim 1 selected from the group consisting of:
(4aS,8aR)-4a,8,8-trimethyl-7-oxo-3,4,4a,7,8,8a-hexahydro-2H-chromene-6-carbonitrile;
(4aR,8aS)-4a,8,8-trimethyl-7-oxo-3,4,4a,7,8,8a-hexahydro-2H-chromene-6-carbonitrile;
(2S,4aS,8aR)-2,4a,8,8-tetramethyl-7-oxo-2,3,4,8a-tetrahydrochromene-6-carbonitrile;
(2R,4aR,8aS)-2,4a,8,8-tetramethyl-7-oxo-2,3,4,8a-tetrahydrochromene-6-carbonitrile;
(2R,4aS,8aR)-2,4a,8,8-tetramethyl-7-oxo-2,3,4,8a-tetrahydrochromene-6-carbonitrile;
(2S,4aR,8aS)-2,4a,8,8-tetramethyl-7-oxo-2,3,4,8a-tetrahydrochromene-6-carbonitrile;
(4aS,8aR)-4a-ethyl-8,8-dimethyl-7-oxo-2,3,4,8a-tetrahydrochromene-6-carbonitrile;
(4aR,8aS)-4a-ethyl-8,8-dimethyl-7-oxo-2,3,4,8a-tetrahydrochromene-6-carbonitrile;
(2S,4aS,8aR)-4a-ethyl-2,8,8-trimethyl-7-oxo-2,3,4,8a-tetrahydrochromene-6-carbonitrile;
(2R,4aR,8aS)-4a-ethyl-2,8,8-trimethyl-7-oxo-2,3,4,8a-tetrahydrochromene-6-carbonitrile;
(2R,4aS,8aR)-4a-ethyl-2,8,8-trimethyl-7-oxo-2,3,4,8a-tetrahydrochromene-6-carbonitrile;
(2S,4aR,8aS)-4a-ethyl-2,8,8-trimethyl-7-oxo-2,3,4,8a-tetrahydrochromene-6-carbonitrile;
(2S,4aS,8aR)-2-ethyl-4a,8,8-trimethyl-7-oxo-2,3,4,8a-tetrahydrochromene-6-carbonitrile;
(2R,4aR,8aS)-2-ethyl-4a,8,8-trimethyl-7-oxo-2,3,4,8a-tetrahydrochromene-6-carbonitrile;
(2R,4aS,8aR)-2-ethyl-4a,8,8-trimethyl-7-oxo-2,3,4,8a-tetrahydrochromene-6-carbonitrile;
(2S,4aR,8aS)-2-ethyl-4a,8,8-trimethyl-7-oxo-2,3,4,8a-tetrahydrochromene-6-carbonitrile;
(2S,4aS,8aR)-2,4a-diethyl-8,8-dimethyl-7-oxo-2,3,4,8a-tetrahydrochromene-6-carbonitrile;
(2R,4aR,8aS)-2,4a-diethyl-8,8-dimethyl-7-oxo-2,3,4,8a-tetrahydrochromene-6-carbonitrile;
(2R,4aS,8aR)-2,4a-diethyl-8,8-dimethyl-7-oxo-2,3,4,8a-tetrahydrochromene-6-carbonitrile;
(2S,4aR,8aS)-2,4a-diethyl-8,8-dimethyl-7-oxo-2,3,4,8a-tetrahydrochromene-6-carbonitrile;
(2R,4aS,8aR)-2-cyclopropyl-4a,8,8-trimethyl-7-oxo-2,3,4,8a-tetrahydrochromene-6-carbonitrile;
(2S,4aR,8aS)-2-cyclopropyl-4a,8,8-trimethyl-7-oxo-2,3,4,8a-tetrahydrochromene-6-carbonitrile;
(2S,4aS,8aR)-2-isopropyl-4a,8,8-trimethyl-7-oxo-2,3,4,8a-tetrahydrochromene-6-carbonitrile;
(2R,4aR,8aS)-2-isopropyl-4a,8,8-trimethyl-7-oxo-2,3,4,8a-tetrahydrochromene-6-carbonitrile;
(2R,4aS,8aR)-2-isopropyl-4a,8,8-trimethyl-7-oxo-2,3,4,8a-tetrahydrochromene-6-carbonitrile;
(2S,4aR,8aS)-2-isopropyl-4a,8,8-trimethyl-7-oxo-2,3,4,8a-tetrahydrochromene-6-carbonitrile;
(2S,4aS,8aR)-2-benzyl-4a,8,8-trimethyl-7-oxo-2,3,4,8a-tetrahydrochromene-6-carbonitrile;
(2R,4aR,8aS)-2-benzyl-4a,8,8-trimethyl-7-oxo-2,3,4,8a-tetrahydrochromene-6-carbonitrile;
(2R,4aS,8aR)-2-benzyl-4a,8,8-trimethyl-7-oxo-2,3,4,8a-tetrahydrochromene-6-carbonitrile;
(2S,4aR,8aS)-2-benzyl-4a,8,8-trimethyl-7-oxo-2,3,4,8a-tetrahydrochromene-6-carbonitrile;
(2S,4aS,8aR)-2,4a,8,8-tetramethyl-3,7-dioxo-4,8a-dihydrochromene-6-carbonitrile;
(2R,4aR,8aS)-2,4a,8,8-tetramethyl-3,7-dioxo-4,8a-dihydrochromene-6-carbonitrile;
(4aS,8aS)-4a,8,8-trimethyl-7-oxo-3,8a-dihydro-2H-1,4-benzodioxine-6-carbonitrile;
(4aR,8aR)-4a,8,8-trimethyl-7-oxo-3,8a-dihydro-2H-1,4-benzodioxine-6-carbonitrile;
4a,8,8,8a-tetramethyl-7-oxo-3,4,4a,7,8,8a-hexahydro-2H-chromene-6-carbonitrile; and
4a,8,8-trimethyl-7-oxo-4a,7,8,8a-tetrahydro-4H-chromene-6-carbonitrile,
and a pharmaceutically acceptable salt thereof.

13. A pharmaceutical composition comprising at least one compound of any one of claims 1 through 12, or a pharmaceutically acceptable salt thereof, and at least one pharmaceutically acceptable excipient.

14. A compound according to any one of claims 1 through 12, or a pharmaceutically acceptable salt thereof, for use in treating a disorder in a subject, wherein the disorder is selected from the group consisting of a neurodegenerative disease, inflammation/an inflammatory disease, an autoimmune disease, an ischemic fibrotic disease, a cancer, premature aging, a cardiovascular disease, a liver disease, a hemoglobinopathy, thalassemia, and a metabolic disorder.

## Patentansprüche

1. Verbindung, dargestellt durch Formel I: 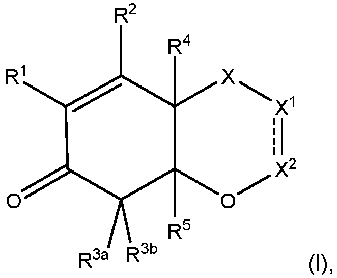 oder ein pharmazeutisch annehmbares Salz davon, wobei
R¹ -CN, -C(O)R^{1a} oder C₁₋₈Alkyl substituiert mit einem oder mehreren Fluoratomen ist;
R^{1a} H, C₁₋₁₂Alkyl, C₂₋₁₂Alkenyl, C₂₋₁₂Alkynyl, ein 3- bis 12-gliedriges Carbocyclyl, ein 3-bis 12-gliedriges Heterocyclyl, -OR^{10a}, -SR^{10a}, -N(R^{10a})₂, -NR^{10a}OR^{10a}, -NR^{10a}S(O)₂R^{10a},-NR^{10a}C(O)R^{10a}, -N(R^{10a})N(R^{10a})₂, -N(R^{10a})C(O)OR^{10a} oder -N(R^{10a})C(O)N(R^{10a})₂ ist, wobei das C₁₋₁₂Alkyl, das C₂₋₁₂Alkenyl, das C₂₋₁₂Alkynyl, das 3- bis 12-gliedrige Carbocyclyl und das 3- bis 12-gliedrige Heterocyclyl jeweils optional substituiert sind mit einem oder mehreren R¹⁵;
R² H, Halo, -NO₂, -CN, -N₃, C₁₋₁₂Alkyl, C₂₋₁₂Alkenyl, C₂₋₁₂Alkynyl, ein 3- bis 12-gliedriges Carbocyclyl, ein 3- bis 12-gliedriges Heterocyclyl, -C(O)R^{2a}, -C(S)R^{2a}, -C(O)OR^{2a},-C(S)SR^{2a}, -C(O)SR^{2a}, -C(S)OR^{2a}, -SC(O)R^{2a}, -OC(S)R^{2a}, - SC(S)R^{2a}, -C(O)N(R^{2a})₂, -OR^{2a}, -SR^{2a}, -N(R^{2a})₂, -N(R^{2a})OR^{2a}, -N(R^{2a})S(O)₂R^{2a}, - N(R^{2a})C(O)R^{2a}, -N(R^{2a})N(R^{2a})₂, -N(R^{2a})C(O)OR^{2a},-N(R^{2a})C(O)N(R^{2a})₂, -S(O)₂R^{2a}, -S(O)R^{2a}, -S(O)N(R^{2a})₂, -S(O)₂N(R^{2a})₂, -N⁺(R^{2a})₃, -S⁺(R^{2a})₂ oder-Si(R^{2a})₃ ist, wobei das C₁₋₁₂Alkyl, das C₂₋₁₂Alkenyl, das C₂₋₁₂Alkynyl, das 3- bis 12-gliedrige Carbocyclyl und das 3- bis 12-gliedrige Heterocyclyl jeweils optional substituiert sind mit einem oder mehreren R²⁵;
R^{3a} H, C₁₋₁₂Alkyl, C₂₋₁₂Alkenyl, C₂₋₁₂Alkynyl, ein 3- bis 12-gliedriges Carbocyclyl, ein 3-bis 12-gliedriges Heterocyclyl, -C(O)R^{30a}, -C(O)OR^{30a}, -C(O)N(R^{30a})₂, -OR^{30a}, -N(R^{30a})₂,-N(R^{30a})OR^{30a}, -N(R^{30a})S(O)₂R^{30a}, -N(R^{30a})C(O)R^{30a}, -N(R^{30a})N(R^{30a})₂, -N(R^{30a})C(O)OR^{30a},-N(R^{30a})C(O)N(R^{30a})₂, -S(O)R^{30a}, -S(O)N(R^{30a})₂ oder - S(O)₂N(R^{30a})₂ ist, wobei das C₁₋₁₂Alkyl, das C₂₋₁₂Alkenyl, das C₂₋₁₂Alkynyl, das 3- bis 12-gliedrige Carbocyclyl und das 3- bis 12-gliedrige Heterocyclyl jeweils optional substituiert sind mit einem oder mehreren R³⁵; und
R^{3b} C₁₋₁₂Alkyl, C₂₋₁₂Alkenyl, C₂₋₁₂Alkynyl, ein 3- bis 12-gliedriges Carbocyclyl, ein 3- bis 12-gliedriges Heterocyclyl, -C(O)R^{30b}, -C(O)OR^{30b}, -C(O)N(R^{30b})₂, -OR^{30b}, -N(R^{30b})₂,-N(R^{30b})OR^{30b}, -N(R^{30b})S(O)₂R^{30b}, -N(R^{30b})C(O)R^{30b}, -N(R^{30b})N(R^{30b})₂, -N(R^{30b})C(O)OR^{30b},-N(R^{30b})C(O)N(R^{30b})₂, -S(O)R^{30b}, -S(O)N(R^{30b})₂ oder - S(O)₂N(R^{30b})₂ ist, wobei das C₁₋₁₂Alkyl, das C₂₋₁₂Alkenyl, das C₂₋₁₂Alkynyl, das 3- bis 12-gliedrige Carbocyclyl und das 3- bis 12-gliedrige Heterocyclyl jeweils optional substituiert sind mit einem oder mehreren R³⁵; oder
R^{3a} und R^{3b} zusammengenommen C₂₋₁₂Alkylen, C₂₋₁₂Alkenylen oder C₂₋₁₂Alkynylen sind, wobei das C₂₋₁₂Alkylen, das C₂₋₁₂Alkenylen und das C₂₋₁₂Alkynylen jeweils optional substituiert sind mit einem oder mehreren R³⁵;
R⁴ H, Halo, -NO₂, -CN, -N₃, C₁₋₁₂Alkyl, C₂₋₁₂Alkenyl, C₂₋₁₂Alkynyl, ein 3- bis 12-gliedriges Carbocyclyl, ein 3- bis 12-gliedriges Heterocyclyl, -C(O)R^{4a}, -C(S)R^{4a}, -C(O)OR^{4a},-C(S)SR^{4a}, -C(O)SR^{4a}, -C(S)OR^{4a}, -SC(O)R^{4a}, -OC(S)R^{4a}, -SC(S)R^{4a}, - C(O)N(R^{4a})₂, -OR^{4a}, -SR^{4a}, -N(R^{4a})₂, -N(R^{4a})OR^{4a}, -N(R^{4a})S(O)₂R^{4a}, - N(R^{4a})C(O)R^{4a}, -N(R^{4a})N(R^{4a})₂, -N(R^{4a})C(O)OR^{4a},-N(R^{4a})C(O)N(R^{4a})₂, -S(O)₂R^{4a}, -S(O)R^{4a}, -S(O)N(R^{4a})₂, -S(O)₂N(R^{4a})₂, -N⁺(R^{4a})₃, -S⁺(R^{4a})₂ oder-Si(R^{4a})₃ ist, wobei das C₁₋₁₂Alkyl, das C₂₋₁₂Alkenyl, das C₂₋₁₂Alkynyl, das 3- bis 12-gliedrige Carbocyclyl und das 3- bis 12-gliedrige Heterocyclyl jeweils optional substituiert sind mit einem oder mehreren R⁴⁵;
R⁵ H, Halo, -NO₂, -CN, -N₃, C₁₋₁₂Alkyl, C₂₋₁₂Alkenyl, C₂₋₁₂Alkynyl, ein 3- bis 12-gliedriges Carbocyclyl, ein 3- bis 12-gliedriges Heterocyclyl, -C(O)R^{5a}, -C(S)R^{5a}, -C(O)OR^{5a},-C(S)SR^{5a}, -C(O)SR^{5a}, -C(S)OR^{5a}, -SC(O)R^{5a}, -OC(S)R^{5a}, -SC(S)R^{5a}, - C(O)N(R^{5a})₂, -OR^{5a}, -SR^{5a}, -N(R^{5a})₂, -N(R^{5a})OR^{5a}, -N(R^{5a})S(O)₂R^{5a}, -N(R^{5a})C(O)R^{5a}, -N(R^{5a})N(R^{5a})₂, -N(R^{5a})C(O)OR^{5a},-N(R^{5a})C(O)N(R^{5a})₂, -S(O)₂R^{5a}, -S(O)R^{5a}, -S(O)N(R^{5a})₂, -S(O)₂N(R^{5a})₂, -N⁺(R^{5a})₃, -S⁺(R^{5a})₂ oder-Si(R^{5a})₃ ist, wobei das C₁₋₁₂Alkyl, das C₂₋₁₂Alkenyl, das C₂₋₁₂Alkynyl, das 3- bis 12-gliedrige Carbocyclyl und das 3- bis 12-gliedrige Heterocyclyl jeweils optional substituiert sind mit einem oder mehreren R⁵⁵;
" 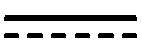 " entweder eine Einfachbindung oder eine Doppelbindung ist, wobei, wenn " 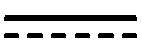 " eine Doppelbindung ist, dann X¹ CR⁶ ist und X² CR⁷ ist; und wenn " 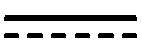 " eine Einfachbindung ist, dann X¹ C(R⁶)₂ ist und X² C(R⁷)₂ ist;
R⁶ bei jedem Auftreten unabhängig H, Halo, -NO₂, -CN, -N₃, C₁₋₁₂Alkyl, C₂₋₁₂Alkenyl, C₂₋₁₂Alkynyl, ein 3- bis 12-gliedriges Carbocyclyl, ein 3- bis 12-gliedriges Heterocyclyl, -C(O)R^{6a},-C(S)R^{6a}, -C(O)OR^{6a}, -C(S)SR^{6a}, -C(O)SR^{6a}, -C(S)OR^{6a}, -SC(O)R^{6a}, -OC(S)R^{6a}, -SC(S)R^{6a},-C(O)N(R^{6a})₂, -OR^{6a}, -SR^{6a}, -N(R^{6a})₂, -N(R^{6a})OR^{6a}, -N(R^{6a})S(O)₂R^{6a}, -N(R^{6a})C(O)R^{6a},-N(R^{6a})N(R^{6a})₂, -N(R^{6a})C(O)OR^{6a}, -N(^{R6a})C(O)N(R^{6a})₂, -S(O)₂R^{6a}, -S(O)R^{6a}, -S(O)N(R^{6a})₂,-S(O)₂N(R^{6a})₂, -N⁺(R^{6a})₃, -S⁺(R^{6a})₂ oder -Si(R^{6a})₃ ist; oder die zwei R⁶-Gruppen zusammengenommen Oxo, C₁₋₁₂Alkyliden oder =NR^{6a} sind, wobei das C₁₋₁₂Alkyl, das C₂₋₁₂Alkenyl, das C₂₋₁₂Alkynyl, das 3- bis 12-gliedrige Carbocyclyl, das 3- bis 12-gliedrige Heterocyclyl und das C₁₋₁₂Alkyliden jeweils optional substituiert sind mit einem oder mehreren R⁶⁵;
R⁷ bei jedem Auftreten unabhängig H, Halo, -NO₂, -CN, -N₃, C₁₋₁₂Alkyl, C₂₋₁₂Alkenyl, C₂₋₁₂Alkynyl, ein 3- bis 12-gliedriges nichtaromatisches Carbocyclyl, ein 3- bis 12-gliedriges Heterocyclyl, -C(O)R^{7a}, -C(S)R^{7a}, -C(O)OR^{7a}, -C(S)SR^{7a}, -C(O)SR^{7a}, -C(S)OR^{7a}, - SC(O)R^{7a},-OC(S)R^{7a}, -SC(S)R^{7a}, -C(O)N(R^{7a})₂, -OR^{7a}, -SR^{7a}, -N(R^{7a})₂, -N(R^{7a})OR^{7a}, -N(R^{7a})S(O)₂R^{7a},-N(R^{7a})C(O)R^{7a}, -N(R^{7a})N(R^{7a})₂, -N(R^{7a})C(O)OR^{7a}, -N(R^{7a})C(O)N(R^{7a})₂, -S(O)₂R^{7a}, -S(O)R^{7a},-S(O)N(R^{7a})₂, -S(O)₂N(R^{7a})₂, -N⁺(R^{7a})₃, -S⁺(R^{7a})₂ oder -Si(R^{7a})₃ ist; oder die zwei R⁷-Gruppen zusammengenommen Oxo, C₁₋₁₂Alkyliden oder =NR^{7a} sind, wobei das C₁₋₁₂Alkyl, das C₂₋₁₂Alkenyl, das C₂₋₁₂Alkynyl, das 3- bis 12-gliedrige nichtaromatische Carbocyclyl, das 3- bis 12-gliedrige Heterocyclyl und das C₁₋₁₂Alkyliden jeweils optional substituiert sind mit einem oder mehreren R⁷⁵;
X -C(R⁸)₂- oder -O- ist;
R⁸ bei jedem Auftreten unabhängig H, Halo, -NO₂, -CN, -N₃, C₁₋₁₂Alkyl, C₂₋₁₂Alkenyl, C₂₋₁₂Alkynyl, ein 3- bis 12-gliedriges Carbocyclyl, ein 3- bis 12-gliedriges Heterocyclyl, -C(O)R^{8a},-C(S)R^{8a}, -C(O)OR^{8a}, -C(S)SR^{8a}, -C(O)SR^{8a}, -C(S)OR^{8a}, -SC(O)R^{8a}, -OC(S)R^{8a}, -SC(S)R^{8a},-C(O)N(R^{8a})₂, -OR^{8a}, -SR^{8a}, -N(R^{8a})₂, -N(R^{8a})OR^{8a}, -N(R^{8a})S(O)₂R^{8a}, -N(R^{8a})C(O)R^{8a},-N(R^{8a})N(R^{8a})₂, -N(R^{8a})C(O)OR^{8a}, -N(R^{8a})C(O)N(R^{8a})₂, -S(O)₂R^{8a}, -S(O)R^{8a}, -S(O)N(R^{8a})₂,-S(O)₂N(R^{8a})₂, -N⁺(R^{8a})₃, -S⁺(R^{8a})₂ oder -Si(R^{8a})₃ ist; oder die zwei R⁸-Gruppen zusammengenommen Oxo, C₁₋₁₂Alkyliden oder =NR^{8a} sind, wobei das C₁₋₁₂Alkyl, das C₂₋₁₂Alkenyl, das C₂₋₁₂Alkynyl, das 3- bis 12-gliedrige Carbocyclyl, das 3- bis 12-gliedrige Heterocyclyl und das C₁₋₁₂Alkyliden jeweils optional substituiert sind mit einem oder mehreren R⁸⁵;
R^{10a}, R^{2a}, R^{30a}, R^{30b}, R^{4a}, R^{5a}, R^{6a}, R^{7a} und R^{8a} bei jedem Auftreten unabhängig ausgewählt sind aus H, C₁₋₁₂Alkyl, C₂₋₁₂Alkenyl, C₂₋₁₂Alkynyl, C₁₋₁₂Alkoxy, C₁₋₁₂Acyl, -Si(R¹⁶)₃, einem 3- bis 12-gliedrigen Carbocyclyl und einem 3- bis 12-gliedrigen Heterocyclyl, wobei das C₁₋₁₂Alkyl, das C₂₋₁₂Alkenyl, das C₂₋₁₂Alkynyl, das C₁₋₁₂Alkoxy, das C₁₋₁₂Acyl, das 3- bis 12-gliedrige Carbocyclyl und das 3- bis 12-gliedrige Heterocyclyl jeweils optional substituiert sind mit einem oder mehreren R¹⁷;
R¹⁶ bei jedem Auftreten unabhängig ausgewählt ist aus H, C₁₋₁₂Alkyl, C₂₋₁₂Alkenyl, C₂₋₁₂Alkynyl, einem 3- bis 12-gliedrigen Carbocyclyl und einem 3- bis 12-gliedrigen Heterocyclyl, wobei das C₁₋₁₂Alkyl, das C₂₋₁₂Alkenyl, das C₂₋₁₂Alkynyl, das 3- bis 12-gliedrige Carbocyclyl und das 3- bis 12-gliedrige Heterocyclyl jeweils optional substituiert sind mit einem oder mehreren R¹⁸;
R¹⁵, R²⁵, R³⁵, R⁴⁵, R⁵⁵, R⁶⁵, R⁷⁵ und R⁸⁵ bei jedem Auftreten unabhängig ausgewählt sind aus Halo, -OH, -CN, C₁₋₁₂Alkyl, C₂₋₁₂Alkenyl, C₂₋₁₂Alkynyl, C₁₋₁₂Alkoxy, einem 3- bis 12-gliedrigen Carbocyclyl und einem 3- bis 12-gliedrigen Heterocyclyl, wobei das C₁₋₁₂Alkyl, das C₂₋₁₂Alkenyl, das C₂₋₁₂Alkynyl, das C₁₋₁₂Alkoxy, das 3- bis 12-gliedrige Carbocyclyl und das 3- bis 12-gliedrige Heterocyclyl jeweils optional substituiert sind mit einem oder mehreren R¹⁹; und
R¹⁷, R¹⁸ und R¹⁹ bei jedem Auftreten unabhängig ausgewählt sind aus Halo, -OH, -CN, C₁₋₁₂Alkyl, C₂₋₁₂Alkenyl, C₂₋₁₂Alkynyl, C₁₋₁₂Alkoxy, einem 3- bis 12-gliedrigen Carbocyclyl und einem 3- bis 12-gliedrigen Heterocyclyl, wobei das C₁₋₁₂Alkyl, das C₂₋₁₂Alkenyl, das C₂₋₁₂Alkynyl, das C₁₋₁₂Alkoxy, das 3- bis 12-gliedrige Carbocyclyl und das 3- bis 12-gliedrige Heterocyclyl jeweils optional substituiert sind mit einer oder mehreren Gruppen unabhängig ausgewählt aus Halo, -OH und C₁₋₄Alkoxy.

2. Verbindung nach Anspruch 1, wobei die Verbindung durch Formel II, III, IV oder V dargestellt ist: 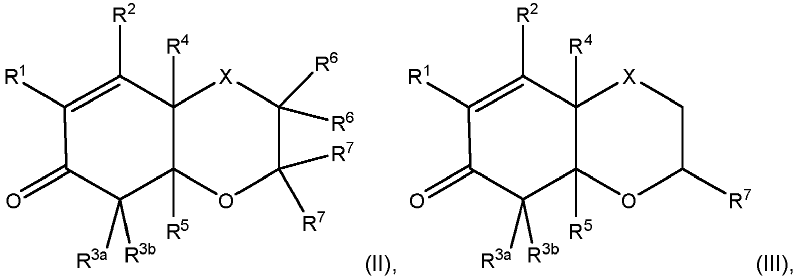 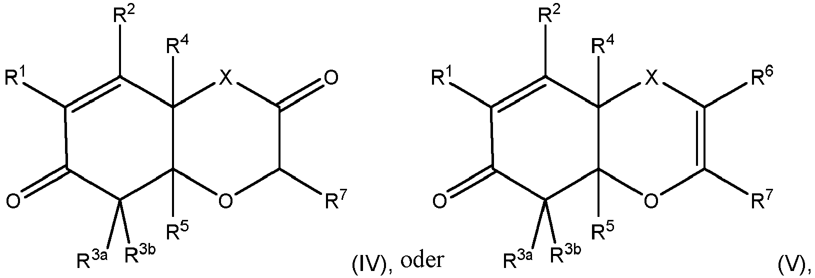 oder einem pharmazeutisch annehmbaren Salz davon.

3. Verbindung nach Anspruch 1 oder 2, wobei die Verbindung durch Formel IIA, IIB, IIC, IID, IIIA, IIIB, IIIC, IIID, IVA, IVB, IVC, IVD, VA, VB, VC oder VD dargestellt ist: 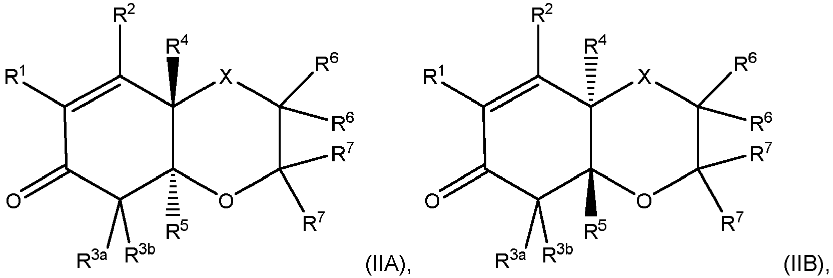 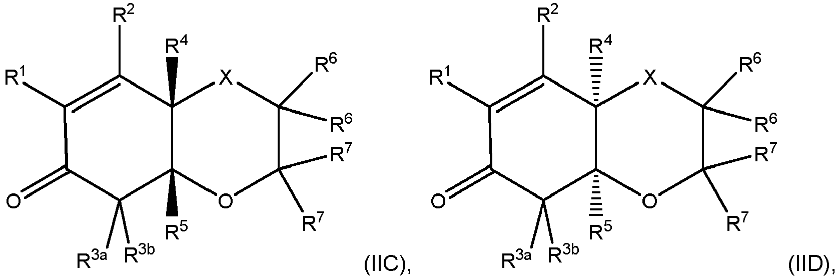 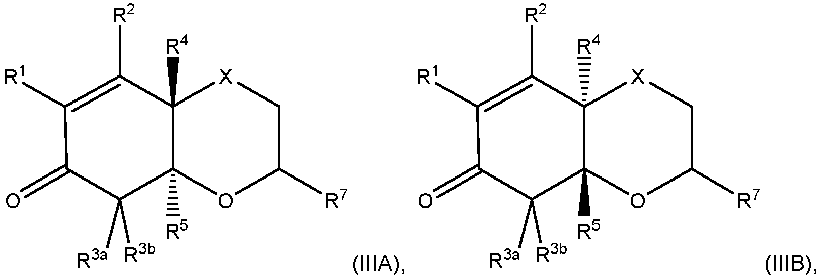 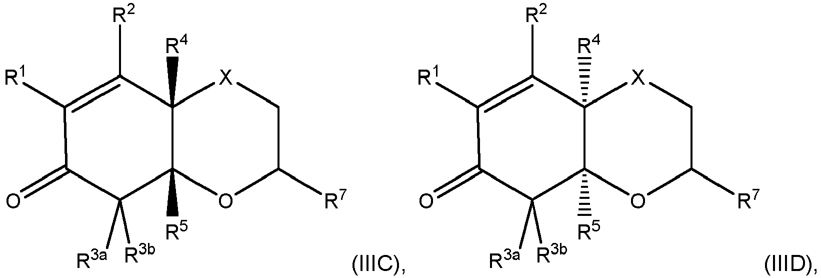 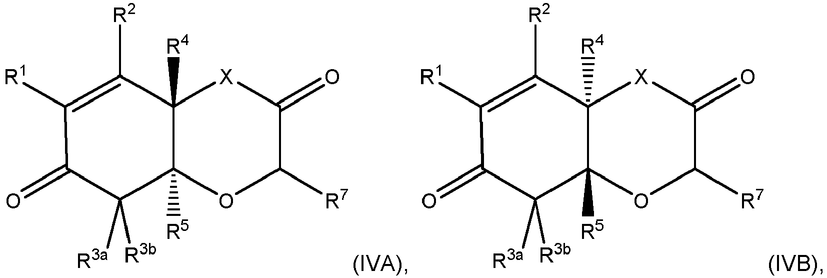 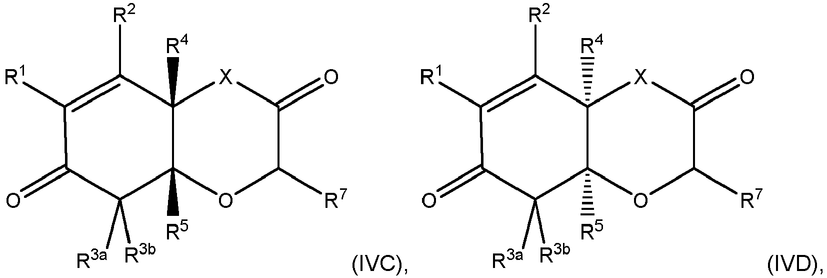 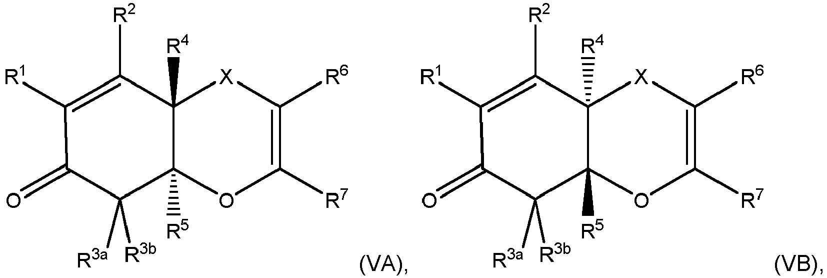 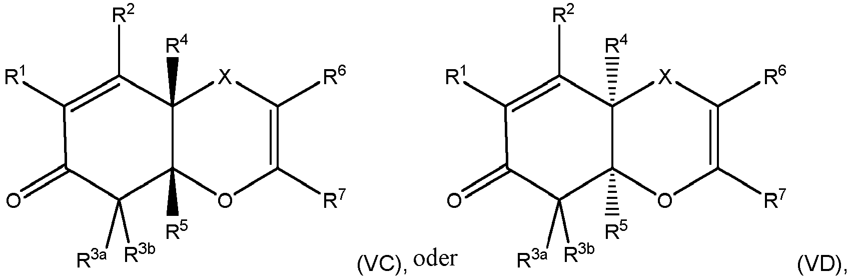 oder einem pharmazeutisch annehmbaren Salz davon.

4. Verbindung nach einem der Ansprüche 1-3 oder pharmazeutisch annehmbares Salz davon, wobei
R⁶ bei jedem Auftreten unabhängig H, Halo, -CN, -OR^{6a}, C₁₋₁₂Alkyl, C₂₋₁₂Alkenyl oder C₂₋₁₂Alkynyl ist, wobei das C₁₋₁₂Alkyl, das C₂₋₁₂Alkenyl und das C₂₋₁₂Alkynyl jeweils optional substituiert sind mit einem bis acht R⁶⁵; oder die zwei R⁶-Gruppen zusammengenommen Oxo sind;
R^{6a} ausgewählt ist aus H, C₁₋₁₂Alkyl, C₂₋₁₂Alkenyl und C₂₋₁₂Alkynyl, wobei das C₁₋₁₂Alkyl, das C₂₋₁₂Alkenyl und das C₂₋₁₂Alkynyl jeweils optional substituiert sind mit einem bis sechs R¹⁷;
R⁷ bei jedem Auftreten unabhängig H, Halo, -CN, -OR^{7a}, C₁₋₁₂Alkyl, C₂₋₁₂Alkenyl, C₂₋₁₂Alkynyl oder ein 3- bis 8-gliedriges nichtaromatisches Carbocyclyl ist, wobei das C₁₋₁₂Alkyl, das C₂₋₁₂Alkenyl, das C₂₋₁₂Alkynyl und das 3- bis 8-gliedrige Carbocyclyl jeweils optional substituiert sind mit einem bis acht R⁷⁵;
R^{7a} ausgewählt ist aus H, C₁₋₁₂Alkyl, C₂₋₁₂Alkenyl und C₂₋₁₂Alkynyl, wobei das C₁₋₁₂Alkyl, das C₂₋₁₂Alkenyl und das C₂₋₁₂Alkynyl jeweils optional substituiert sind mit einem bis sechs R¹⁷;
R⁶⁵ bei jedem Auftreten unabhängig ausgewählt ist aus Halo, -OH, -CN, C₁₋₁₂Alkyl, C_{2- 12}Alkenyl, C₂₋₁₂Alkynyl und C₁₋₁₂Alkoxy, wobei das C₁₋₁₂Alkyl, das C₂₋₁₂Alkenyl, das C_{2- 12}Alkynyl und das C₁₋₁₂Alkoxy jeweils optional substituiert sind mit einer bis acht Gruppen unabhängig ausgewählt aus Halo, -OH und C₁₋₄Alkoxy;
R⁷⁵ bei jedem Auftreten unabhängig ausgewählt ist aus Halo, -OH, -CN, C₁₋₁₂Alkyl, C_{2- 12}Alkenyl, C₂₋₁₂Alkynyl, einem 3- bis 8-gliedrigen Carbocyclyl und C₁₋₁₂Alkoxy, wobei das C_{1- 12}Alkyl, das C₂₋₁₂Alkenyl, das C₂₋₁₂Alkynyl, das 3- bis 8-gliedrige Carbocyclyl und das C_{1- 12}Alkoxy jeweils optional substituiert sind mit einer bis acht Gruppen unabhängig ausgewählt aus Halo, -OH und C₁₋₄Alkoxy; und
R¹⁷ bei jedem Auftreten als ein optionaler Substituent von R^{6a} oder R^{7a} unabhängig ausgewählt ist aus Halo, -CN, -OH, C₁₋₆Alkyl und C₁₋₆Alkoxy, wobei das C₁₋₆Alkyl und das C_{1- 6}Alkoxy jeweils optional substituiert sind mit einem bis sechs Halo.

5. Verbindung nach einem der Ansprüche 1-4 oder pharmazeutisch annehmbares Salz davon, wobei X -C(R⁸)₂- ist;
R⁸ bei jedem Auftreten unabhängig H, Halo, -CN, -OR^{8a}, C₁₋₁₂Alkyl, C₂₋₁₂Alkenyl oder C_{2- 12}Alkynyl ist, wobei das C₁₋₁₂Alkyl, das C₂₋₁₂Alkenyl und das C₂₋₁₂Alkynyl jeweils optional substituiert sind mit einem bis acht R⁸⁵;
R^{8a} ausgewählt ist aus H, C₁₋₁₂Alkyl, C₂₋₁₂Alkenyl und C₂₋₁₂Alkynyl, wobei das C₁₋₁₂Alkyl, das C₂₋₁₂Alkenyl und das C₂₋₁₂Alkynyl jeweils optional substituiert sind mit einem bis sechs R¹⁷;
R⁸⁵ bei jedem Auftreten unabhängig ausgewählt ist aus Halo, -OH, -CN, C₁₋₁₂Alkyl, C_{2- 12}Alkenyl, C₂₋₁₂Alkynyl und C₁₋₁₂Alkoxy, wobei das C₁₋₁₂Alkyl, das C₂₋₁₂Alkenyl, das C₂₋₁₂Alkynyl und das C₁₋₁₂Alkoxy jeweils optional substituiert sind mit einer bis acht Gruppen unabhängig ausgewählt aus Halo, -OH und C₁₋₄Alkoxy; und
R¹⁷ bei jedem Auftreten als ein optionaler Substituent von R^{8a} unabhängig ausgewählt ist aus Halo, -CN, -OH, C₁₋₆Alkyl und C₁₋₆Alkoxy, wobei das C₁₋₆Alkyl und das C₁₋₆Alkoxy jeweils optional substituiert sind mit einem bis sechs Halo.

6. Verbindung nach einem der Ansprüche 1-4 oder pharmazeutisch annehmbares Salz davon, wobei X -O- ist.

7. Verbindung nach einem der Ansprüche 1-6 oder pharmazeutisch annehmbares Salz davon, wobei:
1)
R¹-CN ist;
2)
R¹-CF₃ ist; oder
3)
R¹-C(O)R^{1a} ist;
R^{1a} H, C₁₋₁₂Alkyl, C₂₋₁₂Alkenyl, C₂₋₁₂Alkynyl, ein 3- bis 12-gliedriges Carbocyclyl oder ein 3- bis 12-gliedriges Heterocyclyl ist, wobei das C₁₋₁₂Alkyl, das C₂₋₁₂Alkenyl, das C₂₋₁₂Alkynyl, das 3- bis 12-gliedrige Carbocyclyl und das 3- bis 12-gliedrige Heterocyclyl jeweils optional substituiert sind mit einem bis sechs R¹⁵; und
R¹⁵ bei jedem Auftreten unabhängig ausgewählt ist aus Halo, -OH, C₁₋₁₂Alkyl und C₁₋₁₂Alkoxy, wobei das C₁₋₁₂Alkyl und das C₁₋₁₂Alkoxy jeweils optional substituiert sind mit einer bis drei Gruppen unabhängig ausgewählt aus Halo, -OH und C₁₋₄Alkoxy.

8. Verbindung nach einem der Ansprüche 1-7 oder pharmazeutisch annehmbares Salz davon, wobei
R² H, Halo, -CN, -OR^{2a}, C₁₋₁₂Alkyl, C₂₋₁₂Alkenyl oder C₂₋₁₂Alkynyl ist, wobei das C₁₋₁₂Alkyl, das C₂₋₁₂Alkenyl und das C₂₋₁₂Alkynyl jeweils optional substituiert sind mit einem bis acht R²⁵;
R^{2a} ausgewählt ist aus H, C₁₋₁₂Alkyl, C₂₋₁₂Alkenyl und C₂₋₁₂Alkynyl, wobei das C₁₋₁₂Alkyl, das C₂₋₁₂Alkenyl und das C₂₋₁₂Alkynyl jeweils optional substituiert sind mit einem bis sechs R¹⁷;
R²⁵ bei jedem Auftreten unabhängig ausgewählt ist aus Halo, -OH, -CN, C₁₋₁₂Alkyl, C₂₋₁₂Alkenyl, C₂₋₁₂Alkynyl und C₁₋₁₂Alkoxy, wobei das C₁₋₁₂Alkyl, das C₂₋₁₂Alkenyl, das C₂₋₁₂Alkynyl und das C₁₋₁₂Alkoxy jeweils optional substituiert sind mit einer bis acht Gruppen unabhängig ausgewählt aus Halo, -OH und C₁₋₄Alkoxy;
R^{3a} H, Halo, -CN, -OR^{30a}, C₁₋₁₂Alkyl, C₂₋₁₂Alkenyl oder C₂₋₁₂Alkynyl ist, wobei das C₁₋₁₂Alkyl, das C₂₋₁₂Alkenyl und das C₂₋₁₂Alkynyl jeweils optional substituiert sind mit einem bis acht R¹⁵;
R^{3b} Halo, -CN, -OR^{30b}, C₁₋₁₂Alkyl, C₂₋₁₂Alkenyl oder C₂₋₁₂Alkynyl ist, wobei das C₁₋₁₂Alkyl, das C₂₋₁₂Alkenyl und das C₂₋₁₂Alkynyl jeweils optional substituiert sind mit einem bis acht R³⁵;
R^{30a} und R^{30b} jeweils unabhängig ausgewählt sind aus H, C₁₋₁₂Alkyl, C₂₋₁₂Alkenyl und C₂₋₁₂Alkynyl, wobei das C₁₋₁₂Alkyl, das C₂₋₁₂Alkenyl und das C₂₋₁₂Alkynyl jeweils optional substituiert sind mit einem bis sechs R¹⁷;
R³⁵ bei jedem Auftreten unabhängig ausgewählt ist aus Halo, -OH, -CN, C₁₋₁₂Alkyl, C₂₋₁₂Alkenyl, C₂₋₁₂Alkynyl und C₁₋₁₂Alkoxy, wobei das C₁₋₁₂Alkyl, das C₂₋₁₂Alkenyl, das C₂₋₁₂Alkynyl und das C₁₋₁₂Alkoxy jeweils optional substituiert sind mit einer bis acht Gruppen unabhängig ausgewählt aus Halo, -OH und C₁₋₄Alkoxy;
R⁴ H, Halo, -CN, -OR^{4a}, C₁₋₁₂Alkyl, C₂₋₁₂Alkenyl oder C₂₋₁₂Alkynyl ist, wobei das C₁₋₁₂Alkyl, das C₂₋₁₂Alkenyl und das C₂₋₁₂Alkynyl jeweils optional substituiert sind mit einem bis acht R⁴⁵;
R^{4a} ausgewählt ist aus H, C₁₋₁₂Alkyl, C₂₋₁₂Alkenyl und C₂₋₁₂Alkynyl, wobei das C₁₋₁₂Alkyl, das C₂₋₁₂Alkenyl und das C₂₋₁₂Alkynyl jeweils optional substituiert sind mit einem bis sechs R¹⁷;
R⁴⁵ bei jedem Auftreten unabhängig ausgewählt ist aus Halo, -OH, -CN, C₁₋₁₂Alkyl, C₂₋₁₂Alkenyl, C₂₋₁₂Alkynyl und C₁₋₁₂Alkoxy, wobei das C₁₋₁₂Alkyl, das C₂₋₁₂Alkenyl, das C₂₋₁₂Alkynyl und das C₁₋₁₂Alkoxy jeweils optional substituiert sind mit einer bis acht Gruppen unabhängig ausgewählt aus Halo, -OH und C₁₋₄Alkoxy;
R⁵ H, Halo, -CN, -OR^{5a}, C₁₋₁₂Alkyl, C₂₋₁₂Alkenyl oder C₂₋₁₂Alkynyl ist, wobei das C₁₋₁₂Alkyl, das C₂₋₁₂Alkenyl und das C₂₋₁₂Alkynyl jeweils optional substituiert sind mit einem bis acht R⁵⁵;
R^{5a} ausgewählt ist aus H, C₁₋₁₂Alkyl, C₂₋₁₂Alkenyl und C₂₋₁₂Alkynyl, wobei das C₁₋₁₂Alkyl, das C₂₋₁₂Alkenyl und das C₂₋₁₂Alkynyl jeweils optional substituiert sind mit einem bis sechs R¹⁷;
R⁵⁵ bei jedem Auftreten unabhängig ausgewählt ist aus Halo, -OH, -CN, C₁₋₁₂Alkyl, C₂₋₁₂Alkenyl, C₂₋₁₂Alkynyl und C₁₋₁₂Alkoxy, wobei das C₁₋₁₂Alkyl, das C₂₋₁₂Alkenyl, das C₂₋₁₂Alkynyl und das C₁₋₁₂Alkoxy jeweils optional substituiert sind mit einer bis acht Gruppen unabhängig ausgewählt aus Halo, -OH und C₁₋₄Alkoxy; und
R¹⁷ bei jedem Auftreten unabhängig ausgewählt ist aus Halo, -CN, -OH, C₁₋₆Alkyl und C₁₋₆Alkoxy, wobei das C₁₋₆Alkyl und das C₁₋₆Alkoxy jeweils optional substituiert sind mit einem bis sechs Halo.

9. Verbindung nach einem der Ansprüche 1-3 oder pharmazeutisch annehmbares Salz davon, wobei
R¹-CN oder -CF₃ ist;
R² H, Halo, -OH, -CN, C₁₋₆Alkyl oder C₁₋₆Alkoxy ist, wobei das C₁₋₆Alkyl und das C₁₋₆Alkoxy jeweils optional substituiert sind mit einer bis sechs Gruppen unabhängig ausgewählt aus Halo, -CN, -OH, C₁₋₄Alkyl und C₁₋₄Alkoxy;
R^{3a} und R^{3b} jeweils unabhängig Halo, -OH, -CN, C₁₋₆Alkyl oder C₁₋₆Alkoxy sind,
wobei das C₁₋₆Alkyl und das C₁₋₆Alkoxy jeweils optional substituiert sind mit einer bis sechs Gruppen unabhängig ausgewählt aus Halo, -CN, -OH und C₁₋₄Alkoxy;
R⁴ H, Halo, -OH, -CN, C₁₋₆Alkyl oder C₁₋₆Alkoxy ist, wobei das C₁₋₆Alkyl und das C₁₋₆Alkoxy jeweils optional substituiert sind mit einer bis sechs Gruppen unabhängig ausgewählt aus Halo, -CN, -OH und C₁₋₄Alkoxy;
R⁵ H, Halo, -OH, -CN, C₁₋₆Alkyl oder C₁₋₆Alkoxy ist, wobei das C₁₋₆Alkyl und das C₁₋₆Alkoxy jeweils optional substituiert sind durch eine bis sechs Gruppen unabhängig ausgewählt aus Halo, -CN, -OH und C₁₋₄Alkoxy;
X-C(R⁸)₂- oder -O- ist, wobei R⁸ bei jedem Auftreten unabhängig H, Halo, -CN, -OH, C₁₋₁₂Alkyl, C₂₋₁₂Alkenyl oder C₂₋₁₂Alkynyl ist, wobei das C₁₋₁₂Alkyl, das C₂₋₁₂Alkenyl und das C₂₋₁₂Alkynyl jeweils optional substituiert sind mit einer bis acht Gruppen unabhängig ausgewählt aus Halo, -OH, -CN und C₁₋₄Alkoxy;
" 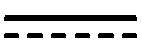 " eine Einfachbindung ist;
X¹C(R⁶)₂ ist, wobei R⁶ bei jedem Auftreten unabhängig H oder C₁₋₄Alkyl ist, oder die zwei R⁶-Gruppen zusammengenommen Oxo sind, wobei das C₁₋₄Alkyl optional substituiert ist mit einer bis sechs Gruppen unabhängig ausgewählt aus Halo, -OH, C₁₋₄Alkoxy und -CN; und
X² C(R⁷)₂ ist, wobei R⁷ bei jedem Auftreten unabhängig H, -OH, Halo, C₁₋₁₂Alkyl, C₁₋₁₂Alkoxy oder ein 3- bis 8-gliedriges Cycloalkyl ist, wobei das C₁₋₁₂Alkyl, das C₁₋₁₂Alkoxy und das 3- bis 8-gliedrige Cycloalkyl jeweils optional substituiert sind mit einer bis sechs Gruppen unabhängig ausgewählt aus Phenyl, Halo, -OH, C₁₋₄Alkoxy und -CN.

10. Verbindung nach Anspruch 9 oder pharmazeutisch annehmbares Salz davon, wobei
R¹ -CN ist;
R² H oder C₁₋₄Alkyl ist;
R^{3a} und R^{3b} jeweils unabhängig C₁₋₆Alkyl sind;
R⁴ H oder C₁₋₄Alkyl ist;
R⁵ H oder C₁₋₄Alkyl ist;
X-C(R⁸)₂- oder -O- ist, wobei R⁸ bei jedem Auftreten unabhängig H oder C₁₋₄Alkyl ist;
" 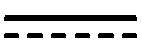 " eine Einfachbindung ist;
X¹C(R⁶)₂ oder -C(O)- ist, wobei R⁶ bei jedem Auftreten unabhängig H oder C₁₋₄Alkyl ist; und
X²-CHR₇- ist, wobei R⁷ H, C₁₋₆Alkyl, Benzyl, Cyclopropyl, Cyclobutyl, Cyclopentyl oder Cyclohexyl ist.

11. Verbindung nach Anspruch 10 oder pharmazeutisch annehmbares Salz davon, wobei
R¹ -CN ist;
R² H ist;
R^{3a} und R^{3b} jeweils unabhängig C₁₋₄Alkyl sind;
R⁴ C₁₋₄Alkyl ist;
R⁵ H ist;
X-CH₂- oder -O- ist;
" 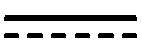 " eine Einfachbindung ist;
X¹-CH₂- oder -C(O)- ist; und
X²-CHR₇- ist, wobei R⁷ H, C₁₋₄Alkyl, Benzyl oder Cyclopropyl ist.

12. Verbindung nach Anspruch 1 ausgewählt aus der Gruppe bestehend aus:
(4aS,8aR)-4a,8,8-Trimethyl-7-oxo-3,4,4a,7,8,8a-hexahydro-2H-chromen-6-carbonitril;
(4aR,8aS)-4a,8,8-Trimethyl-7-oxo-3,4,4a,7,8,8a-hexahydro-2H-chromen-6-carbonitril;
(2S,4aS,8aR)-2,4a,8,8-Tetramethyl-7-oxo-2,3,4,8a-tetrahydrochromen-6-carbonitril;
(2R,4aR,8aS)-2,4a,8,8-Tetramethyl-7-oxo-2,3,4,8a-tetrahydrochromen-6-carbonitril;
(2R,4aS,8aR)-2,4a,8,8-Tetramethyl-7-oxo-2,3,4,8a-tetrahydrochromen-6-carbonitril;
(2S,4aR,8aS)-2,4a,8,8-Tetramethyl-7-oxo-2,3,4,8a-tetrahydrochromen-6-carbonitril;
(4aS,8aR)-4a-Ethyl-8,8-dimethyl-7-oxo-2,3,4,8a-tetrahydrochromen-6-carbonitril;
(4aR,8aS)-4a-Ethyl-8,8-dimethyl-7-oxo-2,3,4,8a-tetrahydrochromen-6-carbonitril;
(2S,4aS,8aR)-4a-Ethyl-2,8,8-trimethyl-7-oxo-2,3,4,8a-tetrahydrochromen-6-carbonitril;
(2R,4aR,8aS)-4a-Ethyl-2,8,8-trimethyl-7-oxo-2,3,4,8a-tetrahydrochromen-6-carbonitril;
(2R,4aS,8aR)-4a-Ethyl-2,8,8-trimethyl-7-oxo-2,3,4,8a-tetrahydrochromen-6-carbonitril;
(2S,4aR,8aS)-4a-Ethyl-2,8,8-trimethyl-7-oxo-2,3,4,8a-tetrahydrochromen-6-carbonitril;
(2S,4aS,8aR)-2-Ethyl-4a,8,8-trimethyl-7-oxo-2,3,4,8a-tetrahydrochromen-6-carbonitril;
(2R,4aR,8aS)-2-Ethyl-4a,8,8-trimethyl-7-oxo-2,3,4,8a-tetrahydrochromen-6-carbonitril;
(2R,4aS,8aR)-2-Ethyl-4a,8,8-trimethyl-7-oxo-2,3,4,8a-tetrahydrochromen-6-carbonitril;
(2S,4aR,8aS)-2-Ethyl-4a,8,8-trimethyl-7-oxo-2,3,4,8a-tetrahydrochromen-6-carbonitril;
(2S,4aS,8aR)-2,4a-Diethyl-8,8-dimethyl-7-oxo-2,3,4,8a-tetrahydrochromen-6-carbonitril;
(2R,4aR,8aS)-2,4a-Diethyl-8,8-dimethyl-7-oxo-2,3,4,8a-tetrahydrochromen-6-carbonitril;
(2R,4aS,8aR)-2,4a-Diethyl-8,8-dimethyl-7-oxo-2,3,4,8a-tetrahydrochromen-6-carbonitril;
(2S,4aR,8aS)-2,4a-Diethyl-8,8-dimethyl-7-oxo-2,3,4,8a-tetrahydrochromen-6-carbonitril;
(2R,4aS,8aR)-2-Cyclopropyl-4a,8,8-trimethyl-7-oxo-2,3,4,8a-tetrahydrochromen-6-carbonitril;
(2S,4aR,8aS)-2-Cyclopropyl-4a,8,8-trimethyl-7-oxo-2,3,4,8a-tetrahydrochromen-6-carbonitril;
(2S,4aS,8aR)-2-Isopropyl-4a,8,8-trimethyl-7-oxo-2,3,4,8a-tetrahydrochromen-6-carbonitril;
(2R,4aR,8aS)-2-Isopropyl-4a,8,8-trimethyl-7-oxo-2,3,4,8a-tetrahydrochromen-6-carbonitril;
(2R,4aS,8aR)-2-Isopropyl-4a,8,8-trimethyl-7-oxo-2,3,4,8a-tetrahydrochromen-6-carbonitril;
(2S,4aR,8aS)-2-Isopropyl-4a,8,8-trimethyl-7-oxo-2,3,4,8a-tetrahydrochromen-6-carbonitril;
(2S,4aS,8aR)-2-Benzyl-4a,8,8-trimethyl-7-oxo-2,3,4,8a-tetrahydrochromen-6-carbonitril;
(2R,4aR,8aS)-2-Benzyl-4a,8,8-trimethyl-7-oxo-2,3,4,8a-tetrahydrochromen-6-carbonitril;
(2R,4aS,8aR)-2-Benzyl-4a,8,8-trimethyl-7-oxo-2,3,4,8a-tetrahydrochromen-6-carbonitril;
(2S,4aR,8aS)-2-Benzyl-4a,8,8-trimethyl-7-oxo-2,3,4,8a-tetrahydrochromen-6-carbonitril;
(2S,4aS,8aR)-2,4a,8,8-Tetramethyl-3,7-dioxo-4,8a-dihydrochromen-6-carbonitril;
(2R,4aR,8aS)-2,4a,8,8-Tetramethyl-3,7-dioxo-4,8a-dihydrochromen-6-carbonitril;
(4aS,8aS)-4a,8,8-Trimethyl-7-oxo-3,8a-dihydro-2H-1,4-benzodioxin-6-carbonitril;
(4aR,8aR)-4a,8,8-Trimethyl-7-oxo-3,8a-dihydro-2H-1,4-benzodioxin-6-carbonitril;
4a,8,8,8a-Tetramethyl-7-oxo-3,4,4a,7,8,8a-hexahydro-2H-chromen-6-carbonitril; und
4a,8,8-Trimethyl-7-oxo-4a,7,8,8a-tetrahydro-4H-chromen-6-carbonitril,
und einem pharmazeutisch annehmbaren Salz davon.

13. Pharmazeutische Zusammensetzung, umfassend zumindest eine Verbindung nach einem der Ansprüche 1 bis 12 oder ein pharmazeutisch annehmbares Salz davon und zumindest einen pharmazeutisch annehmbaren Hilfsstoff.

14. Verbindung nach einem der Ansprüche 1 bis 12 oder pharmazeutisch annehmbares Salz davon zur Verwendung bei der Behandlung einer Störung bei einem Patienten, wobei die Störung ausgewählt ist aus der Gruppe bestehend aus einer neurodegenerativen Erkrankung, Entzündung/einer entzündlichen Erkrankung, einer Autoimmunerkrankung, einer ischämischen fibrotischen Erkrankung, einem Krebs, vorzeitigem Altern, einer kardiovaskulären Erkrankung, einer Lebererkrankung, einer Hämoglobinopathie, Thalassämie und einer Stoffwechselstörung.

## Revendications

1. Composé représenté par la formule I : 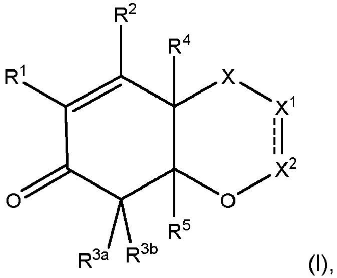 ou sel pharmaceutiquement acceptable de celui-ci, dans laquelle
R¹ représente un groupe -CN, -C(O)R^{1a} ou C₁₋₈alkyle substitué par un ou plusieurs atomes de fluor;
R^{1a} représente un atome H, un groupe C₁₋₁₂alkyle, C₂₋₁₂alcényle, C₂₋₁₂alcynyle, un groupe carbocyclyle de 3 à 12 chaînons, un groupe hétérocyclyle de 3 à 12 chaînons, -OR^{10a}, -SR^{10a}, -N(R^{10a})₂, -NR^{10a}OR^{10a}, -NR^{10a}S(O)₂R^{10a}, -NR^{10a}C(O)R^{10a}, -N(R^{10a})N(R^{10a})₂, -N(R^{10a})C(O)OR^{10a} ou -N(R^{10a})C(O)N(R^{10a})₂, lesdits groupes C₁₋₁₂alkyle, C₂₋₁₂alcényle, C₂₋₁₂alcynyle, carbocyclyle de 3 à 12 chaînons et hétérocyclyle de 3 à 12 chaînons étant chacun éventuellement substitués par un ou plusieurs groupes R¹⁵;
R² représente un atome H, un groupe halogéno, -NO₂, -CN, -N₃, C₁₋₁₂alkyle, C₂₋₁₂alcényle, C₂₋₁₂alcynyle, un groupe carbocyclyle de 3 à 12 chaînons, un groupe hétérocyclyle de 3 à 12 chaînons, -C(O)R^{2a}, -C(S)R^{2a}, -C(O)OR^{2a}, -C(S)SR^{2a}, -C(O)SR^{2a}, -C(S)OR^{2a}, -SC(O)R^{2a}, -OC(S)R^{2a}, -SC(S)R^{2a}, -C(O)N(R^{2a})₂, -OR^{2a}, -SR^{2a}, -N(R^{2a})₂, -N(R^{2a})OR^{2a}, -N(R^{2a})S(O)₂R^{2a}, -N(R^{2a})C(O)R^{2a}, -N(R^{2a})N(R^{2a})₂, -N(R^{2a})C(O)OR^{2a}, -N(R^{2a})C(O)N(R^{2a})₂, -S(O)₂R^{2a}, -S(O)R^{2a}, -S(O)N(R^{2a})₂, -S(O)₂N(R^{2a})₂, -N⁺(R^{2a})₃, -S⁺(R^{2a})₂ ou -Si(R^{2a})₃, lesdits groupes C₁₋₁₂alkyle, C₂₋₁₂alcényle, C₂₋₁₂alcynyle, carbocyclyle de 3 à 12 chaînons et hétérocyclyle de 3 à 12 chaînons étant chacun éventuellement substitués par un ou plusieurs groupes R²⁵;
R^{3a} représente un atome H, un groupe C₁₋₁₂alkyle, C₂₋₁₂alcényle, C₂₋₁₂alcynyle, un groupe carbocyclyle de 3 à 12 chaînons, un groupe hétérocyclyle de 3 à 12 chaînons, -C(O)R^{30a}, -C(O)OR^{30a}, -C(O)N(R^{30a})₂, -OR^{30a}, -N(R^{30a})₂, -N(R^{30a})OR^{30a}, -N(R^{30a})S(O)₂R^{30a}, -N(R^{30a})C(O)R^{30a}, -N(R^{30a})N(R^{30a})₂, -N(R^{30a})C(O)OR^{30a}, -N(R^{30a})C(O)N(R^{30a})₂, -S(O)R^{30a}, -S(O)N(R^{30a})₂ ou -S(O)₂N(R^{30a})₂, lesdits groupes C₁₋₁₂alkyle, C₂₋₁₂alcényle, C₂₋₁₂alcynyle, carbocyclyle de 3 à 12 chaînons et hétérocyclyle de 3 à 12 chaînons étant chacun éventuellement substitués par un ou plusieurs groupes R³⁵ ; et
R^{3b} représente un groupe C₁₋₁₂alkyle, C₂₋₁₂alcényle, C₂₋₁₂alcynyle, un groupe carbocyclyle de 3 à 12 chaînons, un groupe hétérocyclyle de 3 à 12 chaînons, -C(O)R^{30b}, -C(O)OR^{30b}, -C(O)N(R^{30b})₂, -OR^{30b}, -N(R^{30b})₂, -N(R^{30b})OR^{30b}, -N(R^{30b})S(O)₂R^{30b}, -N(R^{30b})C(O)R^{30b}, -N(R^{30b})N(R^{30b})₂, -N(R^{30b})C(O)OR^{30b}, -N(R^{30b})C(O)N(R^{30b})₂, -S(O)R^{30b}, -S(O)N(R^{30b})₂ ou -S(O)₂N(R^{30b})₂, lesdits groupes C₁₋₁₂alkyle, C₂₋₁₂alcényle, C₂₋₁₂alcynyle, carbocyclyle de 3 à 12 chaînons et hétérocyclyle de 3 à 12 chaînons étant chacun éventuellement substitués par un ou plusieurs groupes R³⁵ ; ou
R^{3a} et R^{3b}, pris ensemble, représentent un groupe C₂₋₁₂alkylène, C₂₋₁₂alcénylène ou C₂₋₁₂alcynylène, lesdits groupes C₂₋₁₂alkylène, C₂₋₁₂alcénylène et C₂₋₁₂alcynylène étant chacun éventuellement substitués par un ou plusieurs groupes R³⁵ ;
R⁴ représente un atome H, un groupe halogéno, -NO₂, -CN, -N₃, C₁₋₁₂alkyle, C₂₋₁₂alcényle, C₂₋₁₂alcynyle, un groupe carbocyclyle de 3 à 12 chaînons, un groupe hétérocyclyle de 3 à 12 chaînons, -C(O)R^{4a}, -C(S)R^{4a}, -C(O)OR^{4a}, -C(S)SR^{4a}, -C(O)SR^{4a}, -C(S)OR^{4a}, -SC(O)R^{4a}, -OC(S)R^{4a}, -SC(S)R^{4a}, - C(O)N(R^{4a})₂, -OR^{4a}, -SR^{4a}, -N(R^{4a})₂, -N(R^{4a})OR^{4a}, -N(R^{4a})S(O)₂R^{4a}, -N(R^{4a})C(O)R^{4a}, -N(R^{4a})N(R^{4a})₂, -N(R^{4a})C(O)OR^{4a}, -N(R^{4a})C(O)N(R^{4a})₂, -S(O)₂R^{4a}, -S(O)R^{4a}, -S(O)N(R^{4a})₂, -S(O)₂N(R^{4a})₂, -N⁺(R^{4a})₃, -S⁺(R^{4a})₂ ou -Si(R^{4a})₃, lesdits groupes C₁₋₁₂alkyle, C₂₋₁₂alcényle, C₂₋₁₂alcynyle, carbocyclyle de 3 à 12 chaînons et hétérocyclyle de 3 à 12 chaînons étant chacun éventuellement substitués par un ou plusieurs groupes R⁴⁵ ;
R⁵ représente un atome H, un groupe halogéno, -NO₂, -CN, -N₃, C₁₋₁₂alkyle, C₂₋₁₂alcényle, C₂₋₁₂alcynyle, un groupe carbocyclyle de 3 à 12 chaînons, un groupe hétérocyclyle de 3 à 12 chaînons, -C(O)R^{5a}, -C(S)R^{5a}, -C(O)OR^{5a}, -C(S)SR^{5a}, -C(O)SR^{5a}, -C(S)OR^{5a}, -SC(O)R^{5a}, -OC(S)R^{5a}, -SC(S)R^{5a}, -C(O)N(R^{5a})₂, -OR^{5a}, -SR^{5a}, -N(R^{5a})₂, -N(R^{5a})OR^{5a}, -N(R^{5a})S(O)₂R^{5a}, -N(R^{5a})C(O)R^{5a}, -N(R^{5a})N(R^{5a})₂, -N(R^{5a})C(O)OR^{5a}, -N(R^{5a})C(O)N(R^{5a})₂, -S(O)₂R^{5a}, -S(O)R^{5a}, -S(O)N(R^{5a})₂, -S(O)₂N(R^{5a})₂, -N⁺(R^{5a})₃, -S⁺(R^{5a})₂ ou -Si(R^{5a})₃, lesdits groupes C₁₋₁₂alkyle, C₂₋₁₂alcényle, C₂₋₁₂alcynyle, carbocyclyle de 3 à 12 chaînons et hétérocyclyle de 3 à 12 chaînons étant chacun éventuellement substitués par un ou plusieurs groupes R⁵⁵ ;
" 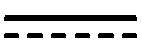 " représente soit une liaison simple soit une double liaison, lorsque " 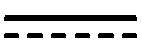 " représente une double liaison, alors X¹ représentant un groupe CR⁶ et X² représentant un groupe CR⁷ ; et lorsque " 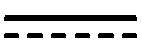 " représente une liaison simple, alors X¹ représentant un groupe C(R⁶)₂ et X² représentant un groupe C(R⁷)₂ ;
R⁶, dans chaque occurrence, représente indépendamment un atome H, un groupe halogéno, -NO₂, -CN, -N₃, C₁₋₁₂alkyle, C₂₋₁₂alcényle, C₂₋₁₂alcynyle, un groupe carbocyclyle de 3 à 12 chaînons, un groupe hétérocyclyle de 3 à 12 chaînons, -C(O)R^{6a}, -C(S)R^{6a}, -C(O)OR^{6a}, -C(S)SR^{6a}, -C(O)SR^{6a}, -C(S)OR^{6a}, -SC(O)R^{6a}, -OC(S)R^{6a}, -SC(S)R^{6a}, -C(O)N(R^{6a})₂, -OR^{6a}, -SR^{6a}, -N(R^{6a})₂, -N(R^{6a})OR^{6a}, -N(R^{6a})S(O)₂R^{6a}, -N(R^{6a})C(O)R^{6a}, -N(R^{6a})N(R)^{6a})₂, -N(R^{6a})C(O)OR^{6a}, -N(R^{6a})C(O)N(R^{6a})₂, -S(O)₂R^{6a}, -S(O)R^{6a}, -S(O)N(R^{6a})₂, -S(O)₂N(R^{6a})₂, -N⁺(R^{6a})₃, -S⁺(R^{6a})₂ ou -Si(R^{6a})₃ ; ou les deux groupes R⁶, pris ensemble, représentent un groupe oxo, C₁₋₁₂alkylidène ou =NR^{6a}, lesdits groupes C₁₋₁₂alkyle, C₂₋₁₂alcényle, C₂₋₁₂alcynyle, carbocyclyle de 3 à 12 chaînons, hétérocyclyle de 3 à 12 chaînons et C₁₋₁₂alkylidéne étant chacun éventuellement substitués par un ou plusieurs groupes R⁶⁵ ;
R⁷, dans chaque occurrence, représente indépendamment un atome H, un groupe halogéno, -NO₂, -CN, -N₃, C₁₋₁₂alkyle, C₂₋₁₂alcényle, C₂₋₁₂alcynyle, un groupe carbocyclyle non aromatique de 3 à 12 chaînons, un groupe hétérocyclyle de 3 à 12 chaînons, -C(O)R^{7a}, -C(S)R^{7a}, -C(O)OR^{7a}, -C(S)SR^{7a}, -C(O)SR^{7a}, -C(S)OR^{7a}, -SC(O)R^{7a}, -OC(S)R^{7a}, -SC(S)R^{7a}, -C(O)N(R^{7a})₂, -OR^{7a}, -SR^{7a}, -N(R^{7a})₂, -N(R^{7a})OR^{7a}, -N(R^{7a})S(O)₂R^{7a}, -N(R^{7a})C(O)R^{7a}, -N(R^{7a})N(R^{7a})₂, -N(R^{7a})C(O)OR^{7a}, -N(R^{7a})C(O)N(R^{7a})₂, -S(O)₂R^{7a}, -S(O)R^{7a}, -S(O)N(R^{7a})₂, -S(O)₂N(R^{7a})₂, -N⁺(R^{7a})₃, -S⁺(R^{7a})₂ ou -Si(R^{7a})₃ ; ou les deux groupes R⁷, pris ensemble, représentent un groupe oxo, C₁₋₁₂alkylidène ou =NR^{7a}, lesdits groupes C₁₋₁₂alkyle, C₂₋₁₂alcényle, C₂₋₁₂alcynyle, carbocyclyle non aromatique de 3 à 12 chaînons, hétérocyclyle de 3 à 12 chaînons et C₁₋₁₂alkylidène étant chacun éventuellement substitués par un ou plusieurs groupes R⁷⁵ ;
X représente un groupe -C(R⁸)₂- ou -O- ;
R⁸, dans chaque occurrence, représente indépendamment un atome H, un groupe halogéno, -NO₂, -CN, -N₃, C₁₋₁₂alkyle, C₂₋₁₂alcényle, C₂₋₁₂alcynyle, un groupe carbocyclyle de 3 à 12 chaînons, un groupe hétérocyclyle de 3 à 12 chaînons, -C(O)R^{8a}, -C(S)R^{8a}, -C(O)OR^{8a}, -C(S)SR^{8a}, -C(O)SR^{8a}, -C(S)OR^{8a}, -SC(O)R^{8a}, -OC(S)R^{8a}, -SC(S)R^{8a}, -C(O)N(R^{8a})₂, -OR^{8a}, -SR^{8a}, -N(R^{8a})₂, -N(R^{8a})OR^{8a}, -N(R^{8a})S(O)₂R^{8a}, -N(R^{8a})C(O)R^{8a}, -N(R^{8a})N(R^{8a})₂, -N(R^{8a})C(O)OR^{8a}, -N(R^{8a})C(O)N(R^{8a})₂, -S(O)₂R^{8a}, -S(O)R^{8a}, -S(O)N(R^{8a})₂, -S(O)₂N(R^{8a})₂, -N⁺(R^{8a})₃, - S⁺(R^{8a})₂ ou -Si(R^{8a})₃ ; ou les deux groupes R⁸ pris ensemble représentent un groupe oxo, C₁₋₁₂alkylidène ou =NR^{8a}, lesdits groupes C₁₋₁₂alkyle, C₂₋₁₂alcényle, C₂₋₁₂alcynyle, carbocyclyle de 3 à 12 chaînons, hétérocyclyle de 3 à 12 chaînons et C₁₋₁₂alkylidène étant chacun éventuellement substitués par un ou plusieurs groupes R⁸⁵ ;
R^{10a,} R^{2a}, R^{30a}, R^{30b}, R^{4a}, R^{5a}, R^{6a}, R^{7a} et R^{8a}, dans chaque occurrence, sont indépendamment choisis parmi un atome H, un groupe C₁₋₁₂alkyle, C₂₋₁₂alcényle, C₂₋₁₂alcynyle, C₁₋₁₂alcoxy, C₁₋₁₂acyle, - Si(R¹⁶)₃, un groupe carbocyclyle de 3 à 12 chaînons et un groupe hétérocyclyle de 3 à 12 chaînons, lesdits groupes C₁₋₁₂alkyle, C₂₋₁₂alcényle, C₂₋₁₂alcynyle, C₁₋₁₂alcoxy, C₁₋₁₂acyle, carbocyclyle de 3 à 12 chaînons et hétérocyclyle de 3 à 12 chaînons étant chacun éventuellement substitués par un ou plusieurs groupes R¹⁷;
R¹⁶, dans chaque occurrence, est indépendamment choisi parmi un atome H, un groupe C₁₋₁₂alkyle, C₂₋₁₂alcényle, C₂₋₁₂alcynyle, un groupe carbocyclyle de 3 à 12 chaînons et un groupe hétérocyclyle de 3 à 12 chaînons, lesdits groupes C₁₋₁₂alkyle, C₂₋₁₂alcényle, C₂₋₁₂alcynyle, carbocyclyle de 3 à 12 chaînons et hétérocyclyle de 3 à 12 chaînons étant chacun éventuellement substitués par un ou plusieurs groupes R¹⁸ ;
R¹⁵, R²⁵, R³⁵, R⁴⁵, R⁵⁵, R⁶⁵, R⁷⁵ et R⁸⁵, dans chaque occurrence, sont indépendamment choisis parmi un groupe halogéno, -OH, -CN, C₁₋₁₂alkyle, C₂₋₁₂alcényle, C₂₋₁₂alcynyle, C₁₋₁₂alcoxy, un groupe carbocyclyle de 3 à 12 chaînons et un groupe hétérocyclyle de 3 à 12 chaînons, lesdits groupes C₁₋₁₂alkyle, C₂₋₁₂alcényle, C₂₋₁₂alcynyle, C₁₋₁₂alcoxy, carbocyclyle de 3 à 12 chaînons et hétérocyclyle de 3 à 12 chaînons étant chacun éventuellement substitués par un ou plusieurs groupes R¹⁹ ; et
R¹⁷, R¹⁸ et R¹⁹, dans chaque occurrence, sont indépendamment choisis parmi un groupe halogéno, -OH, -CN, C₁₋₁₂alkyle, C₂₋₁₂alcényle, C₂₋₁₂alcynyle, C₁₋₁₂alcoxy, un groupe carbocyclyle de 3 à 12 chaînons et un groupe hétérocyclyle de 3 à 12 chaînons, lesdits groupes C₁₋₁₂alkyle, C₂₋₁₂alcényle, C₂₋₁₂alcynyle, C₁₋₁₂alcoxy, carbocyclyle de 3 à 12 chaînons et hétérocyclyle de 3 à 12 chaînons étant chacun éventuellement substitués par un ou plusieurs groupes indépendamment choisis parmi les groupes halogéno, -OH et C₁₋₄alcoxy.

2. Composé selon la revendication 1, ledit composé étant représenté par la formule II, III, IV ou V: 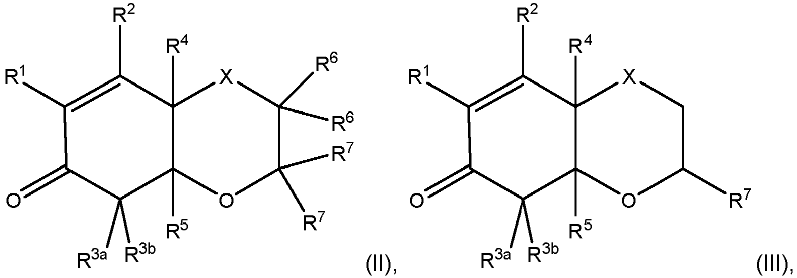 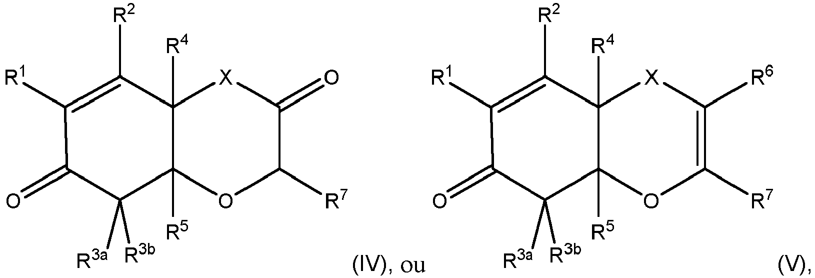 ou sel pharmaceutiquement acceptable de celui-ci.

3. Composé selon la revendication 1 ou 2, ledit composé étant représenté par la formule IIA, IIB, IIC, IID, IIIA, IIIB, IIIC, IIID, IVA, IVB, IVC, IVD, VA, VB, VC ou VD : 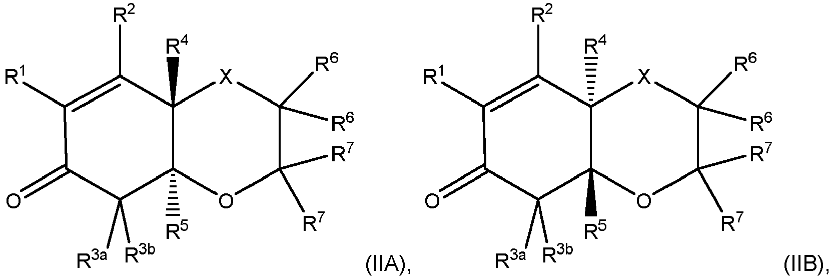 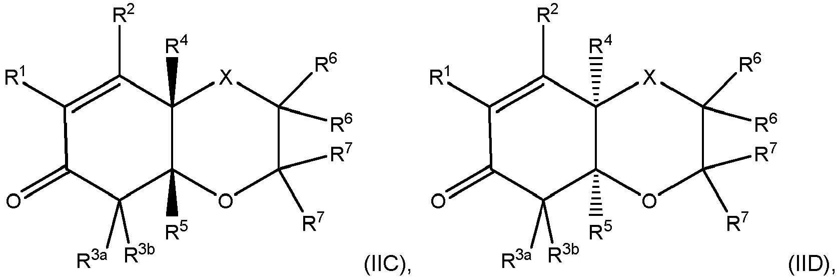 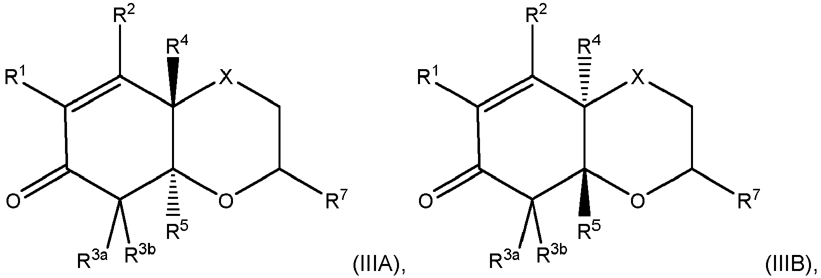 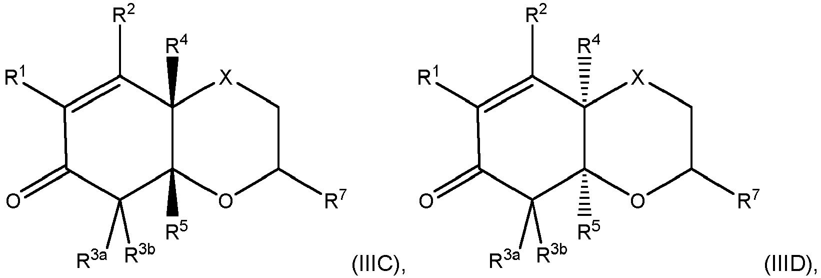 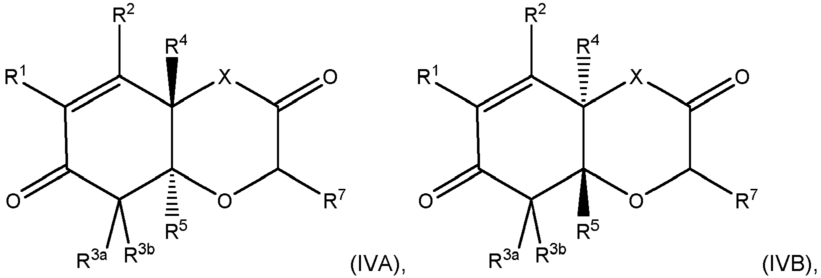 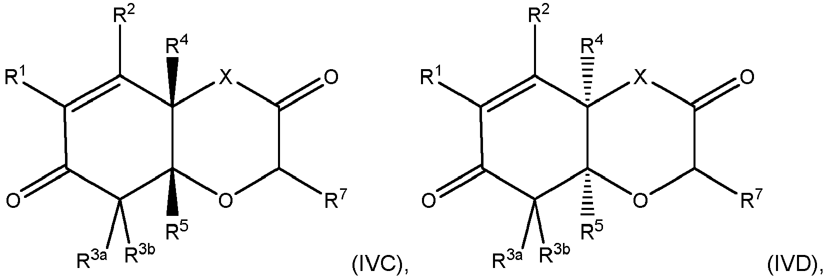 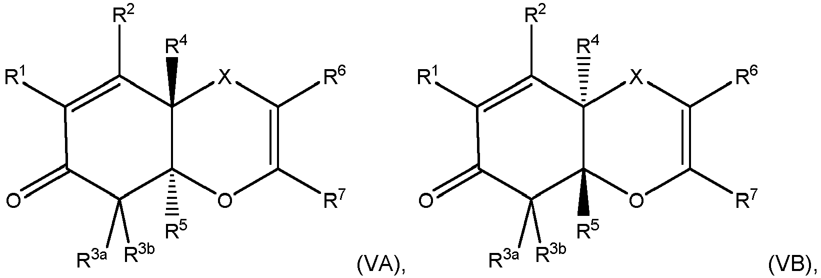 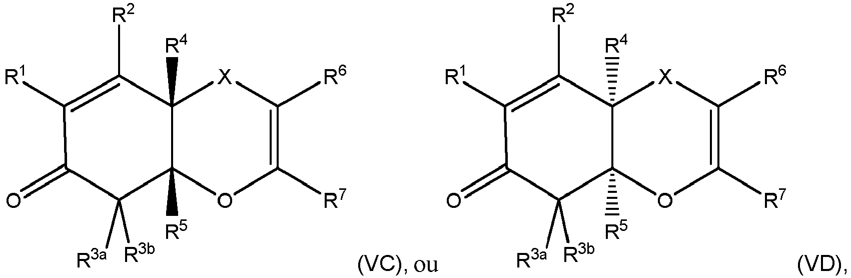 ou sel pharmaceutiquement acceptable de celui-ci.

4. Composé selon l'une quelconque des revendications 1 à 3, ou sel pharmaceutiquement acceptable de celui-ci,
R⁶, dans chaque occurrence, représentant indépendamment un atome H, un groupe halogéno, -CN, -OR^{6a}, C₁₋₁₂alkyle, C₂₋₁₂alcényle ou C₂₋₁₂alcynyle, lesdits groupes C₁₋₁₂alkyle, C₂₋₁₂alcényle et C₂₋₁₂alcynyle étant chacun éventuellement substitués par un à huit groupes R⁶⁵ ; ou les deux groupes R⁶, pris ensemble, formant un groupe oxo ;
R^{6a} étant choisi parmi un atome H, les groupes C₁₋₁₂alkyle, C₂₋₁₂alcényle et C₂₋₁₂alcynyle, lesdits groupes C₁₋₁₂alkyle, C₂₋₁₂alcényle et C₂₋₁₂alcynyle étant chacun éventuellement substitués par un à six groupes R¹⁷ ;
R⁷, dans chaque occurrence, représentant indépendamment un atome H, un groupe halogéno, -CN, -OR^{7a}, C₁₋₁₂alkyle, C₂₋₁₂alcényle, C₂₋₁₂alcynyle ou un groupe carbocyclyle non aromatique de 3 à 8 chaînons, lesdits groupes C₁₋₁₂alkyle, C₂₋₁₂alcényle, C₂₋₁₂alcynyle et carbocyclyle de 3 à 8 chaînons étant chacun éventuellement substitués par un à huit groupes R⁷⁵ ;
R^{7a} étant choisi parmi un atome H, les groupes C₁₋₁₂alkyle, C₂₋₁₂alcényle et C₂₋₁₂alcynyle, lesdits groupes C₁₋₁₂alkyle, C₂₋₁₂alcényle et C₂₋₁₂alcynyle étant chacun éventuellement substitués par un à six groupes R¹⁷ ;
R⁶⁵, dans chaque occurrence, étant indépendamment choisi parmi les groupes halogéno, -OH, -CN, C₁₋₁₂alkyle, C₂₋₁₂alcényle, C₂₋₁₂alcynyle et C₁₋₁₂alcoxy, lesdits groupes C₁₋₁₂alkyle, C₂₋₁₂alcényle, C₂₋₁₂alcynyle et C₁₋₁₂alcoxy étant chacun éventuellement substitués par un à huit groupes indépendamment choisis parmi les groupes halogéno, -OH et C₁₋₄alcoxy ;
R⁷⁵, dans chaque occurrence, étant indépendamment choisi parmi les groupes halogéno, -OH, -CN, C₁₋₁₂alkyle, C₂₋₁₂alcényle, C₂₋₁₂alcynyle, carbocyclyle de 3 à 8 chaînons et C₁₋₁₂alcoxy, lesdits groupes C₁₋₁₂alkyle, C₂₋₁₂alcényle, C₂₋₁₂alcynyle, carbocyclyle de 3 à 8 chaînons et C₁₋₁₂alcoxy étant chacun éventuellement substitués par un à huit groupes indépendamment choisis parmi les groupes halogéno, -OH et C₁₋₄alcoxy ; et
R¹⁷, dans chaque occurrence, en tant que substituant éventuel du groupe R^{6a} ou R^{7a}, étant indépendamment choisi parmi les groupes halogéno, -CN, -OH, C₁₋₆alkyle et C₁₋₆alcoxy, lesdits groupes C₁₋₆alkyle et C₁₋₆alcoxy étant chacun éventuellement substitués par un à six groupes halogéno.

5. Composé selon l'une quelconque des revendications 1 à 4, ou sel pharmaceutiquement acceptable de celui-ci,
X représentant un groupe -C(R⁸)₂- ;
R⁸, dans chaque occurrence, représentant indépendamment un atome H, un groupe halogéno, -CN, -OR^{8a}, C₁₋₁₂alkyle, C₂₋₁₂alcényle ou C₂₋₁₂alcynyle, lesdits groupes C₁₋₁₂alkyle, C₂₋₁₂alcényle et C₂₋₁₂alcynyle étant chacun éventuellement substitués par un à huit groupes R⁸⁵ ;
R^{8a} étant choisi parmi un atome H, les groupes C₁₋₁₂alkyle, C₂₋₁₂alcényle et C₂₋₁₂alcynyle, lesdits groupes C₁₋₁₂alkyle, C₂₋₁₂alcényle et C₂₋₁₂alcynyle étant chacun éventuellement substitués par un à six groupes R¹⁷ ;
R⁸⁵, dans chaque occurrence, étant indépendamment choisi parmi les groupes halogéno, -OH, -CN, C₁₋₁₂alkyle, C₂₋₁₂alcényle, C₂₋₁₂alcynyle et C₁₋₁₂alcoxy, lesdits groupes C₁₋₁₂alkyle, C₂₋₁₂alcényle, C₂₋₁₂alcynyle et C₁₋₁₂alcoxy étant chacun éventuellement substitués par un à huit groupes indépendamment choisis parmi les groupes halogéno, -OH et C₁₋₄alcoxy ; et
R¹⁷, dans chaque occurrence, en tant que substituant éventuel du groupe R^{8a}, étant indépendamment étant choisi parmi les groupes halogéno, -CN, -OH, C₁₋₆alkyle et C₁₋₆alcoxy, lesdits groupes C₁₋₆alkyle et C₁₋₆alcoxy étant chacun éventuellement substitués par un à six groupes halogéno.

6. Composé selon l'une quelconque des revendications 1 à 4, ou sel pharmaceutiquement acceptable de celui-ci, X représentant un groupe -O-.

7. Composé selon l'une quelconque des revendications 1 à 6, ou sel pharmaceutiquement acceptable de celui-ci :
1)
R¹ représentant un groupe -CN ;
2)
R¹ représentant un groupe -CF₃ ; ou
3)
R¹ représentant un groupe -C(O)R^{1a} ;
R^{1a} représentant un atome H, un groupe C₁₋₁₂alkyle, C₂₋₁₂alcényle, C₂₋₁₂alcynyle, un groupe carbocyclyle de 3 à 12 chaînons ou un groupe hétérocyclyle de 3 à 12 chaînons, lesdits groupes C₁₋₁₂alkyle, C₂₋₁₂alcényle, C₂₋₁₂alcynyle, carbocyclyle de 3 à 12 chaînons et hétérocyclyle de 3 à 12 chaînons étant chacun éventuellement substitués par un à six groupes R¹⁵ ; et
R¹⁵, dans chaque occurrence, étant indépendamment choisi parmi les groupes halogéno, -OH, C₁₋₁₂alkyle et C₁₋₁₂alcoxy, lesdits groupes C₁₋₁₂alkyle et C₁₋₁₂alcoxy étant chacun éventuellement substitués par un à trois groupes indépendamment choisis parmi les groupes halogéno, -OH et C₁₋₄alcoxy.

8. Composé selon l'une quelconque des revendications 1 à 7, ou sel pharmaceutiquement acceptable de celui-ci,
R² représentant un atome H, un groupe halogéno, -CN, -OR^{2a}, C₁₋₁₂alkyle, C₂₋₁₂alcényle or C₂₋₁₂alcynyle, lesdits groupes C₁₋₁₂alkyle, C₂₋₁₂alcényle et C₂₋₁₂alcynyle étant chacun éventuellement substitués par un à huit groupes R²⁵ ;
R^{2a} étant choisi parmi un atome H, les groupes C₁₋₁₂alkyle, C₂₋₁₂alcényle et C₂₋₁₂alcynyle, lesdits groupes C₁₋₁₂alkyle, C₂₋₁₂alcényle et C₂₋₁₂alcynyle étant chacun éventuellement substitués par un à six groupes R¹⁷ ;
R²⁵, dans chaque occurrence, étant indépendamment choisi parmi les groupes halogéno, -OH, -CN, C₁₋₁₂alkyle, C₂₋₁₂alcényle, C₂₋₁₂alcynyle et C₁₋₁₂alcoxy, lesdits groupes C₁₋₁₂alkyle, C₂₋₁₂alcényle, C₂₋₁₂alcynyle et C₁₋₁₂alcoxy étant chacun éventuellement substitués par un à huit groupes indépendamment choisis parmi les groupes halogéno, -OH et C₁₋₄alcoxy ;
R^{3a} étant choisi parmi un atome H, un groupe halogéno, -CN, -OR^{30a}, C₁₋₁₂alkyle, C₂₋₁₂alcényle ou C₂₋₁₂alcynyle, lesdits groupes C₁₋₁₂alkyle, C₂₋₁₂alcényle et C₂₋₁₂alcynyle étant chacun éventuellement substitués par un à huit groupes R³⁵ ;
R^{3b} représentant un groupe halogéno, -CN, -OR^{30b}, C₁₋₁₂alkyle, C₂₋₁₂alcényle ou C₂₋₁₂alcynyle, lesdits groupes C₁₋₁₂alkyle, C₂₋₁₂alcényle et C₂₋₁₂alcynyle étant chacun éventuellement substitués par un à huit groupes R³⁵ ;
R^{30a} et R^{30b} étant chacun indépendamment choisis parmi un atome H, les groupes C₁₋₁₂alkyle, C₂₋₁₂alcényle et C₂₋₁₂alcynyle, lesdits groupes C₁₋₁₂alkyle, C₂₋₁₂alcényle et C₂₋₁₂alcynyle étant chacun éventuellement substitués par un à six groupes R¹⁷ ;
R³⁵, dans chaque occurrence, étant indépendamment choisi parmi les groupes halogéno, -OH, -CN, C₁₋₁₂alkyle, C₂₋₁₂alcényle, C₂₋₁₂alcynyle et C₁₋₁₂alcoxy, lesdits groupes C₁₋₁₂alkyle, C₂₋₁₂alcényle, C₂₋₁₂alcynyle et C₁₋₁₂alcoxy étant chacun éventuellement substitués par un à huit groupes indépendamment choisis parmi les groupes halogéno, -OH et C₁₋₄alcoxy ;
R⁴ représentant un atome H, un groupe halogéno, -CN, -OR^{4a}, C₁₋₁₂alkyle, C₂₋₁₂alcényle ou C₂₋₁₂alcynyle, lesdits groupes C₁₋₁₂alkyle, C₂₋₁₂alcényle et C₂₋₁₂alcynyle étant chacun éventuellement substitués par un à huit groupes R⁴⁵ ;
R^{4a} étant choisi parmi un atome H, les groupes C₁₋₁₂alkyle, C₂₋₁₂alcényle et C₂₋₁₂alcynyle, lesdits groupes C₁₋₁₂alkyle, C₂₋₁₂alcényle et C₂₋₁₂alcynyle étant chacun éventuellement substitués par un à six groupes R¹⁷ ;
R⁴⁵, dans chaque occurrence, étant indépendamment choisi parmi les groupes halogéno, -OH, -CN, C₁₋₁₂alkyle, C₂₋₁₂alcényle, C₂₋₁₂alcynyle et C₁₋₁₂alcoxy, lesdits groupes C₁₋₁₂alkyle, C₂₋₁₂alcényle, C₂₋₁₂alcynyle et C₁₋₁₂alcoxy étant chacun éventuellement substitués par un à huit groupes indépendamment choisis parmi les groupes halogéno, -OH et C₁₋₄alcoxy ;
R⁵ représentant un atome H, un groupe halogéno, -CN, -OR^{5a}, C₁₋₁₂alkyle, C₂₋₁₂alcényle ou C₂₋₁₂alcynyle, lesdits groupes C₁₋₁₂alkyle, C₂₋₁₂alcényle et C₂₋₁₂alcynyle étant chacun éventuellement substitués par un à huit groupes R⁵⁵ ;
R^{5a} étant choisi parmi les groupes H, C₁₋₁₂alkyle, C₂₋₁₂alcényle et C₂₋₁₂alcynyle, lesdits groupes C₁₋₁₂alkyle, C₂₋₁₂alcényle et C₂₋₁₂alcynyle étant chacun éventuellement substitués par un à six groupes R¹⁷ ;
R⁵⁵, dans chaque occurrence, étant indépendamment choisi parmi les groupes halogéno, -OH, -CN, C₁₋₁₂alkyle, C₂₋₁₂alcényle, C₂₋₁₂alcynyle et C₁₋₁₂alcoxy, lesdits groupes C₁₋₁₂alkyle, C₂₋₁₂alcényle, C₂₋₁₂alcynyle et C₁₋₁₂alcoxy étant chacun éventuellement substitués par un à huit groupes indépendamment choisis parmi les groupes halogéno, -OH et C₁₋₄alcoxy ; et
R¹⁷, dans chaque occurrence, étant indépendamment choisi parmi les groupes halogéno, -CN, -OH, C₁₋₆alkyle et C₁₋₆alcoxy, lesdits groupes C₁₋₆alkyle et C₁₋₆alkoxy étant chacun éventuellement substitués par un à six groupes halogéno.

9. Composé selon l'une quelconque des revendications 1 à 3, ou sel pharmaceutiquement acceptable de celui-ci,
R¹ représentant un groupe -CN ou -CF₃ ;
R² représentant un atome H, un groupe halogéno, -OH, -CN, C₁₋₆alkyle ou C₁₋₆alcoxy, lesdits groupes C₁₋₆alkyle et C₁₋₆alcoxy étant chacun éventuellement substitués par un à six groupes indépendamment choisis parmi les groupes halogéno, -CN, -OH, C₁₋₄alkyle et C₁₋₄alcoxy ;
R^{3a} et R^{3b} représentant chacun indépendamment un groupe halogéno, -OH, -CN, C₁₋₆alkyle ou C₁₋₆alcoxy, lesdits groupes C₁₋₆alkyle et C₁₋₆alcoxy étant chacun éventuellement substitués par un à six groupes indépendamment choisis parmi les groupes halogéno, -CN, -OH et C₁₋₄alcoxy ;
R⁴ représentant un atome H, un groupe halogéno, -OH, -CN, C₁₋₆alkyle ou C₁₋₆alcoxy, lesdits groupes C₁₋₆alkyle et C₁₋₆alcoxy étant chacun éventuellement substitués par un à six groupes indépendamment choisis parmi les groupes halogéno, -CN, -OH et C₁₋₄alcoxy ;
R⁵ représentant un atome H, un groupe halogéno, -OH, -CN, C₁₋₆alkyle ou C₁₋₆alcoxy, lesdits groupes C₁₋₆alkyle et C₁₋₆alcoxy étant chacun éventuellement substitués par un à six groupes indépendamment choisis parmi les groupes halogéno, -CN, -OH et C₁₋₄alcoxy ;
X représentant un groupe -C(R⁸)₂- ou -O-, dans lequel R⁸, dans chaque occurrence, représente indépendamment un atome H, un groupe halogéno, -CN, -OH, C₁₋₁₂alkyle, C₂₋₁₂alcényle ou C₂₋₁₂alcynyle, lesdits groupes C₁₋₁₂alkyle, C₂₋₁₂alcényle et C₂₋₁₂alcynyle étant chacun éventuellement substitués par un à huit groupes indépendamment choisis parmi les groupes halogéno, -OH, -CN et C₁₋₄alcoxy ;
" 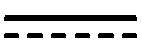 " représentant une liaison simple ;
X¹ représentant un groupe C(R⁶)₂, dans lequel R⁶, dans chaque occurrence, représente indépendamment un atome H ou un groupe C₁₋₄alkyle, ou les deux groupes R⁶, pris ensemble, représentent un groupe oxo, ledit groupe C₁₋₄alkyle étant éventuellement substitué par un à six groupes indépendamment choisis parmi les groupes halogéno, -OH, C₁₋₄alcoxy et -CN ; et
X² représentant un groupe C(R⁷)₂, dans lequel R⁷, dans chaque occurrence, représente indépendamment un atome H, un groupe -OH, halogéno, C₁₋₁₂alkyle, C₁₋₁₂alcoxy, ou un groupe cycloalkyle de 3 à 8 chaînons, lesdits groupes C₁₋₁₂alkyle, C₁₋₁₂alcoxy et cycloalkyle de 3 à 8 chaînons étant chacun éventuellement substitués par un à six groupes indépendamment choisis parmi les groupes phényle, halogéno, -OH, C₁₋₄alcoxy et -CN.

10. Composé selon la revendication 9, ou sel pharmaceutiquement acceptable de celui-ci,
R¹ représentant un groupe -CN ;
R² représentant un atome H ou un groupe C₁₋₄alkyle ;
R^{3a} et R^{3b} représentant chacun indépendamment un groupe C₁₋₆alkyle ;
R⁴ représentant un atome H ou un groupe C₁₋₄alkyle ;
R⁵ représentant un atome H ou un groupe C₁₋₄alkyle ;
X représentant un groupe -C(R⁸)₂- ou -O-, dans lequel R⁸, dans chaque occurrence, représente indépendamment un atome H ou un groupe C₁₋₄alkyle ;
" 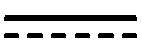 " représentant une liaison simple ;
X¹ représentant un groupe C(R⁶)₂ ou -C(O)-, dans lequel R⁶, dans chaque occurrence, représente indépendamment un atome H ou un groupe C₁₋₄alkyle ; et
X² représentant un groupe -CHR₇-, dans lequel R⁷ représente un atome H, un groupe C₁₋₆alkyle, benzyle, cyclopropyle, cyclobutyle, cyclopentyle ou cyclohexyle.

11. Composé selon la revendication 10, ou sel pharmaceutiquement acceptable de celui-ci,
R¹ représentant un groupe -CN ;
R² représentant un atome H ;
R^{3a} et R^{3b} représentant chacun indépendamment un groupe C₁₋₄alkyle ;
R⁴ représentant un groupe C₁₋₄alkyle ;
R⁵ représentant un atome H ;
X représentant un groupe -CH₂- ou -O- ;
" 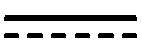 " représentant une liaison simple ;
X¹ représentant un groupe -CH₂- ou -C(O)- ; et
X² représentant un groupe -CHR₇-, dans lequel R⁷ représente un atome H, un groupe C₁₋₄alkyle, benzyle ou cyclopropyle.

12. Composé selon la revendication 1 choisi dans le groupe constitué par :
(4aS,8aR)-4a,8,8-triméthyl-7-oxo-3,4,4a,7,8,8a-hexahydro-2H-chromène-6-carbonitrile ;
(4aR,8aS)-4a,8,8-triméthyl-7-oxo-3,4,4a,7,8,8a-hexahydro-2H-chromène-6-carbonitrile ;
(2S,4aS,8aR)-2,4a,8,8-tétraméthyl-7-oxo-2,3,4,8a-tétrahydrochromène-6-carbonitrile ;
(2R,4aR,8aS)-2,4a,8,8-tétraméthyl-7-oxo-2,3,4,8a-tétrahydrochromène-6-carbonitrile ;
(2R,4aS,8aR)-2,4a,8,8-tétraméthyl-7-oxo-2,3,4,8a-tétrahydrochromène-6-carbonitrile ;
(2S,4aR,8aS)-2,4a,8,8-tétraméthyl-7-oxo-2,3,4,8a-tétrahydrochromène-6-carbonitrile ;
(4aS,8aR)-4a-éthyl-8,8-diméthyl-7-oxo-2,3,4,8a-tétrahydrochromène-6-carbonitrile ;
(4aR,8aS)-4a-éthyl-8,8-diméthyl-7-oxo-2,3,4,8a-tétrahydrochromène-6-carbonitrile ;
(2S,4aS,8aR)-4a-éthyl-2,8,8-triméthyl-7-oxo-2,3,4,8a-tétrahydrochromène-6-carbonitrile ;
(2R,4aR,8aS)-4a-éthyl-2,8,8-triméthyl-7-oxo-2,3,4,8a-tétrahydrochromène-6-carbonitrile ;
(2R,4aS,8aR)-4a-éthyl-2,8,8-triméthyl-7-oxo-2,3,4,8a-tétrahydrochromène-6-carbonitrile ;
(2S,4aR,8aS)-4a-éthyl-2,8,8-triméthyl-7-oxo-2,3,4,8a-tétrahydrochromène-6-carbonitrile ;
(2S,4aS,8aR)-2-éthyl-4a,8,8-triméthyl-7-oxo-2,3,4,8a-tétrahydrochromène-6-carbonitrile ;
(2R,4aR,8aS)-2-éthyl-4a,8,8-triméthyl-7-oxo-2,3,4,8a-tétrahydrochromène-6-carbonitrile ;
(2R,4aS,8aR)-2-éthyl-4a,8,8-triméthyl-7-oxo-2,3,4,8a-tétrahydrochromène-6-carbonitrile ;
(2S,4aR,8aS)-2-éthyl-4a,8,8-triméthyl-7-oxo-2,3,4,8a-tétrahydrochromène-6-carbonitrile ;
(2S,4aS,8aR)-2,4a-diéthyl-8,8-diméthyl-7-oxo-2,3,4,8a-tétrahydrochromène-6-carbonitril e ;
(2R,4aR,8aS)-2,4a-diéthyl-8,8-diméthyl-7-oxo-2,3,4,8a-tétrahydrochromène-6-carbonitri le ;
(2R,4aS,8aR)-2,4a-diéthyl-8,8-diméthyl-7-oxo-2,3,4,8a-tétrahydrochromène-6-carbonitri le ;
(2S,4aR,8aS)-2,4a-diéthyl-8,8-diméthyl-7-oxo-2,3,4,8a-tétrahydrochromène-6-carbonitril e ;
(2R,4aS,8aR)-2-cyclopropyl-4a,8,8-triméthyl-7-oxo-2,3,4,8a-tétrahydrochromène-6-carbonitrile ;
(2S,4aR,8aS)-2-cyclopropyl-4a,8,8-triméthyl-7-oxo-2,3,4,8a-tétrahydrochromène-6-carbonitrile ;
(2S,4aS,8aR)-2-isopropyl-4a,8,8-triméthyl-7-oxo-2,3,4,8a-tétrahydrochromène-6-carbonitrile ;
(2R,4aR,8aS)-2-isopropyl-4a,8,8-triméthyl-7-oxo-2,3,4,8a-tétrahydrochromène-6-carbonitrile ;
(2R,4aS,8aR)-2-isopropyl-4a,8,8-triméthyl-7-oxo-2,3,4,8a-tétrahydrochromène-6-carbonitrile ;
(2S,4aR,8aS)-2-isopropyl-4a,8,8-triméthyl-7-oxo-2,3,4,8a-tétrahydrochromène-6-carbonitrile ;
(2S,4aS,8aR)-2-benzyl-4a,8,8-triméthyl-7-oxo-2,3,4,8a-tétrahydrochromène-6-carbonitril e ;
(2R,4aR,8aS)-2-benzyl-4a,8,8-triméthyl-7-oxo-2,3,4,8a-tétrahydrochromène-6-carbonitri le ;
(2R,4aS,8aR)-2-benzyl-4a,8,8-triméthyl-7-oxo-2,3,4,8a-tétrahydrochromène-6-carbonitri le ;
(2S,4aR,8aS)-2-benzyl-4a,8,8-triméthyl-7-oxo-2,3,4,8a-tétrahydrochromène-6-carbonitril e;
(2S,4aS,8aR)-2,4a,8,8-tétraméthyl-3,7-dioxo-4,8a-dihydrochromène-6-carbonitrile ;
(2R,4aR,8aS)-2,4a,8,8-tétraméthyl-3,7-dioxo-4,8a-dihydrochromène-6-carbonitrile ;
(4aS,8aS)-4a,8,8-triméthyl-7-oxo-3,8a-dihydro-2H-1,4-benzodioxine-6-carbonitrile ;
(4aR,8aR)-4a,8,8-triméthyl-7-oxo-3,8a-dihydro-2H-1,4-benzodioxine-6-carbonitrile ;
4a,8,8,8a-tétraméthyl-7-oxo-3,4,4a,7,8,8a-hexahydro-2H-chromène-6-carbonitrile ; et
4a,8,8-triméthyl-7-oxo-4a,7,8,8a-tétrahydro-4H-chromène-6-carbonitrile,
et sel pharmaceutiquement acceptable de celui-ci.

13. Composition pharmaceutique comprenant au moins un composé selon l'une quelconque des revendications 1 à 12, ou un sel pharmaceutiquement acceptable de celui-ci, et au moins un excipient pharmaceutiquement acceptable.

14. Composé selon l'une quelconque des revendications 1 à 12, ou sel pharmaceutiquement acceptable de celui-ci, pour une utilisation dans le traitement d'un trouble chez un sujet, ledit trouble étant choisi dans le groupe constitué par une maladie neurodégénérative, une inflammation/une maladie inflammatoire, une maladie autoimmune, une maladie fibrotique ischémique, un cancer, une vieillissement prématuré, une maladie cardiovasculaire, une maladie hépatique, une hémoglobinopathie, une thalassémie et un trouble métabolique.
